(19) Europäisches Patentamt / European Patent Office / Office européen des brevets

(11) EP 3 980 510 B1

(12) EUROPEAN PATENT SPECIFICATION

(45) Date of publication and mention of the grant of the patent:
**08.11.2023 Bulletin 2023/45**

(21) Application number: **20729745.8**

(22) Date of filing: **02.06.2020**

(51) International Patent Classification (IPC):
***C07C 69/653*** $^{(2006.01)}$ ***C09K 19/04*** $^{(2006.01)}$
***C09K 19/30*** $^{(2006.01)}$ ***C09K 19/12*** $^{(2006.01)}$
***C09K 19/34*** $^{(2006.01)}$ ***C07C 69/54*** $^{(2006.01)}$

(52) Cooperative Patent Classification (CPC):
**C09K 19/04; C07C 69/54; C07C 69/653;**
C09K 2019/0444; C09K 2019/0448;
C09K 2019/0459; C09K 2019/0466;
C09K 2019/123; C09K 2019/3004; C09K 2019/301;
C09K 2019/3422

(86) International application number:
**PCT/EP2020/065144**

(87) International publication number:
**WO 2020/245084 (10.12.2020 Gazette 2020/50)**

# (54) LIQUID CRYSTAL MIXTURE AND LIQUID CRYSTAL DISPLAY

FLÜSSIGKRISTALLMISCHUNG UND FLÜSSIGKRISTALLANZEIGE

MÉLANGE DE CRISTAUX LIQUIDES ET DISPOSITIF D'AFFICHAGE À CRISTAUX LIQUIDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.06.2019 EP 19178130**

(43) Date of publication of application:
**13.04.2022 Bulletin 2022/15**

(73) Proprietor: **Merck Patent GmbH**
**64293 Darmstadt (DE)**

(72) Inventors:
- **LIETZAU, Lars**
  **64380 ROSSDORF (DE)**
- **SIEMIANOWSKI, Simon**
  **64380 ROSSDORF (DE)**

(56) References cited:
**EP-A1- 3 466 918**
**WO-A1-2018/047850**
**WO-A1-2019/206791**
**US-A1- 2006 172 090**
**US-A1- 2011 240 917**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 980 510 B1

## Description

The invention relates to compounds of formula I,

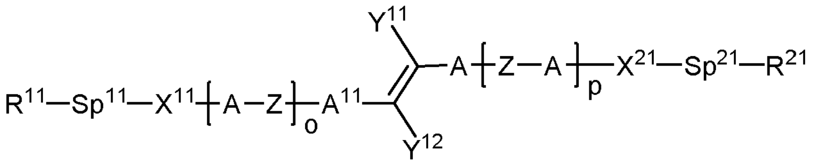

I

wherein $R^{11}$, $R^{21}$, $A^{11}$, A, Z, $X^{11}$, $X^{21}$, $Y^{11}$, $Y^{12}$, $Sp^{11}$, $Sp^{21}$, o and p have one of the meanings as given in claim 1. The invention further relates to a method of production of said compounds, to the use of said compounds in LC media and to LC media comprising one or more compounds of formula I. Further, the invention relates to a method of production of such LC media, to the use of such media in LC devices, and to a LC device comprising a LC medium according to the present invention. The present invention further relates to a process for the fabrication such liquid crystal display and to the use of the liquid crystal mixtures according to the invention for the fabrication of such liquid crystal display.

### Background and Prior Art

Liquid-crystalline media have been used for decades in electro-optical displays for information display. The liquid crystal displays used at present are usually those of the TN ("twisted nematic") type. However, these have the disadvantage of a strong viewing-angle dependence of the contrast.

In addition, so-called VA ("vertically aligned") displays are known which have a broader viewing angle. The LC cell of a VA display contains a layer of an LC medium between two transparent electrodes, where the LC medium usually has a negative value of the dielectric (DC) anisotropy. In the switched-off state, the molecules of the LC layer are aligned perpendicular to the electrode surfaces (homeotropically) or have a tilted homeotropic alignment. On application of an electrical voltage to the two electrodes, a realignment of the LC molecules parallel to the electrode surfaces takes place. Furthermore, so-called IPS ("in plane switching") displays and later, FFS ("fringe-field switching") displays have been reported (see, inter alia, S.H. Jung et al., Jpn. J. Appl. Phys., Volume 43, No. 3, 2004, 1028), which contain two electrodes on the same substrate, one of which is structured in a comb-shaped manner and the other is unstructured. A strong, so-called "fringe field" is thereby generated, i.e. a strong electric field close to the edge of the electrodes, and, throughout the cell, an electric field which has both a strong vertical component and a strong horizontal component. FFS displays have a low viewing-angle dependence of the contrast. FFS displays usually contain an LC medium with positive dielectric anisotropy, and an alignment layer, usually of polyimide, which provides planar alignment to the molecules of the LC medium.

Furthermore, FFS displays have been disclosed (see S.H. Lee et al., Appl. Phys. Lett. 73(20), 1998, 2882-2883 and S.H. Lee et al., Liquid Crystals 39(9), 2012, 1141-1148), which have similar electrode design and layer thickness as FFS displays, but comprise a layer of an LC medium with negative dielectric anisotropy instead of an LC medium with positive dielectric anisotropy. The LC medium with negative dielectric anisotropy shows a more favorable director orientation that has less tilt and more twist orientation compared to the LC medium with positive dielectric anisotropy, as a result of which these displays have a higher transmission.

A further development are the so-called PS (polymer sustained) or PSA (polymer sustained alignment) displays, for which the term "polymer stabilised" is also occasionally used. The PSA displays are distinguished by the shortening of the response times without significant adverse effects on other parameters, such as, in particular, the favourable viewing-angle dependence of the contrast.

In these displays, a small amount (for example 0.3% by weight, typically < 1% by weight) of one or more polymerizable compound(s) is added to the LC medium and, after introduction into the LC cell, is polymerised or crosslinked *in situ,* usually by UV photopolymerization, between the electrodes with or without an applied electrical voltage. The addition of polymerizable mesogenic or liquid-crystalline compounds, also known as reactive mesogens or "RMs", to the LC mixture has proven particularly suitable. PSA technology has hitherto been employed principally for LC media having negative dielectric anisotropy.

Unless indicated otherwise, the term "PSA" is used below as representative of PS displays and PSA displays.

In the meantime, the PSA principle is being used in diverse classical LC displays. Thus, for example, PSA-VA, PSA-OCB, PSA-IPS, PSA-FFS and PSA-TN displays are known. The polymerisation of the polymerizable compound(s) preferably takes place with an applied electrical voltage in the case of PSA-VA and PSA-OCB displays, and with or without an applied electrical voltage in the case of PSA-IPS displays. As can be demonstrated in test cells, the PS(A) method results in a 'pretilt' in the cell. In the case of PSA-OCB displays, for example, it is possible for the bend structure

to be stabilised so that an offset voltage is unnecessary or can be reduced. In the case of PSA-VA displays, the pretilt has a positive effect on the response times. A standard MVA or PVA pixel and electrode layout can be used for PSA-VA displays. In addition, however, it is also possible, for example, to manage with only one structured electrode side and no protrusions, which significantly simplifies production and at the same time results in very good contrast at the same time as very good light transmission.

PSA-VA displays are described, for example, in JP 10-036847 A, EP 1 170 626 A2, US 6,861,107, US 7,169,449, US 2004/0191428 A1,

US 2006/0066793 A1 and US 2006/0103804 A1. PSA-OCB displays are described, for example, in T.-J- Chen et al., Jpn. J. Appl. Phys. 45, 2006, 2702-2704 and S. H. Kim, L.-C- Chien, Jpn. J. Appl. Phys. 43, 2004, 7643-7647. PSA-IPS displays are described, for example, in US 6,177,972 and Appl. Phys. Lett. 1999, 75(21), 3264. PSA-TN displays are described, for example, in Optics Express 2004, 12(7), 1221. PSA-VA-IPS displays are disclosed, for example, in WO 2010/089092 A1.

Like the conventional LC displays described above, PSA displays can be operated as active-matrix or passive-matrix displays. In the case of active-matrix displays, individual pixels are usually addressed by integrated, non-linear active elements, such as, for example, transistors (for example thin-film transistors or "TFTs"), while in the case of passive-matrix displays, individual pixels are usually addressed by the multiplex method, both methods being known from the prior art.

In the prior art, polymerizable compounds of the following formula, for example, are used for PSA-VA:

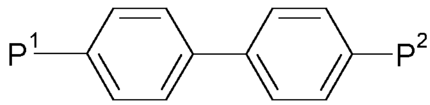

in which P denotes a polymerizable group, usually an acrylate or methacrylate group, as described, for example, in US 7,169,449.

Below the polymer layer which induces the above mentioned pretilt, an orientation layer - usually a polyimide - provides the initial alignment of the liquid crystal regardless of the polymer stabilisation step of the production process.

The effort for the production of a polyimide layer, treatment of the layer and improvement with bumps or polymer layers is relatively great. A simplifying technology which on the one hand reduces production costs and on the other hand helps to optimise the image quality (viewing-angle dependence, contrast, response times) would therefore be desirable. Rubbed polyimide has been used for a long time to align liquid crystals. The rubbing process causes a number of problems: mura, contamination, problems with static discharge, debris, etc.

Photoalignment is a technology for achieving liquid crystal (LC) alignment that avoids rubbing by replacing it with a light-induced orientational ordering of the alignment surface. This can be achieved through the mechanisms of photodecomposition, photodimerization, and photoisomerization (N.A. Clark et al. Langmuir 2010, 26(22), 17482-17488, and literature cited therein) by means of polarised light. However, still a suitably derivatised polyimide layer is required that comprises the photoreactive group. A further improvement would be to avoid the use of polyimide at all. For VA displays this was achieved by adding a self-alignment agent to the LC that induces homeotropic alignment *in situ* by a self-assembling mechanism as disclosed in WO 2012/104008 and WO 2012/038026.

N.A. Clark et al. Langmuir 2010, 26(22), 17482-17488 have shown that it is possible to self-assemble a compound of the following structure

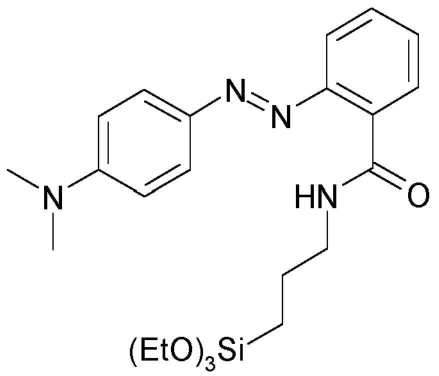

onto a substrate to give a monolayer that is able to be photoaligned to induce homogeneous alignment of a liquid crystal. However, a separate step of self-assembly before manufacture of the LC cell is required and the nature of the azo-group

causes reversibility of the alignment when exposed to light.

Another functional group known to enable photoalignment is the phenylethenylcarbonyloxy group (cinnamate). Photocrosslinkable cinnamates are known from the prior art, e.g. of the following structure

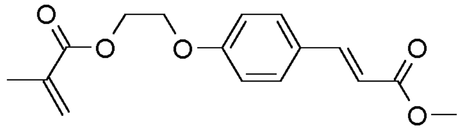

as disclosed in EP0763552. From such compounds, polymers can be obtained, for example the following

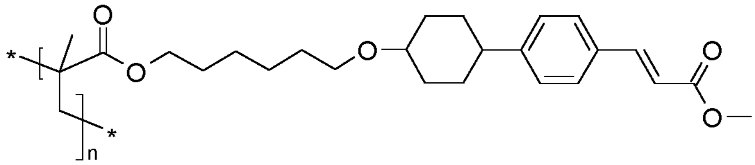

This material was used in a photoalignment process, as disclosed in WO 99/49360, to give an orientation layer for liquid crystals. A disadvantage of orientation layers obtained by this process is that they give lower voltage holding ratios (VHR) than polyimides.

In WO 00/05189 polymerizable direactive mesogenic cinnamates are disclosed for the use in polymerizable LC mixtures for e.g. optical retarders.

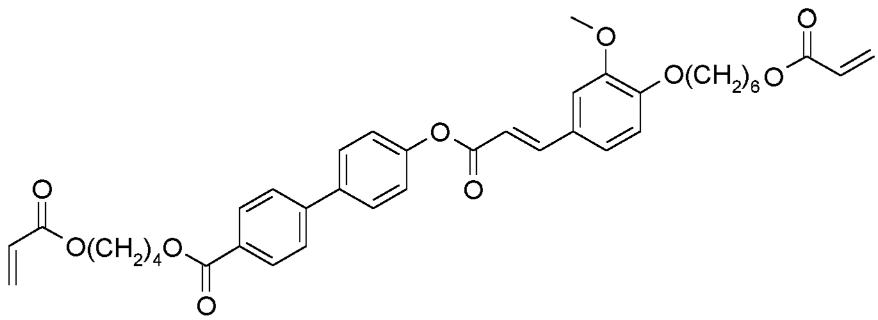

A structurally related compound of the following formula

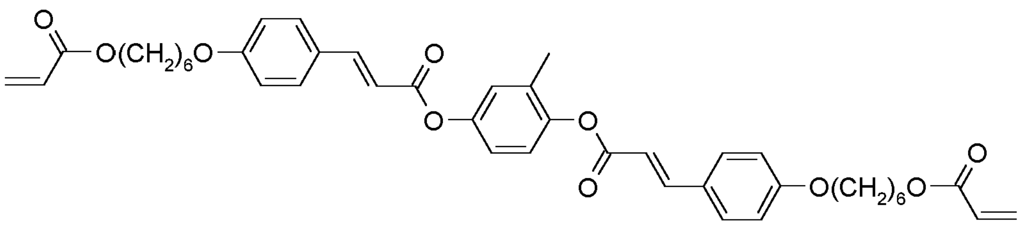

comprising two cinnamic acid moieties is disclosed in GB 2 306 470 A for the use as component in liquid crystalline polymer films. This type of compound has not been used or proposed for the use as photoalignment agent.

A very similar compound is published in B.M.I. van der Zande et al., Liquid Crystals, Vol. 33, No. 6, June 2006, 723-737, in the field of liquid crystalline polymers for patterned retarders, and has the following structure:

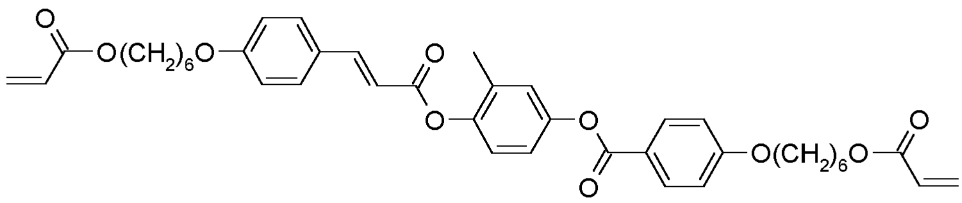

WO 2017/102068 A1 discloses the same structure for the purpose of a polyimide-free homogeneous photoalignment method.

Further, M.H. Lee et al. published in Liquid Crystals (https://doi.org/10.1080/02678292.2018.1441459) a polyimide-free homogeneous photoalignment method induced by polymerizable liquid crystal containing cinnamate moiety of the following formula:

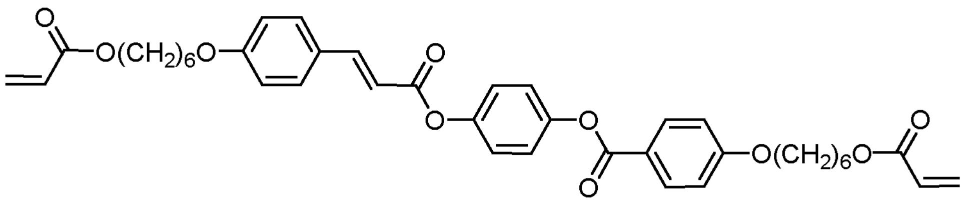

WO 2018/008581 A1 provides a liquid crystal display device that is equipped with: a liquid crystal layer containing a liquid crystal material; a sealing material positioned so as to surround the liquid crystal layer when seen from a planar view; a pair of substrates which sandwich the liquid crystal layer and are joined to one another by the sealing material; and an alignment control layer positioned so as to contact the liquid crystal layer in the region surrounded by the sealing material when seen from a planar view. Therein, the alignment control layer aligns the liquid crystal material in the horizontal direction relative to the substrate surface, and contains a polymer containing a unit derived from at least a specific first monomer of the following formulae:

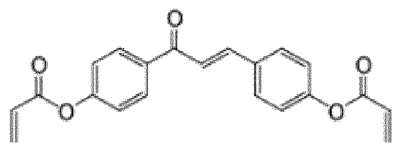
(2-1)

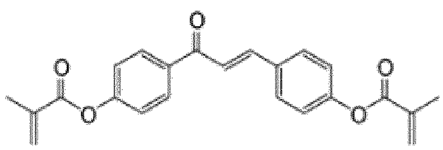
(2-2)

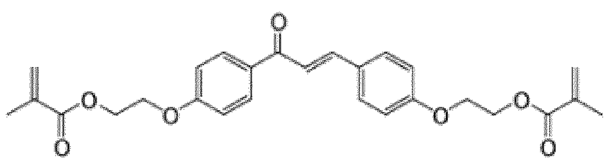
(2-3)

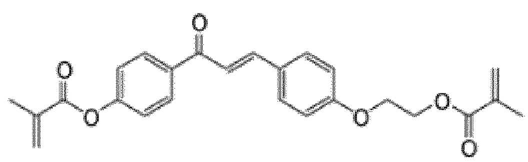
(2-4)

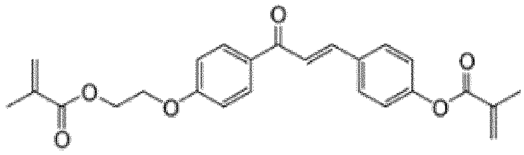

(2-5)

WO 2018/139507 A1 addresses the problem of controlling the alignment of liquid crystal molecules of a liquid crystal display element not having an alignment film by using a non-colored alignable monomer of the following formulae:

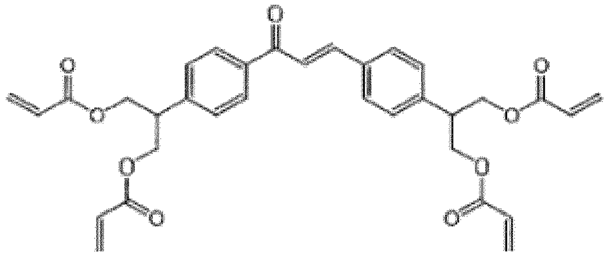

(A-2-3)

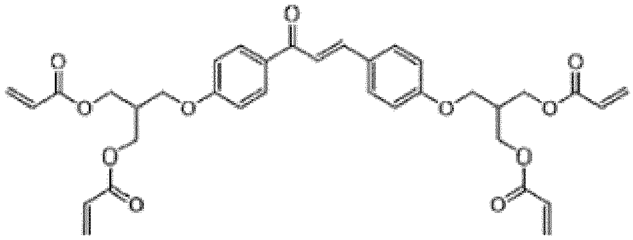

(A-2-4)

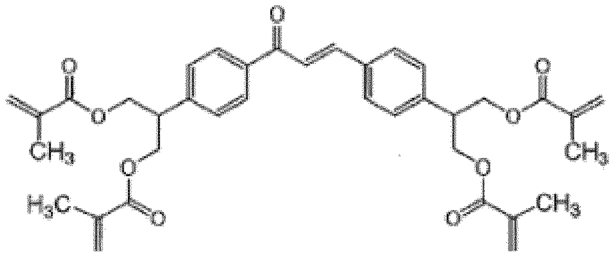

(A-2-5)

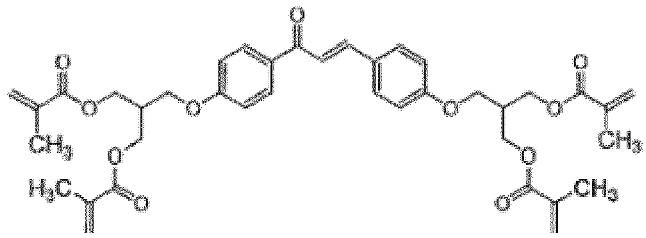

(A-2-6)

Both types of the above-suggested monomers are limited in their solubility in modern liquid crystalline mixtures and limited in their processability especially in view of modern methods for mass production.

EP 3 466 918 A1 describes so called self-aligning LC additives for the purpose of providing an LCD or LC element that do not require an additional alignment layer.

Thus, there is a great demand for new photoreactive mesogens that enable photoalignment of a liquid crystal mixture in *situ, i.e.* after assembly of the display, by means of linearly polarized light.

In addition to this requirement, the corresponding photoreactive mesogen should provide, preferably at the same time, a liquid crystal display having favourable high dark state and a favourable high voltage holding ratio. Furthermore, the amount of photoreactive mesogens in the nematic LC medium should be a low as possible and the process for the production should be obtainable from a process that is compatible with common mass production processes, e.g. in terms of favourable short processing times.

Other aims of the present invention are immediately evident to the person skilled in the art from the following detailed description.

Surprisingly, the inventors have found out that one or more of the above-mentioned aims can be achieved by providing a compound according to claim 1.

Terms and Definitions

A photoreactive group according to the present invention is a functional group of a molecule that causes a change of the geometry of the molecule either by bond rotation, skeletal rearrangement or atom- or group- transfer, or by dimerization, upon irradiation with light of a suitable wavelength that can be absorbed by the molecule.

The term "mesogenic group" as used herein is known to the person skilled in the art and described in the literature, and means a group which, due to the anisotropy of its attracting and repelling interactions, essentially contributes to causing a liquid-crystal (LC) phase in low-molecular-weight or polymeric substances. Compounds containing mesogenic groups (mesogenic compounds) do not necessarily have to have an LC phase themselves. It is also possible for mesogenic compounds to exhibit LC phase behaviour only after mixing with other compounds and/or after polymerisation. Typical mesogenic groups are, for example, rigid rod- or disc-shaped units. An overview of the terms and definitions used in connection with mesogenic or LC compounds is given in Pure Appl. Chem. 2001, 73(5), 888 and C. Tschierske, G. Pelzl, S. Diele, Angew. Chem. 2004, 116, 6340-6368.

A photoreactive mesogen according to the present invention is a mesogenic compound comprising one or more photoreactive groups.

Examples of photoreactive groups are -C=C- double bonds and azo groups (-N=N-).

Examples of molecular structures and sub-structures comprising such photoreactive groups are stilbene, (1,2-difluoro-2-phenyl-vinyl)-benzene, cinnamate, 4-phenylbut-3-en-2-one, chalcone, coumarin, chromone, pentalenone and azobenzene.

According to the present application, the term "linearly polarised light" means light, which is at least partially linearly polarized. Preferably, the aligning light is linearly polarized with a degree of polarization of more than 5:1. Wavelengths, intensity and energy of the linearly polarised light are chosen depending on the photosensitivity of the photoalignable material. Typically, the wavelengths are in the UV-A, UV-B and/or UV-C range or in the visible range. Preferably, the linearly polarised light comprises light of wavelengths less than 450 nm, more preferably less than 420 nm at the same time the linearly polarised light preferably comprises light of wavelengths longer than 280nm, preferably more than 320nm, more preferably over 350nm.

The term "organic group" denotes a carbon or hydrocarbon group.

The term "carbon group" denotes a mono- or polyvalent organic group containing at least one carbon atom, where this either contains no further atoms (such as, for example, -C≡C-) or optionally contains one or more further atoms, such as, for example, N, O, S, P, Si, Se, As, Te or Ge (for example carbonyl, etc.). The term "hydrocarbon group" denotes a carbon group which additionally contains one or more H atoms and optionally one or more heteroatoms, such as, for example, N, O, S, P, Si, Se, As, Te or Ge.

"Halogen" denotes F, Cl, Br or I.

A carbon or hydrocarbon group can be a saturated or unsaturated group. Unsaturated groups are, for example, aryl, alkenyl or alkynyl groups. A carbon or hydrocarbon radical having 3 or more atoms can be straight-chain, branched and/or cyclic and may also contain spiro links or condensed rings.

The terms "alkyl", "aryl", "heteroaryl", etc., also encompass polyvalent groups, for example alkylene, arylene, heteroarylene, etc.

The term "aryl" denotes an aromatic carbon group or a group derived therefrom. The term "heteroaryl" denotes "aryl" as defined above, containing one or more heteroatoms.

Preferred carbon and hydrocarbon groups are optionally substituted alkyl, alkenyl, alkynyl, alkoxy, alkylcarbonyl, alkoxycarbonyl, alkylcarbonyloxy and alkoxycarbonyloxy having 1 to 40, preferably 1 to 25, particularly preferably 1 to 18, C atoms, optionally substituted aryl or aryloxy having 6 to 40, preferably 6 to 25, C atoms, or optionally substituted alkylaryl, arylalkyl, alkylaryloxy, arylalkyloxy, arylcarbonyl, aryloxycarbonyl, arylcarbonyloxy and aryloxycarbonyloxy having 6 to 40, preferably 6 to 25, C atoms.

Further preferred carbon and hydrocarbon groups are $C_1$-$C_{40}$ alkyl, $C_2$-$C_{40}$ alkenyl, $C_2$-$C_{40}$ alkynyl, $C_3$-$C_{40}$ allyl, $C_4$-$C_{40}$ alkyldienyl, $C_4$-$C_{40}$ polyenyl, $C_6$-$C_{40}$ aryl, $C_6$-$C_{40}$ alkylaryl, $C_6$-$C_{40}$ arylalkyl, $C_6$-$C_{40}$ alkylaryloxy, $C_6$-$C_{40}$ arylalkyloxy, $C_2$-$C_{40}$ heteroaryl, $C_4$-$C_{40}$ cycloalkyl, $C_4$-$C_{40}$ cycloalkenyl, etc. Particular preference is given to $C_1$-$C_{22}$ alkyl, $C_2$-$C_{22}$ alkenyl, $C_2$-$C_{22}$ alkynyl, $C_3$-$C_{22}$ allyl, $C_4$-$C_{22}$ alkyldienyl, $C_6$-$C_{12}$ aryl, $C_6$-$C_{20}$ arylalkyl and $C_2$-$C_{20}$ heteroaryl.

Further preferred carbon and hydrocarbon groups are straight-chain, branched or cyclic alkyl radicals having 1 to 40, preferably 1 to 25, C atoms, which are unsubstituted or mono- or polysubstituted by F, C!, Br, I or CN and in which one more non-adjacent $CH_2$ groups may each be replaced, independently of one another, by -C($R^z$)=C($R^z$)-, -C≡C-, -N($R^z$)-, -O-, -S-, -CO-, -CO-O-, -O-CO-, -O-CO-O- in such a way that O and/or S atoms are not linked directly

to one another.

$R^z$ preferably denotes H, halogen, a straight-chain, branched or cyclic alkyl chain having 1 to 25 C atoms, in which, in addition, one or more non-adjacent C atoms may be replaced by -O-, -S-, -CO-, -CO-O-, -O-CO- or -O-CO-O- and in which one or more H atoms may be replaced by fluorine, an optionally substituted aryl or aryloxy group having 6 to 40 C atoms, or an optionally substituted heteroaryl or heteroaryloxy group having 2 to 40 C atoms.

Preferred alkyl groups are, for example, methyl, ethyl, n-propyl, isopropyl, n-butyl, isobutyl, s-butyl, t-butyl, 2-methylbutyl, n-pentyl, s-pentyl, cyclopentyl, n-hexyl, cyclohexyl, 2-ethylhexyl, n-heptyl, cycloheptyl, n-octyl, cyclooctyl, n-nonyl, n-decyl, n-undecyl, n-dodecyl, trifluoromethyl, perfluoro-n-butyl, 2,2,2-trifluoroethyl, perfluorooctyl and perfluorohexyl.

Preferred alkenyl groups are, for example, ethenyl, propenyl, butenyl, pentenyl, cyclopentenyl, hexenyl, cyclohexenyl, heptenyl, cycloheptenyl, octenyl and cyclooctenyl.

Preferred alkynyl groups are, for example, ethynyl, propynyl, butynyl, pentynyl, hexynyl and octynyl.

Preferred alkoxy groups are, for example, methoxy, ethoxy, 2-methoxy-ethoxy, n-propoxy, i-propoxy, n-butoxy, i-butoxy, s-butoxy, t-butoxy, 2-methylbutoxy, n-pentoxy, n-hexoxy, n-heptoxy, n-octoxy, n-nonoxy, n-decoxy, n-undecoxy and n-dodecoxy.

Preferred amino groups are, for example, dimethylamino, methylamino, methylphenylamino and phenylamino.

Aryl and heteroaryl groups can be monocyclic or polycyclic, i.e. they can contain one ring (such as, for example, phenyl) or two or more rings, which may also be fused (such as, for example, naphthyl) or covalently bonded (such as, for example, biphenyl), or contain a combination of fused and linked rings. Heteroaryl groups contain one or more heteroatoms, preferably selected from O, N, S and Se. A ring system of this type may also contain individual non-conjugated units, as is the case, for example, in the fluorene basic structure.

Particular preference is given to mono-, bi- or tricyclic aryl groups having 6 to 25 C atoms and mono-, bi- or tricyclic heteroaryl groups having 2 to 25 C atoms, which optionally contain fused rings and are optionally substituted. Preference is furthermore given to 5-, 6- or 7-membered aryl and heteroaryl groups, in which, in addition, one or more CH groups may be replaced by N, S or O in such a way that O atoms and/or S atoms are not linked directly to one another.

Preferred aryl groups are derived, for example, from the parent structures benzene, biphenyl, terphenyl, [1,1':3',1"]terphenyl, naphthalene, anthracene, binaphthyl, phenanthrene, pyrene, dihydropyrene, chrysene, perylene, tetracene, pentacene, benzopyrene, fluorene, indene, indenofluorene, spirobifluorene, etc.

Preferred heteroaryl groups are, for example, 5-membered rings, such as pyrrole, pyrazole, imidazole, 1,2,3-triazole, 1,2,4-triazole, tetrazole, furan, thiophene, selenophene, oxazole, isoxazole, 1,2-thiazole, 1,3-thiazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 6-membered rings, such as pyridine, pyridazine, pyrimidine, pyrazine, 1,3,5-triazine, 1,2,4-triazine, 1,2,3-triazine, 1,2,4,5-tetrazine, 1,2,3,4-tetrazine, 1 ,2,3,5-tetrazine, or condensed groups, such as indole, isoindole, indolizine, indazole, benzimidazole, benzotriazole, purine, naphthimid-azole, phenanthrimidazole, pyridimidazole, pyrazinimidazole, quinoxalin-imidazole, benzoxazole, naphthoxazole, anthroxazole, phenanthroxazole, isoxazole, benzothiazole, benzofuran, isobenzofuran, dibenzofuran, quinoline, isoquinoline, pteridine, benzo-5,6-quinoline, benzo-6,7-quinoline, benzo-7,8-quinoline, benzoisoquinoline, acridine, phenothiazine, phenoxazine, benzopyridazine, benzopyrimidine, quinoxaline, phenazine, naphthyridine, azacarbazole, benzocarboline, phenanthridine, phenanthroline, thieno[2,3b]thiophene, thieno[3,2b]thiophene, dithienothiophene, dihydrothieno [3,4-b]-1 ,4-dioxin, isobenzothiophene, dibenzothiophene, benzothiadiazothiophene, or combinations of these groups. The heteroaryl groups may also be substituted by alkyl, alkoxy, thioalkyl, fluorine, fluoroalkyl or further aryl or heteroaryl groups.

The (non-aromatic) alicyclic and heterocyclic groups encompass both saturated rings, i.e. those containing exclusively single bonds, and also partially unsaturated rings, i.e. those which may also contain multiple bonds. Heterocyclic rings contain one or more heteroatoms, preferably selected from Si, O, N, S and Se.

The (non-aromatic) alicyclic and heterocyclic groups can be monocyclic, i.e. contain only one ring (such as, for example, cyclohexane), or polycyclic, i.e. contain a plurality of rings (such as, for example, decahydronaphthalene or bicyclooctane). Particular preference is given to saturated groups. Preference is furthermore given to mono-, bi- or tricyclic groups having 3 to 25 C atoms, which optionally contain fused rings and are optionally substituted. Preference is furthermore given to 5-, 6-, 7- or 8-membered carbocyclic groups, in which, in addition, one or more C atoms may be replaced by Si and/or one or more CH groups may be replaced by N and/or one or more non-adjacent $CH_2$ groups may be replaced by -O- and/or -S-.

Preferred alicyclic and heterocyclic groups are, for example, 5-membered groups, such as cyclopentane, tetrahydrofuran, tetrahydrothiofuran, pyrrolidine, 6-membered groups, such as cyclohexane, silinane, cyclohexene, tetrahydropyran, tetrahydrothiopyran, 1,3-dioxane, 1,3-dithiane, piperidine, 7-membered groups, such as cycloheptane, and fused groups, such as tetrahydronaphthalene, decahydronaphthalene, indane, bicyclo[1.1.1]pentane-1,3-diyl, bicyclo[2.2.2]octane-1,4-diyl, spiro[3.3]heptane-2,6-diyl, octahydro-4,7-methanoindane-2,5-diyl.

The aryl, heteroaryl, carbon and hydrocarbon radicals optionally have one or more substituents, which are preferably selected from the group comprising silyl, sulfo, sulfonyl, formyl, amine, imine, nitrile, mercapto, nitro, halogen,

$C_{1-12}$ alkyl, $C_{6-12}$ aryl, $C_{1-12}$ alkoxy, hydroxyl, or combinations of these groups.

Preferred substituents are, for example, solubility-promoting groups, such as alkyl or alkoxy, and electron-withdrawing groups, such as fluorine, nitro or nitrile.

Preferred substituents, unless stated otherwise, also referred to as "L" above and below, are F, C!, Br, I, -CN, -$NO_2$, -NCO, -NCS, -OCN, -SCN, - C(=O)N($R^z$)$_2$, -C(=O)Y', -C(=O)$R^z$, -N($R^z$)$_2$, in which $R^z$ has the meaning indicated above, and $Y^1$ denotes halogen, optionally substituted silyl or aryl having 6 to 40, preferably 6 to 20, C atoms, and straight-chain or branched alkyl, alkoxy, alkylcarbonyl, alkoxycarbonyl, alkylcarbonyloxy or alkoxycarbonyloxy having 1 to 25 C atoms, preferably 2 to 12, in which one or more H atoms may optionally be replaced by F or Cl.

"Substituted silyl or aryl" preferably means substituted by halogen, -CN, $R^{y1}$, -O$R^{y1}$, -CO-$R^{y1}$, -CO-O-$R^{y1}$, -O-CO-$R^{y1}$ or -O-CO-O-$R^{y1}$, in which $R^{y1}$ has the meaning indicated above.

Particularly preferred substituents L are, for example, F, Cl, CN, $CH_3$, $C_2H_5$, -CH($CH_3$)$_2$, $OCH_3$, $OC_2H_5$, $CF_3$, $OCF_3$, $OCHF_2$, $OC_2F_5$, furthermore phenyl.

Above and below "halogen" denotes F, Cl, Br or I.

Above and below, the terms "alkyl", "aryl", "heteroaryl", etc., also encompass polyvalent groups, for example alkylene, arylene, heteroarylene, etc.

The term "director" is known in prior art and means the preferred orientation direction of the long molecular axes (in case of calamitic compounds) or short molecular axes (in case of discotic compounds) of the liquid-crystalline molecules. In case of uniaxial ordering of such anisotropic molecules, the director is the axis of anisotropy.

The term "alignment" or "orientation" relates to alignment (orientation ordering) of anisotropic units of material such as small molecules or fragments of big molecules in a common direction named "alignment direction". In an aligned layer of liquid-crystalline material, the liquid-crystalline director coincides with the alignment direction so that the alignment direction corresponds to the direction of the anisotropy axis of the material.

The term "planar orientation/alignment", for example in a layer of an liquid-crystalline material, means that the long molecular axes (in case of calamitic compounds) or the short molecular axes (in case of discotic compounds) of a proportion of the liquid-crystalline molecules are oriented substantially parallel (about 180°) to the plane of the layer.

The term "homeotropic orientation/alignment", for example in a layer of a liquid-crystalline material, means that the long molecular axes (in case of calamitic compounds) or the short molecular axes (in case of discotic compounds) of a proportion of the liquid-crystalline molecules are oriented at an angle $\theta$ ("tilt angle") between about 80° to 90° relative to the plane of the layer.

The terms "uniform orientation" or "uniform alignment" of an liquid-crystalline material, for example in a layer of the material, mean that the long molecular axes (in case of calamitic compounds) or the short molecular axes (in case of discotic compounds) of the liquid-crystalline molecules are oriented substantially in the same direction. In other words, the lines of liquid-crystalline director are parallel.

The wavelength of light generally referred to in this application is 550 nm, unless explicitly specified otherwise.

The birefringence $\Delta n$ herein is defined by the following equation

$$\Delta n = n_e - n_o$$

wherein $n_e$ is the extraordinary refractive index and $n_o$ is the ordinary refractive index and the effective average refractive index $n_{av.}$ is given by the following equation

$$n_{av.} = [(2\ n_o^2 + n_e^2)/3]^{1/2}$$

The extraordinary refractive index $n_e$ and the ordinary refractive index $n_o$ can be measured using an Abbe refractometer.

In the present application the term "dielectrically positive" is used for compounds or components with $\Delta\varepsilon > 3.0$, "dielectrically neutral" with $-1.5 \leq \Delta\varepsilon \leq 3.0$ and "dielectrically negative" with $\Delta\varepsilon < -1.5$. $\Delta\varepsilon$ is determined at a frequency of 1 kHz and at 20°C. The dielectric anisotropy of the respective compound is determined from the results of a solution of 10 % of the respective individual compound in a nematic host mixture. In case the solubility of the respective compound in the host medium is less than 10 % its concentration is reduced by a factor of 2 until the resultant medium is stable enough at least to allow the determination of its properties. Preferably, the concentration is kept at least at 5 %, however, to keep the significance of the results as high as possible. The capacitance of the test mixtures are determined both in a cell with homeotropic and with homogeneous alignment. The cell gap of both types of cells is approximately 20 $\mu$m. The voltage applied is a rectangular wave with a frequency of 1 kHz and a root mean square value typically of 0.5 V to 1.0 V; however, it is always selected to be below the capacitive threshold of the respective test mixture.

$\Delta\varepsilon$ is defined as ($\varepsilon_{||} - \varepsilon_{\perp}$), whereas $\varepsilon_{av.}$ is ($\varepsilon_{||} + 2\ \varepsilon_{\perp}$) / 3. The dielectric permittivity of the compounds is

determined from the change of the respective values of a host medium upon addition of the compounds of interest. The values are extrapolated to a concentration of the compounds of interest of 100 %. A typical host medium is ZLI-4792 or ZLI-2857 both commercially available from Merck, Darmstadt.

For the present invention,

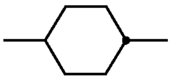 , 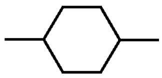 , 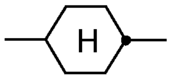 and 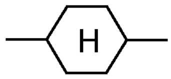

denote trans-1,4-cyclohexylene,

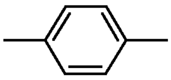 and 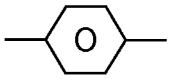

denote 1,4-phenylene.

For the present invention the groups -CO-O-, -COO- -C(=O)O- or $-CO_2$-denote an ester group of formula

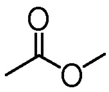 ,

and the groups -O-CO- -OCO-, -OC(=O)-, $-O_2C$- or -OOC- denote an ester group of formula

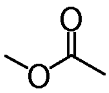 .

Furthermore, the definitions as given in C. Tschierske, G. Pelzl and S. Diele, Angew. Chem. 2004, 116, 6340-6368 shall apply to non-defined terms related to liquid crystal materials in the instant application.

<u>Detailed description</u>

In detail, the present invention relates to compounds or photoreactive mesogens of formula I,

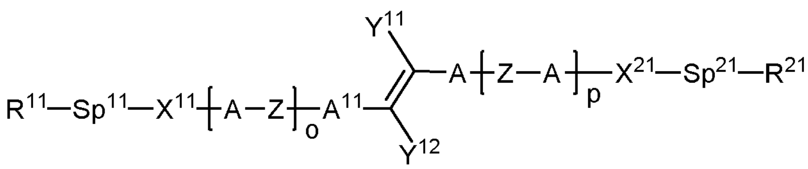 I

wherein

$A^{11}$ denotes a radical selected from the following groups: a) a group consisting of 1,4-phenylene and 1,3-phenylene, wherein, in addition, one or two CH groups may be replaced by N and wherein, in addition, one or more H atoms may be replaced by L, b) a group selected from the group consisting of

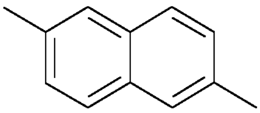 , 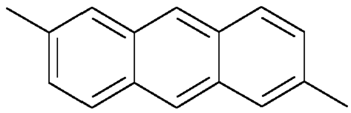 ,

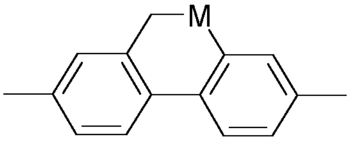 , 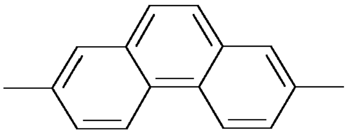 ,

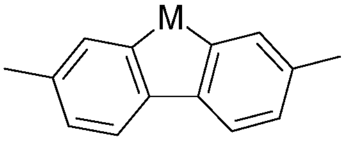 , 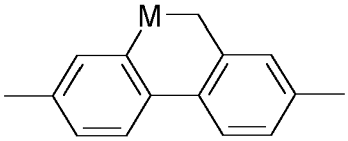 ,

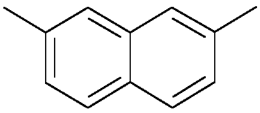 , 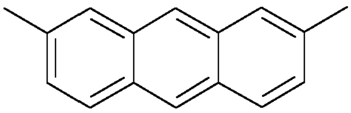 ,

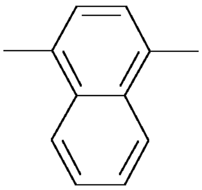 , 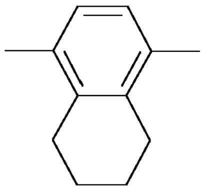 ,

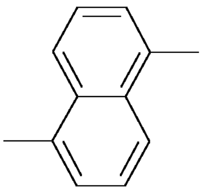 ,

where, in addition, one or more H atoms in these radicals may be replaced by L, and/or one or more double bonds may be replaced by single bonds, and/or one or more CH groups may be replaced by N,

A have each, independently of one another, in each occurrence one of the meanings for $A^{11}$ or a) group consisting of trans-1 ,4-cyclohexylene, 1,4-cyclohexenylene, wherein, in addition, one or more non-adjacent $CH_2$ groups may be replaced by -O-and/or -S- and wherein, in addition, one or more H atoms may be replaced by F, or b) a group consisting of tetrahydropyran-2,5-diyl, 1,3-dioxane-2,5-diyl, tetrahydrofuran-2,5-diyl, cyclobutane-1,3-diyl, piperidine-1,4-diyl, thiophene-2,5-diyl and selenophene-2,5-diyl, each of which may also be mono- or polysubstituted by L,

L on each occurrence, identically or differently, denotes -OH, -F, -Cl, -Br, -I, -CN, $-NO_2$, $SF_5$, -NCO, -NCS, -OCN, -SCN, $-C(=O)N(R^z)_2$, $-C(=O)R^z$, $-N(R^z)_2$, optionally substituted silyl, optionally substituted aryl having 6 to 20 C atoms, or straight-chain or branched or cyclic alkyl, alkoxy, alkylcarbonyl, alkoxycarbonyl, alkylcarbonyloxy or alkoxycarbonyloxy having 1 to 25 C atoms, preferably 1 to 12 C atoms, more preferably 1 to 6 C atoms , in which, in addition, one or more H atoms may be replaced by F or Cl, or $X^{21}$-$Sp^{21}$-$R^{21}$,

M denotes -O-, -S-, $-CH_2-$, $-CHR^z-$ or $-CR^yR^z-$, and

$R^y$ and $R^z$ each, independently of one another, denote H, CN, F or alkyl having 1-12 C atoms, wherein one or more H atoms may be replaced by F, preferably H, methyl, ethyl, propyl, butyl, more preferably H or methyl, in particular H,

$Y^{11}$ and $Y^{12}$ each, independently of one another, denote H, F, phenyl or optionally fluorinated alkyl having 1-12 C atoms, wherein one or more H atoms may be replaced by F, preferably H, F, methyl, ethyl, propyl,

butyl, more preferably H, F or methyl, in particular H or F,

Z denotes, independently of each other, in each occurrence, a single bond, -COO-, -OCO-, -O-CO-O-, $-OCH_2-$, $-CH_2O-$, $-OCF_2-$, $-CF_2O-$, $-(CH_2)_n-$ wherein one or more non-adjacent groups may be replaced by O or S, $-CF_2CF_2-$, -CH=CH-, -CF=CF-, -CH=CH-COO-, -OCO-CH=CH-, -CO-S-, -S-CO-, -CS-S-, -S-CS-, -S-CSS- or -C≡C-, preferably a single bond, -COO-,-OCO-, $-OCF_2-$, $-CF_2O-$, or $-(CH_2)_n-$, more preferably a single bond, -COO-,or -OCO-,

n denotes an integer between 2 and 8, preferably 2,

o and p denote each and independently 0, 1 or 2, preferably 1,

$X^{11}$ and $X^{21}$ denote independently from one another, in each occurrence a single bond, -CO-O-, -O-CO-, -O-COO-, -O-, -CH=CH-, -C≡C-, $-CF_2-O-$, $-O-CF_2-$, $-CF_2-CF_2-$, $-CH_2-O-$, $-O-CH_2-$, -CO-S-, -S-CO-, -CS-S-, -S-CS-, -S-CSS- or -S-, preferably, a single bond -CO-O-, -O-CO-, -O-COO-, or -O-, more preferably a single bond or -O-,

$Sp^{11}$ and $Sp^{21}$ denote each and independently, in each occurrence a single bond or a spacer group comprising 1 to 20 C atoms, preferably 2 to 20 C atoms, wherein one or more non-adjacent and non-terminal $CH_2$ groups may also be replaced by -O-, -S-, -NH-, $-N(CH_3)-$, -CO-, -O-CO-, -S-CO-, -O-COO-, -CO-S-, -CO-O-, $-CF_2-$, $-CF_2O-$, $-OCF_2-$ -C(OH)-, -CH(alkyl)-, -CH(alkenyl)-, -CH(alkoxyl)-, -CH(oxaalkyl)-, -CH=CH- or -C≡C-, however in such a way that no two O-atoms are adjacent to one another and no two groups selected from -O-CO-, -S-CO-, -O-COO-, -CO-S-, -CO-O- and -CH=CH- are adjacent to each other, preferably alkylene having 1 to 20, preferably 1 to 12, C atoms, which is optionally mono- or polysubstituted by F, C!, Br, I or CN, more preferably straight-chain ethylene, propylene, butylene, pentylene, hexylene, heptylene, octylene, nonylene, decylene, undecylene, dodecylene,

$R^{11}$ and $R^{21}$ denotes $P^{11}$ or under the condition that at least one of $R^{11}$ and $R^{21}$ denotes $P^{21}$, more preferably both $P^{21}$,

$P^{11}$ each and independently from another in each occurrence a polymerizable group selected from the group consisting of acrylate, methacrylate, fluoroacrylate, furthermore vinyloxy, chloroacrylate, oxetane and epoxide groups,

$P^{21}$ denotes a group

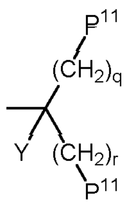

preferably a group

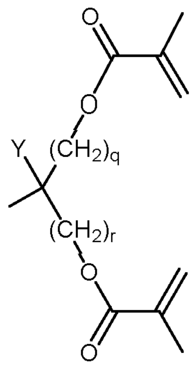

, or

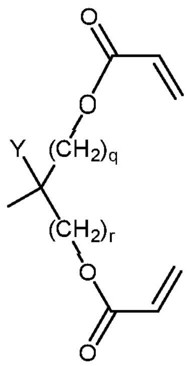

Y denotes H, F, phenyl or optionally fluorinated alkyl having 1-12 C atoms, preferably H, methyl, ethyl, propyl, butyl, more preferably H, methyl ethyl or propyl, in particular H, methyl, or ethyl, especially methyl or ethyl,

q and r denotes each and independently an integer from 0 to 8, preferably q+r ≥ 1 and ≤ 16, more preferably q and r each and independently denotes an integer from1 to 8.

Further prefrerred are compounds of formula I, wherein

$Sp^{21}$ denotes a spacer group comprising 1 to 20 C atoms, preferably 2 to 20 C atoms, wherein one or more non-adjacent and non-terminal $CH_2$ groups may also be replaced by -O-, -S-, -NH-, -N($CH_3$)-, -CO-, -O-CO-, -S-CO-, -O-COO-, -CO-S-, -CO-O-, -$CF_2$-, -$CF_2$O-, -O$CF_2$- -C(OH)-, -CH(alkyl)-, -CH(alkenyl)-, -CH(alkoxyl)-, -CH(oxaalkyl)-, -CH=CH- or -C≡C-, however in such a way that no two O-atoms are adjacent to one another and no two groups selected from -O-CO-, -S-CO-, -O-COO-, -CO-S-, -CO-O- and -CH=CH- are adjacent to each other, if $X^{21}$ denotes single bond and if at the same time $R^{11}$ denotes $P^{21}$ and Y denotes H.

Further prefrerred are compounds of formula I, wherein

$Sp^{21}$ denotes a spacer group comprising 2 to 20 C atoms, wherein one or more non-adjacent and non-terminal $CH_2$ groups may also be replaced by -O-, -S-, -NH-, -N($CH_3$)-, -CO-, -O-CO-, -S-CO-, -O-COO-, -CO-S-, -CO-O-, -$CF_2$-, -$CF_2$O-, -O$CF_2$- -C(OH)-, -CH(alkyl)-, -CH(alkenyl)-,-CH(alkoxyl)-, -CH(oxaalkyl)-, -CH=CH- or -C≡C-, however in such a way that no two O-atoms are adjacent to one another and no two groups selected from -O-CO-, -S-CO-, -O-COO-, -CO-S-, -CO-O- and -CH=CH- are adjacent to each other, if $X^{21}$ denotes -O- and if at the same time $R^{11}$ denotes $P^{21}$ and Y denotes H.

In the instant application, polymerizable groups (P) are groups that are suitable for a polymerisation reaction, such as, for example, free-radical or ionic chain polymerisation, polyaddition or polycondensation, or for a polymer-analogous reaction, for example addition or condensation onto a main polymer chain. Particular preference is given to groups for chain polymerisation, in particular those containing a C=C double bond or -C≡C-triple bond, and groups which are suitable for polymerisation with ring opening, such as, for example, oxetane or epoxide groups.

According to the invention, groups $P^{11}$ are selected from the group consisting of acrylate, methacrylate, fluoroacrylate, furthermore vinyloxy, chloroacrylate, oxetane and epoxide groups, and of these preferably an vinyloxy, acrylate or methacrylate group.

The compounds of formula I are preferably selected from compounds of the sub-formulae I-1 to I-9,

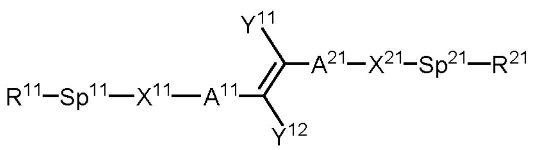

I-1

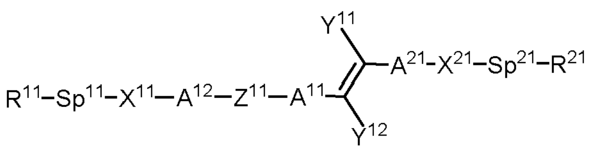

I-2

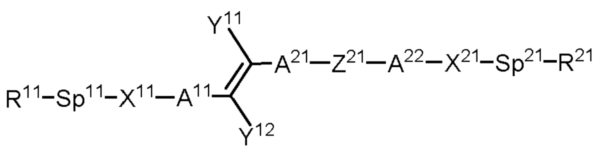

I-3

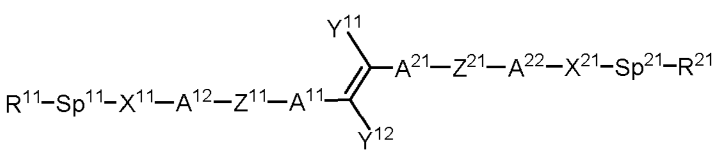

I-4

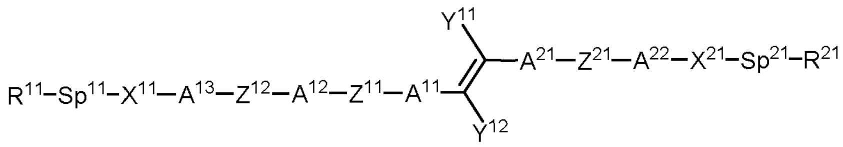

I-5

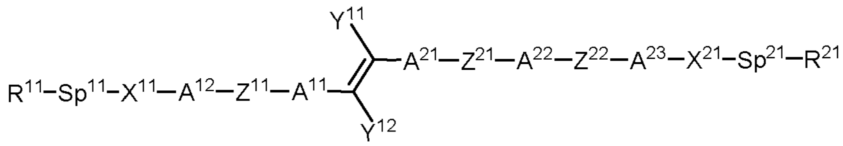

I-6

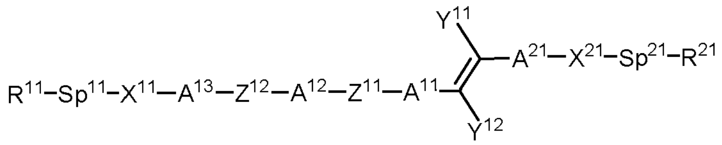

I-7

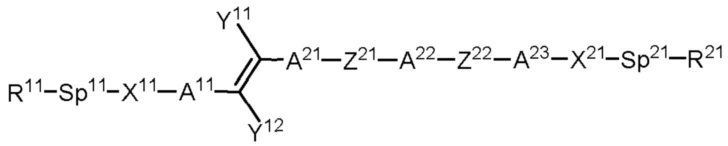

I-8

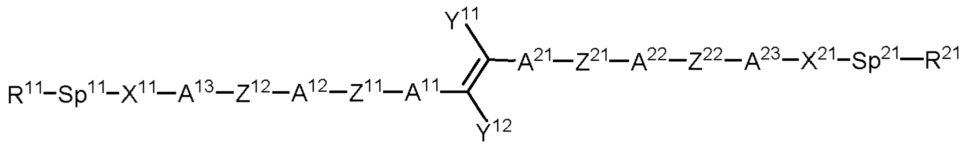

I-9

wherein $R^{11}$, $R^{21}$, $A^{11}$, $X^{11}$, $X^{21}$, $Y^{11}$, $Y^{12}$, $Sp^{11}$, and $Sp^{21}$ have one of the meanings as given above in formula I, $A^{12}$ to $A^{23}$ have one of the meanings for A in formula I, and $Z^{11}$ to $Z^{22}$ have one of the meanings for Z as given above under formula I.

Further prefrerred are compounds of formula I-1, wherein

$Sp^{21}$ denotes a spacer group comprising 1 to 20 C atoms, preferably 2 to 20 C atoms, wherein one or more non-adjacent and non-terminal $CH_2$ groups may also be replaced by -O-, -S-, -NH-, -N($CH_3$)-, -CO-, -O-CO-, -S-CO-, -O-COO-, -CO-S-, -CO-O-, -$CF_2$-, -$CF_2$O-, -O$CF_2$- -C(OH)-, -CH(alkyl)-, -CH(alkenyl)-, -CH(alkoxyl)-, -CH(oxaalkyl)-, -CH=CH- or -C≡C-, however in such a way that no two O-atoms are adjacent to one another and no two groups selected from -O-CO-, -S-CO-, -O-COO-, -CO-S-, -CO-O- and -CH=CH- are adjacent to each other, if $X^{21}$ denotes single bond and if at the same time $R^{21}$ denotes $P^{21}$ and Y denotes H.

Further prefrerred are compounds of formula I, wherein

$Sp^{21}$ denotes a spacer group comprising 2 to 20 C atoms, wherein one or more non-adjacent and non-terminal $CH_2$ groups may also be replaced by -O-, -S-, -NH-, -N($CH_3$)-, -CO-, -O-CO-, -S-CO-, -O-COO-, -CO-S-, -CO-O-, -$CF_2$-, -$CF_2$O-, -O$CF_2$- -C(OH)-, -CH(alkyl)-, -CH(alkenyl)-,-CH(alkoxyl)-, -CH(oxaalkyl)-, -CH=CH- or -C≡C-, however in such a way that no two O-atoms are adjacent to one another and no two groups selected from -O-CO-, -S-CO-, -O-COO-, -CO-S-, -CO-O- and -CH=CH- are adjacent to each other, if $X^{21}$ denotes -O- and if at the same time $R^{21}$ denotes $P^{21}$ and Y denotes H.

Further preferred compounds of formula I are selected from the following compounds of formulae I-1a to I-4c.

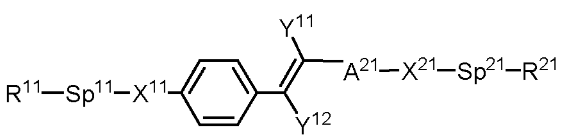

I-1a

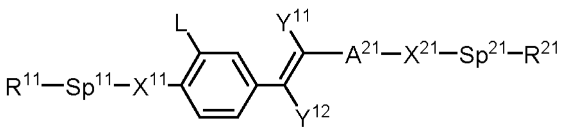
I-1b

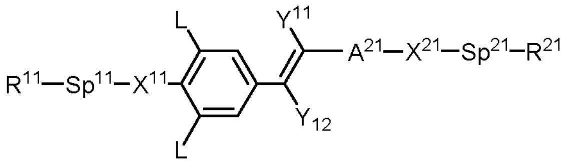
I-1c

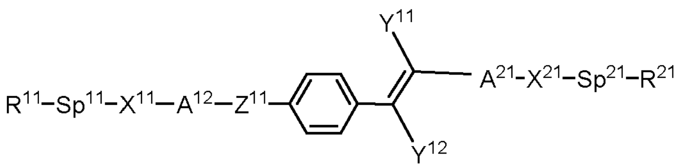
I-2a

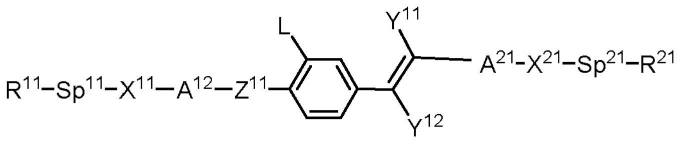
I-2b

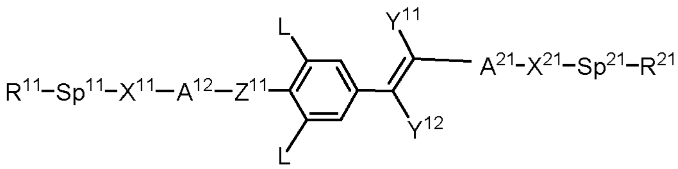
I-2c

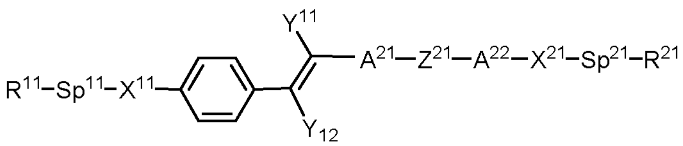
I-3a

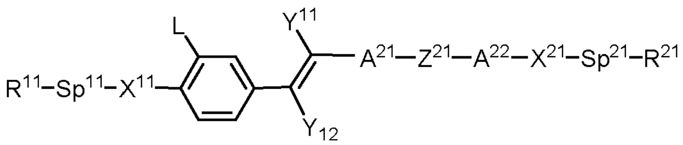
I-3b

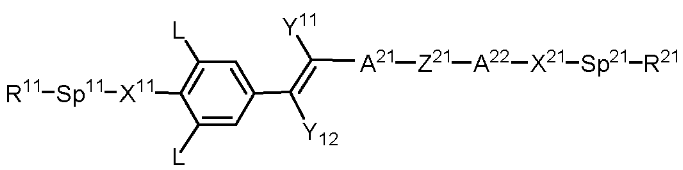
I-3c

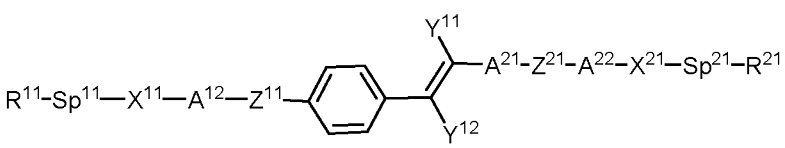
I-4a

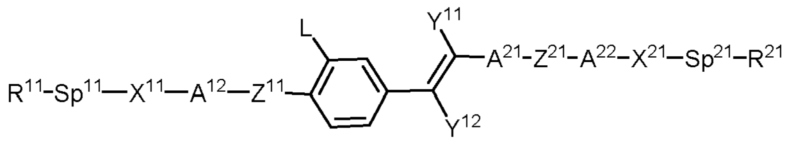

I-4b

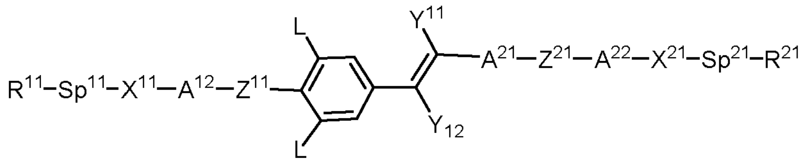

I-4c

wherein L, $R^{11}$, $R^{21}$, $A^{11}$, $X^{11}$, $X^{21}$, $Y^{11}$, $Y^{12}$, $Sp^{11}$, and $Sp^{21}$ have one of the meanings as given above in formula I, $A^{12}$ to $A^{23}$ have one of the meanings for A in formula I, and $Z^{11}$ to $Z^{22}$ have one of the meanings for Z as given above under formula I.

Further prefrerred are compounds of formula I-1a, wherein

$Sp^{21}$ denotes a spacer group comprising 1 to 20 C atoms, preferably 2 to 20 C atoms, wherein one or more non-adjacent and non-terminal $CH_2$ groups may also be replaced by -O-, -S-, -NH-, -N($CH_3$)-, -CO-, -O-CO-, -S-CO-, -O-COO-, -CO-S-, -CO-O-, -$CF_2$-, -$CF_2$O-, -O$CF_2$- -C(OH)-, -CH(alkyl)-, -CH(alkenyl)-, -CH(alkoxyl)-, -CH(oxaalkyl)-, -CH=CH- or -C≡C-, however in such a way that no two O-atoms are adjacent to one another and no two groups selected from -O-CO-, -S-CO-, -O-COO-, -CO-S-, -CO-O- and -CH=CH- are adjacent to each other, if $X^{21}$ denotes single bond and if at the same time $R^{21}$ denotes $P^{21}$ and Y denotes H.

Further prefrerred are compounds of formula I, wherein

$Sp^{21}$ denotes a spacer group comprising 2 to 20 C atoms, wherein one or more non-adjacent and non-terminal $CH_2$ groups may also be replaced by -O-, -S-, -NH-, -N($CH_3$)-, -CO-, -O-CO-, -S-CO-, -O-COO-, -CO-S-, -CO-O-, -$CF_2$-, -$CF_2$O-, -O$CF_2$- -C(OH)-, -CH(alkyl)-, -CH(alkenyl)-,-CH(alkoxyl)-, -CH(oxaalkyl)-, -CH=CH- or -C≡C-, however in such a way that no two O-atoms are adjacent to one another and no two groups selected from -O-CO-, -S-CO-, -O-COO-, -CO-S-, -CO-O- and -CH=CH- are adjacent to each other, if $X^{21}$ denotes -O- and if at the same time $R^{21}$ denotes $P^{21}$ and Y denotes H.

Further preferred compounds of formula I are selected from the following compounds:

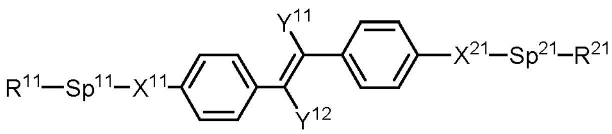

I-1a-1

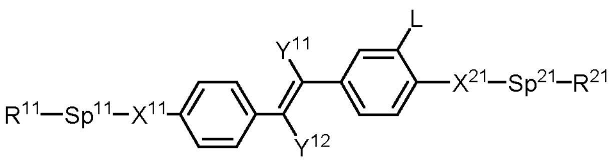

I-1a-2

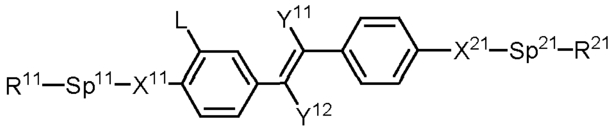

I-1b-1

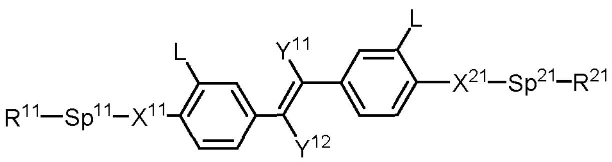

I-1b-2

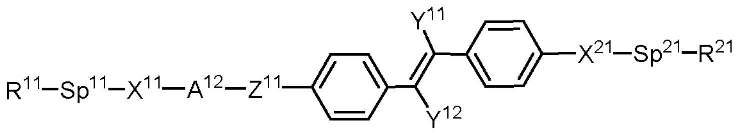

I-2a-1

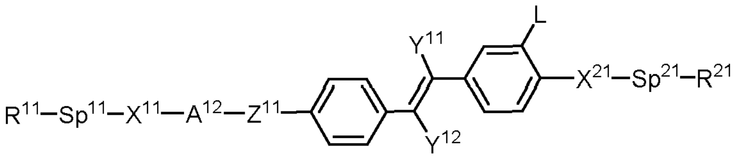

I-2a-2

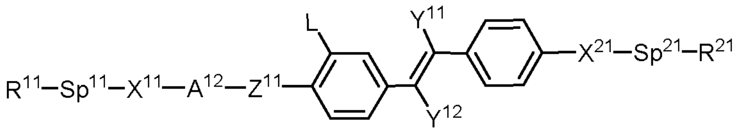

I-2b-1

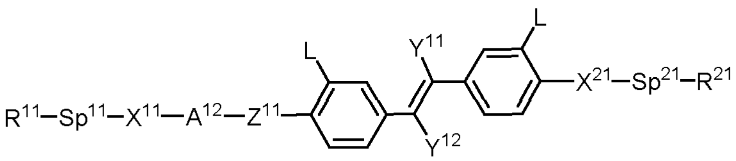

I-2b-2

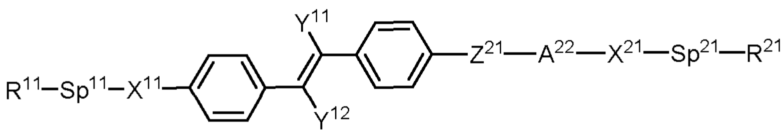

I-3a-1

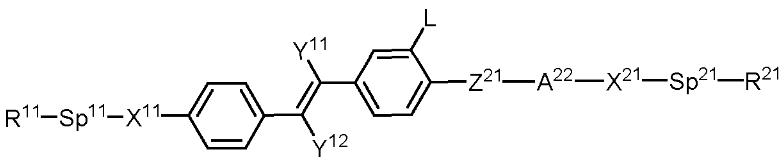

I-3a-2

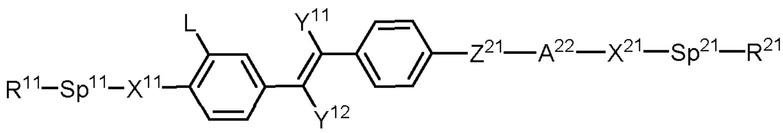

I-3b-1

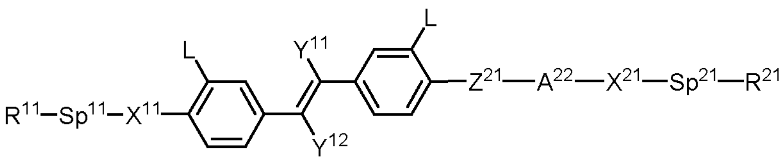

I-3b-2

wherein L, $R^{11}$, $R^{21}$, $A^{11}$, $Y^{11}$, $Y^{12}$, $X^{11}$, $X^{21}$, $Sp^{11}$, and $Sp^{21}$ have one of the meanings as given above in formula I, $A^{12}$ and $A^{22}$ have one of the meanings for A in formula I, and $Z^{11}$ and $Z^{22}$ have one of the meanings for Z as given above under formula I.

Further prefrerred are compounds of formula I-1 a-1, wherein

$Sp^{21}$ denotes a spacer group comprising 1 to 20 C atoms, preferably 2 to 20 C atoms, wherein one or more non-adjacent and non-terminal $CH_2$ groups may also be replaced by -O-, -S-, -NH-, -N($CH_3$)-, -CO-, -O-CO-, -S-CO-, -O-COO-, -CO-S-, -CO-O-, -$CF_2$-, -$CF_2$O-, -O$CF_2$- -C(OH)-, -CH(alkyl)-, -CH(alkenyl)-, -CH(alkoxyl)-, -CH(oxaalkyl)-, -CH=CH- or -C≡C-, however in such a way that no two O-atoms are adjacent to one another and no two groups selected from -O-CO-, -S-CO-, -O-COO-, -CO-S-, -CO-O- and -CH=CH- are adjacent to each other, if $X^{21}$ denotes single bond and if at the same time $R^{21}$ denotes $P^{21}$ and Y denotes H.

Further prefrerred are compounds of formula I, wherein

$Sp^{21}$ denotes a spacer group comprising 2 to 20 C atoms, wherein one or more non-adjacent and non-terminal $CH_2$ groups may also be replaced by -O-, -S-, -NH-, -N($CH_3$)-, -CO-, -O-CO-, -S-CO-, -O-COO-, -CO-S-, -CO-O-, -$CF_2$-, -$CF_2$O-, -O$CF_2$- -C(OH)-, -CH(alkyl)-, -CH(alkenyl)-,-CH(alkoxyl)-, -CH(oxaalkyl)-, -CH=CH- or -C≡C-, however in such a way that no two O-atoms are adjacent to one another and no two groups selected from -O-CO-, -S-CO-, -O-COO-, -CO-S-, -CO-O- and -CH=CH- are adjacent to each other, if $X^{21}$ denotes -O- and if at the same time $R^{21}$ denotes $P^{21}$ and Y denotes H.

Further preferred compounds of formula I-2a-1 to I-3b-2 are selected from the following compounds:

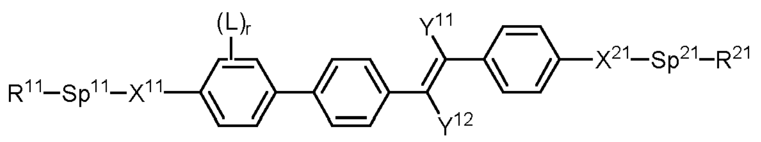

I-2a-1a

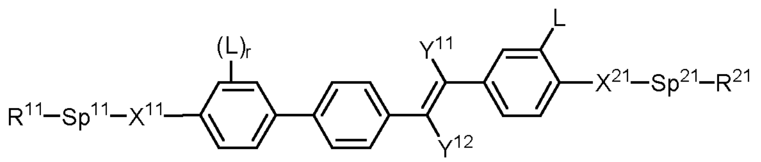

I-2a-2a

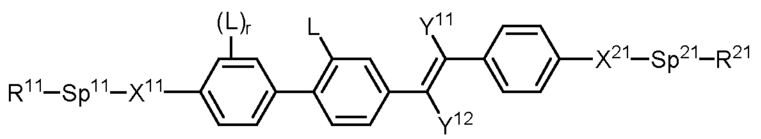

I-2b-1a

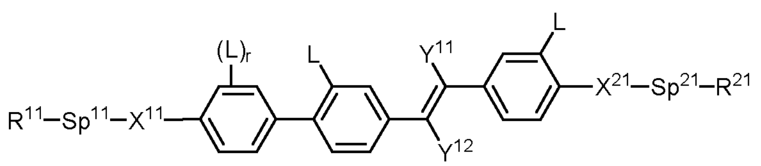

I-2b-2a

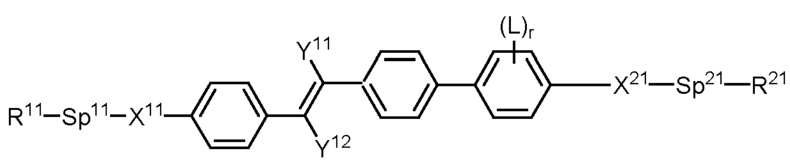

I-3a-1a

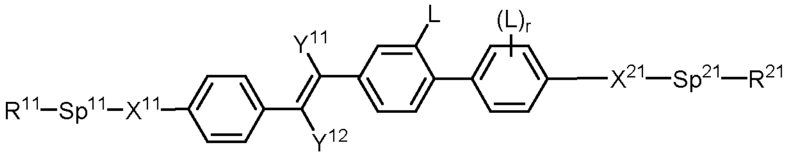

I-3a-2a

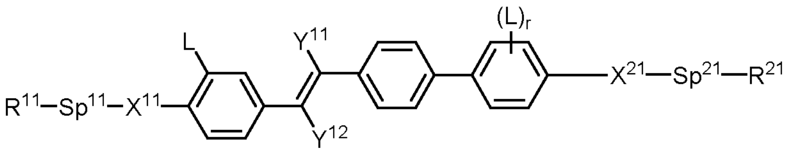

I-3b-1a

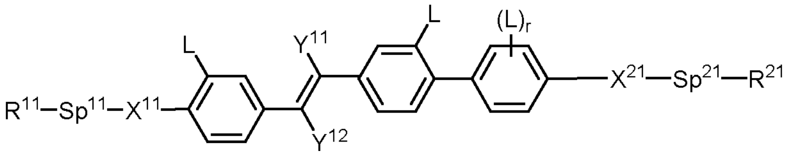

I-3b-2a

wherein L, $R^{11}$, $R^{21}$, $Y^{11}$, $Y^{12}$, $X^{11}$, $X^{21}$, $Sp^{11}$, and $Sp^{12}$ have one of the meanings as given above in formula I, and

the group

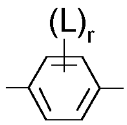

is each and independently

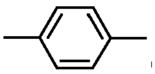 ,

or

denotes

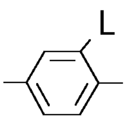 , 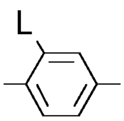 or 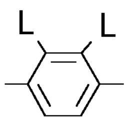 ,

furthermore

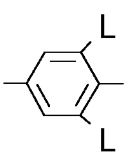 ,

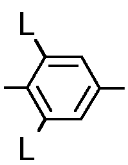 , 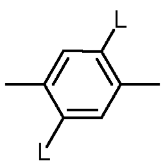 , or 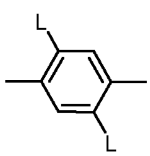 ,

wherein L have one of the meanings as given above in formula I, and preferably denotes F, Cl, $OCH_3$, $COCH_3$ or

alkyl having 1 to 6 C Atoms, such as methyl, ethyl, propyl, butyl, pentyl, hexyl, cyclopropyl, cylobutyl, cyclopentyl, cyclohexyl, or $X^{21}$-$Sp^{21}$-$R^{21}$.

Further preferred compound of formula I and subformulae thereof are selected from compounds wherein the groups -$X^{11}$-$Sp^{11}$-$R^{11}$ and $X^{21}$-$Sp^{21}$-$R^{21}$ are selected from different groups, such as

- $X^{11}$-$Sp^{11}$-$P^{11}$ and -$X^{21}$-$Sp^{21}$-$P^{21}$,
- $X^{11}$-$Sp^{11}$-$P^{21}$ and -$X^{21}$-$Sp^{21}$-$P^{11}$,
- $P^{11}$ and -$X^{21}$-$Sp^{21}$-$P^{21}$, or
- $X^{11}$-$Sp^{11}$-$P^{21}$ and -$P^{11}$.

However it is likewise preferred that the groups -$X^{11}$-$Sp^{11}$-$R^{11}$ and $X^{21}$-$Sp^{21}$-$R^{21}$ are identical, such as -$X^{11}$-$Sp^{11}$-$P^{21}$ and -$X^{21}$-$Sp^{21}$-$P^{21}$.

Especially preferred compounds of formula I are selected from compounds of the following sub-formula:

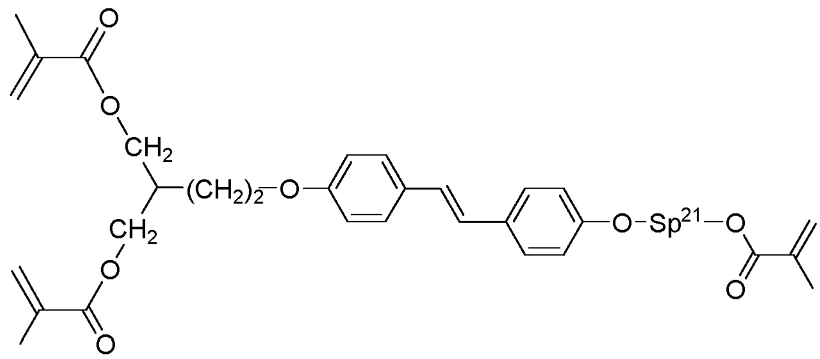

I-1a-1-1

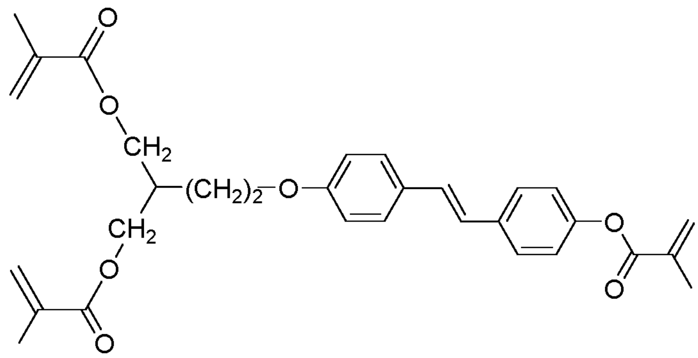

I-1a-1-2

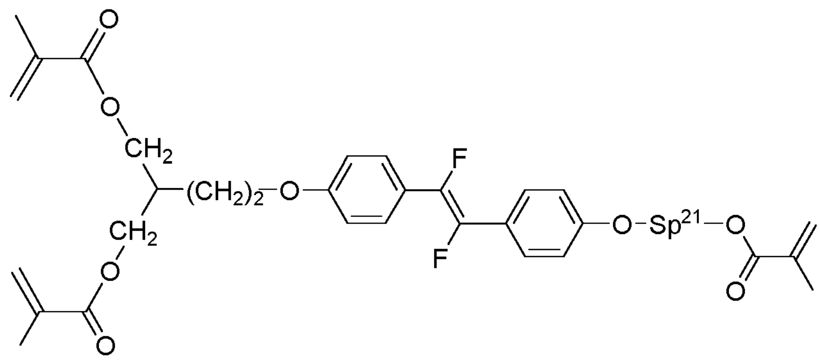

I-1a-1-3

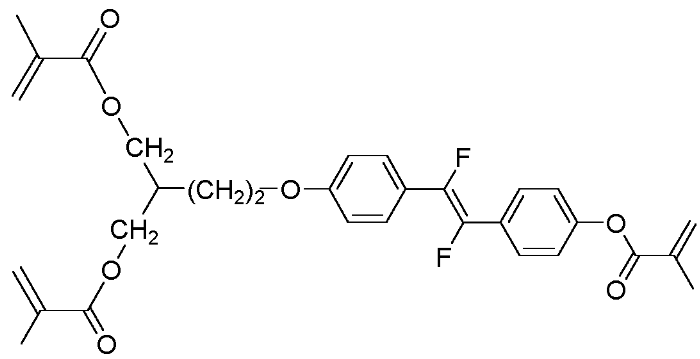

I-1a-1-4

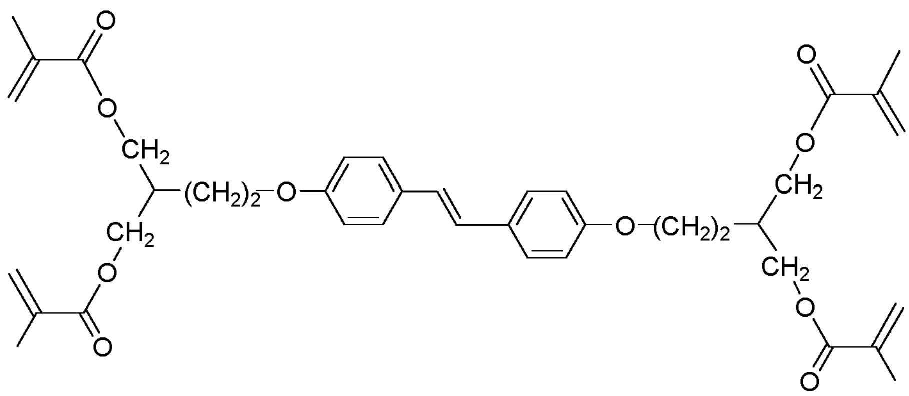

I-1a-1-5

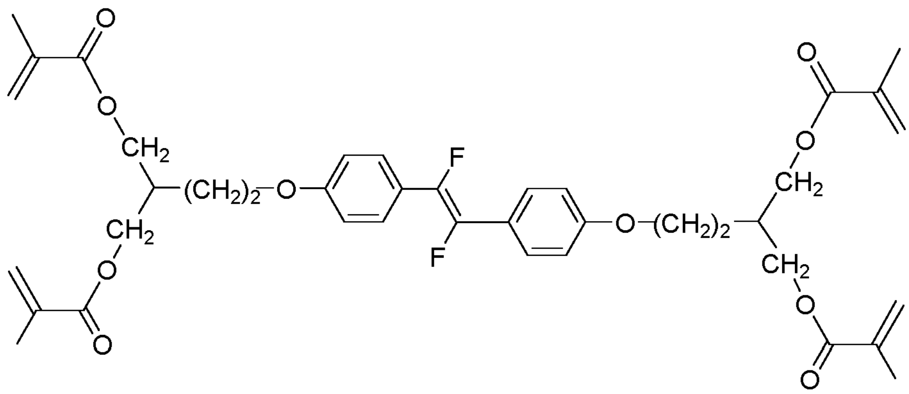

I-1a-1-6

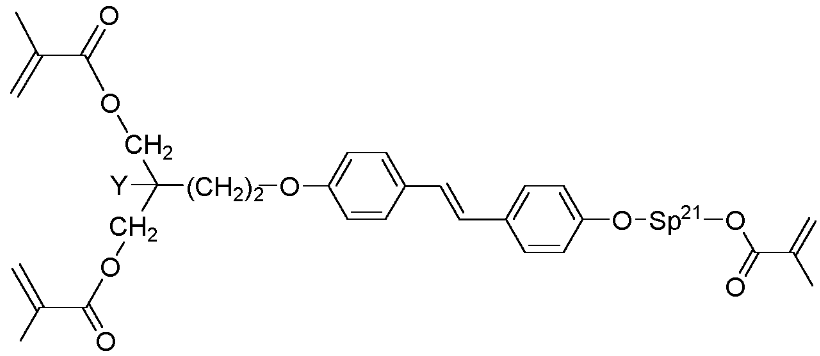

I-1a-1-7

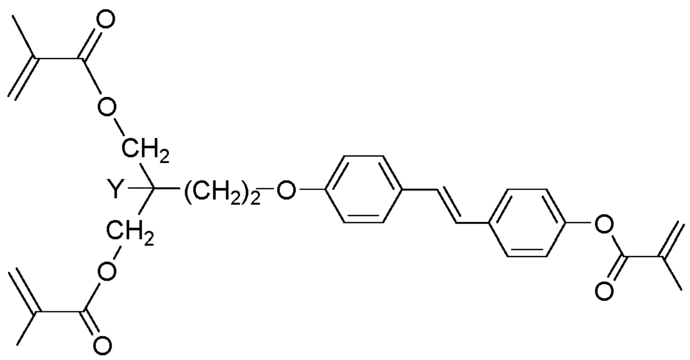

I-1a-1-8

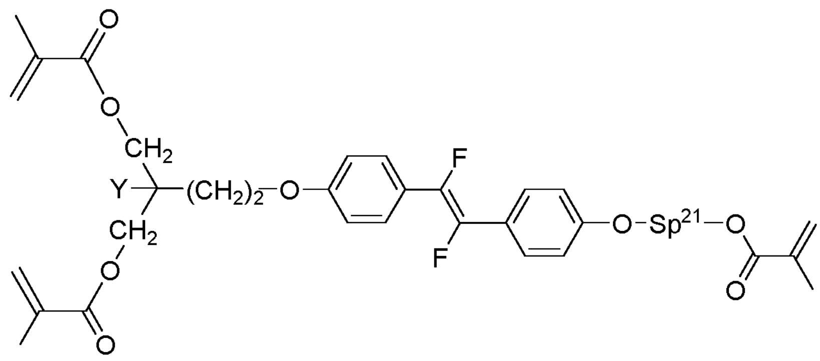

I-1a-1-9

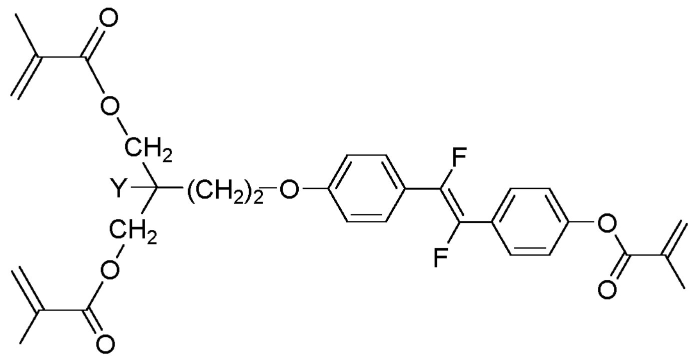

I-1a-1-10

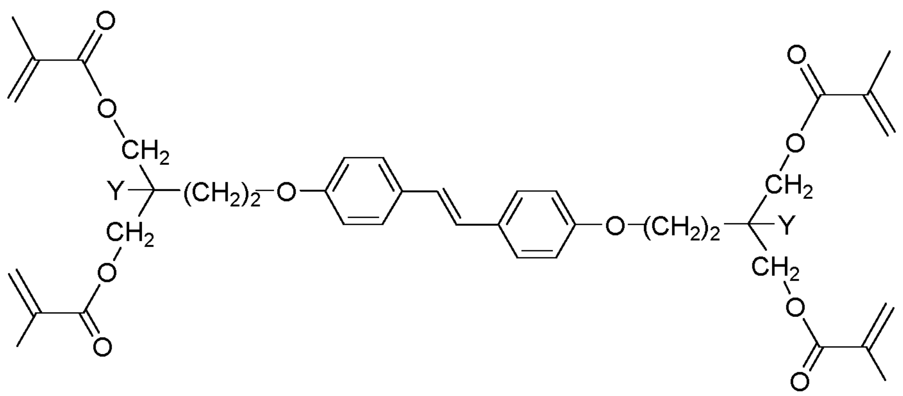

I-1a-1-11

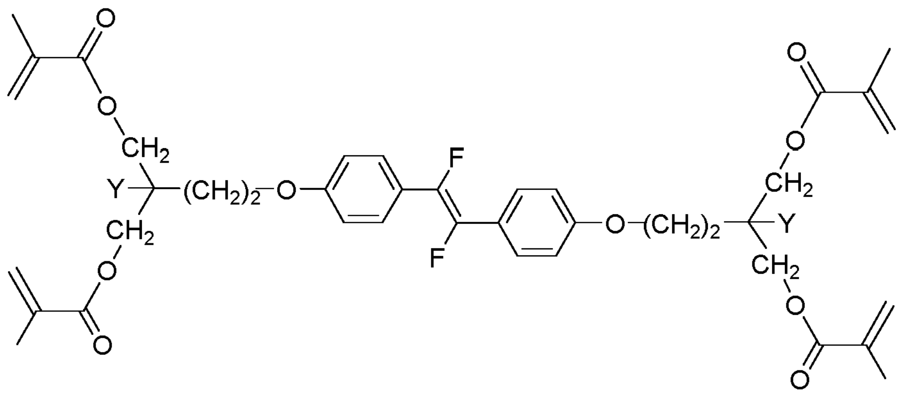

I-1a-1-12

wherein $Sp^{11}$ and $Sp^{21}$ have one of the meanings as given above in formula I, and Y denotes methyl or ethyl.

In particular preferred compounds of formula I are selected from compounds of the formulae I-1a-1-3, I-1a-1-4, I-1a-1-6, I-1a-1-9, I-1a-1-10, or I-1a-1-12.

Preferred intermediate compounds (**6**) from which the compounds of formula I are preferably synthesised, are obtainable or obtained according to or in analogy to the procedure described in the following scheme:

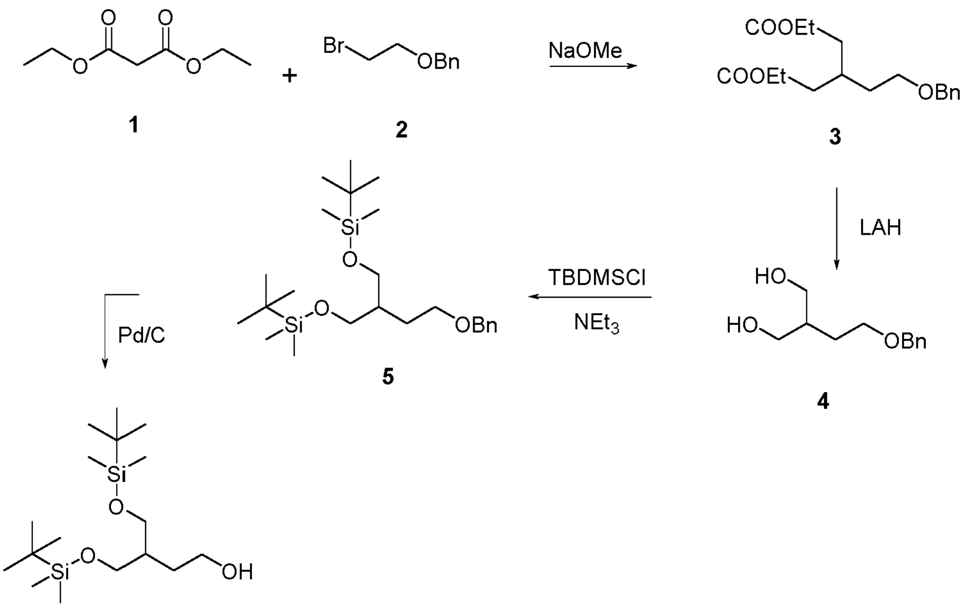


The compounds of formula I are prepared by methods known per se, as described in the literature (for example in the standard works, such as Houben-Weyl, Methoden der organischen Chemie [Methods of Organic Chemistry], Georg-Thieme-Verlag, Stuttgart), to be precise under reaction conditions which are known and suitable for the said reactions. Use can also be made here of variants known per se, which are not mentioned here in greater detail.

Preferably the compounds of formula I are synthesised, are obtainable or obtained according to or in analogy to the procedure described in the following scheme:

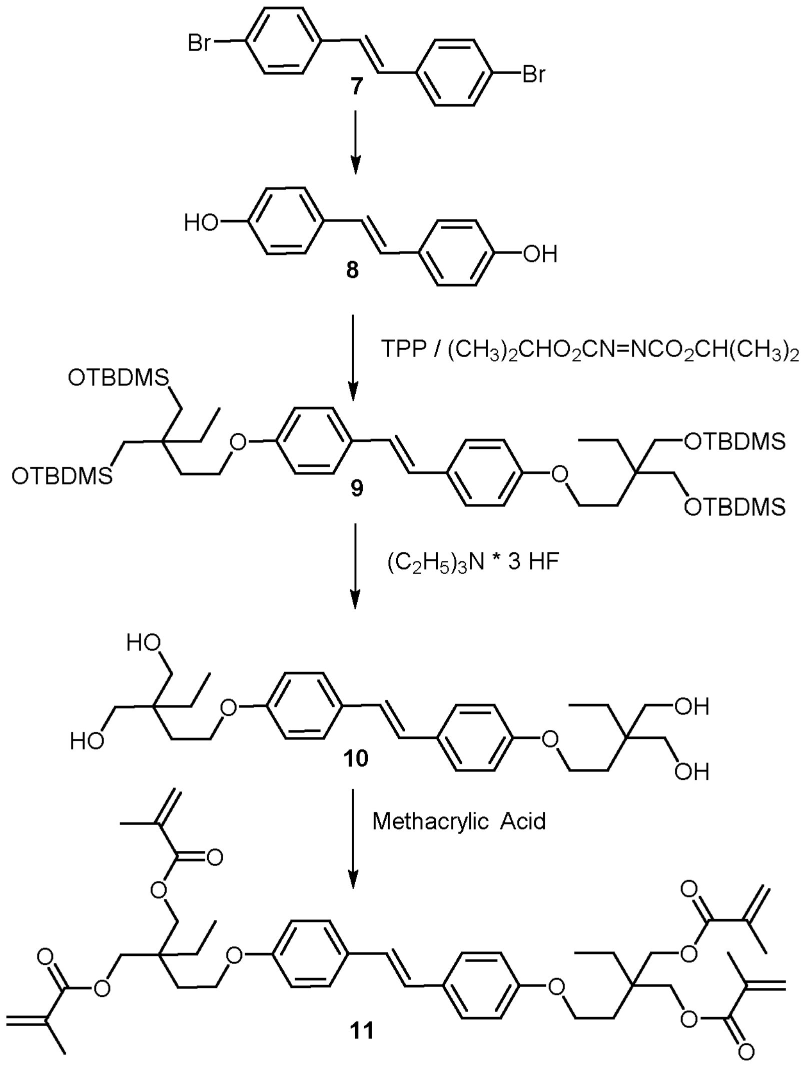


In another preferred embodiment, the compounds of formula I are synthesised, are obtainable or obtained according to or in analogy to the procedure described in the following scheme:

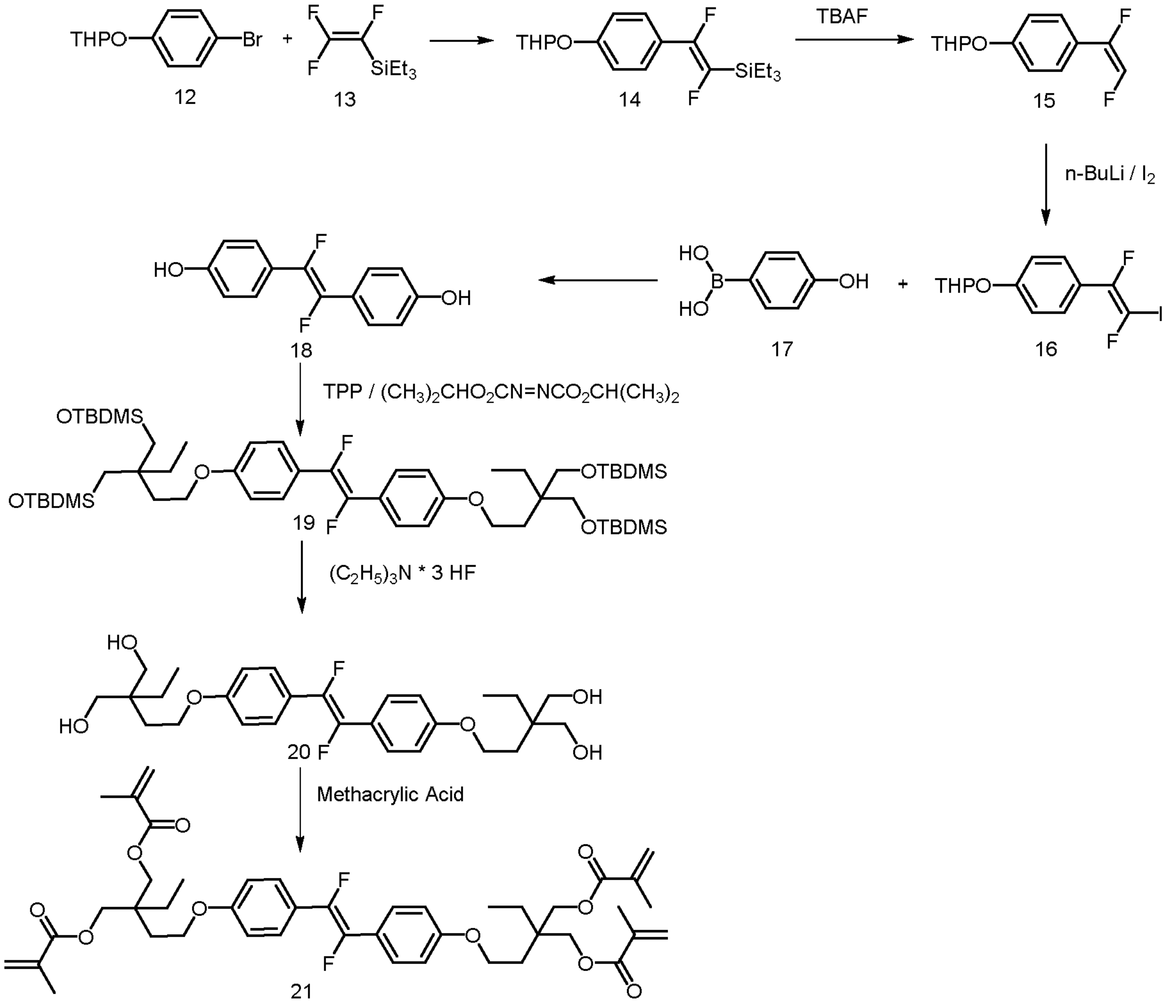


12 13 14 15 16 17 18 19 20 21

The compounds of formula I and subformulae thereof can be preferably utilized in a mixture comprising one or more mesogenic or liquid-crystalline compounds.

Therefore, the present invention relates to the use compounds of formula I and subformulae thereof in a liquid crystal mixture.

Further the present invention relates to liquid crystal mixtures comprising a photoalignment component A) comprising one or more photoreactive mesogens of formula I, and a liquid-crystalline component B), hereinafter also referred to as "LC host mixture", comprising one or more mesogenic or liquid-crystalline compounds.

The media according to the invention preferably comprise from 0.01 to 10%, particularly preferably from 0.05 to 5% and most preferably from 0.1 to 3% of component A) comprising compounds of formula I according to the invention.

The media preferably comprise one, two or three, more preferably one or two and most preferably one compound of the formula I according to the invention.

In a preferred embodiment component A) consists of compounds of formula I.

In a preferred embodiment, the LC-host mixture (component B) according to the present invention comprises one or more, preferably two or more, low-molecular-weight (i.e. monomeric or unpolymerized) compounds. The latter are stable or unreactive with respect to a polymerisation reaction or photoalignment under the conditions used for the polymerisation of the polymerizable compounds or photoalignment of the photoreactive mesogen of formula I.

In principle, a suitable host mixture is any dielectrically negative or positive LC mixture which is suitable for use in conventional VA, IPS or FFS displays.

Suitable LC mixtures are known to the person skilled in the art and are described in the literature. LC media for VA displays having negative dielectric anisotropy are described in for example EP 1 378 557 A1.

Suitable LC mixtures having positive dielectric anisotropy which are suitable for LCDs and especially for IPS displays are known, for example, from JP 07-181 439 (A), EP 0 667 555, EP 0 673 986, DE 195 09 410, DE 195 28 106, DE 195 28 107, WO 96/23 851, WO 96/28 521 and WO2012/079676.

Preferred embodiments of the liquid-crystalline medium having negative or positive dielectric anisotropy according to the invention are indicated below and explained in more detail by means of the working examples.

The LC host mixture is preferably a nematic LC mixture, and preferably does not have a chiral LC phase.

In a preferred embodiment of the present invention the LC medium contains an LC host mixture with negative dielectric anisotropy. Preferred embodiments of such an LC medium, and the corresponding LC host mixture, are those of sections a)-z) below:

a) LC medium which comprises one or more compounds of the formulae CY and/or PY:

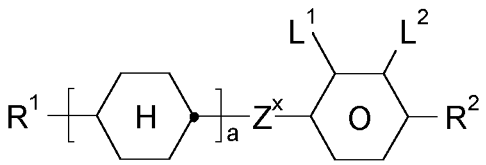

CY

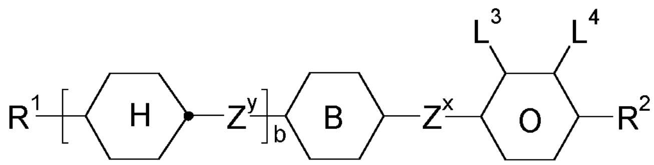

PY

wherein

a denotes 1 or 2,

b denotes 0 or 1,

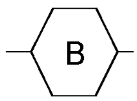

denotes

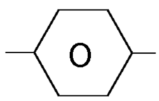

or

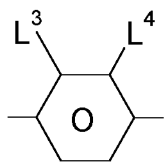

$R^1$ and $R^2$ each, independently of one another, denote alkyl having 1 to 12 C atoms, where, in addition, one or two non-adjacent $CH_2$ groups may be replaced by -O-, -CH=CH-, -CO-, -OCO- or -COO- in such a way that O atoms are not linked directly to one another, preferably alkyl or alkoxy having 1 to 6 C atoms,

$Z^x$ and $Z^y$ each, independently of one another, denote $-CH_2CH_2-$, -CH=CH-, $-CF_2O-$, $-OCF_2-$, $-CH_2O-$, $-O CH_2-$, -CO-O-, -O-CO-, $-C_2F_4-$, -CF=CF-, $-CH=CH-CH_2O-$ or a single bond, preferably a single bond,

$L^{1,4}$ each, independently of one another, denote F, Cl, $OCF_3$, $CF_3$, $CH_3$, $CH_2F$, $CHF_2$.

Preferably, both $L^1$ and $L^2$ denote F or one of $L^1$ and $L^2$ denotes F and the other denotes C!, or both $L^3$ and $L^4$ denote F or one of $L^3$ and $L^4$ denotes F and the other denotes Cl.

The compounds of the formula CY are preferably selected from the group consisting of the following sub-formulae:

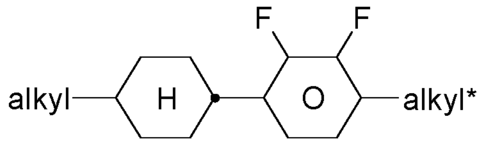

CY1

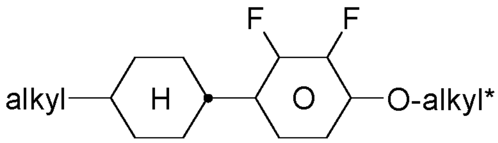

CY2

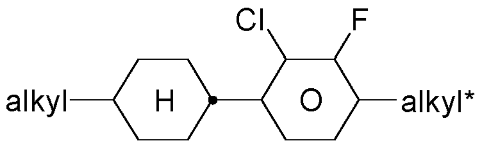

CY3

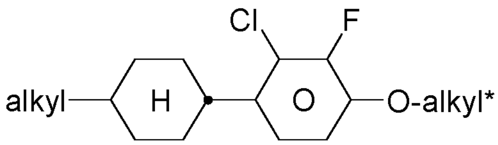

CY4

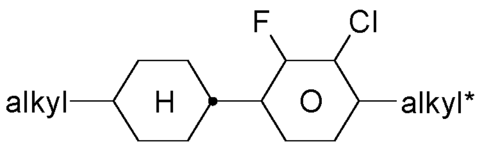

CY5

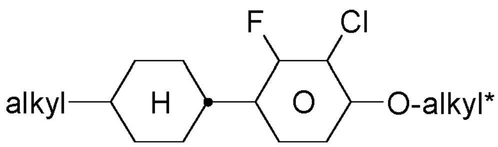

CY6

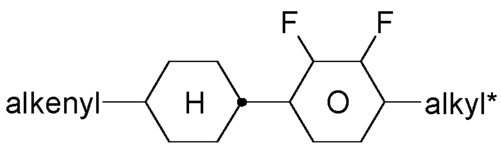

CY7

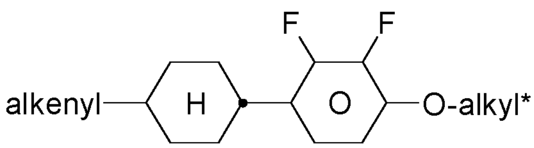

CY8

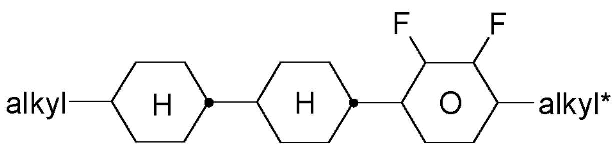

CY9

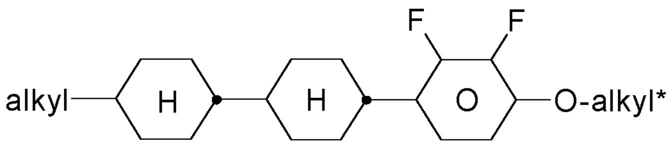

CY10

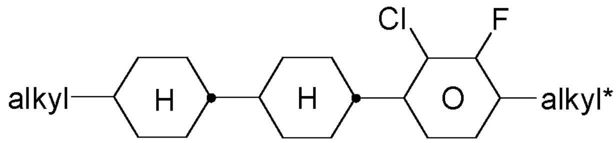

CY11

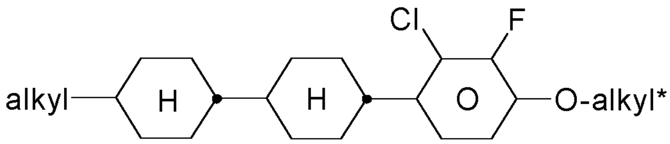

CY12

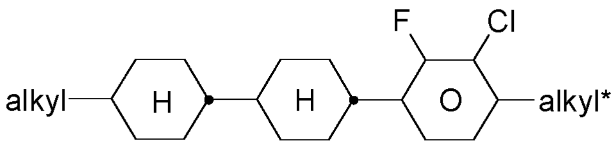

CY13

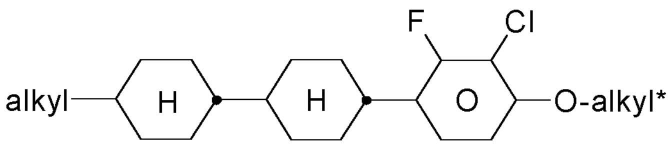

CY14

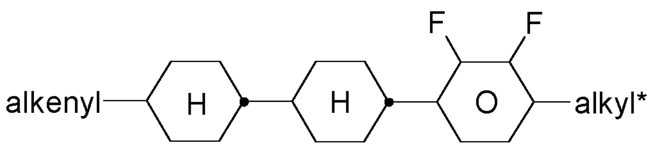

CY15

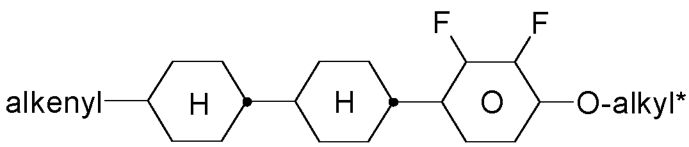

CY16

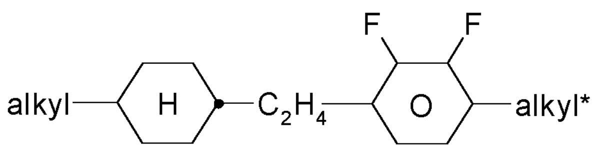

CY17

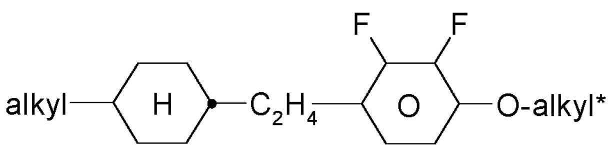

CY18

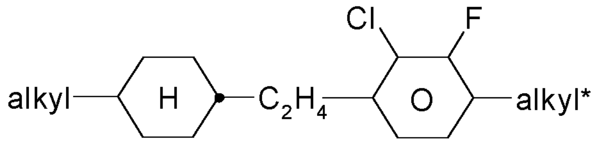

CY19

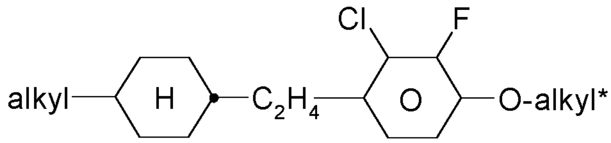

CY20

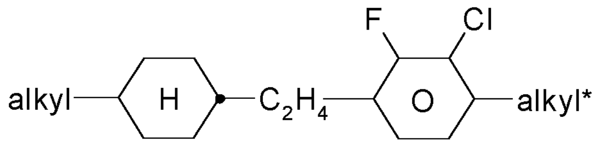

CY21

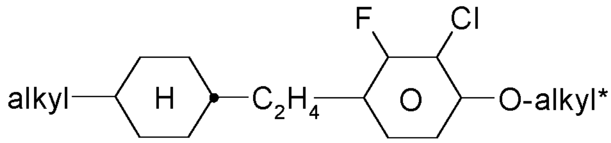

CY22

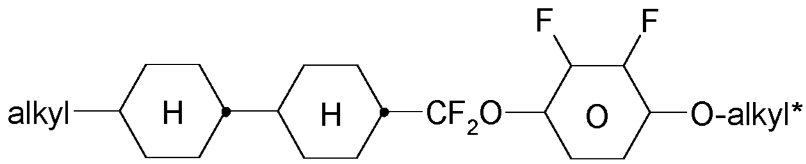

CY23

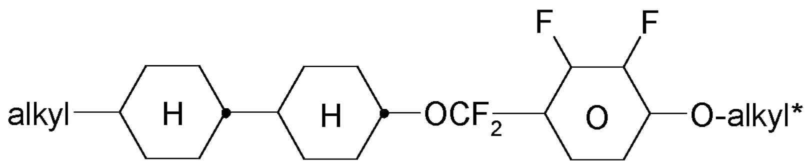

CY24

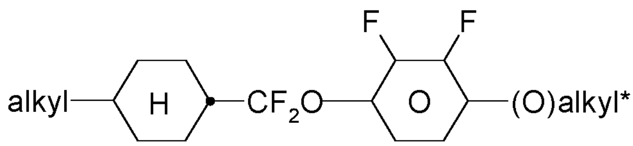

CY25

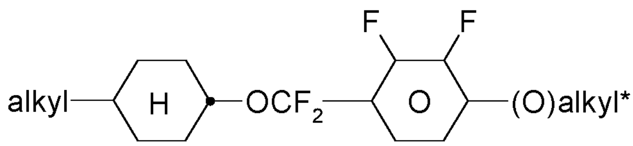

CY26

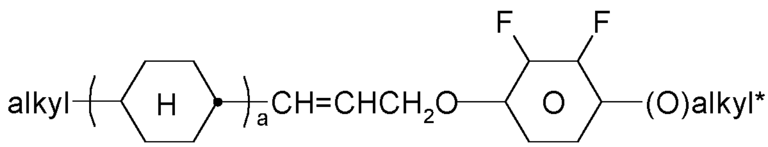

CY27

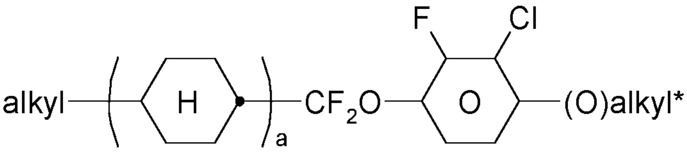

CY28

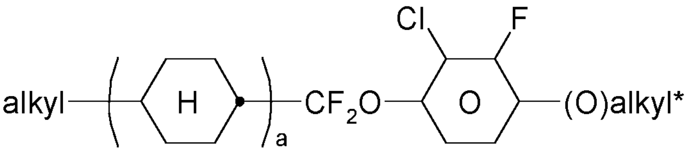

CY29

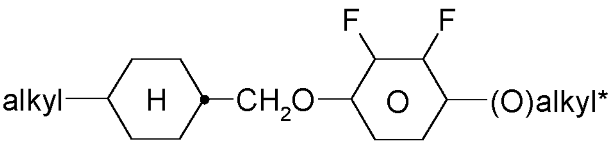

CY30

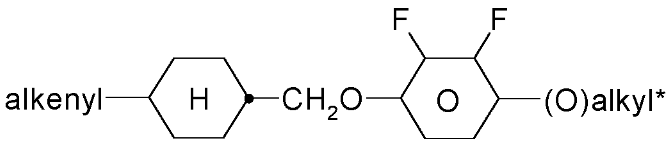

CY31

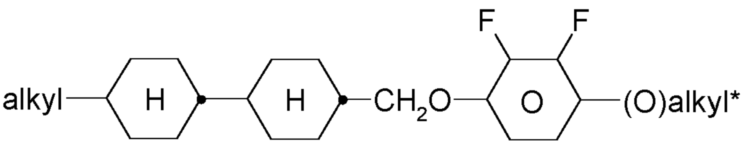

CY32

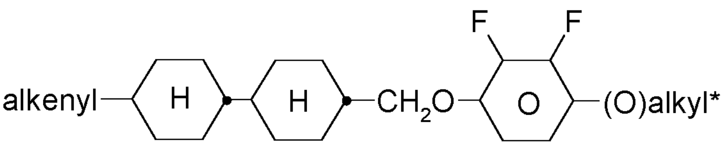

CY33

wherein a denotes 1 or 2, alkyl and alkyl* each, independently of one another, denote a straight-chain alkyl radical having 1-6 C atoms, and alkenyl denotes a straight-chain alkenyl radical having 2-6 C atoms, and (O) denotes an oxygen atom or a single bond. Alkenyl preferably denotes $CH_2$=CH-, $CH_2$=$CHCH_2CH_2$-, $CH_3$-CH=CH-, $CH_3$-$CH_2$-CH=CH-, $CH_3$-$(CH_2)_2$-CH=CH-, $CH_3$-$(CH_2)_3$-CH=CH- or $CH_3$-CH=CH-$(CH_2)_2$-.

The compounds of the formula PY are preferably selected from the group consisting of the following sub-formulae:

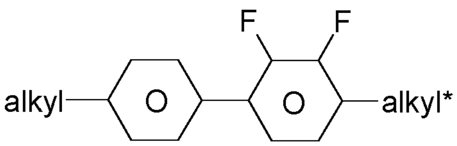

PY1

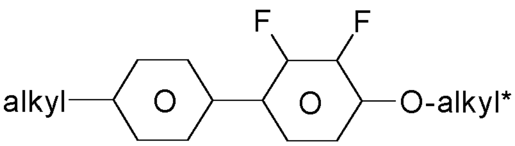

PY2

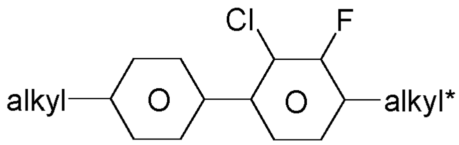

PY3

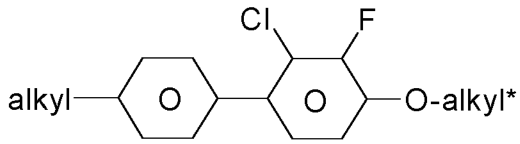

PY4

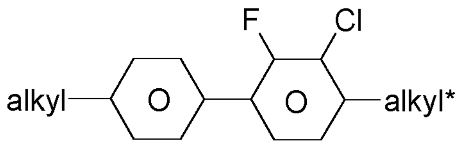

PY5

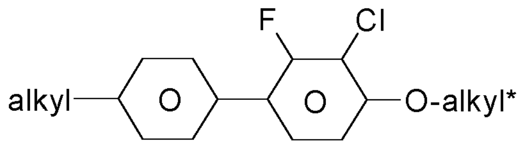

PY6

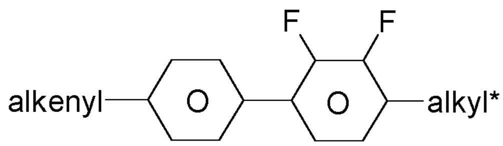

PY7

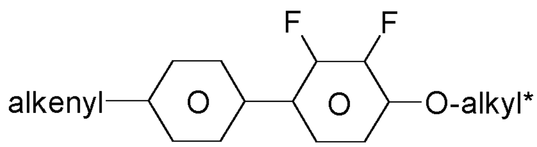

PY8

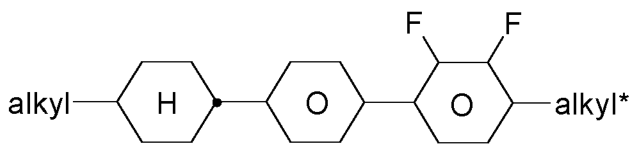

PY9

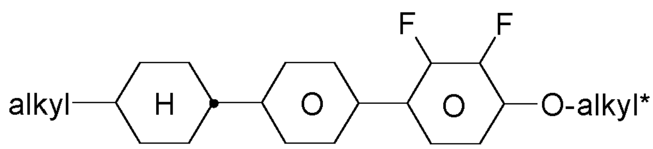

PY10

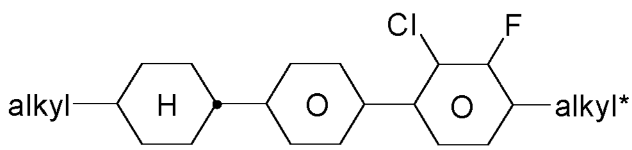

PY11

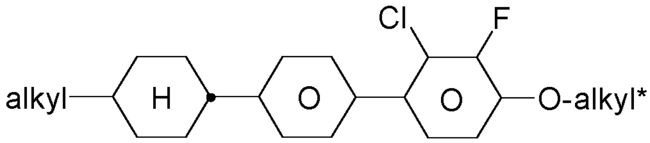
PY12

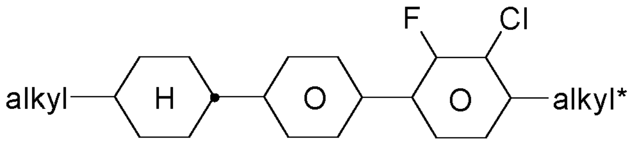
PY13

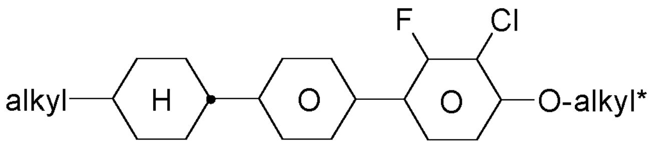
PY14

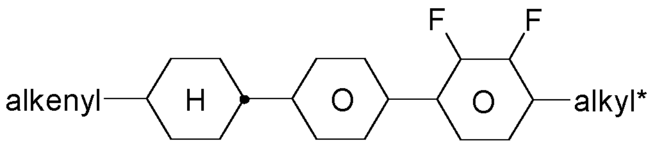
PY15

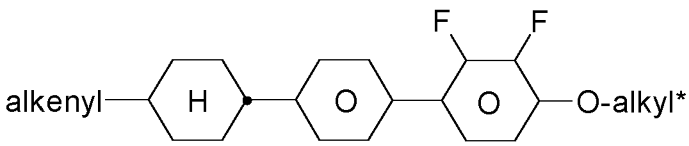
PY16

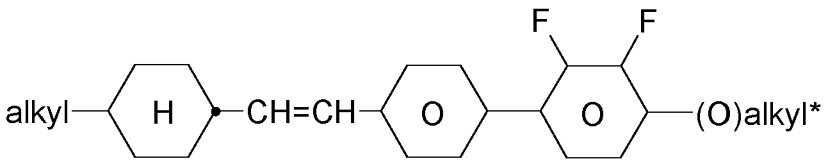
PY17

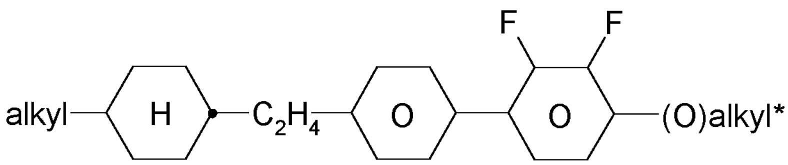
PY18

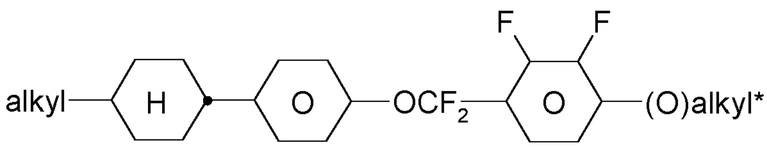
PY19

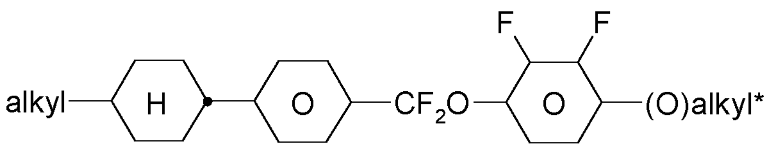
PY20

wherein alkyl and alkyl* each, independently of one another, denote a straight-chain alkyl radical having 1-6 C atoms, and alkenyl denotes a straight-chain alkenyl radical having 2-6 C atoms, and (O) denotes an oxygen atom or a single

bond. Alkenyl preferably denotes $CH_2$=CH-, $CH_2$=$CHCH_2CH_2$-, $CH_3$-CH=CH-, $CH_3$-$CH_2$-CH=CH-, $CH_3$-$(CH_2)_2$-CH=CH-, $CH_3$-$(CH_2)_3$-CH=CH- or $CH_3$-CH=CH-$(CH_2)_2$-.

b) LC medium which additionally comprises one or more compounds of the following formula:

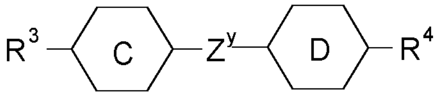

ZK

in which the individual radicals have the following meanings:

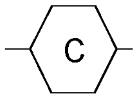

denotes

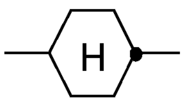

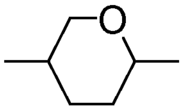

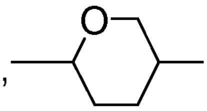

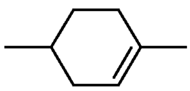

or

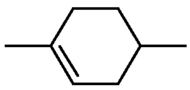

,

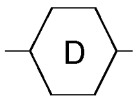

denotes

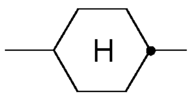

or

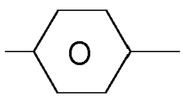

,

$R^3$ and $R^4$ each, independently of one another, denote alkyl having 1 to 12 C atoms, in which, in addition, one or two non-adjacent $CH_2$ groups may be replaced by -O-, -CH=CH-, -CO-, -O-CO- or -CO-O- in such a way that O atoms are not linked directly to one another,

$Z^y$ denotes -$CH_2CH_2$-, -CH=CH-, -$CF_2O$-, -$OCF_2$-, -$CH_2O$-, -$OCH_2$-, -CO-O-, -O-CO-, -$C_2F_4$-, -CF=CF-, -CH=CH-$CH_2O$- or a single bond, preferably a single bond.

The compounds of the formula ZK are preferably selected from the group consisting of the following sub-formulae:

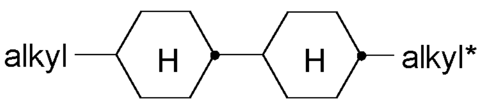

ZK1

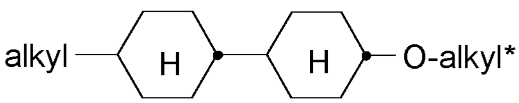

ZK2

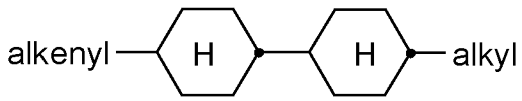

ZK3

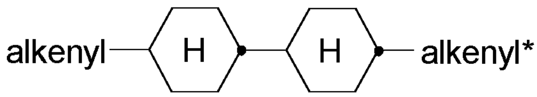

ZK4

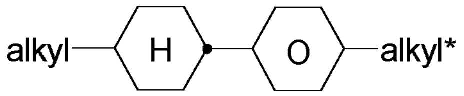

ZK5

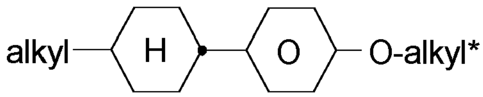

ZK6

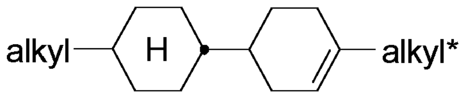

ZK7

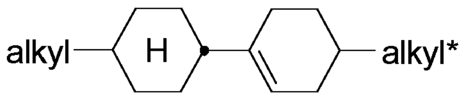

ZK8

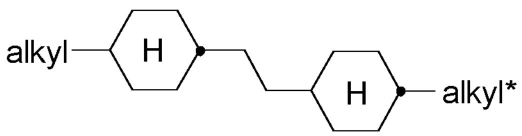

ZK9

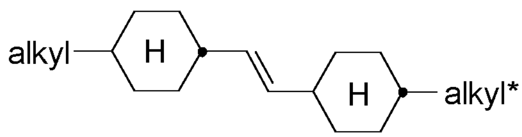

ZK10

in which alkyl and alkyl* each, independently of one another, denote a straight-chain alkyl radical having 1-6 C atoms, and alkenyl denotes a straight-chain alkenyl radical having 2-6 C atoms. Alkenyl preferably denotes $CH_2$=CH-, $CH_2$=$CHCH_2CH_2$-, $CH_3$-CH=CH-, $CH_3$-$CH_2$-CH=CH-, $CH_3$-$(CH_2)_2$-CH=CH-, $CH_3$-$(CH_2)_3$-CH=CH- or $CH_3$-CH=CH-$(CH_2)_2$-.

Especially preferred are compounds of formula ZK1 and ZK3.

Particularly preferred compounds of formula ZK are selected from the following sub-formulae:

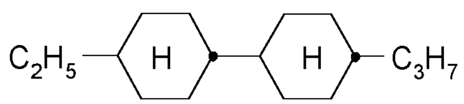

ZK1a

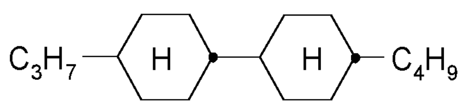

ZK1b

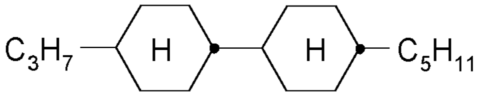 ZK1c

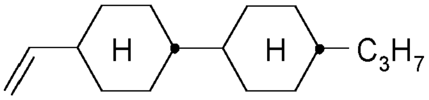 ZK3a

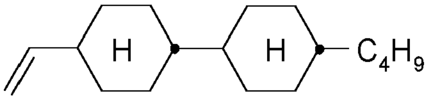 ZK3b

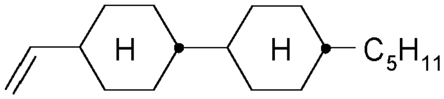 ZK3c

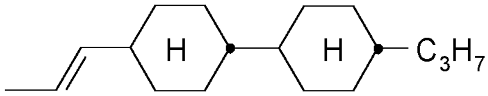 ZK3d

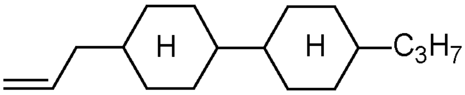 ZK3e

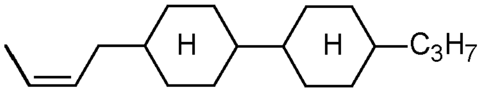 ZK3f

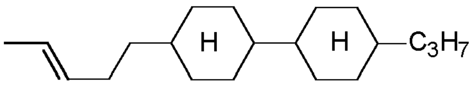 ZK3g

wherein the propyl, butyl and pentyl groups are straight-chain groups.

Most preferred are compounds of formula ZK1a and ZK3a.

c) LC medium which additionally comprises one or more compounds of the following formula:

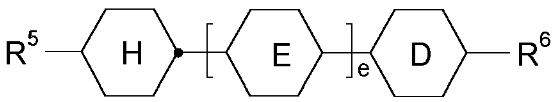 DK

in which the individual radicals on each occurrence, identically or differently, have the following meanings:

$R^5$ and $R^6$ each, independently of one another, denote alkyl having 1 to 12 C atoms, where, in addition, one or two non-adjacent $CH_2$ groups may be replaced by -O-, -CH=CH-, -CO-, -OCO- or -COO- in such a way that O atoms are not linked directly to one another, preferably alkyl or alkoxy having 1 to 6 C atoms,

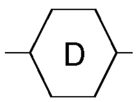

denotes

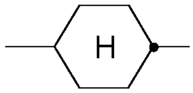 or 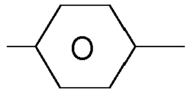 ,

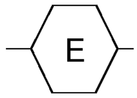

denotes

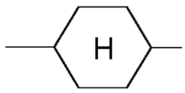 , 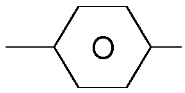 or 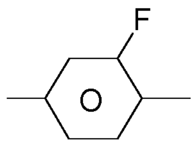 ,

and

e denotes 1 or 2.

The compounds of the formula DK are preferably selected from the group consisting of the following sub-formulae:

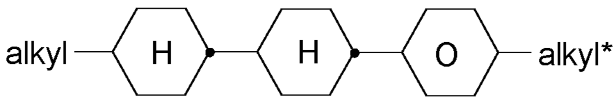 DK1

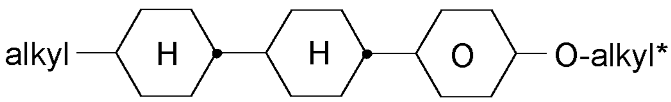 DK2

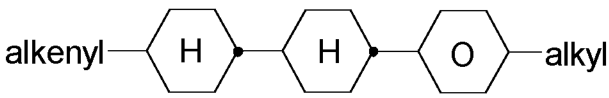 DK3

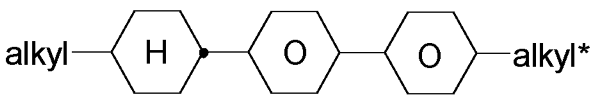 DK4

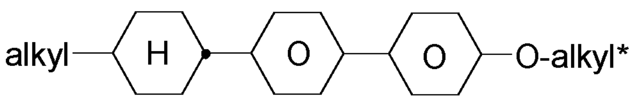 DK5

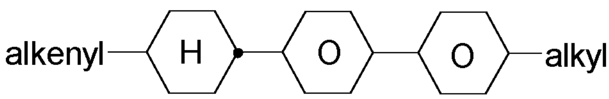 DK6

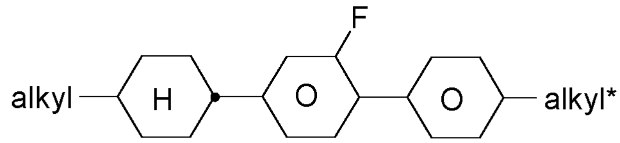

DK7

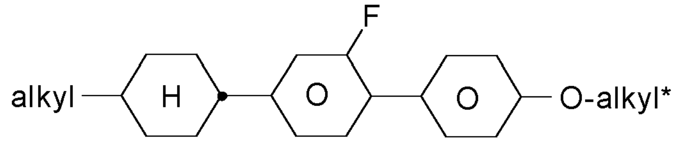

DK8

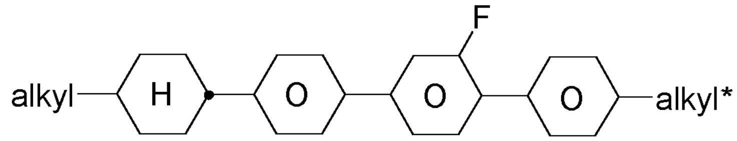

DK9

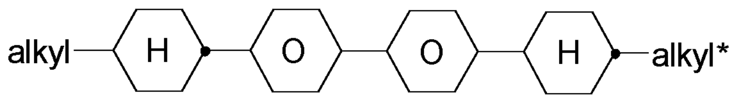

DK10

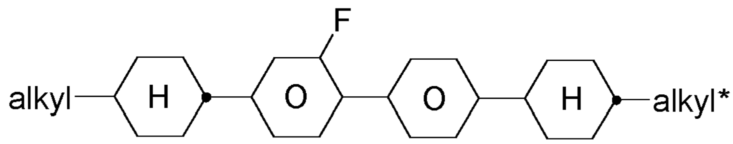

DK11

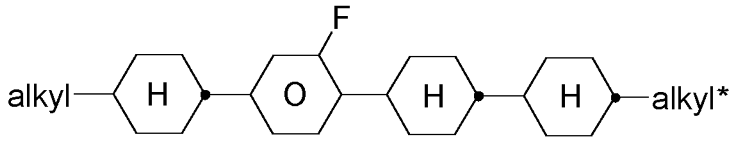

DK12

in which alkyl and alkyl* each, independently of one another, denote a straight-chain alkyl radical having 1-6 C atoms, and alkenyl denotes a straight-chain alkenyl radical having 2-6 C atoms. Alkenyl preferably denotes $CH_2=CH-$, $CH_2=CHCH_2CH_2-$, $CH_3-CH=CH-$, $CH_3-CH_2-CH=CH-$, $CH_3-(CH_2)_2-CH=CH-$, $CH_3-(CH_2)_3-CH=CH-$ or $CH_3-CH=CH-(CH_2)_2-$.

d) LC medium which additionally comprises one or more compounds of the following formula:

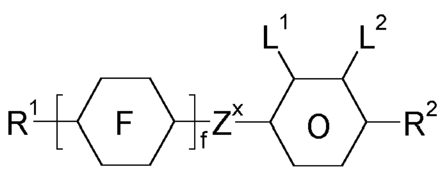

LY

in which the individual radicals have the following meanings:

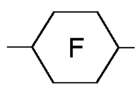

denotes

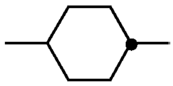, 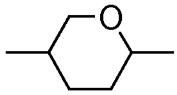, 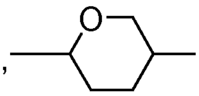,

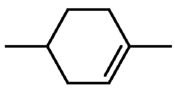 or 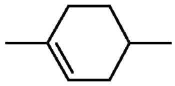

with at least one ring F being different from cyclohexylene,

f denotes 1 or 2,

$R^1$ and $R^2$ each, independently of one another, denote alkyl having 1 to 12 C atoms, where, in addition, one or two non-adjacent $CH_2$ groups may be replaced by -O-, -CH=CH-, -CO-, -OCO- or -COO- in such a way that O atoms are not linked directly to one another,

$Z^x$ denotes $-CH_2CH_2-$, -CH=CH-, $-CF_2O-$, $-OCF_2-$, $-CH_2O-$, -O $CH_2-$, -CO-O-, -O-CO-, $-C_2F_4-$, -CF=CF-, $-CH=CH-CH_2O-$ or a single bond, preferably a single bond,

$L^1$ and $L^2$ each, independently of one another, denote F, Cl, $OCF_3$, $CF_3$, $CH_3$, $CH_2F$, $CHF_2$.

Preferably, both radicals $L^1$ and $L^2$ denote F or one of the radicals $L^1$ and $L^2$ denotes F and the other denotes Cl.

The compounds of the formula LY are preferably selected from the group consisting of the following sub-formulae:

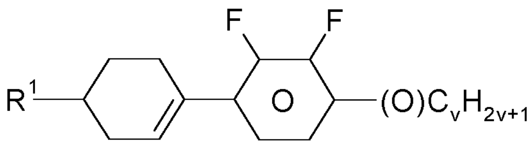

LY1

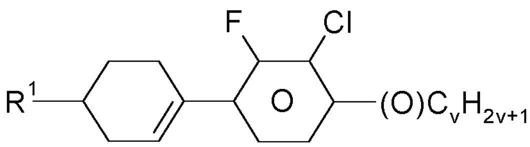

LY2

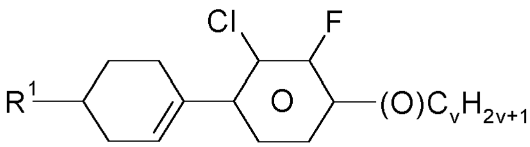

LY3

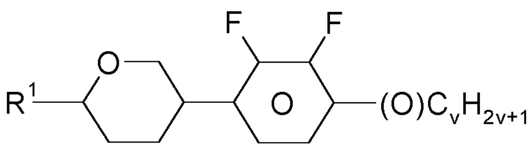

LY4

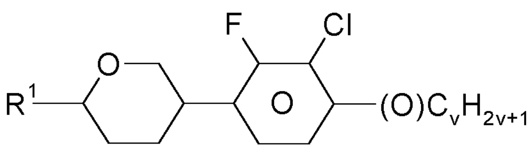

LY5

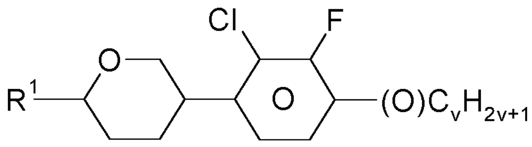

LY6

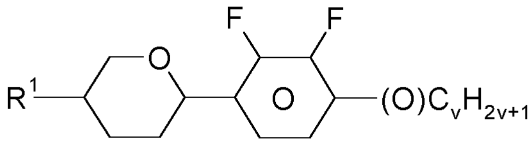

LY7

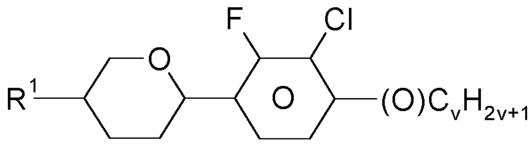

LY8

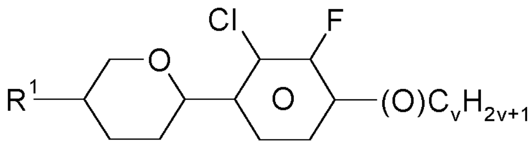

LY9

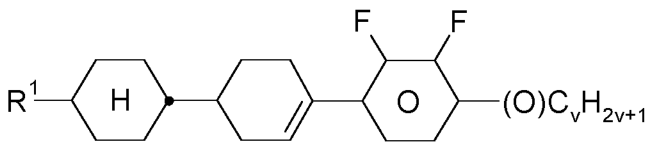

LY10

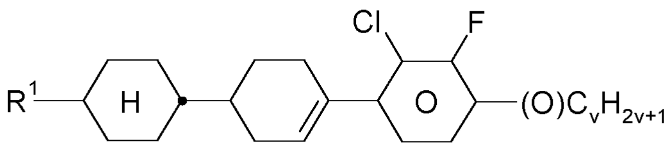

LY11

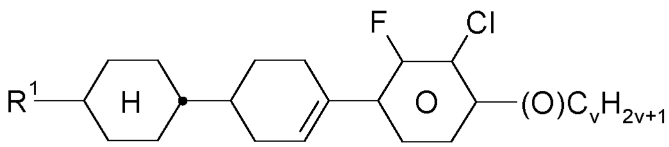

LY12

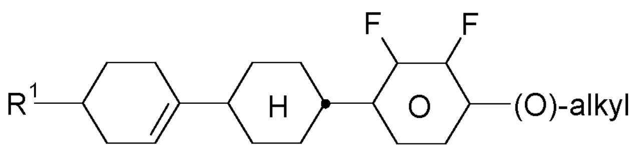

LY13

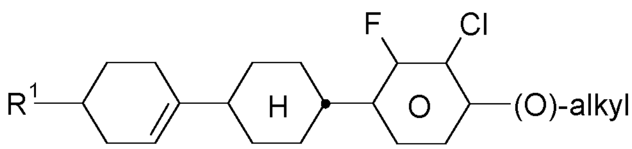

LY14

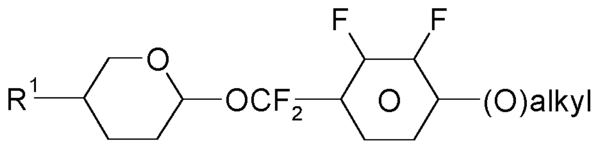

LY15

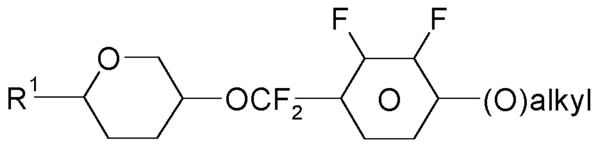

LY16

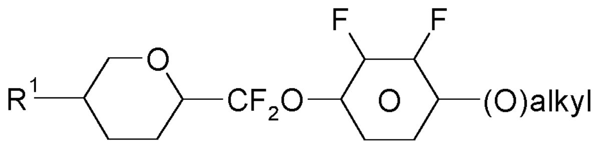

LY17

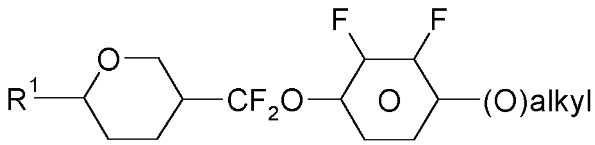

LY18

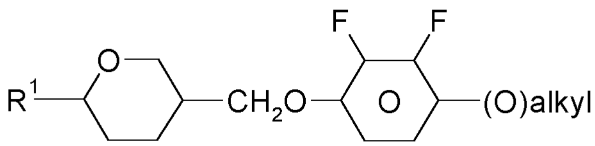

LY19

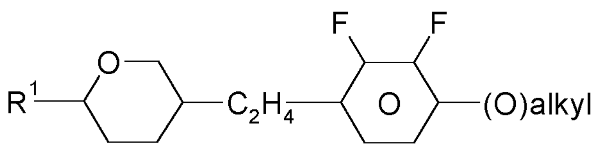

LY20

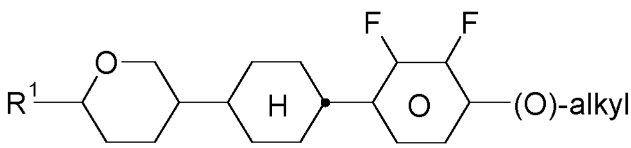

LY21

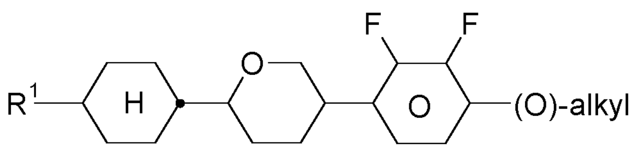

LY22

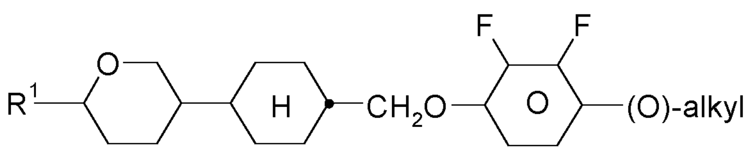

LY23

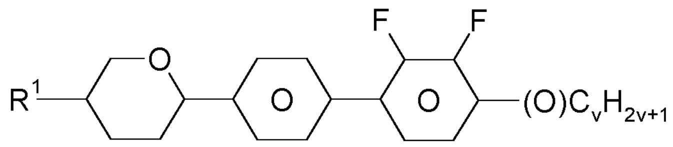

LY24

in which $R^1$ has the meaning indicated above, alkyl denotes a straight-chain alkyl radical having 1-6 C atoms, (O) denotes an oxygen atom or a single bond, and v denotes an integer from 1 to 6. $R^1$ preferably denotes straight-chain alkyl having 1 to 6 C atoms or straight-chain alkenyl having 2 to 6 C atoms, in particular $CH_3$, $C_2H_5$, n-$C_3H_7$, n-$C_4H_9$, n-$C_5H_{11}$, $CH_2$=CH-, $CH_2$=CH$CH_2CH_2$-, $CH_3$-CH=CH-, $CH_3$-$CH_2$-CH=CH-, $CH_3$-$(CH_2)_2$-CH=CH-, $CH_3$-$(CH_2)_3$-CH=CH- or $CH_3$-CH=CH-$(CH_2)_2$-.

e) LC medium which additionally comprises one or more compounds selected from the group consisting of the following formulae:

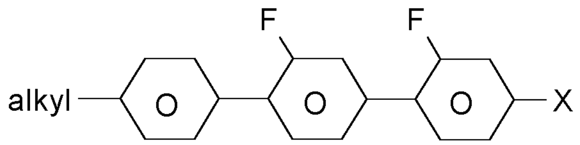

G1

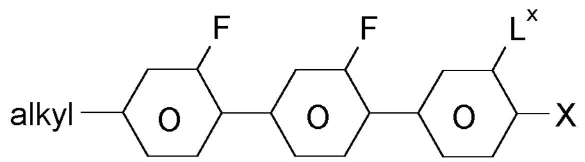

G2

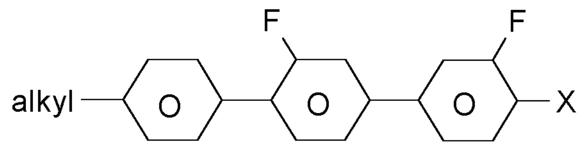

G3

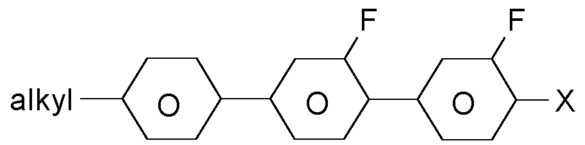

G4

in which alkyl denotes $C_{1-6}$-alkyl, $L^x$ denotes H or F, and X denotes F, Cl, $OCF_3$, $OCHF_2$ or OCH=$CF_2$. Particular preference is given to compounds of the formula G1 in which X denotes F.

f) LC medium which additionally comprises one or more compounds selected from the group consisting of the following formulae:

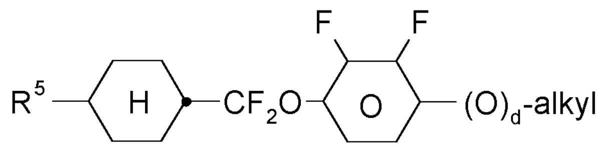

Y1

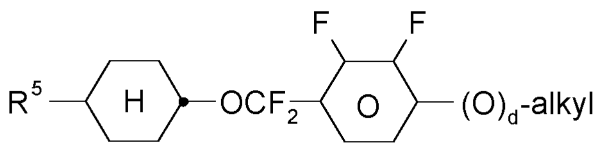

Y2

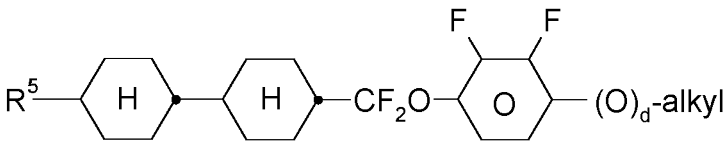
Y3

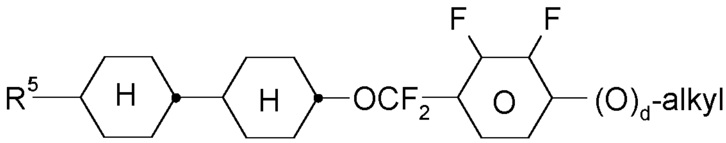
Y4

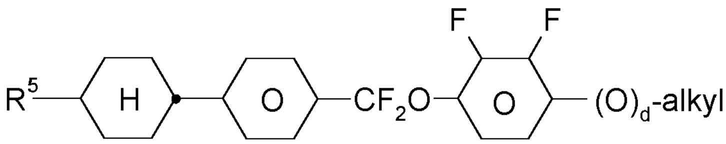
Y5

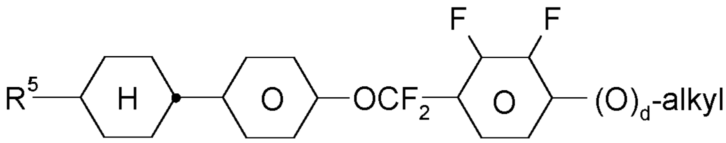
Y6

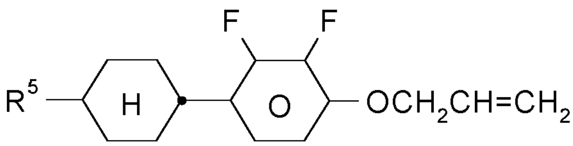
Y7

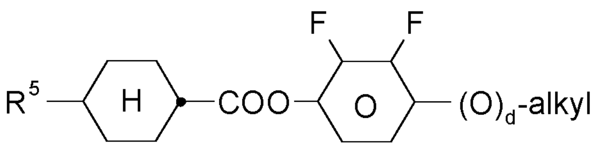
Y8

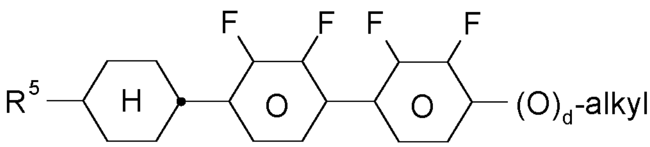
Y9

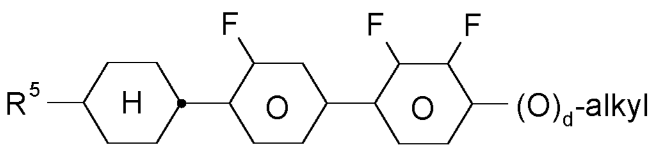
Y10

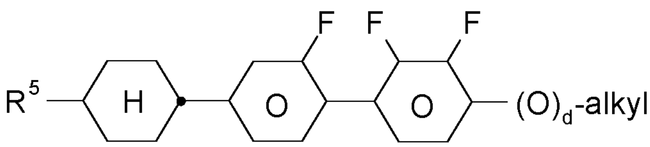
Y11

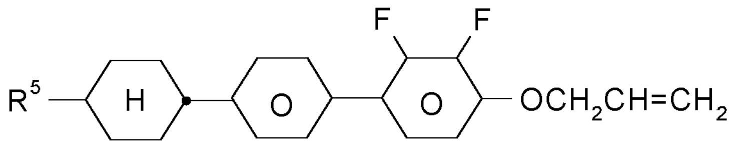

Y12

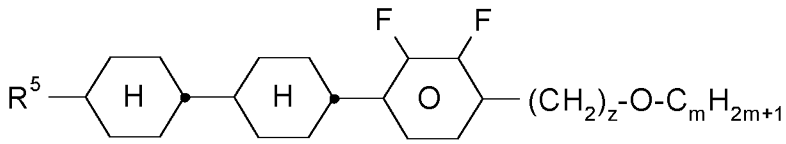

Y13

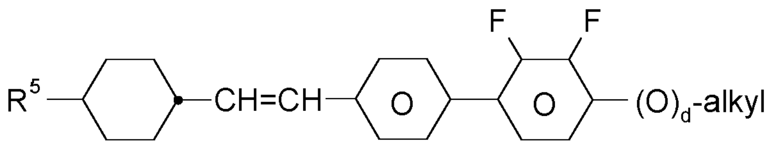

Y14

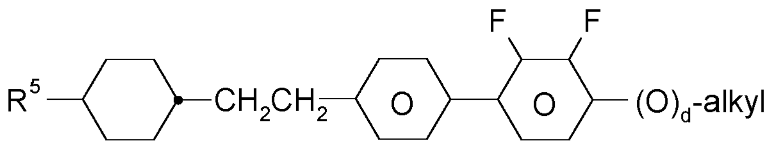

Y15

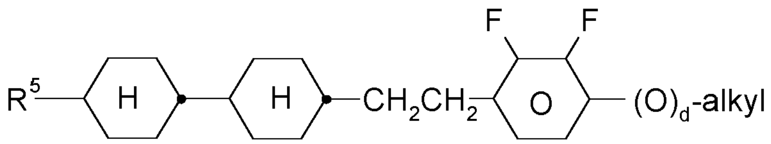

Y16

in which $R^5$ has one of the meanings indicated above for $R^1$, alkyl denotes $C_{1-6}$-alkyl, d denotes 0 or 1, and z and m each, independently of one another, denote an integer from 1 to 6. $R^5$ in these compounds is particularly preferably $C_{1-6}$-alkyl or -alkoxy or $C_{2-6}$-alkenyl, d is preferably 1. The LC medium according to the invention preferably comprises one or more compounds of the above-mentioned formulae in amounts of > 5% by weight.

g) LC medium which additionally comprises one or more biphenyl compounds selected from the group consisting of the following formulae:

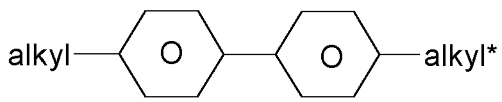

B1

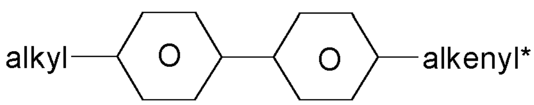

B2

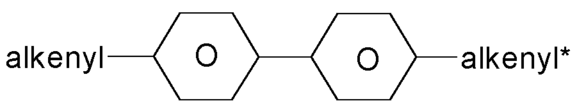

B3

in which alkyl and alkyl* each, independently of one another, denote a straight-chain alkyl radical having 1-6 C atoms, and alkenyl and alkenyl* each, independently of one another, denote a straight-chain alkenyl radical having 2-6 C atoms. Alkenyl and alkenyl* preferably denote $CH_2=CH-$, $CH_2=CHCH_2CH_2-$, $CH_3-CH=CH-$, $CH_3-CH_2-CH=CH-$, $CH_3-(CH_2)_2-CH=CH-$, $CH_3-(CH_2)_3-CH=CH-$ or $CH_3-CH=CH-(CH_2)_2-$.

The proportion of the biphenyls of the formulae B1 to B3 in the LC mixture is preferably at least 3% by weight, in particular $\geq$ 5% by weight.

The compounds of the formula B2 are particularly preferred.

The compounds of the formulae B1 to B3 are preferably selected from the group consisting of the following sub-formulae:

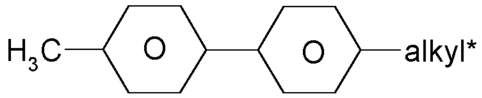

B1a

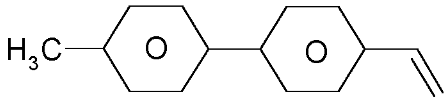

B2a

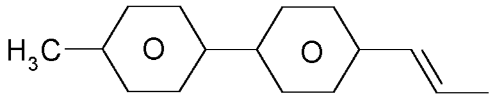

B2b

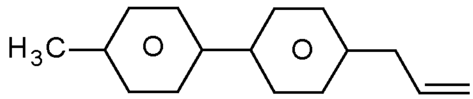

B2c

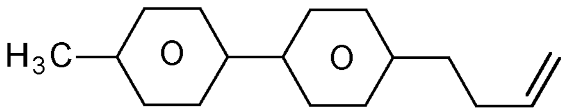

B2d

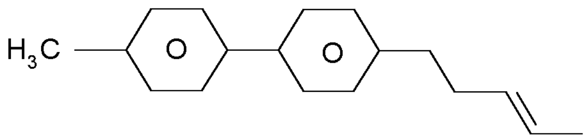

B2e

in which alkyl* denotes an alkyl radical having 1-6 C atoms. The medium according to the invention particularly preferably comprises one or more compounds of the formulae B1a and/or B2e.

h) LC medium which additionally comprises one or more terphenyl compounds of the following formula:

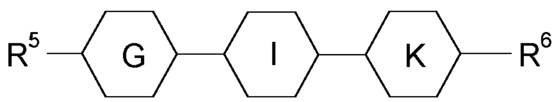

T

in which $R^5$ and $R^6$ each, independently of one another, have one of the meanings indicated above, and

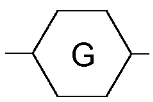 , 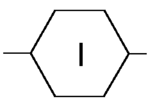 and 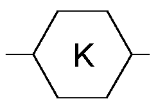

each, independently of one another, denote

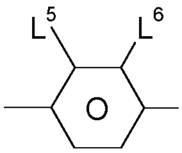 , 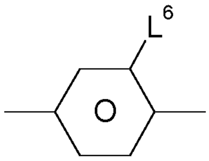 , 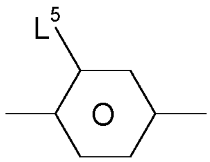 or 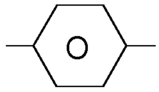

in which $L^5$ denotes F or Cl, preferably F, and $L^6$ denotes F, Cl, $OCF_3$, $CF_3$, $CH_3$, $CH_2F$ or $CHF_2$, preferably F.

The compounds of the formula T are preferably selected from the group consisting of the following sub-formulae:

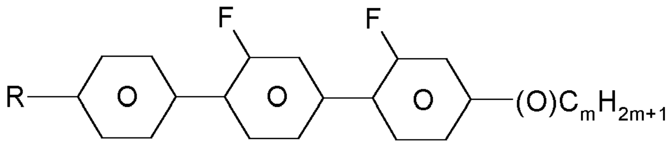

T1

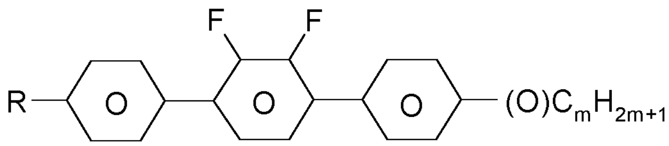

T2

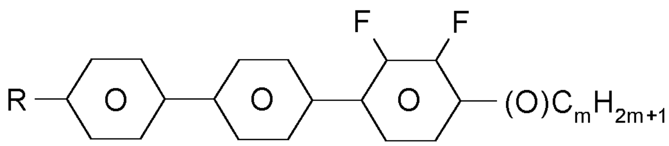

T3

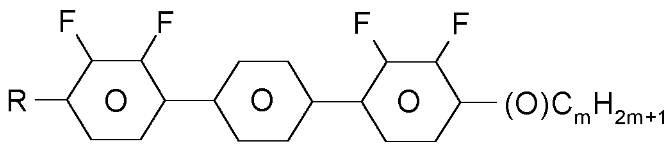

T4

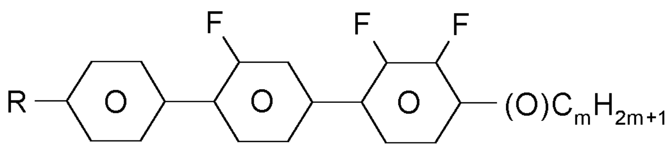

T5

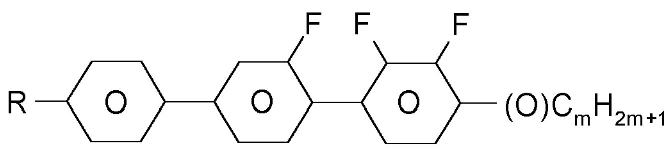

T6

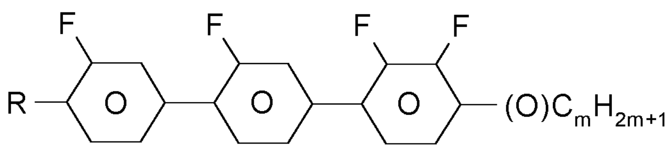

T7

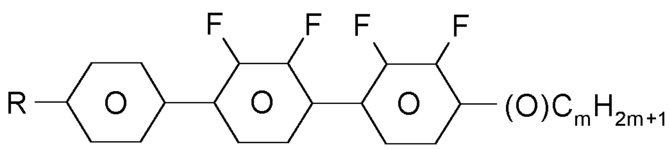

T8

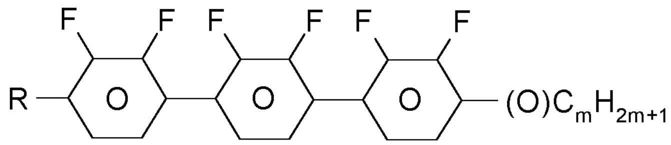

T9

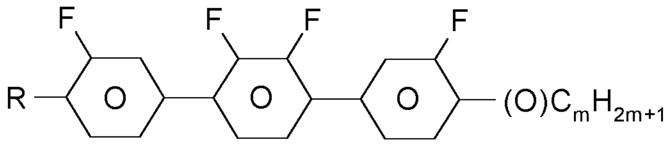

T10

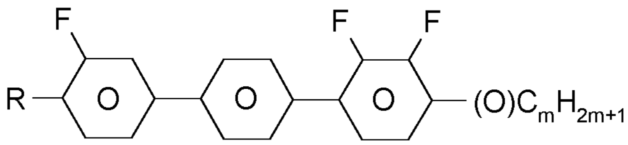

T11

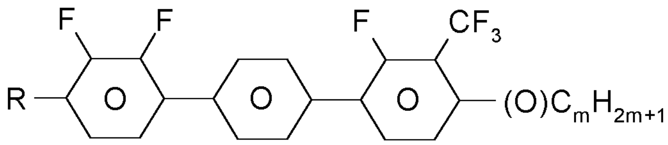

T12

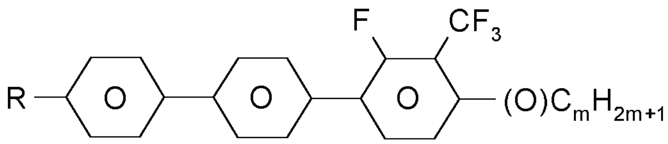

T13

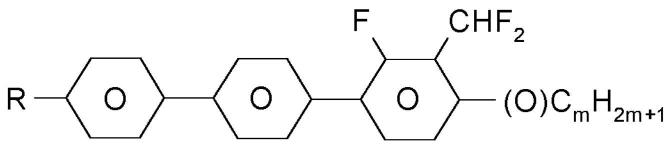

T14

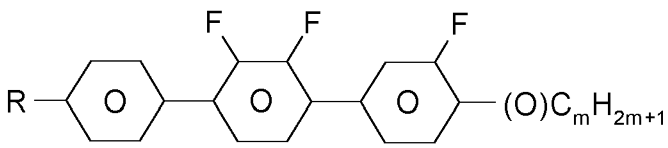

T15

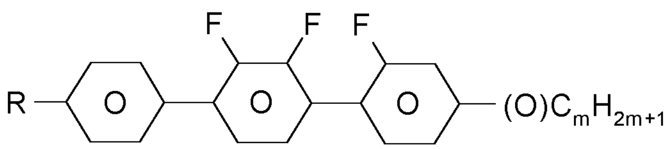

T16

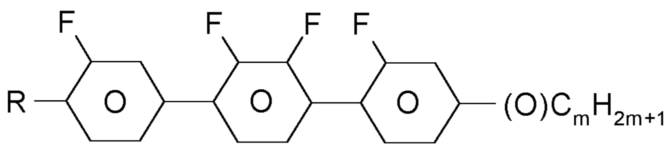

T17

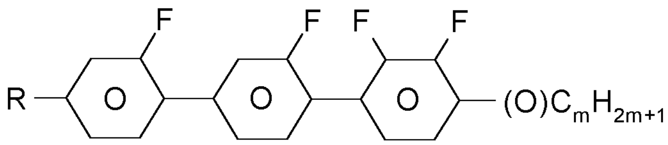

T18

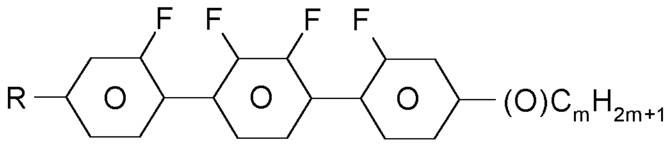

T19

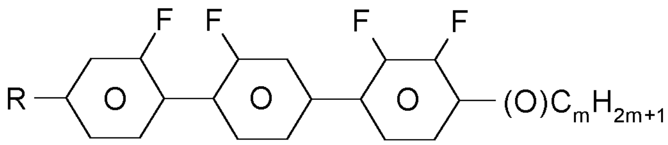

T20

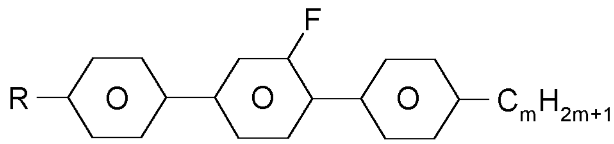

T21

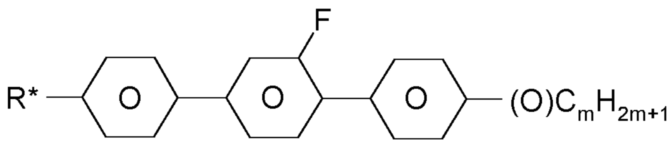

T22

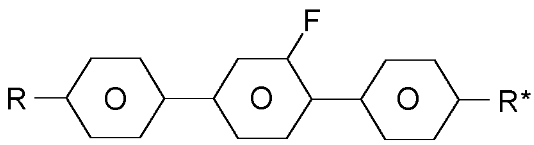

T23

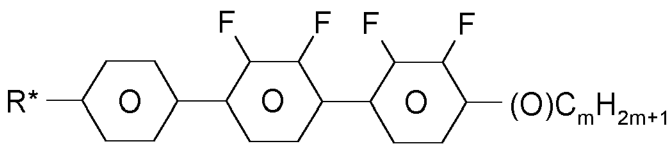

T24

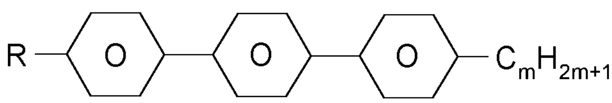

T25

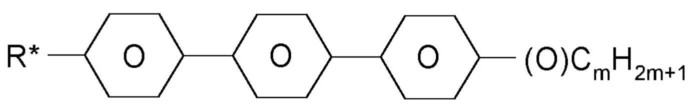

T26

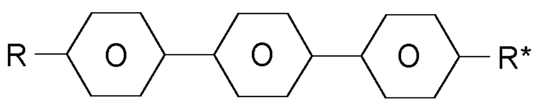

T27

in which R denotes a straight-chain alkyl or alkoxy radical having 1-7 C atoms, R* denotes a straight-chain alkenyl radical

having 2-7 C atoms, (O) denotes an oxygen atom or a single bond, and m denotes an integer from 1 to 6. R* preferably denotes $CH_2=CH-$, $CH_2=CHCH_2CH_2-$, $CH_3-CH=CH-$, $CH_3-CH_2-CH=CH-$, $CH_3-(CH_2)_2-CH=CH-$, $CH_3-(CH_2)_3-CH=CH-$ or $CH_3-CH=CH-(CH_2)_2-$.

R preferably denotes methyl, ethyl, propyl, butyl, pentyl, hexyl, methoxy, ethoxy, propoxy, butoxy or pentoxy.

The LC medium according to the invention preferably comprises the terphenyls of the formula T and the preferred sub-formulae thereof in an amount of 0.5-30% by weight, in particular 1-20% by weight.

Particular preference is given to compounds of the formulae T1, T2, T3 and T21. In these compounds, R preferably denotes alkyl, furthermore alkoxy, each having 1-5 C atoms.

The terphenyls are preferably employed in mixtures according to the invention if the Δn value of the mixture is to be ≥ 0.1. Preferred mixtures comprise 2-20% by weight of one or more terphenyl compounds of the formula T, preferably selected from the group of compounds T1 to T22.

i) LC medium which additionally comprises one or more compounds selected from the group consisting of the following formulae:

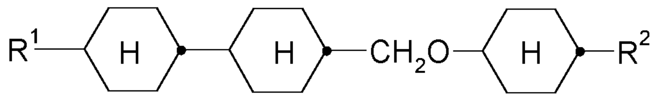
O1

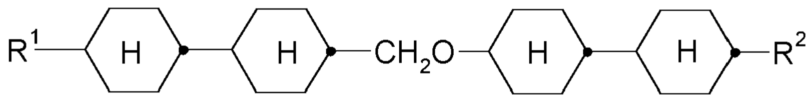
O2

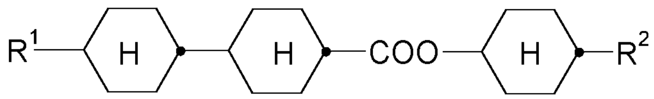
O3

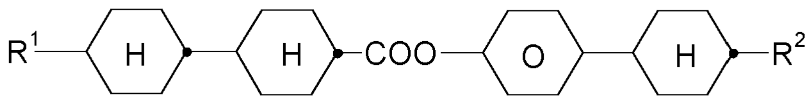
O4

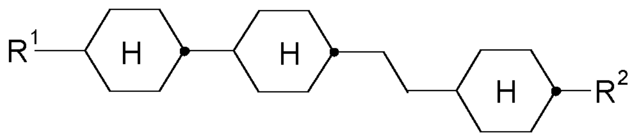
O5

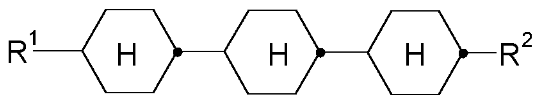
O6

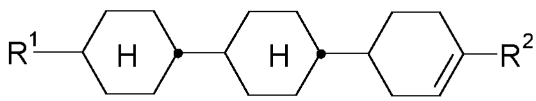
O7

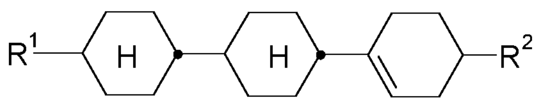
O8

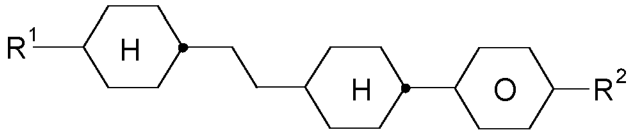

O9

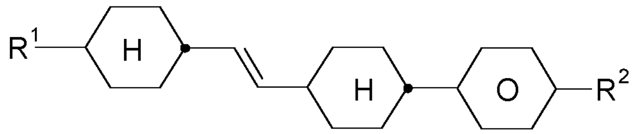

O10

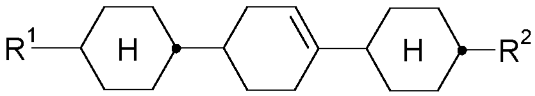

O11

in which $R^1$ and $R^2$ have the meanings indicated above and preferably each, independently of one another, denote straight-chain alkyl having 1 to 6 C atoms or straight-chain alkenyl having 2 to 6 C atoms.

Preferred media comprise one or more compounds selected from the formulae O1, O3 and O4.

k) LC medium which additionally comprises one or more compounds of the following formula:

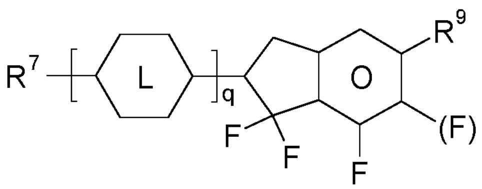

FI

in which

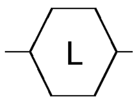

denotes

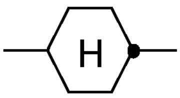 , 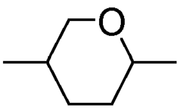 , 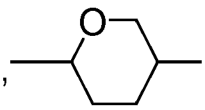 , 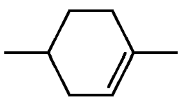

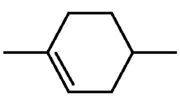 , 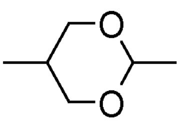 , 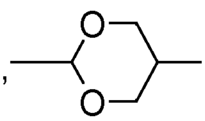 , 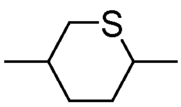

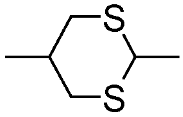 , 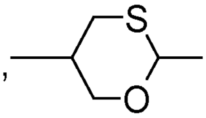 ,

$R^9$ denotes H, $CH_3$, $C_2H_5$ or n-$C_3H_7$, (F) denotes an optional fluorine substituent, and q denotes 1, 2 or 3, and $R^7$ has one of the meanings indicated for $R^1$, preferably in amounts of > 3% by weight, in particular ≥ 5% by weight and very particularly preferably 5-30% by weight.

Particularly preferred compounds of the formula FI are selected from the group consisting of the following sub-formulae:

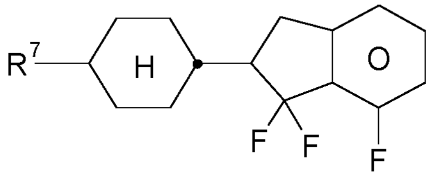

FI1

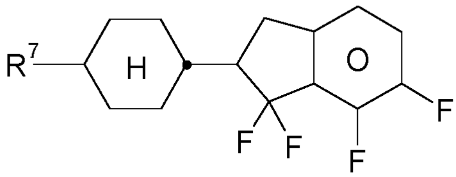

FI2

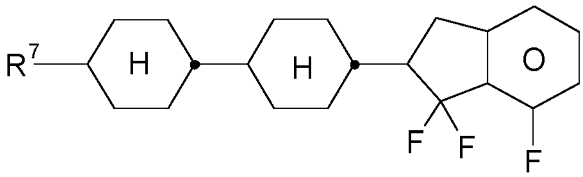

FI3

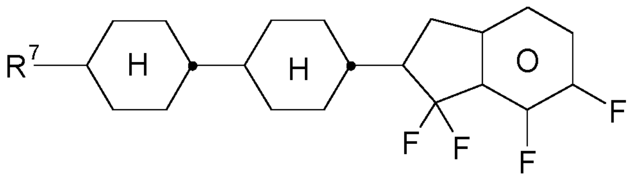

FI4

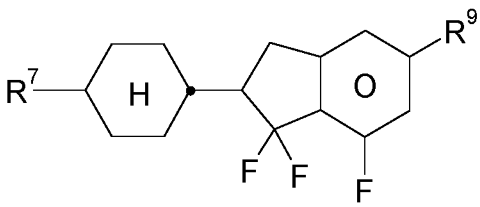

FI5

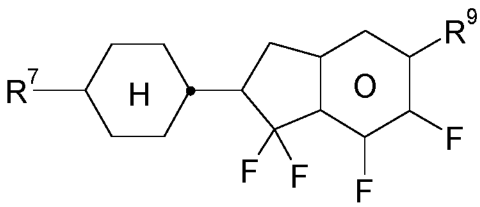

FI6

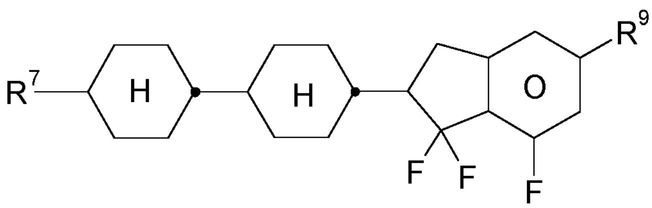

FI7

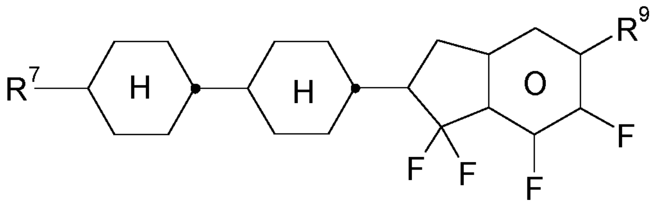

FI8

in which $R^7$ preferably denotes straight-chain alkyl, and $R^9$ denotes $CH_3$, $C_2H_5$ or n-$C_3H_7$. Particular preference is given to the compounds of the formulae FI1, FI2 and FI3.

l) LC medium which additionally comprises one or more compounds selected from the group consisting of the following formulae:

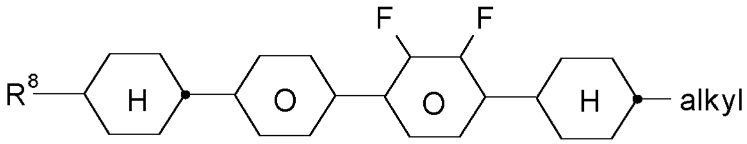

VK1

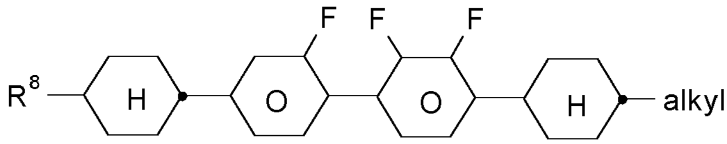

VK2

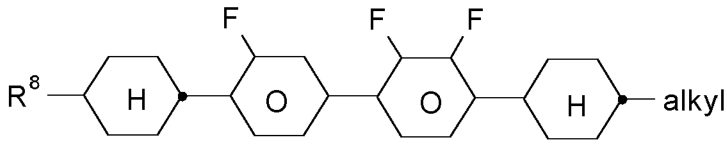

VK3

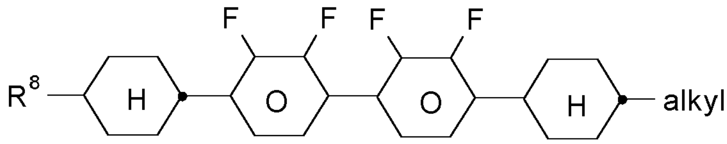

VK4

in which $R^8$ has the meaning indicated for $R^1$, and alkyl denotes a straight-chain alkyl radical having 1-6 C atoms.

m) LC medium which additionally comprises one or more compounds which contain a tetrahydronaphthyl or naphthyl unit, such as, for example, the compounds selected from the group consisting of the following formulae:

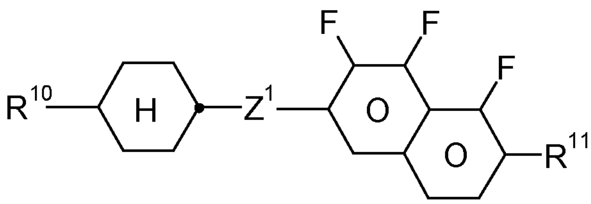

N1

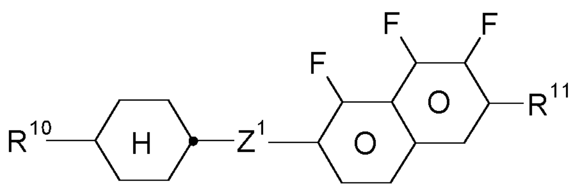

N2

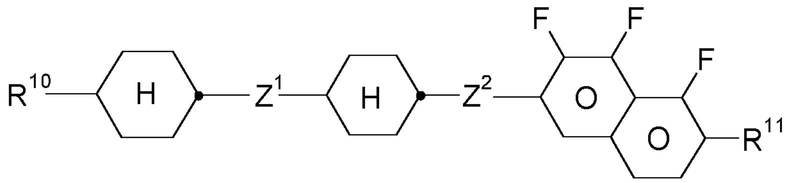
N3

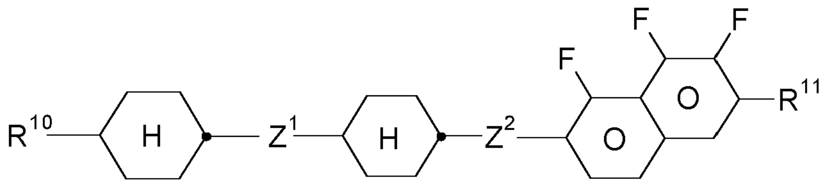
N4

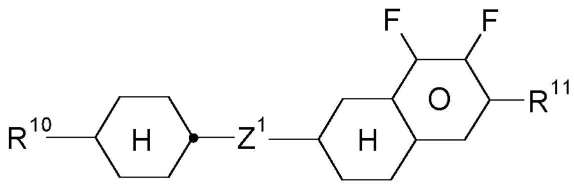
N5

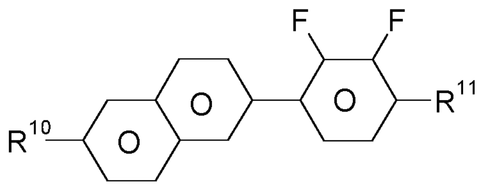
N6

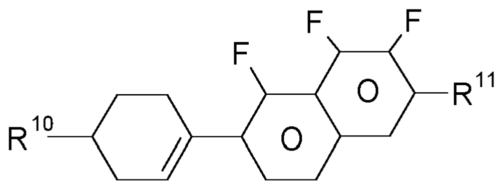
N7

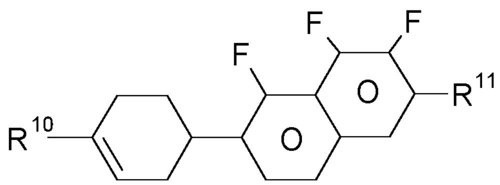
N8

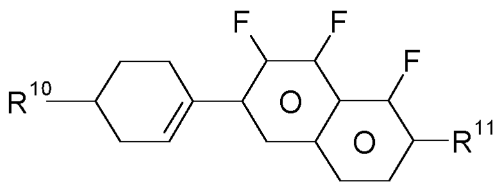
N9

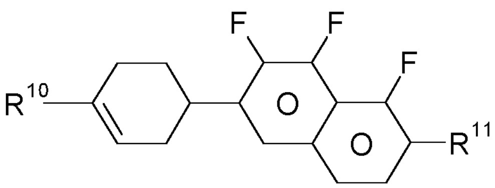
N10

in which

$R^{10}$ and $R^{11}$ each, independently of one another, denote alkyl having 1 to 12 C atoms, where, in addition, one or two non-adjacent $CH_2$ groups may be replaced by -O-, -CH=CH-, -CO-, -OCO- or -COO- in such a way that O atoms are not linked directly to one another, preferably alkyl or alkoxy having 1 to 6 C atoms,

and $R^{10}$ and $R^{11}$ preferably denote straight-chain alkyl or alkoxy having 1 to 6 C atoms or straight-chain alkenyl having 2 to 6 C atoms, and

$Z^1$ and $Z^2$ each, independently of one another, denote $-C_2H_4-$, -CH=CH-, $-(CH_2)_4-$, $-(CH_2)_3O-$, $-O(CH_2)_3-$, $-CH=CH-CH_2CH_2-$, $-CH_2CH_2CH=CH-$, $-CH_2O-$, $-OCH_2-$, -CO-O-, -O-CO-, $-C_2F_4-$, -CF=CF-, -CF=CH-, -CH=CF-, $-CH_2-$ or a single bond.

n) LC medium which additionally comprises one or more difluorodibenzochromans and/or chromans of the following formulae:

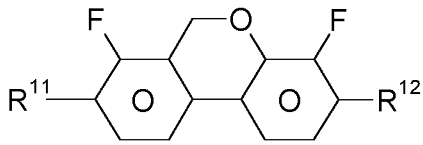

BC

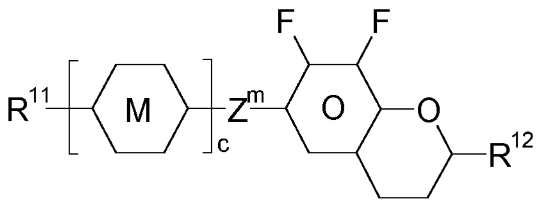

CR

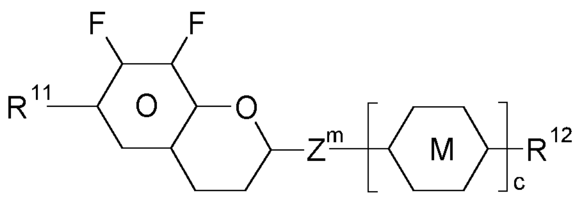

RC

in which

$R^{11}$ and $R^{12}$ each, independently of one another, have one of the meanings indicated above for $R^{11}$ under formula N1
ring M is trans-1,4-cyclohexylene or 1,4-phenylene,
$Z^m$ $-C_2H_4-$, $-CH_2O-$, $-OCH_2-$, -CO-O- or -O-CO-,
c is 0, 1 or 2,

preferably in amounts of 3 to 20% by weight, in particular in amounts of 3 to 15% by weight.

Particularly preferred compounds of the formulae BC, CR and RC are selected from the group consisting of the following sub-formulae:

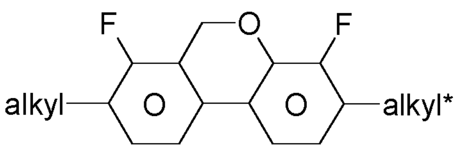

BC1

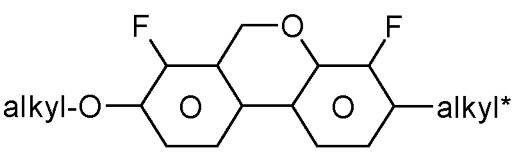

BC2

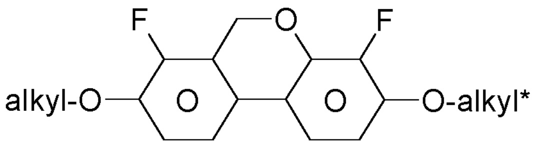

BC3

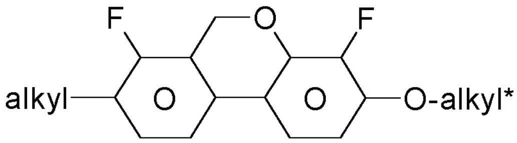

BC4

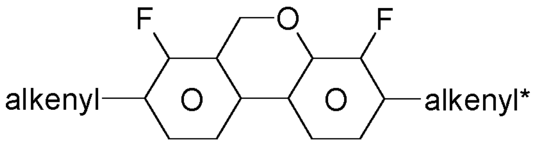

BC5

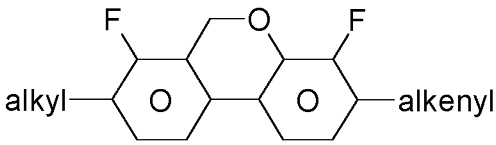

BC6

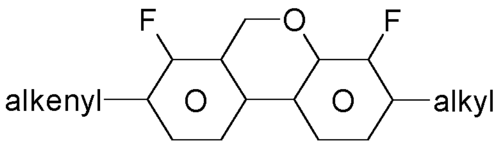

BC7

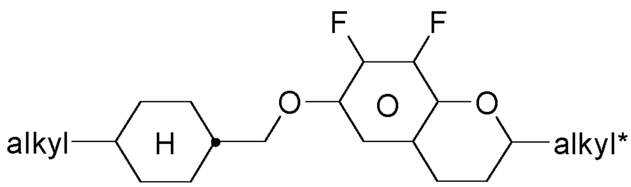

CR1

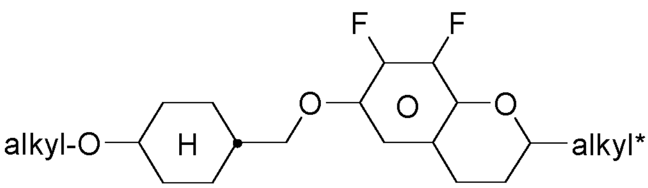

CR2

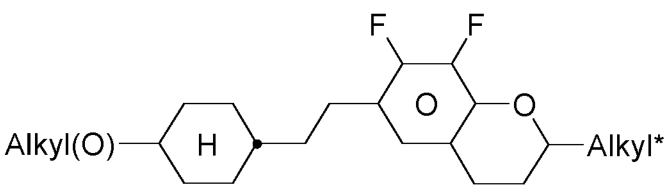

CR3

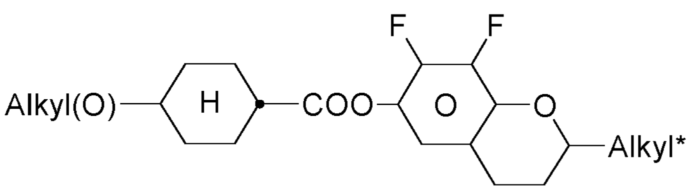

CR4

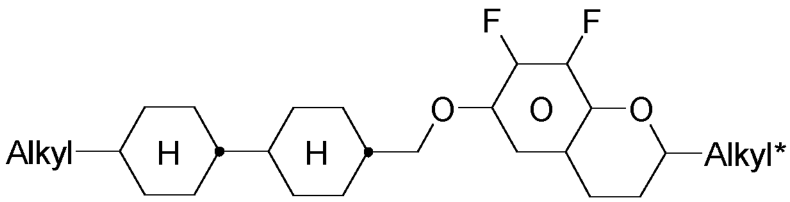

CR5

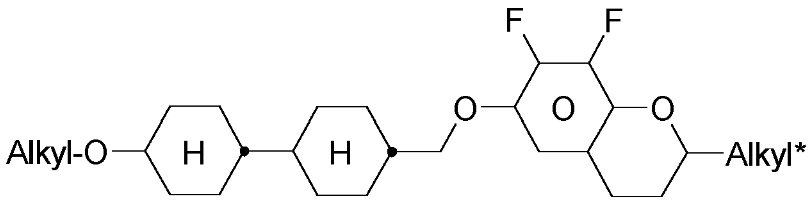

CR6

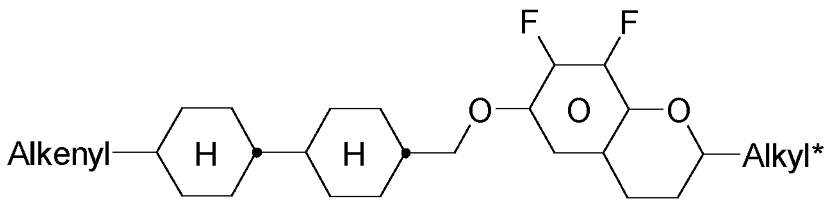

CR7

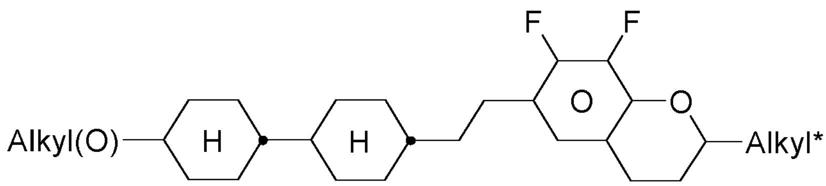

CR8

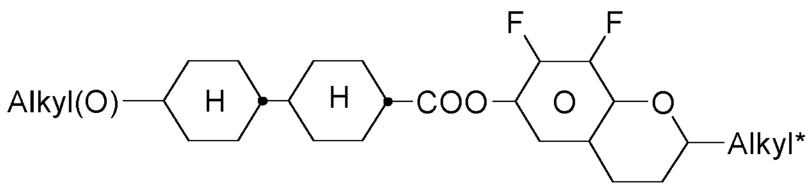

CR9

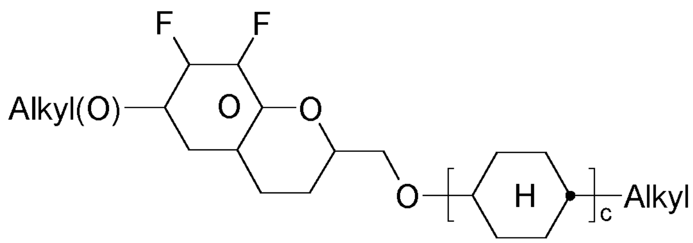

RC1

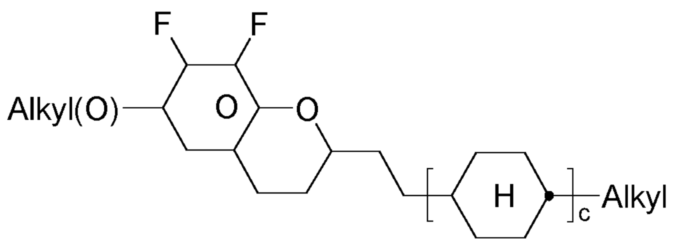

RC2

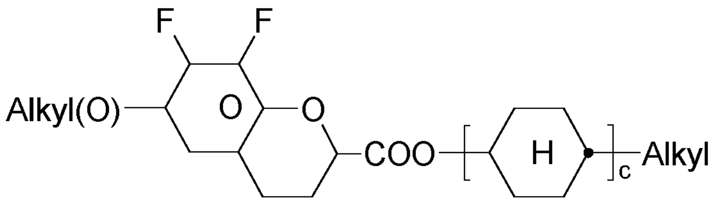

RC3

in which alkyl and alkyl* each, independently of one another, denote a straight-chain alkyl radical having 1-6 C atoms, (O) denotes an oxygen atom or a single bond, c is 1 or 2, and alkenyl and alkenyl* each, independently of one another, denote a straight-chain alkenyl radical having 2-6 C atoms. Alkenyl and alkenyl* preferably denote $CH_2$=CH-, $CH_2$=$CHCH_2CH_2$-, $CH_3$-CH=CH-, $CH_3$-$CH_2$-CH=CH-, $CH_3$-$(CH_2)_2$-CH=CH-, $CH_3$-$(CH_2)_3$-CH=CH- or $CH_3$-CH=CH-$(CH_2)_2$-.

Very particular preference is given to mixtures comprising one, two or three compounds of the formula BC-2.

o) LC medium which additionally comprises one or more fluorinated phenanthrenes and/or dibenzofurans of the following formulae:

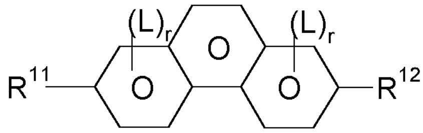

PH

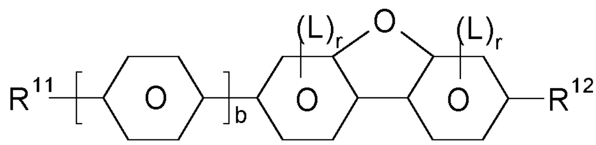

BF

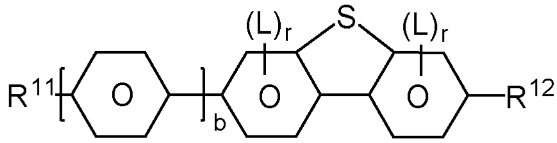

BS

in which $R^{11}$ and $R^{12}$ each, independently of one another, have one of the meanings indicated above for $R^{11}$ under formula N1, b denotes 0 or 1, L denotes F, and r denotes 1, 2 or 3.

Particularly preferred compounds of the formulae PH and BF are selected from the group consisting of the following sub-formulae:

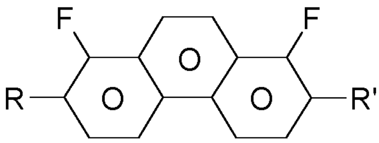

PH1

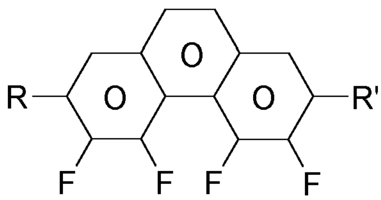

PH2

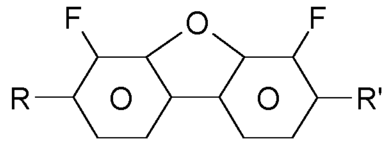

BF1

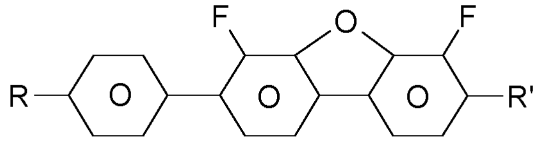

BF2

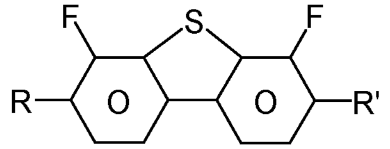

BS1

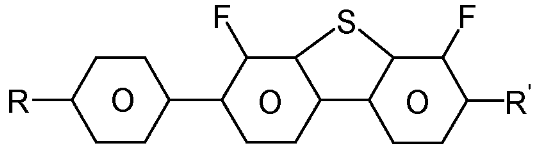

BS2

in which R and R' each, independently of one another, denote a straight-chain alkyl or alkoxy radical having 1-7 C atoms.

p) LC medium which additionally comprises one or more monocyclic compounds of the following formula

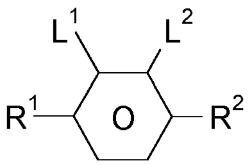

Y

wherein

$R^1$ and $R^2$ each, independently of one another, denote alkyl having 1 to 12 C atoms, where, in addition, one or two non-adjacent $CH_2$ groups may be replaced by -O-, -CH=CH-, -CO-, -OCO- or -COO- in such a way that O atoms are not linked directly to one another, preferably alkyl or alkoxy having 1 to 6 C atoms,

$L^1$ and $L^2$ each, independently of one another, denote F, Cl, $OCF_3$, $CF_3$, $CH_3$, $CH_2F$, $CHF_2$.

Preferably, both $L^1$ and $L^2$ denote F or one of $L^1$ and $L^2$ denotes F and the other denotes Cl,

The compounds of the formula Y are preferably selected from the group consisting of the following sub-formulae:

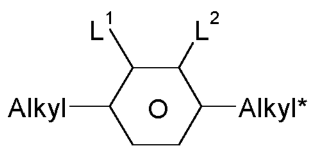

Y1

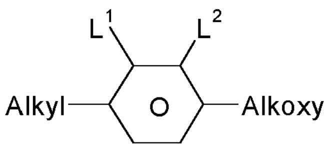

Y2

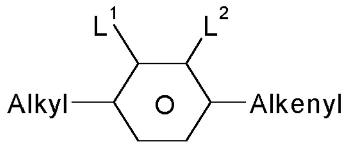

Y3

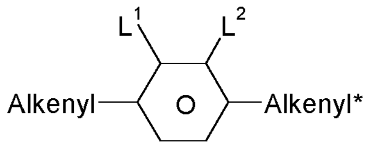

Y4

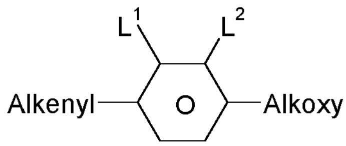

Y5

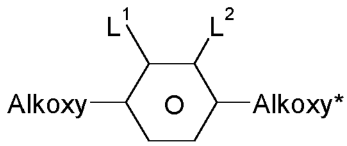

Y6

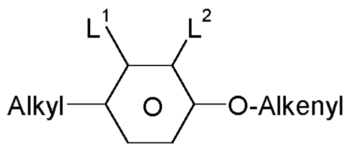

Y7

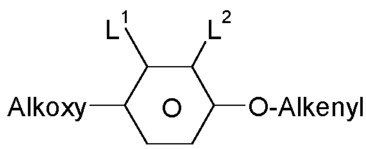

Y8

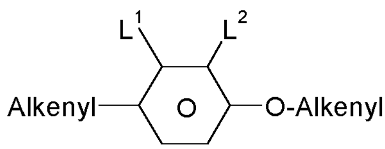

Y9

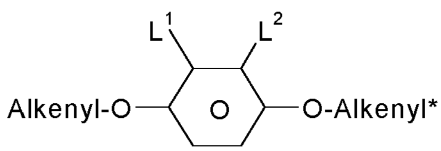

Y10,

in which, Alkyl and Alkyl* each, independently of one another, denote a straight-chain alkyl radical having 1-6 C atoms, Alkoxy denotes a straight-chain alkoxy radical having 1-6 C atoms, Alkenyl and Alkenyl* each, independently of one another, denote a straight-chain alkenyl radical having 2-6 C atoms, and O denotes an oxygen atom or a single bond. Alkenyl and Alkenyl* preferably denote $CH_2$=CH-, $CH_2$=$CHCH_2CH_2$-, $CH_3$-CH=CH-, $CH_3$-$CH_2$-CH=CH-, $CH_3$-$(CH_2)_2$-CH=CH-, $CH_3$-$(CH_2)_3$-CH=CH- or $CH_3$-CH=CH-$(CH_2)_2$-.

Particularly preferred compounds of the formula Y are selected from the group consisting of the following sub-formulae:

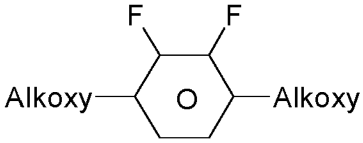

Y6A

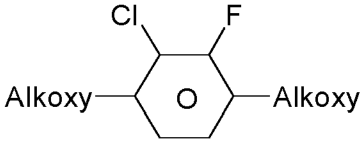

Y6B

wherein Alkoxy preferably denotes straight-chain alkoxy with 3, 4, or 5 C atoms.

q) LC medium which, apart from the stabilisers according to the invention, in particular of the formula I or sub-formulae thereof and the comonomers, comprises no compounds which contain a terminal vinyloxy group ($-O-CH=CH_2$).

r) LC medium which comprises 1 to 5, preferably 1, 2 or 3, stabilisers, preferably selected from stabilisers according to the invention, in particular of the formula I or sub-formulae thereof.

s) LC medium in which the proportion of stabilisers, in particular of the formula I or sub-formulae thereof, in the mixture as a whole is 1 to 1500ppm, preferably 100 to 1000ppm.

t) LC medium which comprises 1 to 8, preferably 1 to 5, compounds of the formulae CY1, CY2, PY1 and/or PY2. The proportion of these compounds in the mixture as a whole is preferably 5 to 60%, particularly preferably 10 to 35%. The content of these individual compounds is preferably in each case 2 to 20%.

u) LC medium which comprises 1 to 8, preferably 1 to 5, compounds of the formulae CY9, CY10, PY9 and/or PY10. The proportion of these compounds in the mixture as a whole is preferably 5 to 60%, particularly preferably 10 to 35%. The content of these individual compounds is preferably in each case 2 to 20%.

v) LC medium which comprises 1 to 10, preferably 1 to 8, compounds of the formula ZK, in particular compounds of the formulae ZK1, ZK2 and/or ZK6. The proportion of these compounds in the mixture as a whole is preferably 3 to 25%, particularly preferably 5 to 45%. The content of these individual compounds is preferably in each case 2 to 20%.

w) LC medium in which the proportion of compounds of the formulae CY, PY and ZK in the mixture as a whole is greater than 70%, preferably greater than 80%.

x) LC medium in which the LC host mixture contains one or more compounds containing an alkenyl group, preferably selected from the group consisting of formula CY, PY and LY, wherein one or both of $R^1$ and $R^2$ denote straight-chain alkenyl having 2-6 C atoms, formula ZK and DK, wherein one or both of $R^3$ and $R^4$ or one or both of $R^5$ and $R^6$ denote straight-chain alkenyl having 2-6 C atoms, and formula B2 and B3, very preferably selected from formulae CY15, CY16, CY24, CY32, PY15, PY16, ZK3, ZK4, DK3, DK6, B2 and B3, most preferably selected from formulae ZK3, ZK4, B2 and B3. The concentration of these compounds in the LC host mixture is preferably from 2 to 70%, very preferably from 3 to 55%.

y) LC medium which contains one or more, preferably 1 to 5, compounds selected of formula PY1-PY8, very preferably of formula PY2. The proportion of these compounds in the mixture as a whole is preferably 1 to 30%, particularly preferably 2 to 20%. The content of these individual compounds is preferably in each case 1 to 20%.

z) LC medium which contains one or more, preferably 1, 2 or 3, compounds of formula T2. The content of these compounds in the mixture as a whole is preferably 1 to 20%.

In another preferred embodiment of the present invention the LC medium contains an LC host mixture with positive dielectric anisotropy. Preferred embodiments of such an LC medium, and the corresponding LC host mixture, are those of sections aa) - mmm) below:

aa) LC-medium, characterised in that it comprises one or more compounds selected from the group of compounds of the formulae II and III

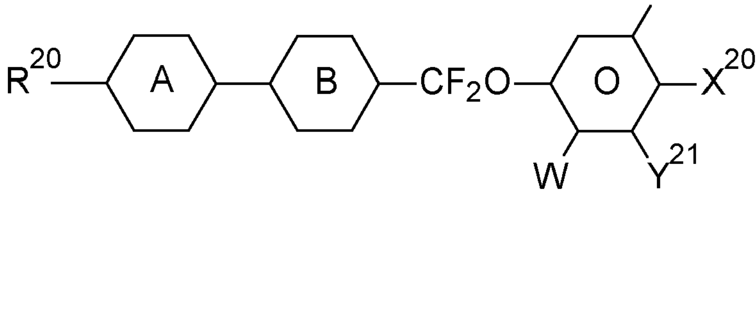

II

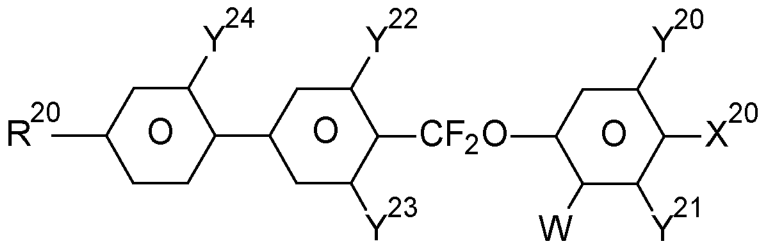

III

wherein

R20 each, identically or differently, denote a halogenated or unsubstituted alkyl or alkoxy radical having 1 to 15 C atoms, where, in addition, one or more $CH_2$ groups in these radicals may each be replaced, independently of one another, by -C≡C-, $-CF_2O-$, -CH=CH-,

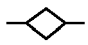, 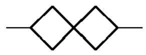,

-O-, -CO-O- or -O-CO- in such a way that O atoms are not linked directly to one another,

X20 each, identically or differently, denote F, Cl, CN, $SF_5$, SCN, NCS, a halogenated alkyl radical, a halogenated alkenyl radical, a halogenated alkoxy radical or a halogenated alkenyloxy radical, each having up to 6 C atoms, and

Y20-24 each, identically or differently, denote H or F;

W denotes H or methyl,

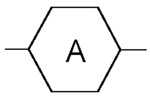 and 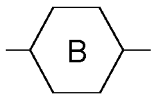

each, independently of one another, denote

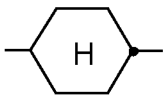,

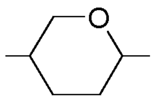 or 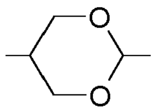.

The compounds of the formula II are preferably selected from the following formulae:

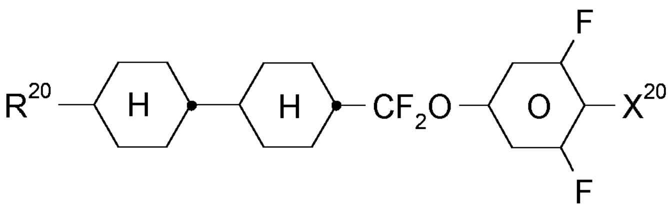

IIa

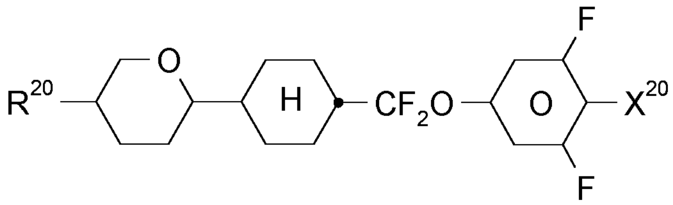

IIb

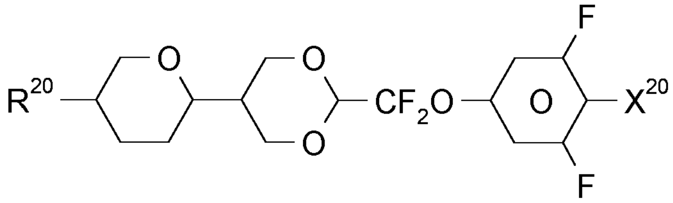

IIc

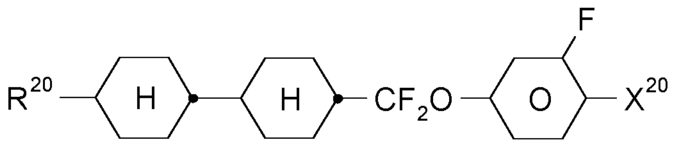

IId

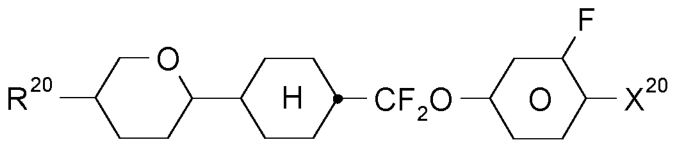

IIe

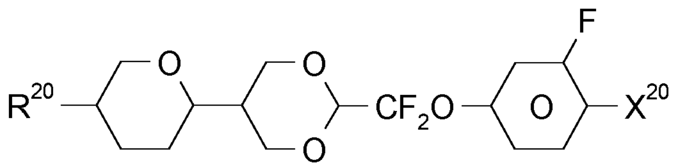

IIf

wherein $R^{20}$ and $X^{20}$ have the meanings indicated above.

$R^{20}$ preferably denotes alkyl having 1 to 6 C atoms. $X^{20}$ preferably denotes F. Particular preference is given to compounds of the formulae IIa and IIb, in particular compounds of the formulae IIa and IIb wherein X denotes F.

The compounds of the formula III are preferably selected from the following formulae:

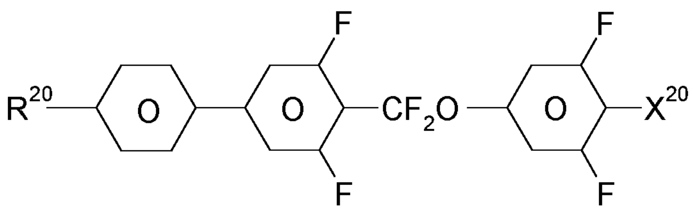

IIIa

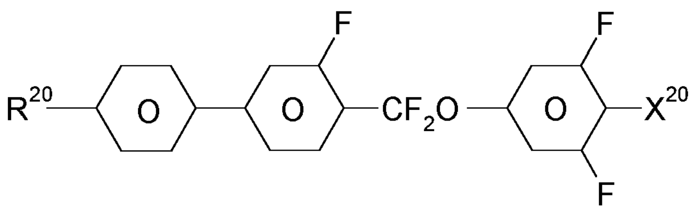

IIIb

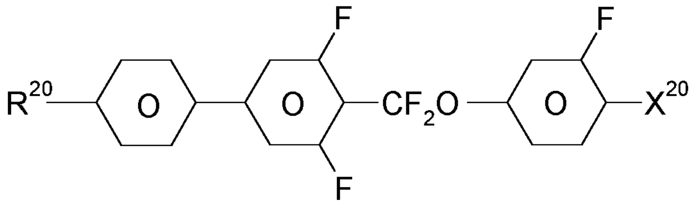

IIIc

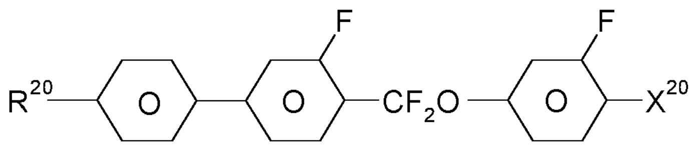

IIId

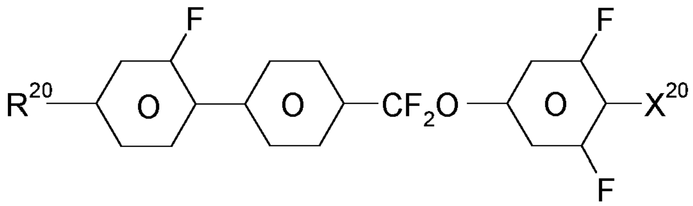

IIIe

wherein $R^{20}$ and $X^{20}$ have the meanings indicated above.

$R^{20}$ preferably denotes alkyl having 1 to 6 C atoms. $X^{20}$ preferably denotes F. Particular preference is given to compounds of the formulae IIIa and IIIe, in particular compounds of the formula IIIa;

bb) LC-medium additionally comprising one or more compounds selected from the following formulae:

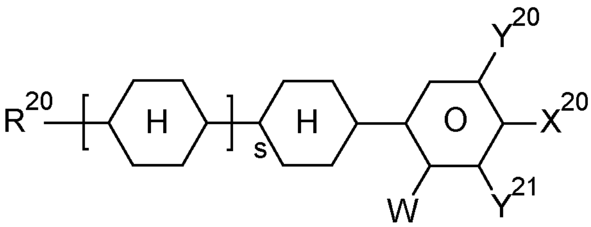

IV

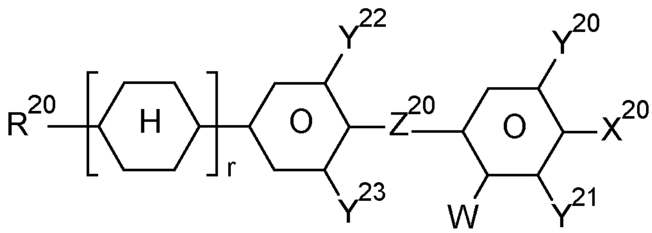

V

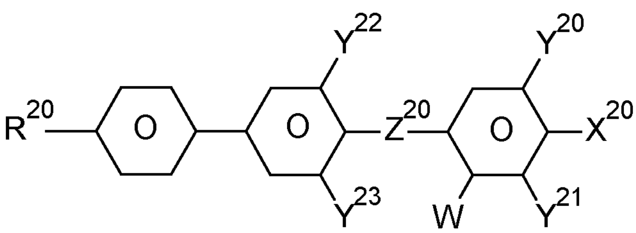

VI

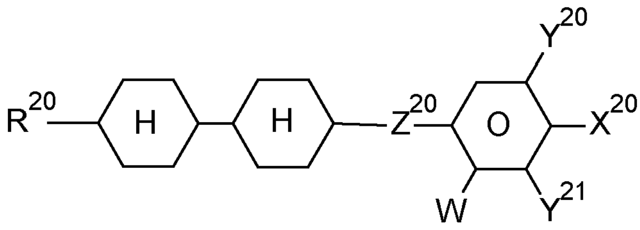

VII

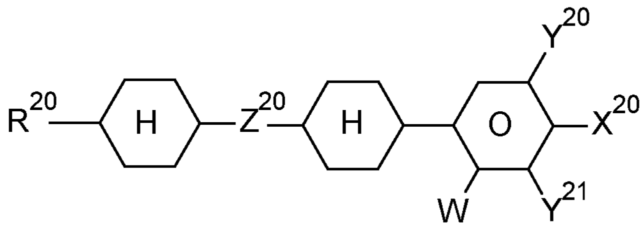

VIII

wherein

| | |
|---|---|
| $R^{20}$, $X^{20}$, W and $Y^{20-23}$ | have the meanings indicated above under formula II, and |
| $Z^{20}$ | denotes $-C_2H_4-$, $-(CH_2)_4-$, -CH=CH-, -CF=CF, $-C_2F_4-$, $-CH_2CF_2-$, $-CF_2CH_2-$, $-CH_2O-$, $-OCH_2-$, -COO- or $-OCF_2-$, in formulae V and VI also a single bond, in formulae V and VIII also $-CF_2O-$, |
| r | denotes 0 or 1, and |
| s | denotes 0 or 1; |

- The compounds of the formula IV are preferably selected from the following formulae:

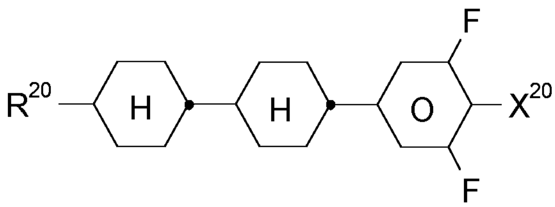

IVa

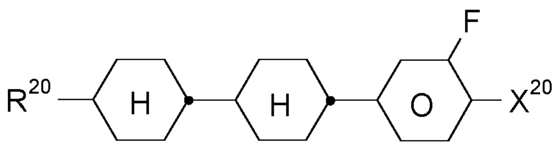

IVb

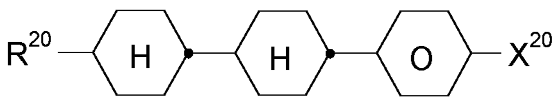

IVc

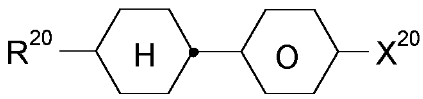

IVd

wherein $R^{20}$ and $X^{20}$ have the meanings indicated above.

$R^{20}$ preferably denotes alkyl having 1 to 6 C atoms. $X^{20}$ preferably denotes F or $OCF_3$, furthermore $OCF=CF_2$

or Cl;

- The compounds of the formula V are preferably selected from the following formulae:

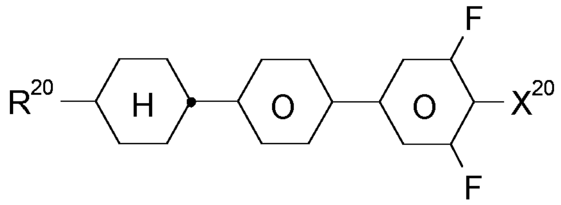

Va

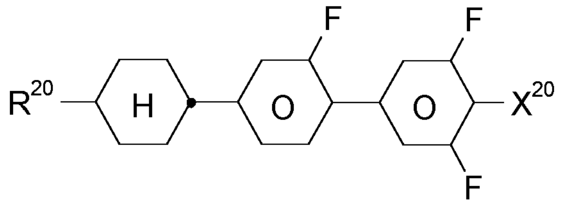

Vb

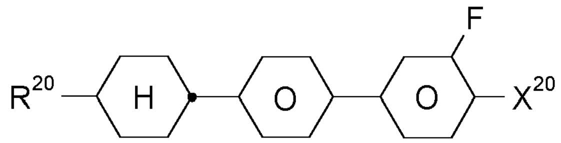

Vc

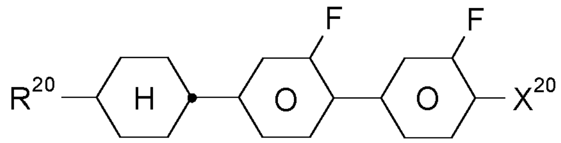

Vd

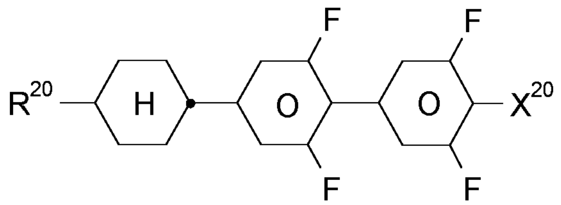

Ve

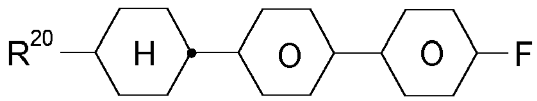

Vf

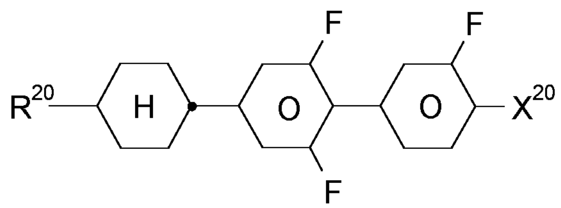

Vg

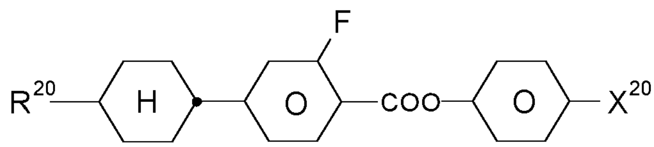

Vh

wherein $R^{20}$ and $X^{20}$ have the meanings indicated above.

$R^{20}$ preferably denotes alkyl having 1 to 6 C atoms. $X^{20}$ preferably denotes F and $OCF_3$, furthermore $OCHF_2$, $CF_3$, $OCF=CF_2$ and $OCH=CF_2$;

- The compounds of the formula VI are preferably selected from the following formulae:

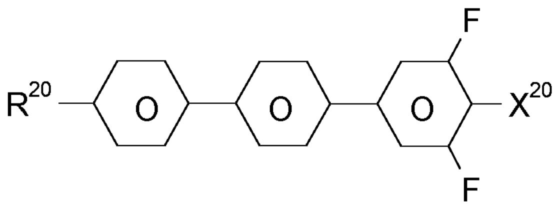

VIa

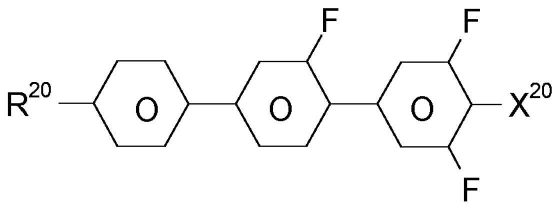

VIb

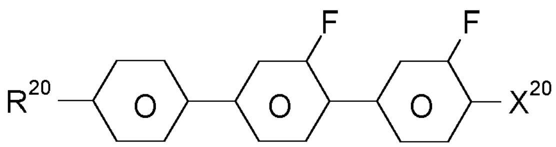

VIc

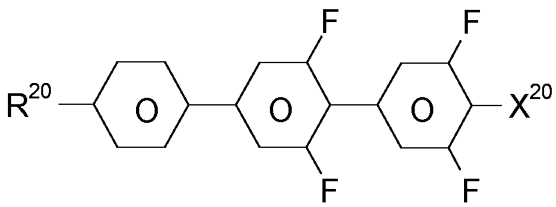

VId

wherein $R^{20}$ and $X^{20}$ have the meanings indicated above.

$R^{20}$ preferably denotes alkyl having 1 to 6 C atoms. $X^{20}$ preferably denotes F, furthermore $OCF_3$, $CF_3$, $CF=CF_2$, $OCHF_2$ and $OCH=CF_2$;

- The compounds of the formula VII are preferably selected from the following formulae:

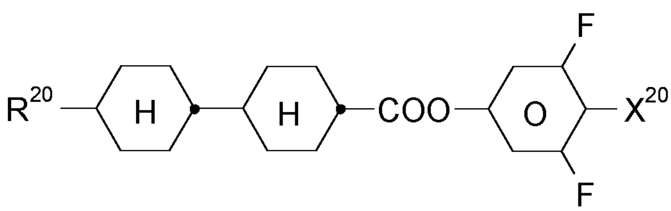

VIIa

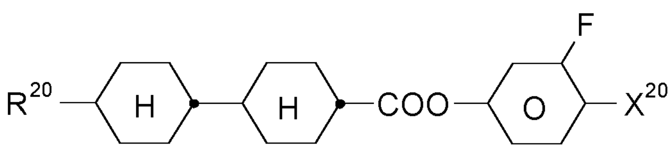

VIIb

wherein $R^{20}$ and $X^{20}$ have the meanings indicated above.

$R^{20}$ preferably denotes alkyl having 1 to 6 C atoms. $X^{20}$ preferably denotes F, furthermore $OCF_3$, $OCHF_2$ and $OCH{=}CF_2$.

cc) The medium additionally comprises one or more compounds selected from the formulae ZK1 to ZK10 given above. Especially preferred are compounds of formula ZK1 and ZK3. Particularly preferred compounds of formula ZK are selected from the sub-formulae ZK1a, ZK1b, ZK1c, ZK3a, ZK3b, ZK3c and ZK3d.

dd) The medium additionally comprises one or more compounds selected from the formulae DK1 to DK12 given above. Especially preferred compounds are DK3.

ee) The medium additionally comprises one or more compounds selected from the following formulae:

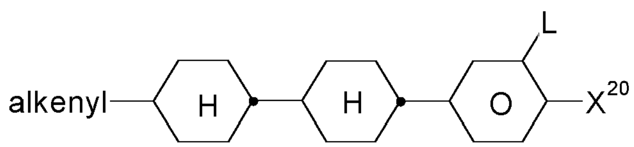

IX

wherein $X^{20}$ has the meanings indicated above, and

L denotes H or F,

"alkenyl" denotes $C_{2-6}$-alkenyl.

ff) The compounds of the formulae DK-3a and IX are preferably selected from the following formulae:

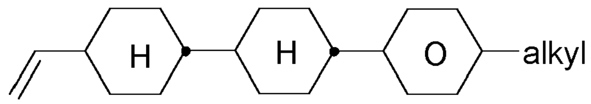

DK3a

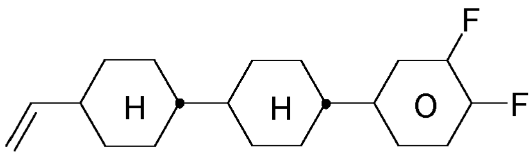

IXa

wherein "alkyl" denotes $C_{1-6}$-alkyl, preferably n-$C_3H_7$, n-$C_4H_9$ or n-$C_5H_{11}$, in particular n-$C_3H_7$.

gg) The medium additionally comprises one or more compounds selected from the formulae B1, B2 and B3 given above, preferably from the formula B2. The compounds of the formulae B1 to B3 are particularly preferably selected from the formulae B1a, B2a, B2b and B2c.

hh) The medium additionally comprises one or more compounds selected from the following formula:

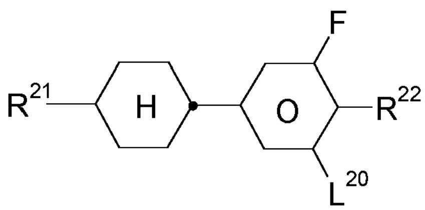

X

wherein $L^{20}$ denotes H or F, and $R^{21}$ and $R^{22}$ each, identically or differently, denote n-alkyl, alkoxy, oxaalkyl, fluoroalkyl or alkenyl, each having up to 6 C atoms, and preferably each, identically or differently, denote alkyl having 1 to 6 C atoms.

ii) The medium comprises one or more compounds of the following formulae:

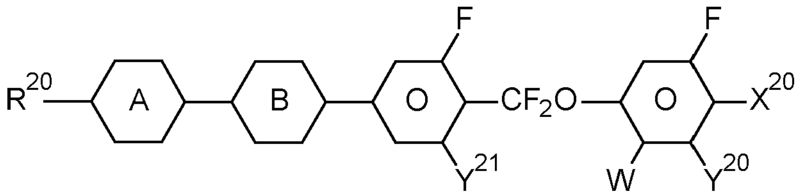

XI

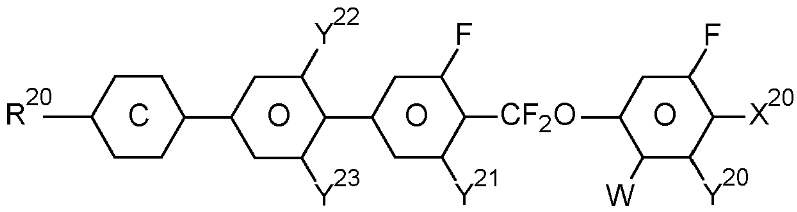

XII

Wherein W, $R^{20}$, $X^{20}$ and $Y^{20-23}$ have the meanings indicated in formula III, and

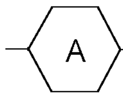 and 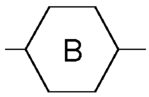

each, independently of one another, denote

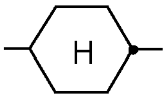 , 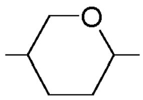 or 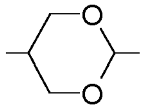

and

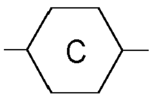

denotes

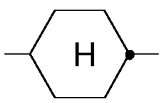 , 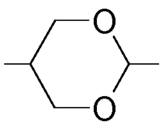 , 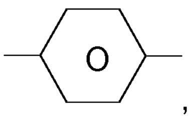 ,

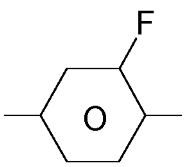 or 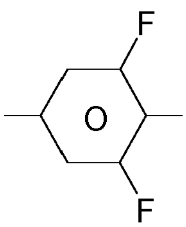

The compounds of the formulae XI and XII are preferably selected from the following formulae:

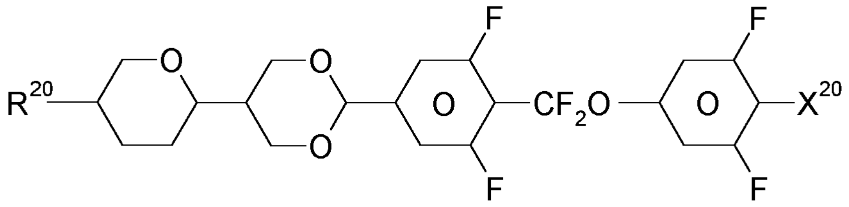

XIa

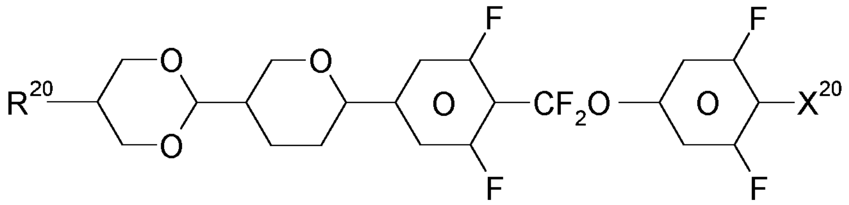

XIb

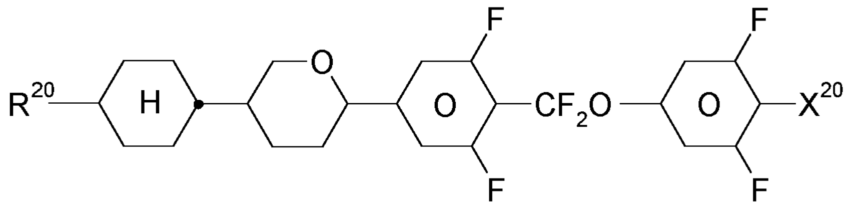

XIc

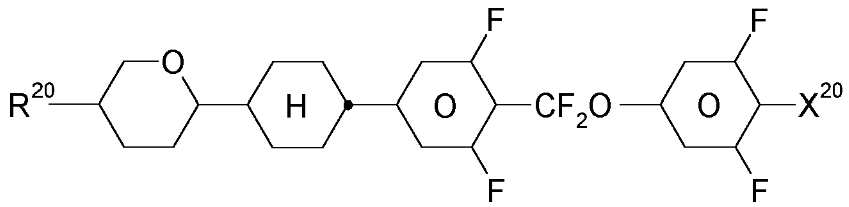

XId

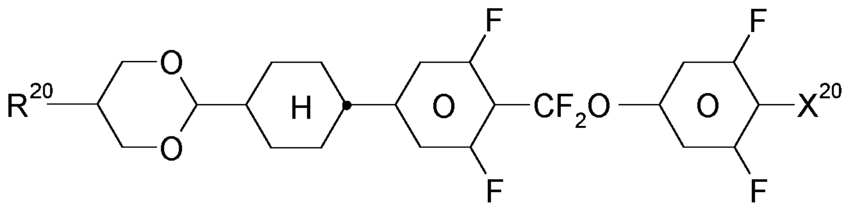

XIe

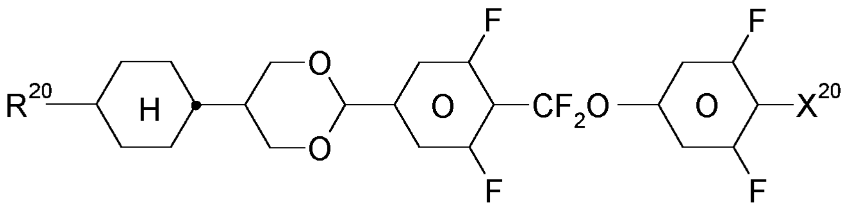

XIf

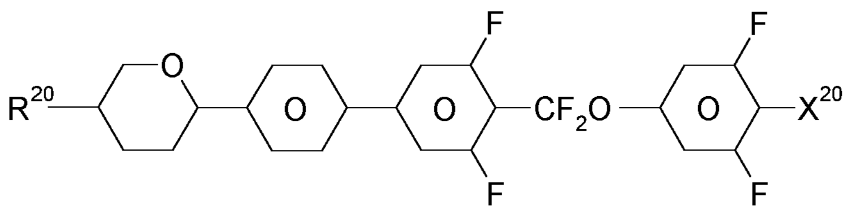

XIIa

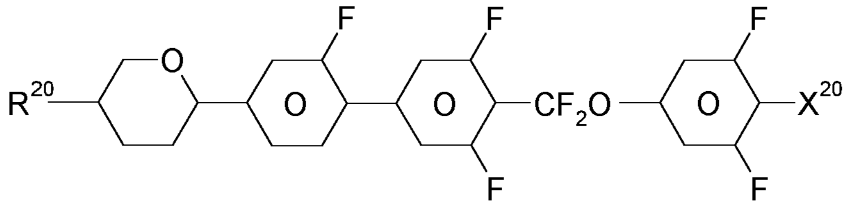
XIIb

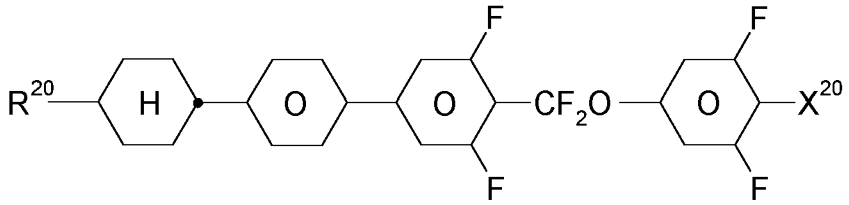
XIIc

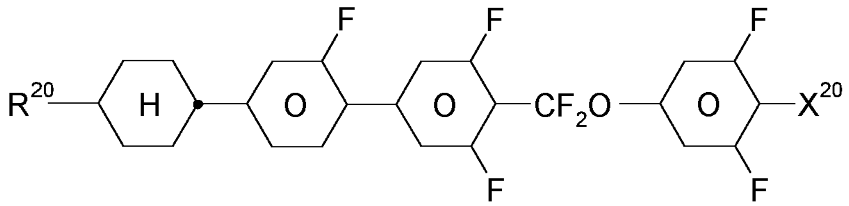
XIId

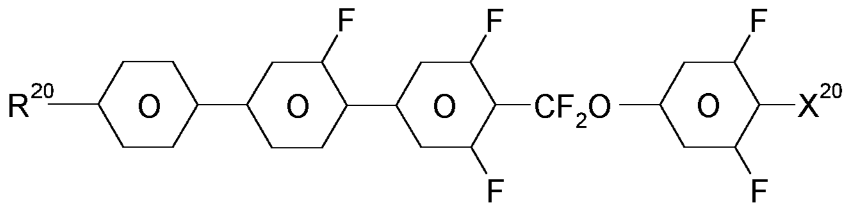
XIIe

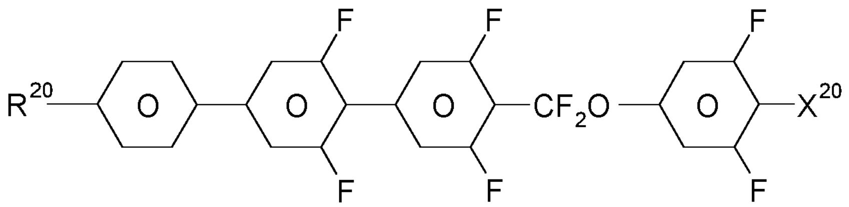
XIIf

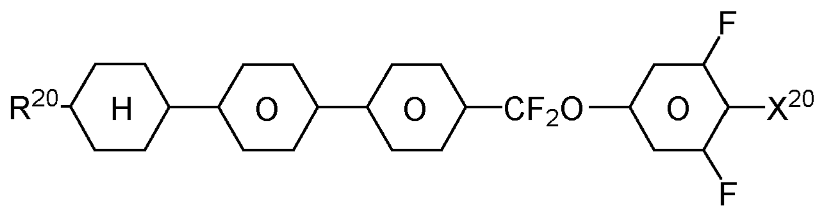
XIIg

wherein $R^{20}$ and $X^{20}$ have the meaning indicated above and preferably $R^{20}$ denotes alkyl having 1 to 6 C atoms and $X^{20}$ denotes F.

The mixture according to the invention particularly preferably comprises at least one compound of the formula XIIa and/or XIIe.

jj) The medium comprises one or more compounds of formula T given above, preferably selected from the group of compounds of the formulae T21 toT23 and T25 to T27.

Particular preference is given to the compounds of the formulae T21 to T23. Very particular preference is given to the compounds of the formulae

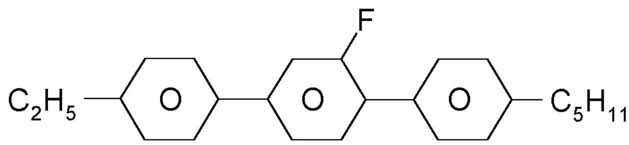

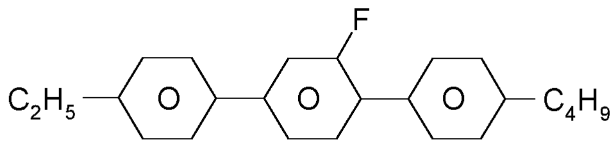

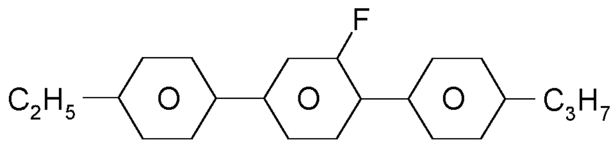

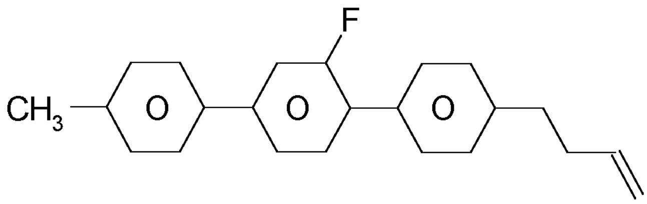

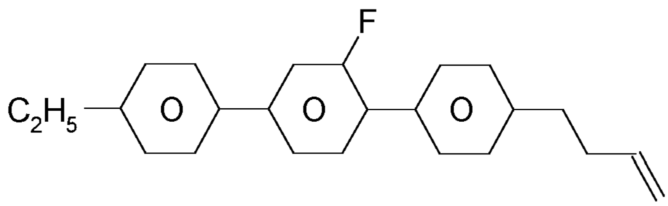

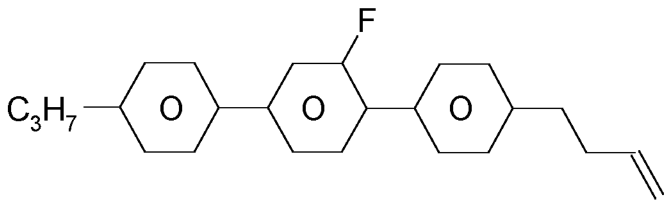

.

kk) The medium comprises one or more compounds selected from the group of formulae DK9, DK10 and DK11 given above.

ll) The medium additionally comprises one or more compounds selected from the following formulae:

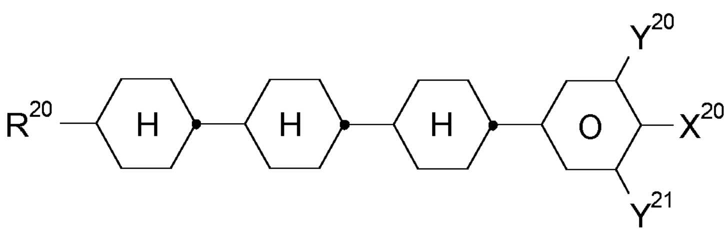

XIII

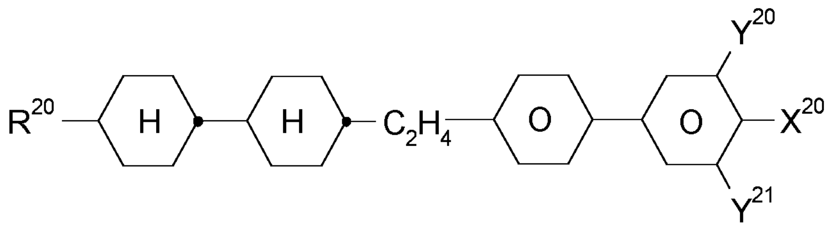

XIV

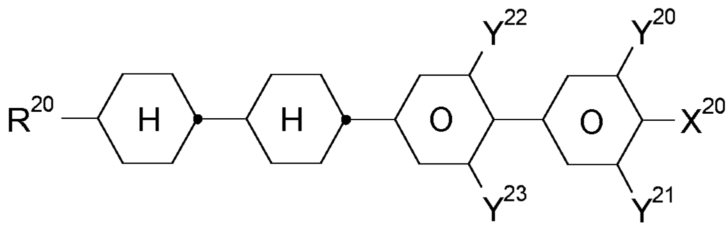

XV

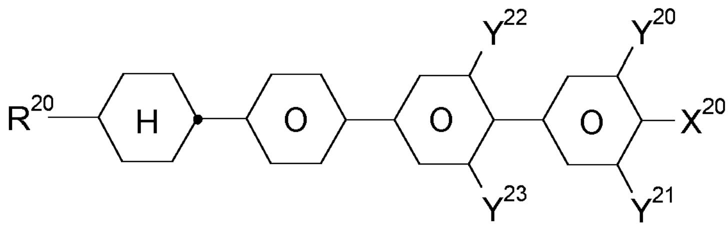

XVI

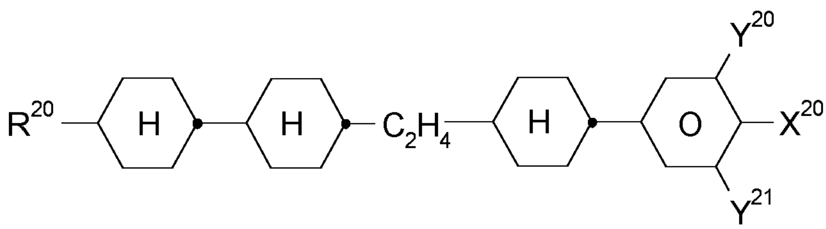

XVII

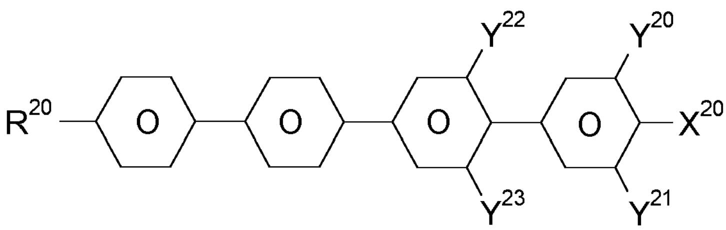

XVIII

wherein $R^{20}$ and $X^{20}$ each, independently of one another, have one of the meanings indicated above, and $Y^{20-23}$ each, independently of one another, denote H or F. $X^{20}$ is preferably F, Cl, $CF_3$, $OCF_3$ or $OCHF_2$. $R^{20}$ preferably denotes alkyl, alkoxy, oxaalkyl, fluoroalkyl or alkenyl, each having up to 6 C atoms.

The mixture according to the invention particularly preferably comprises one or more compounds of the formula XVIII-a,

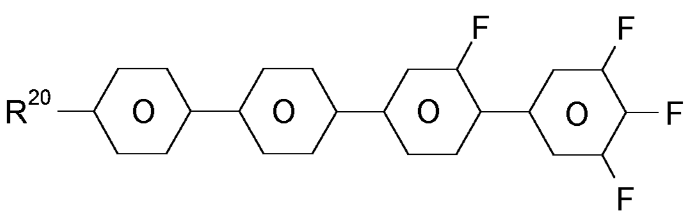

XVIII-a

wherein $R^{20}$ has the meanings indicated above. $R^{20}$ preferably denotes straight-chain alkyl, in particular ethyl, n-propyl, n-butyl and n-pentyl and very particularly preferably n-propyl. The compound(s) of the formula XVIII, in particular of the formula XVIII-a, is (are) preferably employed in the mixtures according to the invention in amounts of 0.5-20% by weight, particularly preferably 1-15% by weight.

mm) The medium additionally comprises one or more compounds of the formula XIX,

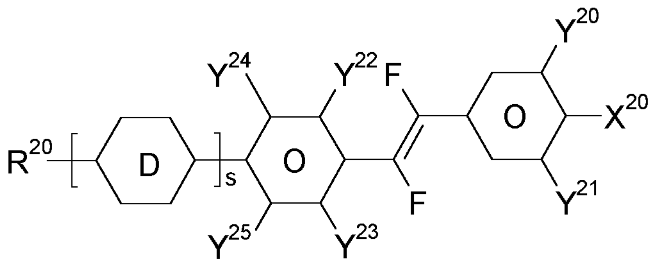

XIX

wherein $R^{20}$, $X^{20}$ and $Y^{20-25}$ have the meanings indicated in formula I, s denotes 0 or 1, and

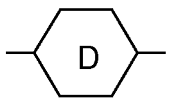

denotes

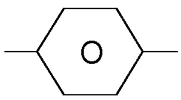 , 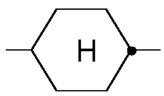 or 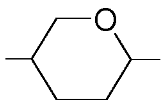 .

In the formula XIX, $X^{20}$ may also denote an alkyl radical having 1-6 C atoms or an alkoxy radical having 1-6 C atoms. The alkyl or alkoxy radical is preferably straight-chain.

$R^{20}$ preferably denotes alkyl having 1 to 6 C atoms. $X^{20}$ preferably denotes F;

- The compounds of the formula XIX are preferably selected from the following formulae:

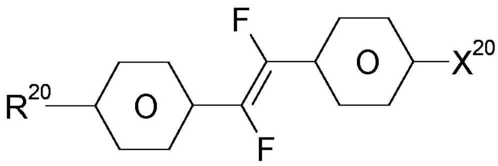

XIXa

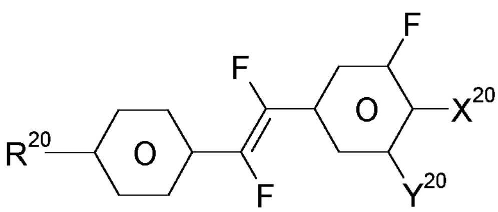

XIXb

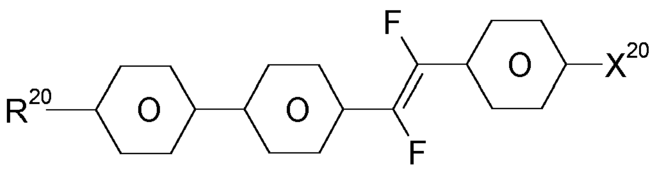

XIXc

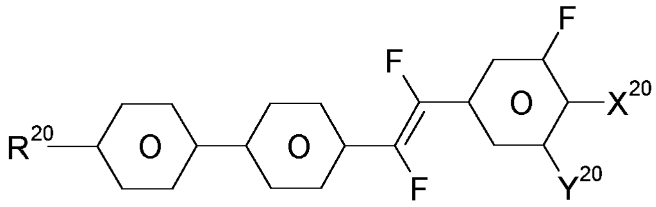

XIXd

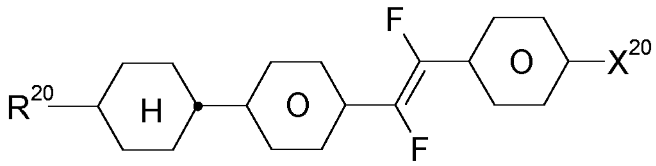

XIXe

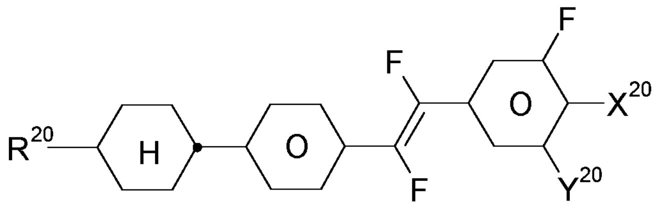

XIXf

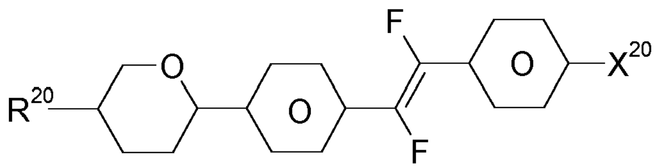

XIXg

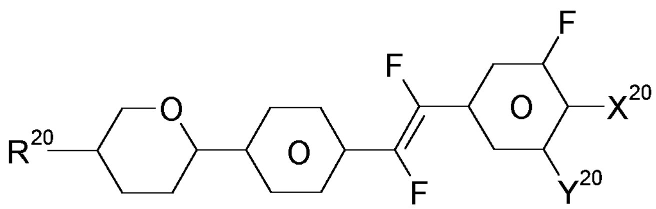

XIXh

wherein $R^{20}$, $X^{20}$ and $Y^{20}$ have the meanings indicated above. $R^{20}$ preferably denotes alkyl having 1 to 6 C atoms. $X^{20}$ preferably denotes F, and $Y^{20}$ is preferably F;

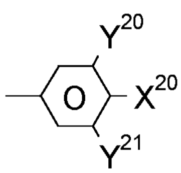

is preferably

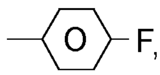, 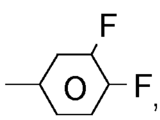, 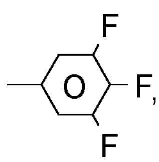,

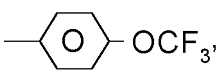, 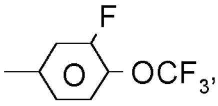, 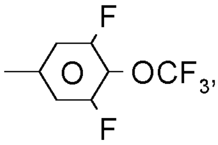, 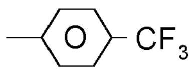,

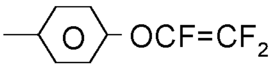, 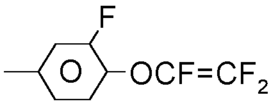, 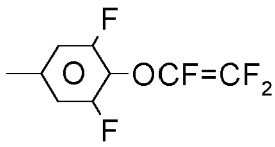,

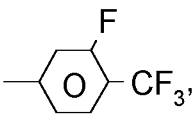, 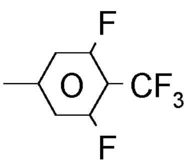, 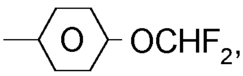, 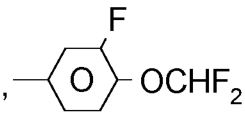,

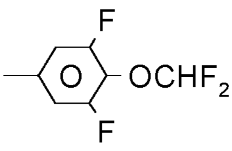, 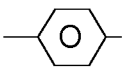, 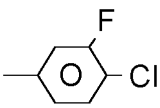 or 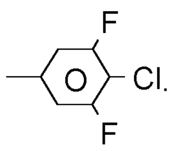.

- $R^{20}$ is straight-chain alkyl or alkenyl having 2 to 6 C atoms;

nn) The medium comprises one or more compounds of the formulae G1 to G4 given above, preferably selected from G1 and G2 wherein alkyl denotes $C_{1-6}$-alkyl, $L^x$ denotes H and X denotes F or Cl. In G2, X particularly preferably denotes Cl.

oo) The medium comprises one or more compounds of the following formulae:

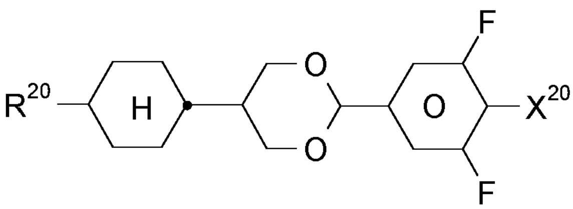

XX

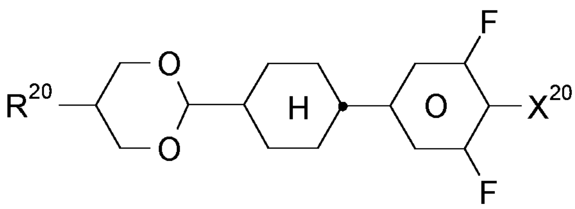

XXI

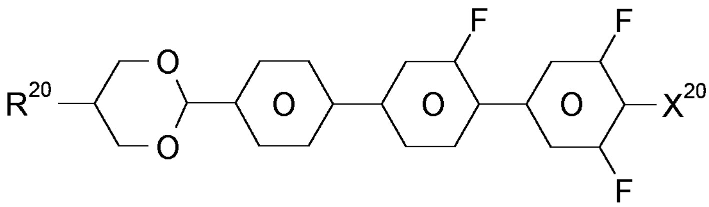

XXII

wherein $R^{20}$ and $X^{20}$ have the meanings indicated above. $R^{20}$ preferably denotes alkyl having 1 to 6 C atoms.

$X^{20}$ preferably denotes F. The medium according to the invention particularly preferably comprises one or more compounds of the formula XXII wherein $X^{20}$ preferably denotes F. The compound(s) of the formulae XX - XXII is (are) preferably employed in the mixtures according to the invention in amounts of 1-20% by weight, particularly preferably 1-15% by weight. Particularly preferred mixtures comprise at least one compound of the formula XXII.

pp) The medium comprises one or more compounds of the following pyrimidine or pyridine compounds of the formulae

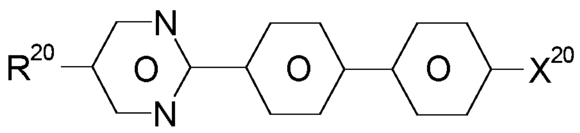

M-1

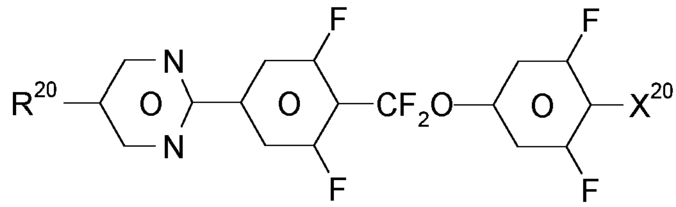

M-2

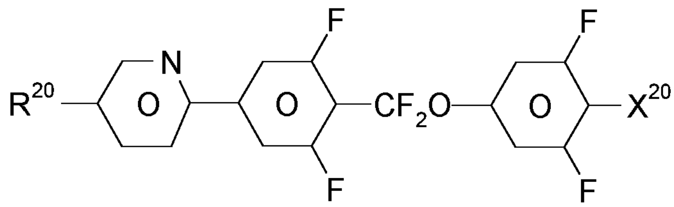

M-3

wherein $R^{20}$ and $X^{20}$ have the meanings indicated above. $R^{20}$ preferably denotes alkyl having 1 to 6 C atoms. $X^{20}$ preferably denotes F. The medium according to the invention particularly preferably comprises one or more compounds of the formula M-1, wherein $X^{20}$ preferably denotes F. The compound(s) of the formulae M-1 - M-3 is (are) preferably employed in the mixtures according to the invention in amounts of 1-20% by weight, particularly preferably 1-15% by weight.

Further preferred embodiments are indicated below:

qq) The medium comprises two or more compounds of the formula XII, in particular of the formula XIIe;

rr) The medium comprises 2-30% by weight, preferably 3-20% by weight, particularly preferably 3-15% by weight, of compounds of the formula XII;

ss) Besides the compounds of the formulae XII, the medium comprises further compounds selected from the group of the compounds of the formulae II, III, IX-XIII, XVII and XVIII;

tt) The proportion of compounds of the formulae II, III, IX-XI, XIII, XVII and XVIII in the mixture as a whole is 40 to 95% by weight;

uu) The medium comprises 10-50% by weight, particularly preferably 12-40% by weight, of compounds of the formulae II and/or III;

vv) The medium comprises 20-70% by weight, particularly preferably 25-65% by weight, of compounds of the formulae IX-XIII;

ww) The medium comprises 4-30% by weight, particularly preferably 5-20% by weight, of compounds of the formula XVII;

xx) The medium comprises 1-20% by weight, particularly preferably 2-15% by weight, of compounds of the formula XVIII;

yy) The medium comprises at least two compounds of the formulae

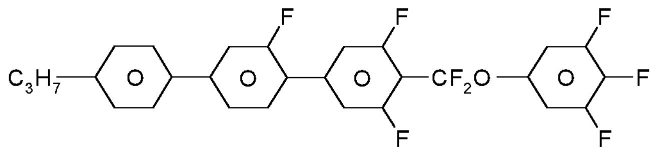
XIIe-1

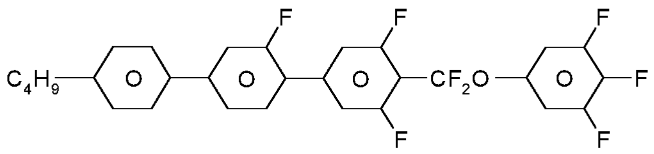
XIIe-2

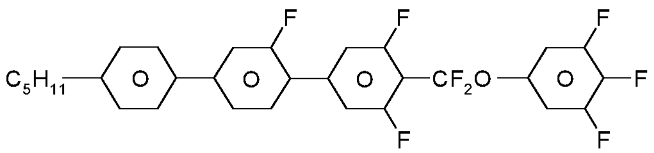
XIIe-3.

zz) The medium comprises at least two compounds of the formulae

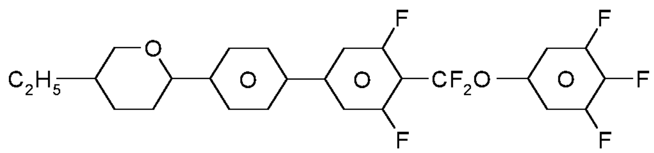
XIIa-1

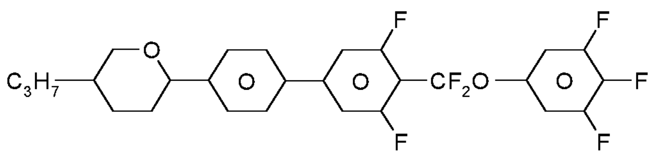
XIIa-2

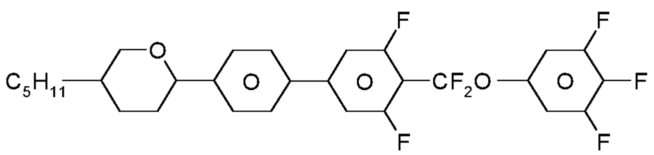
XIIa-3.

aaa) The medium comprises at least two compounds of the formula XIIa and at least two compounds of the formula XIIe.

bbb) The medium comprises at least one compound of the formula XIIa and at least one compound of the formula XIIe and at least one compound of the formula IIIa.

ccc) The medium comprises at least two compounds of the formula XIIa and at least two compounds of the formula XIIe and at least one compound of the formula IIIa.

ddd) The medium comprises in total ≥ 25% by weight, preferably ≥ 30% by weight, of one or more compounds of the formula XII.

eee) The medium comprises ≥ 20% by weight, preferably ≥ 24% by weight, preferably 25-60% by weight, of compounds of the formula ZK3, in particular the compound of the formula ZK3a,

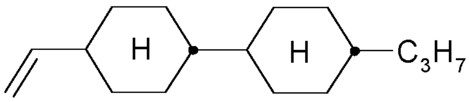

ZK3a

fff) The medium comprises at least one compound selected from the group of compounds ZK3a, ZK3b and ZK3c, preferably ZK3a, in combination with compound ZK3d

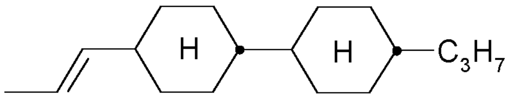

ZK3d

ggg) The medium comprises at least one compound of the formula DPGU-n-F.

hhh) The medium comprises at least one compound of the formula CDUQU-n-F.

iii) The medium comprises at least one compound of the formula CPU-n-OXF.

jjj) The medium comprises at least one compound of the formula CPGU-3-OT.

kkk) The medium comprises at least one compound of the formula PPGU-n-F.

lll) The medium comprises at least one compound of the formula PGP-n-m, preferably two or three compounds.

mmm) The medium comprises at least one compound of the formula PGP-2-2V having the structure

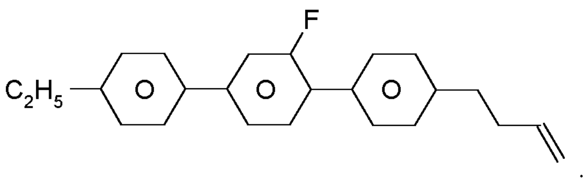

.

In a preferred embodiment, the liquid crystal mixture according to the present invention further comprises a polymerizable component C) comprising one or more polymerizable compounds.

The polymerizable compounds can be selected from isotropic or mesogenic polymerizable compounds known to the skilled person in the art.

Preferably, the polymerizable component C) comprises one or more polymerizable compounds of formula P,

$$P^a\text{-}(Sp^a)_{s1}\text{-}A^2\text{-}(Z^a\text{-}A^1)_{n2}\text{-}(Sp^b)_{s2}\text{-}P^b \qquad P$$

wherein the individual radicals have the following meanings:

| | |
|---|---|
| $P^a$, $P^b$ | each, independently of one another, denote a polymerizable group, |
| $Sp^a$, $Sp^b$ | on each occurrence, identically or differently, denote a spacer group, |
| s1, s2 | each, independently of one another, denote 0 or 1, |
| $A^1$, $A^2$ | each, independently of one another, denote a radical selected from the following groups: |
| a) | the group consisting of trans-1,4-cyclohexylene, 1,4-cyclohexenylene and 4,4'-bicyclohexylene, wherein, in addition, one or more non-adjacent $CH_2$ groups may be replaced by -O- and/or -S- and wherein, in addition, one or more H atoms may be replaced by F, |
| b) | the group consisting of 1,4-phenylene and 1,3-phenylene, wherein, in addition, one or two CH groups may be replaced by N and wherein, in addition, one or more H atoms may be replaced by L, |

c) the group consisting of tetrahydropyran-2,5-diyl, 1,3-dioxane-2,5-diyl, tetrahydrofuran-2,5-diyl, cyclobutane-1,3-diyl, piperidine-1,4-diyl, thiophene-2,5-diyl and selenophene-2,5-diyl, each of which may also be monoor polysubstituted by L,

d) the group consisting of saturated, partially unsaturated or fully unsaturated, and optionally substituted, polycyclic radicals having 5 to 20 cyclic C atoms, one or more of which may, in addition, be replaced by heteroatoms, preferably selected from the group consisting of

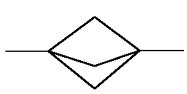 , 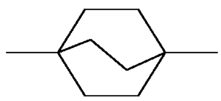 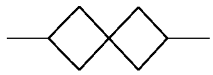 ,

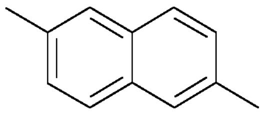 , 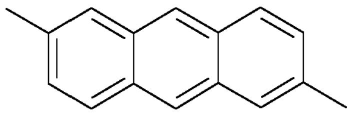 ,

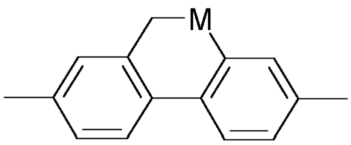 , 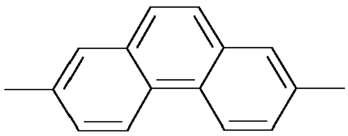 ,

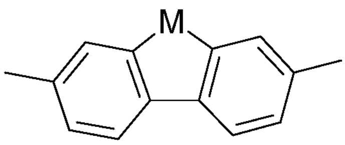 , 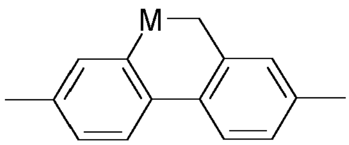 ,

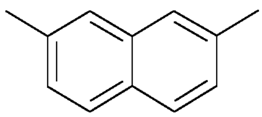 , 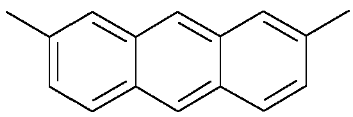 ,

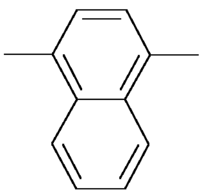 , 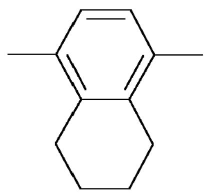 ,

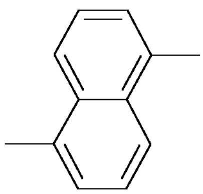 , 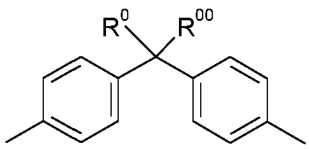

where, in addition, one or more H atoms in these radicals may be replaced by L, and/or one or more double bonds may be replaced by single bonds, and/or one or more CH groups may be replaced by N,

n2 denotes 0, 1, 2 or 3,

$Z^a$ in each case, independently of one another, denotes -CO-O-, -O-CO-, $-CH_2O-$, $-OCH_2-$, $-CF_2O-$,$-OCF_2-$, or $-(CH_2)_n-$, where n is 2, 3 or 4, -O-, -CO-, $-C(R^yR^z)-$, $-CH_2CF_2-$, $-CF_2CF_2-$ or a single bond,

L on each occurrence, identically or differently, denotes F, Cl, CN, SCN, $SF_5$ or straight-chain or branched, in each case optionally fluorinated, alkyl, alkoxy, alkylcarbonyl, alkoxycarbonyl, alkylcarbonyloxy or alkoxycarbonyloxy having 1 to 12 C atoms,

$R^y$, $R^z$ each, independently of one another, denote H, F or straight-chain or branched alkyl having 1 to 12 C atoms, wherein, in addition, one or more H atoms may be replaced by F,

M denotes -O-, -S-, $-CH_2-$, $-CHY^1-$ or $-CY^1Y^2-$, and

$Y^1$ and $Y^2$ each, independently of one another, have one of the meanings indicated above for $R^y$ or denote Cl or CN.

Preferred spacer groups $Sp^{a,b}$ are selected from the formula Sp"-X", so that the radicals P-Sp- and $P^{a/b}$-$Sp^{a/b}$- conforms to the formulae P-Sp"-X"- and $P^{a/b}$-Sp"-X"-, respectively, wherein

Sp" denotes alkylene having 1 to 20, preferably 1 to 12, C atoms, which is optionally mono- or polysubstituted by F, Cl, Br, I or CN and wherein, in addition, one or more nonadjacent $CH_2$ groups may each be replaced, independently of one another, by -O-, -S-, -NH-, -N(R°)-, $-Si(R^{00}R^{000})-$, -CO-, -CO-O-, -O-CO-, -O-CO-O-, -S-CO-, -CO-S-, $-N(R^{00})-CO-O-$, $-O-CO-N(R^{00})-$, $-N(R^{00})-CO-N(R^{00})-$, -CH=CH- or -C≡C- in such a way that O and/or S atoms are not linked directly to one another,

X" denotes -O-, -S-, -CO-, -CO-O-, -O-CO-, -O-CO-O-, $-CO-N(R^{00})-$, $-N(R^{00})-CO-$, $-N(R^{00})-CO-N(R^{00})-$, $-OCH_2-$, $-CH_2O-$, $-SCH_2-$, $-CH_2S-$, $-CF_2O-$, $-OCF_2-$, $-CF_2S-$, $-SCF_2-$, $-CF_2CH_2-$, $-CH_2CF_2-$, $-CF_2CF_2-$, -CH=N-, -N=CH-, -N=N-, $-CH=CR^0-$, $-CY^3=CY^4-$, -C≡C-, -CH=CH-CO-O-, -O-CO-CH=CH- or a single bond,

$R^0$, $R^{00}$ and $R^{000}$ each, independently of one another, denote H or alkyl having 1 to 12 C atoms, and

$Y^3$ and $Y^4$ each, identically or differently, denote H, F, Cl or CN.

X" is preferably -O-, -S-, -CO-, -C(O)O-, -OC(O)-, -O-C(O)O-, $-CO-NR^0-$, $-NR^0-CO-$, $-NR^0-CO-NR^0-$ or a single bond.

Typical spacer groups Sp" are, for example, $-(CH_2)_{p1}-$, $-(CH_2CH_2O)_{q1}-CH_2CH_2-$, $-CH_2CH_2-S-CH_2CH_2-$, $-CH_2CH_2-NH-CH_2CH_2-$ or $-(SiR^{00}R^{000}-O)_{p1}-$, wherein p1 is an integer from 1 to 12, q1 is an integer from 1 to 3, and $R^{00}$ and $R^{000}$ have the meanings indicated above.

Particularly preferred groups -Sp"-X"- are $-(CH_2)_{p1}-$, $-(CH_2)_{p1}-O-$, $-(CH_2)_{p1}-O-CO-$, $-(CH_2)_{p1}-O-CO-O-$, wherein p1 and q1 have the meanings indicated above.

Particularly preferred groups Sp" are, for example, in each case straight-chain ethylene, propylene, butylene, pentylene, hexylene, heptylene, octylene, nonylene, decylene, undecylene, dodecylene, octadecylene, ethyleneoxyethylene, methyleneoxybutylene, ethylenethioethylene, ethylene-N-methyliminoethylene, 1-methylalkylene, ethenylene, propenylene and butenylene.

Particularly preferred monomers of formula P are the following:

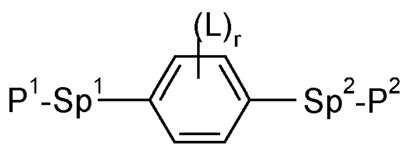

P1

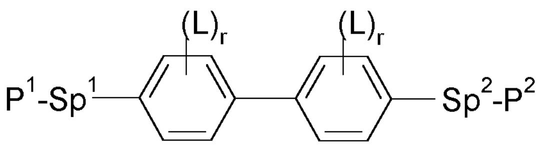

P2

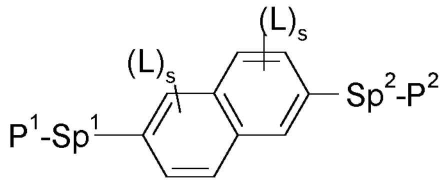
P3

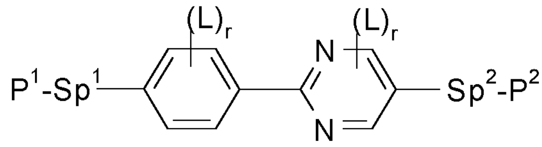
P4

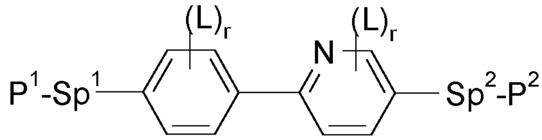
P5

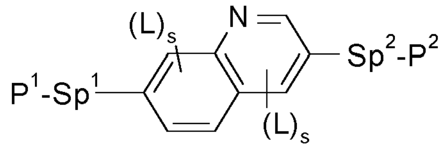
P6

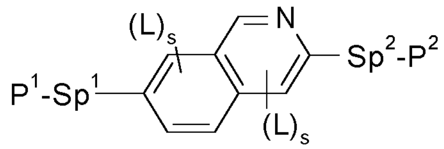
P7

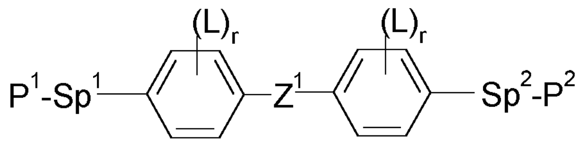
P8

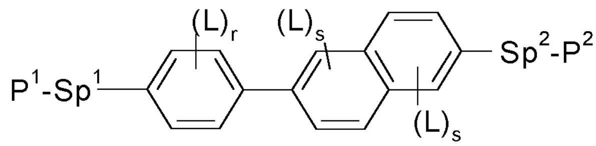
P9

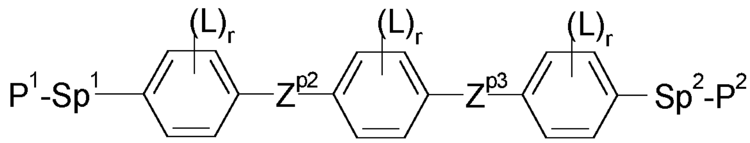
P10

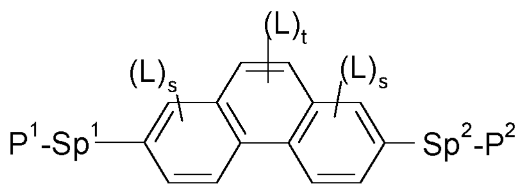
P11

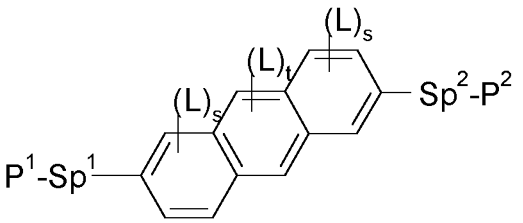

P12

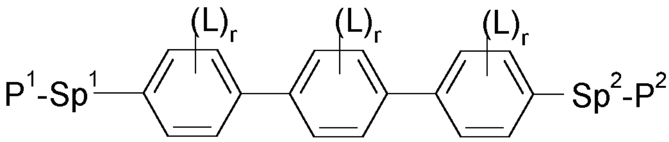

P13

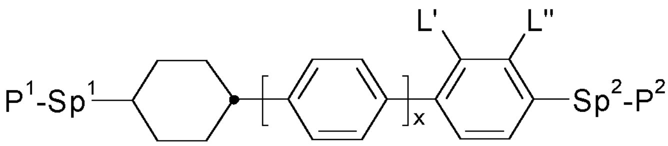

P14

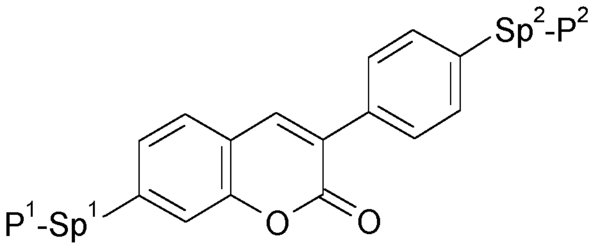

P15

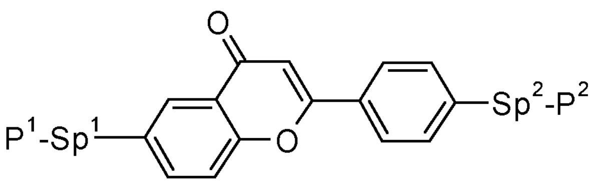

P16

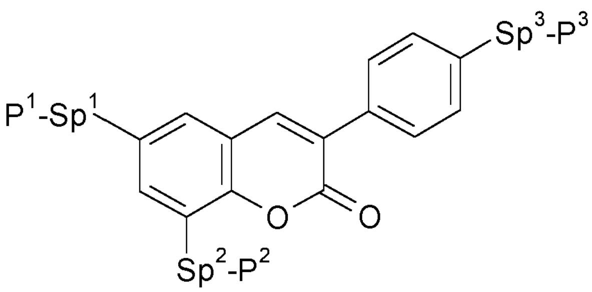

P17

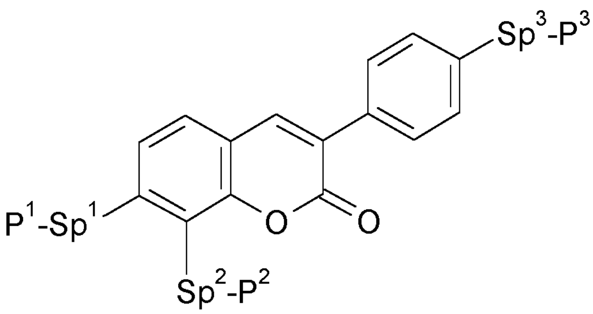

P18

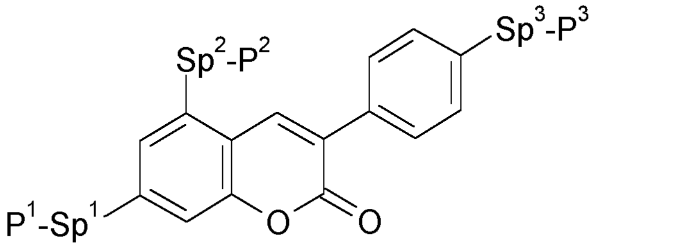

P19

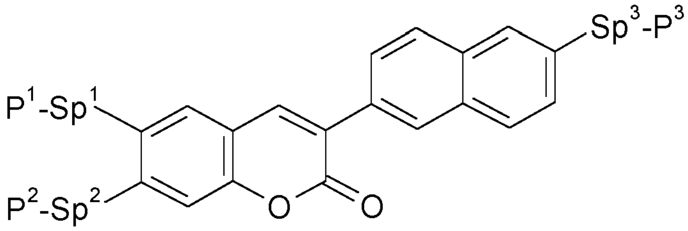

P20

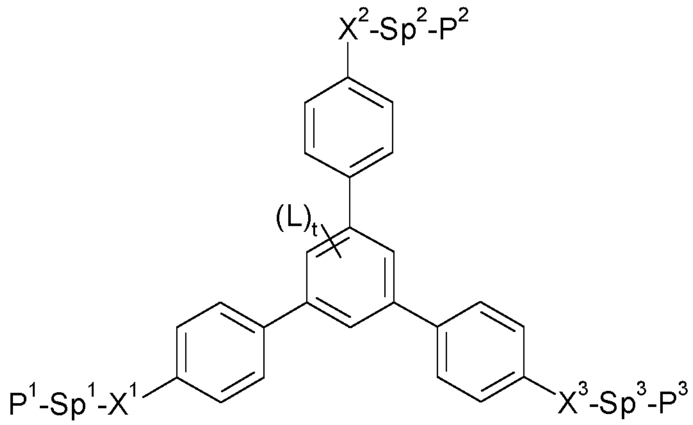

P21

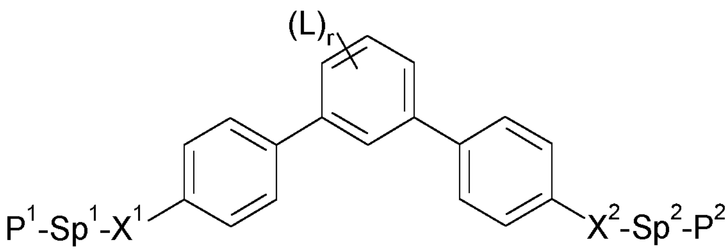

P22

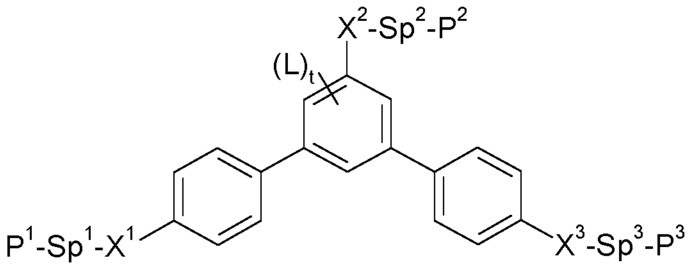

P23

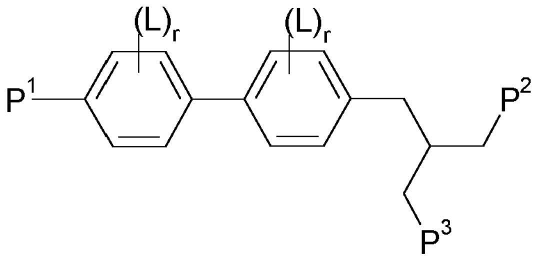

P24

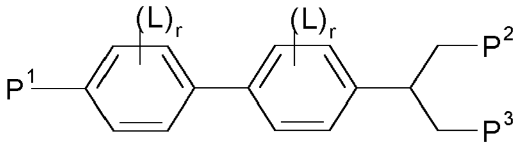

P25

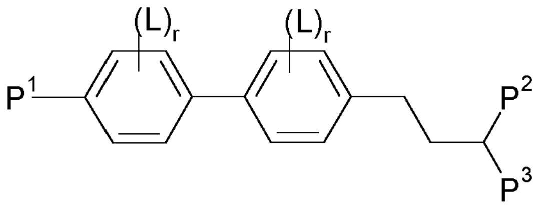

P26

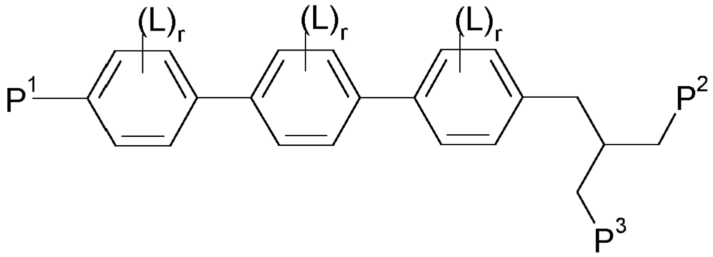

P27

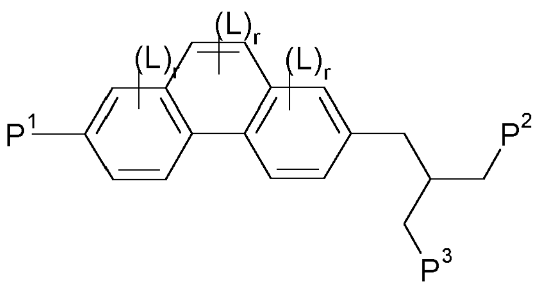

P28

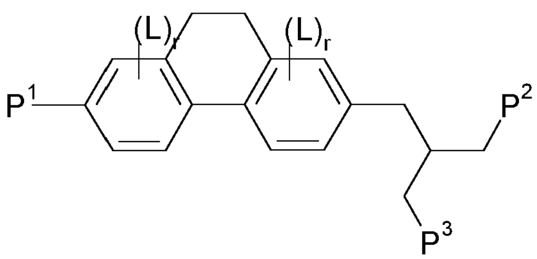

P29

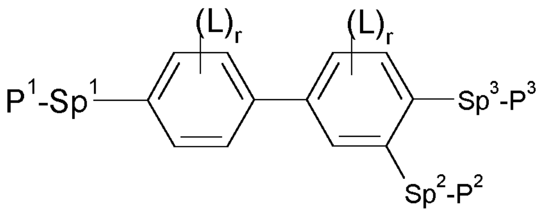

P30

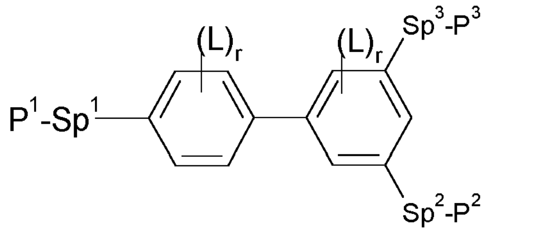

P31

wherein the individual radicals have the following meanings:

| | |
|---|---|
| $P^1$ to $P^3$ | each, independently of one another, denote a polymerizable group as defined for formula P, preferably an acrylate, methacrylate, fluoroacrylate, oxetane, vinyloxy or epoxide group, |
| $Sp^1$ to $Sp^3$ | each, independently of one another, denote a single bond or a spacer group, preferably having one of the meanings indicated above and below for $Sp^a$, and particularly preferably $-(CH_2)_{p1}-$, $-(CH_2)_{p1}-O-$, $-(CH_2)_{p1}-CO-O-$or $-(CH_2)_{p1}-O-CO-O-$, wherein p1 is an integer from 1 to 12, and where the linking to the adjacent ring in the lastmentioned groups takes place via the O atom, where, in addition, one or more of the radicals $P^1$-$Sp^1$- , $P^2$-$Sp^2$- and $P^3$-$Sp^3$- may denote a radical $R^{aa}$, with the proviso that at least one of the radicals $P^1$-$Sp^1$- , $P^2$-$Sp^2$- and $P^3$-$Sp^3$- present does not denote $R^{aa}$, |
| $R^{aa}$ | denotes H, F, Cl, CN or straight-chain or branched alkyl having 1 to 25 C atoms, wherein, in addition, one or more nonadjacent $CH_2$ groups may each be replaced, independently of one another, by $C(R^0)=C(R^{00})-$, $-C\equiv C-$, $-N(R^0)-$, -O-, -S-, -CO-, -CO-O-, -O-CO-, -O-CO-O- in such a way that O and/or S atoms are not linked directly to one another, and wherein, in addition, one or more H atoms may be replaced by F, Cl, CN or $P^1$-$Sp^1$-, particularly preferably straight-chain or branched, optionally mono- or polyfluorinated alkyl, alkoxy, alkenyl, alkynyl, alkylcarbonyl, alkoxycarbonyl or alkylcarbonyloxy having 1 to 12 C atoms (where the alkenyl and alkynyl radicals have at least two C atoms and the branched radicals have at least three C atoms), |
| $R^0$, $R^{00}$ | each, independently of one another, denote H or alkyl having 1 to 12 C atoms, |
| $R^y$ and $R^z$ | each, independently of one another, denote H, F, $CH_3$ or $CF_3$, |
| $Z^{p1}$ | denotes -O-, -CO-, $-C(R^yR^z)-$ or $-CF_2CF_2-$, |
| $Z^{p2}$ and $Z^{p3}$ | each, independently of one another, denote -CO-O-, -O-CO-, $-CH_2O-$, $-OCH_2-$, $-CF_2O-$, $-OCF_2-$ or $-(CH_2)_{n3}-$, where n3 is 2, 3 or 4, |
| L | on each occurrence, identically or differently, denotes F, Cl, CN, SCN, $SF_5$ or straight-chain or branched, optionally mono- or polyfluorinated alkyl, alkoxy, alkenyl, alkynyl, alkylcarbonyl, alkoxycarbonyl, alkylcarbonyloxy or alkoxycarbonyloxy having 1 to 12 C atoms, preferably F, |
| L' and L" | each, independently of one another, denote H, F or Cl, |
| r | denotes 0, 1, 2, 3 or 4, |
| s | denotes 0, 1, 2 or 3, |
| t | denotes 0, 1 or 2, and |

x denotes 0 or 1.

In a particularly preferred embodiment of the present invention the LC mixture, or component C), comprises one or more compounds of formula P10-1.

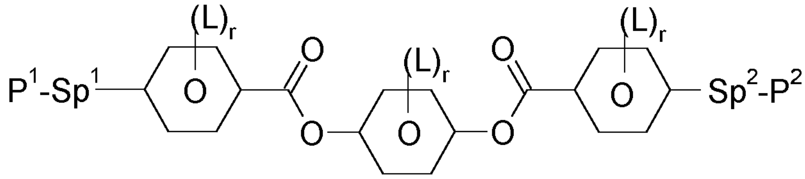

P10-1

wherein the parameters are defined as described above and $P^1$ and $P^2$ preferably denote acrylate or methacrylate.

Particularly preferred compounds of formula P10-1 are selected from the group of the following subformulae

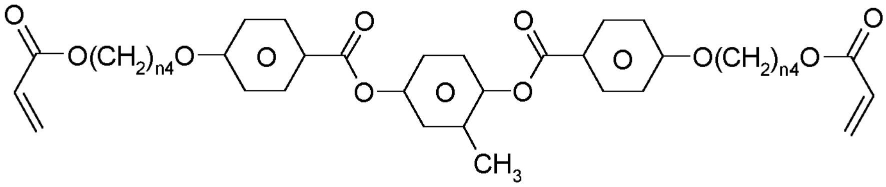

P10-1-1

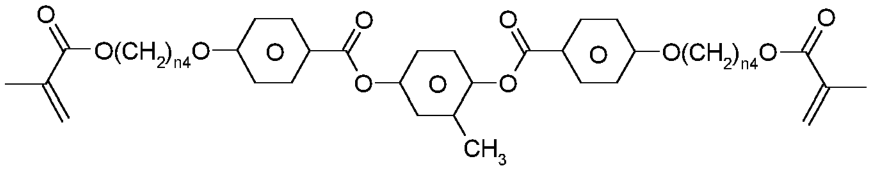

P10-1-2

wherein each n4 denote independently of each other an integer between 2 and 10, preferably 3,4,5 or 6.

In another preferred embodiment, the polymerizable component C) comprises one or more polymerizable compounds of formula CC,

$P^a$-$Sp^a$-$(A^p)_{n2}$-$Sp^b$-$P^b$ CC

wherein the individual radicals have the following meanings:

$P^a$, $P^b$ each, independently of one another, denote a polymerizable group, preferably each and idependently selected from the group consisting of acrylate, methacrylate, ethacrylate, fluoroacrylate, vinyl¬oxy, chloro¬acry-late, oxetane, or epoxide groups

$Sp^a$, $Sp^b$ on each occurrence, identically or differently, denote a spacer group or a single bond,

$A^p$ each and idependently from another, in each occurrence,

a group selected from 5, 6 or 7-membered alicyclic groups wherein, in addition, one or more non-adjacent $CH_2$ groups may be replaced by -NH-, -O- and/or -S-, wherein one or more non-adjacent -$CH_2$-$CH_2$- groups may be replaced by -CH=CH-, and wherein one or more H atoms may be replaced by F, preferably 5-membered groups such as cyclopentane, cyclopentane, tetrahydrofuran, tetrahydrothiofuran, pyrrolidine, or 6-membered groups, such as cyclohexane, silinane, cyclohexene, tetrahydropyran, tetrahydrothiopyran, 1,3-dioxane, 1,3-dithiane, piperidine, or 7-membered groups, such as cycloheptane, trans-1,4-cyclohexylene, more preferably 1,4-cyclohexylene or 1,4-cyclohexenylene,

n2 denotes 0, 1, 2 or 3, preferably 1 or 2.

Preferred spacer groups $Sp^{a,b}$ are selected from the formula Sp"-X", so that the radicals P-Sp- and $P^{a/b}$-$Sp^{a/b}$- conforms to the formulae P-Sp"-X"-and $P^{a/b}$-Sp"-X"-, respectively, wherein

Sp" denotes alkylene having 1 to 20, preferably 1 to 12, C atoms, which is optionally mono- or polysubstituted by F, Cl, Br, I or CN and wherein, in addition, one or more non-adjacent $CH_2$ groups may each be replaced, independently of one another, by -O-, -S-, -NH-, -N(R°)-, -Si($R^{00}R^{000}$)-, -CO-,

-CO-O-, -O-CO-, -O-CO-O-, -S-CO-, -CO-S-, -N($R^{00}$)-CO-O-, -O-CO-N($R^{00}$)-, -N($R^{00}$)-CO-N($R^{00}$)-, -CH=CH- or -C=C- in such a way that O and/or S atoms are not linked directly to one another, or a single bond,

X" denotes -O-, -S-, -CO-, -CO-O-, -O-CO-, -O-CO-O-, -CO-N($R^{00}$)-, -N($R^{00}$)-CO-, -N($R^{00}$)-CO-N($R^{00}$)-, $-OCH_2-$, $-CH_2O-$, $-SCH_2-$, $-CH_2S-$, $-CF_2O-$, $-OCF_2-$, $-CF_2S-$, $-SCF_2-$, $-CF_2CH_2-$, $-CH_2CF_2-$, $-CF_2CF_2-$, -CH=N-, -N=CH-, -N=N-, -CH=CR$^0$-, -CY$^3$=CY$^4$-, -C≡C-, -CH=CH-CO-O-, -O-CO-CH=CH- or a single bond, preferably a singlebond

$R^0$, $R^{00}$ and $R^{000}$ each, independently of one another, denote H or alkyl having 1 to 12 C atoms, and

$Y^3$ and $Y^4$ each, identically or differently, denote H, F, Cl or CN.

X" is preferably -O-, -S-, -CO-, -C(O)O-, -OC(O)-, -O-C(O)O-, -CO-NR$^0$-, -NR$^0$-CO-, -NR$^0$-CO-NR$^0$- or a single bond.

Typical spacer groups Sp" are, for example, a single bon, $-(CH_2)_{p1}-$, $-(CH_2CH_2O)_{q1}-CH_2CH_2-$, $-CH_2CH_2-S-CH_2CH_2-$, $-CH_2CH_2-NH-CH_2CH_2-$ or $-(SiR^{00}R^{000}-O)_{p1}-$, wherein p1 is an integer from 1 to 12, q1 is an integer from 1 to 3, and $R^{00}$ and $R^{000}$ have the meanings indicated above.

Particularly preferred groups -Sp"-X"- are a single bond, $-(CH_2)_{p1}-$, $-(CH_2)_{p1}-O-$, $-(CH_2)_{p1}-O-CO-$, $-(CH_2)_{p1}-O-CO-O-$, wherein p1 and q1 have the meanings indicated above.

Particularly preferred groups Sp" are, for example, in each case straight-chain methylene, ethylene, propylene, butylene, pentylene, hexylene, heptylene, octylene, nonylene, decylene, undecylene, dodecylene, octadecylene, ethyleneoxyethylene, methyleneoxybutylene, ethylenethioethylene, ethylene-N-methyliminoethylene, 1-methylalkylene, ethenylene, propenylene and butenylene.

Particularly preferred monomers of formula CC are the following:

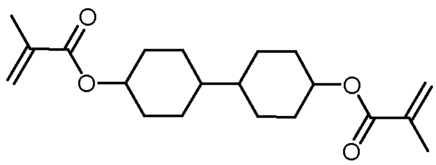

CC-1

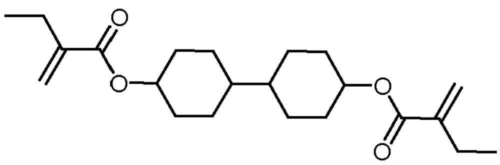

CC-2

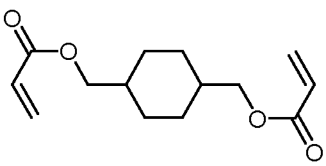

CC-3

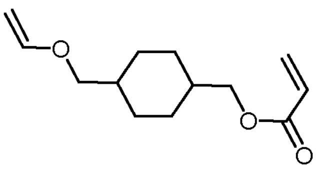

CC-4

In another preferred embodiment, the polymerizable component C) in accordance with the present invention may comprise, preferably consists of, one or more compounds of formula Palk,

P"-Sp'-P" Palk

wherein the individual radicals have the meanings :

P", P" each, independently of one another, denote a polymerizable group as given in formula CC,

Sp' denotes a spacer group,

Preferred spacer groups Spare selected from the formula -X"-Sp"-X"-

Sp" denotes alkylene having 1 to 25, preferably 1 to 20, C atoms, which is optionally mono- or polysubstituted by F, Cl, Br, I, or CN and wherein, in addition, one or more non-adjacent $CH_2$ groups may each be replaced, independently of one another, by -O-, -S-, -NH-, -N($R^0$)-, -Si($R^{00}R^{000}$)-, -CO-, -CO-O-, -O-CO-, -O-CO-O-, -S-CO-, -CO-S-, -N($R^{00}$)-CO-O-, -O-CO-N($R^{00}$)-, -N($R^{00}$)-CO-N($R^{00}$)-, -CH=CH- or -C≡C- in such a way that O and/or S atoms are not linked directly to one another,

X" denotes each and idependently -O-, -S-, -CO-, -CO-O-, -O-CO-, -O-CO-O-, -CO-N($R^{00}$)-, -N($R^{00}$)-CO-, -N($R^{00}$)-CO-N($R^{00}$)-, -$OCH_2$-, -$CH_2O$-, -$SCH_2$-, -$CH_2S$-, -$CF_2O$-, -$OCF_2$-, -$CF_2S$-, -$SCF_2$-, -$CF_2CH_2$-, -$CH_2CF_2$-, -$CF_2CF_2$-, -CH=N-, -N=CH-, -N=N-, -CH=C$R^0$-, -C$Y^3$=C$Y^4$-, -C≡C-, -CH=CH-CO-O-, -O-CO-CH=CH- or a single bond,

$R^0$, $R^{00}$ and $R^{000}$ each, independently of one another, denote H or alkyl having 1 to 12 C atoms, and

$Y^3$ and $Y^4$ each, identically or differently, denote H, F, Cl or CN.

X" is preferably -O-, -S-, -CO-, -C(O)O-, -OC(O)-, -O-C(O)O-, -CO-N$R^0$-, -N$R^0$-CO-, -N$R^0$-CO-N$R^0$- or a single bond.

Typical spacer groups Sp" are, for example, -$(CH_2)_{p1}$-, -$(CH_2CH_2O)_{q1}$-$CH_2CH_2$-, -$CH_2CH_2$-S-$CH_2CH_2$-, -$CH_2CH_2$-NH-$CH_2CH_2$- or -(Si$R^{00}R^{000}$-O)$_{p1}$-, wherein p1 and q1 are integers from 1 to 20, and $R^{00}$ and $R^{000}$ have the meanings indicated above.

Particularly preferred groups X"-Sp"-X"- are -$(CH_2)_{p1}$-, -$(CH_2CH_2O)_{q1}$-$CH_2CH_2$- -$(CH_2)_{p1}$-O-, -$(CH_2)_{p1}$-O-CO-, -$(CH_2)_{p1}$-O-CO-O-, -O-$(CH_2)_{p1}$-O-, -O-CO-$(CH_2)_{p1}$-O-CO-, -O-CO-O-$(CH_2)_{p1}$-O-CD-O-, -O-$(CH_2)_{p1}$-, -O-CO-$(CH_2)_{p1}$-, -O-CO-O-$(CH_2)_{p1}$-, wherein p1 and q1 have the meanings indicated above.

Particularly preferred groups Sp" are, for example, in each case straight-chain ethylene, propylene, butylene, pentylene, hexylene, heptylene, octylene, nonylene, decylene, undecylene, dodecylene, octadecylene, ethyleneoxyethylene, methyleneoxybutylene, ethylenethioethylene, ethylene-N-methyliminoethylene, 1-methylalkylene, ethenylene, propenylene and butenylene,

furthermore -$(CH_2CH_2O)_{q1}$-$(CH_2CH_2)_{p1}$-, -$(CH_2CH_2)_{p1}$-$(OCH_2CH_2)_{q1}$-, or -$(CH_2CH_2)_{p1}$-$(CH_2CH_2O)_{q1}$-$(CH_2CH_2)r_1$ wherein p1, q1 and r1 each and independently denotes 1, 2, 3, 4, 5, 6, 7, 8, 9, 10, 11 or 12.

Particularly preferred monomers of formula P are the following:

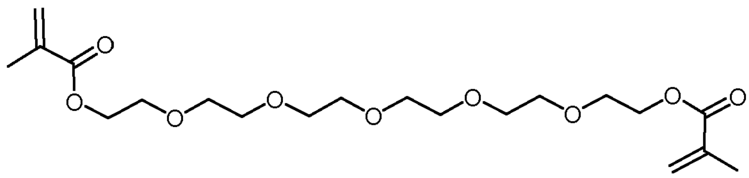

P-1

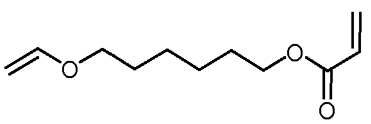

P-2

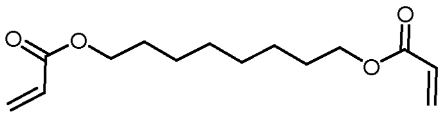

P-3

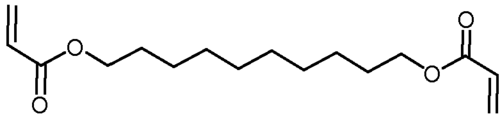

P-4

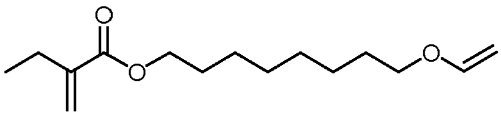

P-5

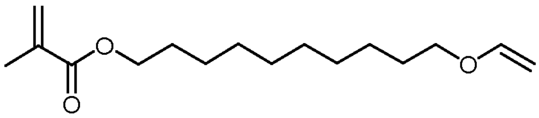

P-6

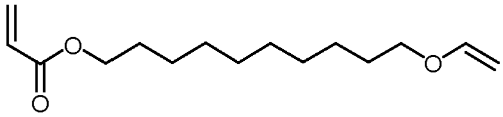

P-7

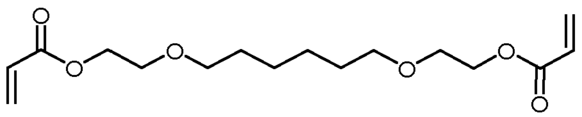

P-8

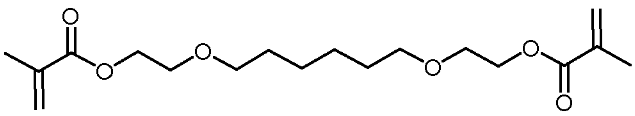

P-9

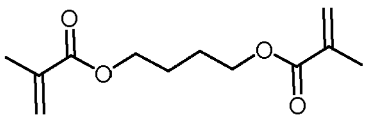

P-10

The polymerizable compounds of formulae I, P, CC and Palk are also suitable for polymerisation without an initiator, which is associated with considerable advantages, such as, for example, lower material costs and, in particular, reduced contamination of the LC medium by possible residual amounts of the initiator or degradation products thereof. The polymerisation can thus also be carried out without addition of an initiator. The LC medium thus, in a preferred embodiment, comprises no polymerisation initiator.

The polymerizable component C) or the LC medium as a whole may also comprise one or more stabilisers in order to prevent undesired spontaneous polymerisation of the RMs, for example during storage or transport. Suitable types and amounts of stabilisers are known to the person skilled in the art and are described in the literature. Particularly suitable are, for example, the commercially available stabilisers from the Irganox® series (BASF SE), such as, for example, Irganox® 1076. If stabilisers are employed, their proportion, based on the total amount of the RMs or the polymerizable component, is preferably 10 - 10,000 ppm, particularly preferably 50 - 1000 ppm.

The media according to the invention preferably comprise from 0.01 to 10%, particularly preferably from 0.05 to 7.5% and most preferably from 0.1 to 5% of the compounds of component C) comprising compounds of formula P according to the invention. The media preferably comprise one, two or three, more preferably one or two and most preferably one compound of the formula P according to the invention.

By means of suitable additives, the liquid-crystalline phases of the present invention can be modified in such a way that they can be used in all types of liquid-crystal display element that have been disclosed hitherto. Additives of this type are known to the person skilled in the art and are described in detail in the literature (H. Kelker/ R. Hatz,

Handbook of Liquid Crystals, Verlag Chemie, Weinheim, 1980). For example, pleochroic dyes can be added for the production of coloured guest-host systems or substances can be added in order to modify the dielectric anisotropy, the viscosity and/or the alignment of the nematic phases.

The media according to the invention are prepared in a manner conventional per se. In general, the components are dissolved in one another, preferably at elevated temperature.

Accordingly the present invention relates further to method for the production of an LC medium according to the present invention, comprising the step of mixing one or more compounds of formula I with a liquid-crystalline component B) comprising one or more mesogenic or liquid-crystalline compounds as described above.

The present invention further relates to a process for the fabrication of liquid crystal displays comprising at least the steps of:

- providing a first substrate which includes a pixel electrode and a common electrode for generating an electric field substantially parallel to a surface of the first substrate in the pixel region;
- providing a second substrate, the second substrate being disposed opposite to the first substrate;
- interposing a liquid crystal mixture between the first substrate and the second substrate, the liquid crystal mixture comprising one or more compounds of formula I, component B) and optionally component C);
- irradiating the liquid crystal mixture with linearly polarised light causing photoalignment of the liquid crystal;
- curing the polymerizable compounds of the liquid crystal mixture by irradiation with ultraviolet light or visible light having a wavelength of 450nm or below.

The present invention further relates to the use of the liquid crystal mixtures according to the invention for the fabrication of a liquid crystal display.

The present invention further relates to liquid crystal displays fabricated by the process described above.

In the following, the production process according to the present invention is described in greater detail.

The first substrate includes a pixel electrode and a common electrode for generating an electric field substantially parallel to a surface of the first substrate in the pixel region. Various kinds of displays having at least two electrodes on one substrate are known to the skilled person wherein the most significant difference is that either both the pixel electrode and the common electrode are structured, as it is typical for IPS displays, or only the pixel electrode is structured and the common electrode is unstructured, which is the case for FFS displays.

It has to be understood that the present invention refers to any kind of electrode configurations suitable for generating an electric field substantially parallel to a surface of the first substrate in the pixel region; mentioned above, i.e. IPS as well as FFS displays.

The process according to the present invention is independent of the kind of substrate or material of the surface which is in contact with the liquid crystal mixture according to the invention, during and after this process. Examples of materials used for the substrates or surfaces are organic polymers including polyimide, indium tin oxide (ITO), indium zinc oxide (IZO), silicon nitride ($SiN_x$) and silicon dioxide($SiO_2$). The process is especially suitable for the use in displays containing substrates that do not have a polyimide layer on one or more of the surfaces that are in contact with the liquid crystal.

In case one or more substrates contain a polyimide layer, the polyimide can be rubbed or not rubbed, preferably not rubbed.

Hence, the invention relates to a display produced by the process according to the invention in which the substrates contain a rubbed or unrubbed polyimide layer, preferably an unrubbed polyimide layer.

The invention further relates to a display produced by the process according to the invention in which none or only one of the top and bottom substrates contains a polyimide layer.

In one embodiment of the present invention the liquid crystal composition is injected between the first and second substrates or is filled into the cell by capillary force after combining the first and second substrates. In an alternative embodiment, the liquid crystal composition may be interposed between the first and second substrates by combining the second substrate to the first substrate after loading the liquid crystal composition on the first substrate. Preferably, the liquid crystal is dispensed dropwise onto a first substrate in a process known as "one drop filling" (ODF) process, as disclosed in for example JPS63-179323 and JPH10-239694, or using the Ink Jet Printing (IJP) method.

In a preferred embodiment, the process according to the invention contains a process step where the liquid crystal inside the display panel is allowed to rest for a period of time in order to evenly redistribute the liquid crystal medium inside the panel (herein referred to as "annealing").

However it is likewise preferred that the annealing step is combined with a previous step, such as edge sealant pre-curing. In which case a 'separate' annealing step may not be necessary at all.

For the production of the displays according to the present invention, the photoreactive mesogen of formula I is preferably allowed to redistribute in the panel. After filling and assembly, the display panel is annealed for a time between 1 min and 3h, preferably between 2 min and 1h and most preferably between 5 min and 30 min. The annealing

is preferably performed at room temperature.

In an alternative embodiment, the annealing is performed at elevated temperature, preferably at above 20°C and below 140°C, more preferably above 40°C and below 100°C and most preferably above 50°C and below 80°C.

Preferably the annealing is performed at elevated temperature, preferably 30°C above the clearing point of the utilized host mixture, more preferably 20°C above the clearing point of the utilized host mixture, and most preferably 5 to 15°C above the clearing point of the utilized host mixture.

In a preferred embodiment, one or more of the process steps of filling the display, annealing, photoalignment and curing of the polymerizable compound is performed at a temperature above the clearing point of the liquid crystal host mixture.

During the photoalignment of the liquid crystal inside the liquid crystal panel, anisotropy is induced by exposing the display or the liquid crystal layer to linearly polarised light.

In a preferred embodiment of the present invention the photoreactive component A) comprising one or more compounds of formula I, is photoaligned in a first step using linearly polarised light and in a second step further cured using linearly polarized or unpolarised UV light. In the second step the optional component C) is also further cured.

In another preferred embodiment, the linearly polarised light applied according to the inventive process is ultraviolet light which enables simultaneous photoalignment and photocuring of the photoreactive component A) comprising one or more compounds of formula I, and, if present, photocuring of the polymerizable component C).

Photoalignment of the photoreactive compounds of formula I and curing of the polymerizable groups of compounds of formula I and the curing of the optional polymerizable compounds of formula P can be performed simultaneously or stepwise. In case the process is split into different steps, the individual steps can be performed at the same temperature or at different temperatures.

After the photoalignment and curing step(s) a so-called "post-curing" step can optionally be performed by irradiation with UV-light and/or visible light (both either linearly or unpolarised) at reduced temperature in order to remove unreacted polymerizable compounds. The post-curing is preferably performed at above 0°C and below the clearing point of the utilized LC mixture, preferably 20°C and below 60°C°C, and most preferably above 20°C and below 40°C.

The polymerizable compounds are optionally polymerised or crosslinked (if a polymerizable compound contains two or more polymerizable groups) with the application of an electrical field. The polymerisation can be carried out in one or more steps.

Suitable and preferred polymerisation methods for component C) are, for example, thermal or photopolymerization, preferably photopolymerization, in particular UV photopolymerization. One or more initiators can optionally also be added here. Suitable conditions for the polymerisation and suitable types and amounts of initiators are known to the person skilled in the art and are described in the literature. Suitable for free-radical polymerisation are, for example, the commercially available photoinitiators Irgacure651®, Irgacure184®, Irgacure907®, Irgacure369® or Darocure1 1730 (BASF SE). If an initiator is employed, its proportion is preferably 0.001 to 5% by weight, particularly preferably 0.001 to 1% by weight.

The present invention also relates to electro-optical liquid-crystal display elements containing a liquid-crystalline medium according to the invention, which is preferably homogeneously aligned. In a preferred embodiment the liquid crystal display is of the IPS or FFS mode.

Further combinations of the embodiments and variants of the invention in accordance with the description arise from the claims.

The invention is explained in greater detail below with reference to working examples, but without intending to be restricted thereby. The person skilled in the art will be able to glean from the examples working details that are not given in detail in the general description, generalise them in accordance with general expert knowledge and apply them to a specific problem.

Besides the usual and well-known abbreviations, the following abbreviations are used:
C: crystalline phase; N: nematic phase; Sm: smectic phase; I: isotropic phase. The numbers between these symbols show the transition temperatures of the substance concerned.

Temperature data are in °C, unless indicated otherwise.

Physical, physicochemical or electro-optical parameters are determined by generally known methods, as described, inter alia, in the brochure "Merck Liquid Crystals - Licristal® - Physical Properties of Liquid Crystals - Description of the Measurement Methods", 1998, Merck KGaA, Darmstadt.

Above and below, $\Delta n$ denotes the optical anisotropy (589 nm, 20°C) and $\Delta\varepsilon$ denotes the dielectric anisotropy (1 kHz, 20°C). The dielectric anisotropy $\Delta\varepsilon$ is determined at 20°C and 1 kHz. The optical anisotropy $\Delta n$ is determined at 20°C and a wavelength of 589.3 nm.

The $\Delta\varepsilon$ and $\Delta n$ values and the rotational viscosity ($\gamma_1$) of the compounds according to the invention are obtained by linear extrapolation from liquid-crystalline mixtures consisting of 5 to 10% of the respective compound according to the invention and 90-95% of the commercially available liquid-crystal mixture ZLI-2857 (for $\Delta\varepsilon$) or ZLI-4792 (for $\Delta n$, $\gamma_1$) (mixtures, Merck KGaA, Darmstadt).

The compounds used in the present invention are prepared by methods known per se, as described in the literature (for example in the standard works, such as Houben-Weyl, Methoden der organischen Chemie [Methods of Organic Chemistry], Georg-Thieme-Verlag, Stuttgart), to be precise under reaction conditions which are known and suitable for the said reactions. Use can also be made here of variants known per se, which are not mentioned here in greater detail.

In the present invention and especially in the following examples, the structures of the mesogenic compounds are indicated by means of abbreviations, also called acronyms. In these acronyms, the chemical formulae are abbreviated as follows using Tables A to C below. All groups $C_nH_{2n+1}$, $C_mH_{2m+1}$ and $C_lH_{2l+1}$ or $C_nH_{2n-1}$, $C_mH_{2m-1}$ and $C_lH_{2l-1}$ denote straight-chain alkyl or alkenyl, preferably 1E-alkenyl, each having n, m and I C atoms respectively. Table A lists the codes used for the ring elements of the core structures of the compounds, while Table B shows the linking groups. Table C gives the meanings of the codes for the left-hand or right-hand end groups. The acronyms are composed of the codes for the ring elements with optional linking groups, followed by a first hyphen and the codes for the left-hand end group, and a second hyphen and the codes for the right-hand end group. Table D shows illustrative structures of compounds together with their respective abbreviations.

**Table A: Ring elements**

| | | | |
|---|---|---|---|
| **C** | 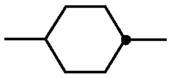 | | |
| **P** | 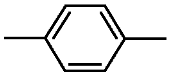 | | |
| **D** | 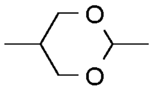 | **DI** | 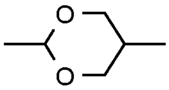 |
| **A** | 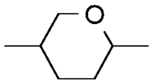 | **AI** | 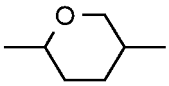 |
| **G** | 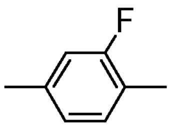 | **GI** | 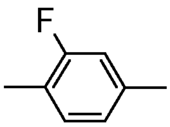 |
| **U** | 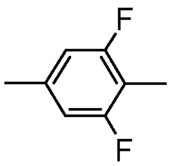 | **UI** | 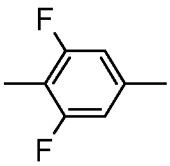 |
| **Y** | 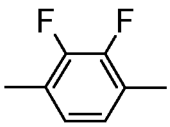 | | |
| **M** | 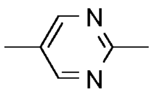 | **MI** | 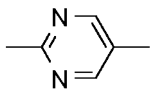 |
| **N** | 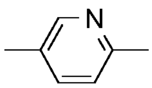 | **NI** | 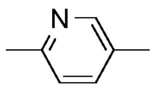 |
| **Np** | 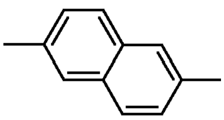 | **dH** | 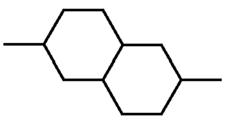 |

(continued)

| | | | |
|---|---|---|---|
| **N3f** | 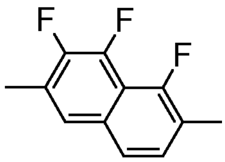 | **N3fI** | 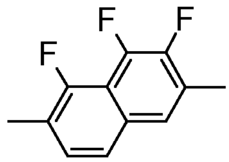 |
| **tH** | 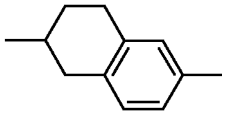 | **tHI** | 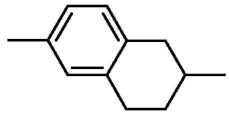 |
| **tH2f** | 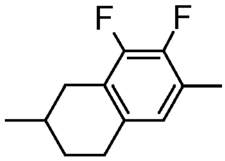 | **tH2fI** | 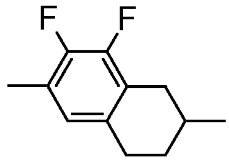 |
| **K** | 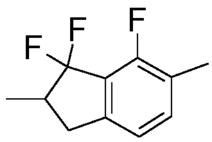 | **KI** | 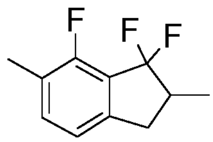 |
| **L** | 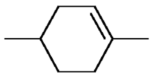 | LI | 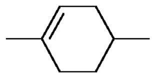 |
| **F** | 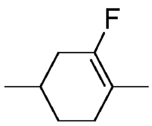 | **FI** | 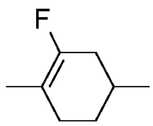 |
| **Nf** | 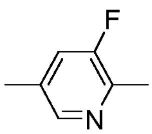 | NfI | 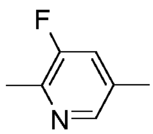 |

**Table B: Linking groups**

| | | | |
|---|---|---|---|
| **E** | $-CH_2CH_2-$ | **Z** | -CO-O- |
| **V** | -CH=CH- | **ZI** | -O-CO- |
| **X** | -CF=CH- | **O** | $-CH_2-O-$ |
| **XI** | -CH=CF- | **OI** | $-O-CH_2-$ |
| **B** | -CF=CF- | **Q** | $-CF_2-O-$ |
| **T** | -C≡C- | **QI** | $-O-CF_2-$ |
| **W** | $-CF_2CF_2-$ | **T** | -C≡C- |

**Table C: End groups**

| **Left-hand side** | | **Right-hand side** | |
|---|---|---|---|
| | | **Use alone** | |
| **-n-** | $C_nH_{2n+1}-$ | **-n** | $-C_nH_{2n+1}$ |
| **-nO-** | $C_nH_{2n+1}-O-$ | **-nO** | $-O-C_nH_{2n+1}$ |
| **-V-** | $CH_2=CH-$ | **-V** | $-CH=CH_2$ |
| **-nV-** | $C_nH_{2n+1}-CH=CH-$ | **-nV** | $-C_nH_{2n}-CH=CH_2$ |
| **-Vn-** | $CH_2=CH-C_nH_{2n+1}-$ | **-Vn** | $-CH=CH-C_nH_{2n+1}$ |
| **-nVm-** | $C_nH_{2n+1}-CH=CH-C_mH_{2m}-$ | **-nVm** | $-C_nH_{2n}-CH=CH-C_mH_{2m+1}$ |

(continued)

| Left-hand side | | Right-hand side | |
|---|---|---|---|
| | | **Use alone** | |
| **-N-** | N≡C- | **-N** | -C≡N |
| **-S-** | S=C=N- | **-S** | -N=C=S |
| **-F-** | F- | **-F** | -F |
| **-CL-** | Cl- | **-CL** | -Cl |
| **-M-** | $CFH_2$- | **-M** | -$CFH_2$ |
| **-D-** | $CF_2H$- | **-D** | -$CF_2H$ |
| **-T-** | $CF_3$- | **-T** | -$CF_3$ |
| **-MO-** | $CFH_2O$ - | **-OM** | -$OCFH_2$ |
| **-DO-** | $CF_2HO$ - | **-OD** | -$OCF_2H$ |
| **-TO-** | $CF_3O$ - | **-OT** | -$OCF_3$ |
| **-FXO-** | $CF_2$=CH-O- | **-OXF** | -O-CH=$CF_2$ |
| **-A-** | H-C≡C- | **-A** | -C≡C-H |
| **-nA-** | $C_nH_{2n+1}$-C≡C- | **-An** | -C≡C-Cn$H_{2n+1}$ |
| **-NA-** | N≡C-C≡C- | **-AN** | -C≡C-C≡N |
| | **Use together with one another and with others** | | |
| **-...A...-** | -C≡- | **-...A...** | -C≡- |
| **-...V...-** | CH=CH- | **-...V...** | -CH=CH- |
| **-...Z...-** | -CO-O- | **-...Z...** | -CO-O- |
| **-...ZI...-** | -O-CO- | **-...ZI...** | -O-CO- |
| **-...K...-** | -CO- | **-...K...** | -CO- |
| **-...W...-** | -CF=CF- | **-...W...** | -CF=CF- |

wherein n and m each denote integers, and the three dots "..." are place-holders for other abbreviations from this table.

The following table shows illustrative structures together with their respective abbreviations. These are shown in order to illustrate the meaning of the rules for the abbreviations. They furthermore represent compounds which are preferably used.

## Table D: Illustrative structures

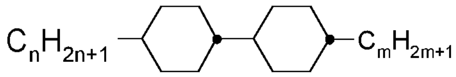

**CC-n-m**

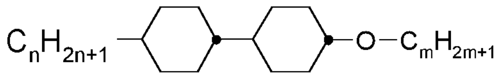

**CC-n-Om**

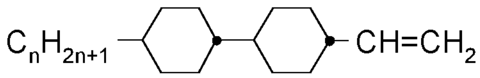

**CC-n-V**

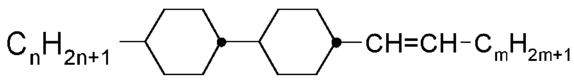

**CC-n-Vm**

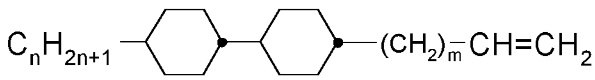

**CC-n-mV**

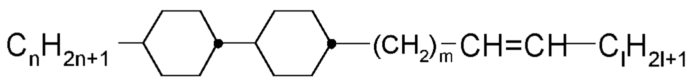

**CC-n-mVl**

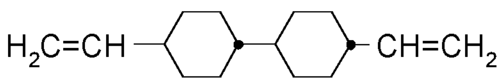

**CC-V-V**

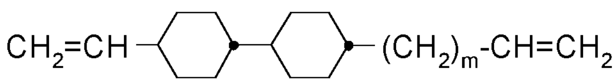

**CC-V-mV**

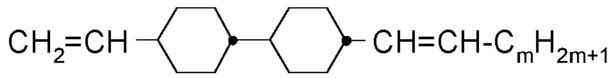

**CC-V-Vm**

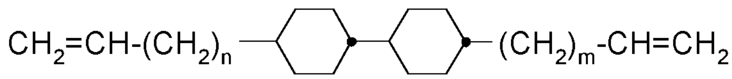

**CC-Vn-mV**

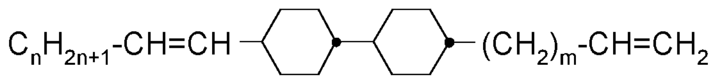

**CC-nV-mV**

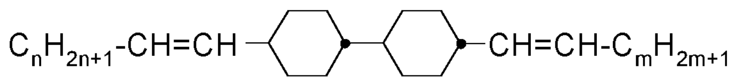

**CC-nV-Vm**

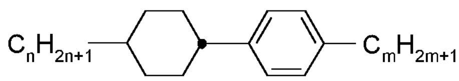

**CP-n-m**

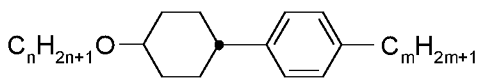

**CP-nO-m**

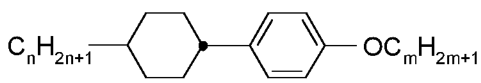

**CP-n-Om**

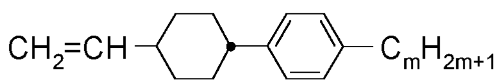

**CP-V-m**

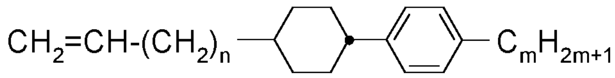

**CP-Vn-m**

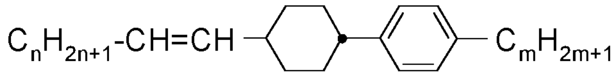

**CP-nV-m**

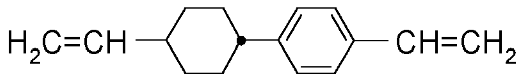

**CP-V-V**

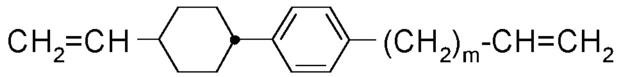

**CP-V-mV**

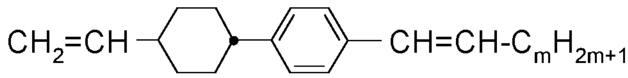

**CP-V-Vm**

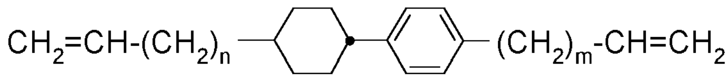

**CP-Vn-mV**

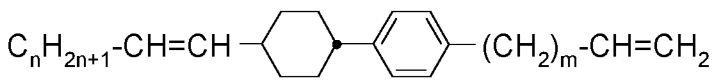

**CP-nV-mV**

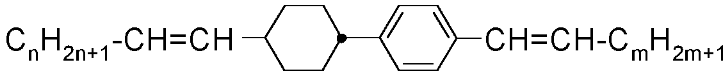

**CP-nV-Vm**

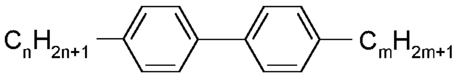

**PP-n-m**

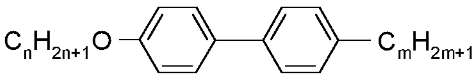

**PP-nO-m**

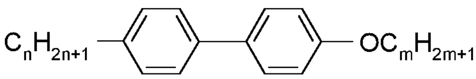

**PP-n-Om**

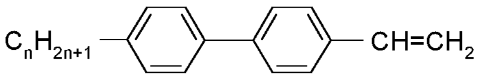

**PP-n-V**

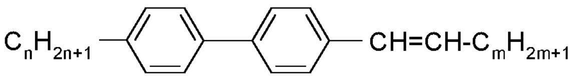

**PP-n-Vm**

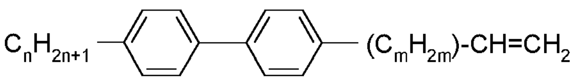

**PP-n-mV**

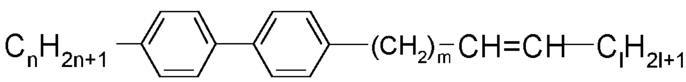

**PP-n-mVl**

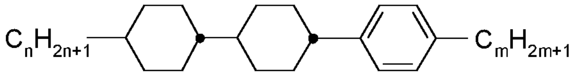

**CCP-n-m**

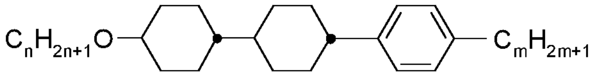

**CCP-nO-m**

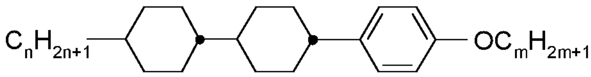

**CCP-n-Om**

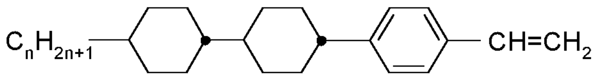

**CCP-n-V**

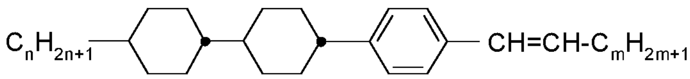

**CCP-n-Vm**

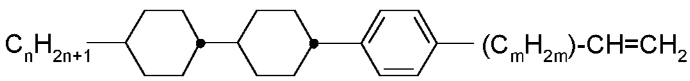

**CCP-n-mV**

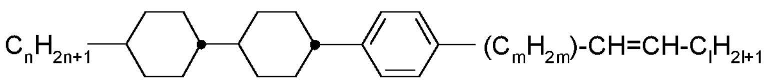

**CCP-n-mVl**

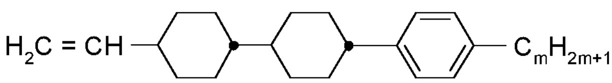

**CCP-V-m**

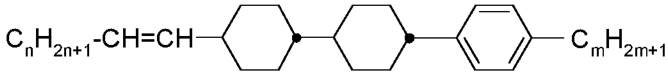

**CCP-nV-m**

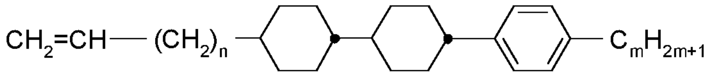

**CCP-Vn-m**

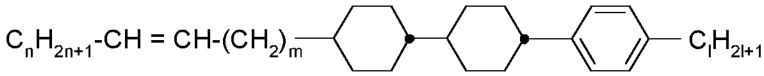

**CCP-nVm-l**

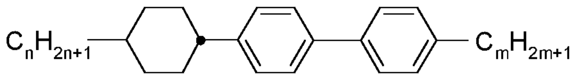

**CPP-n-m**

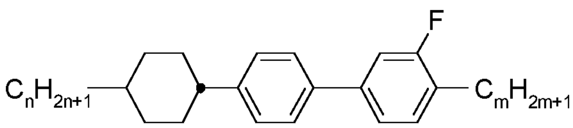

**CPG-n-m**

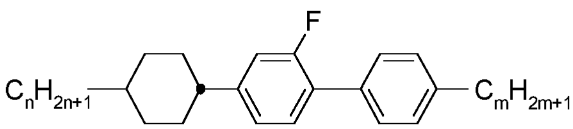

**CGP-n-m**

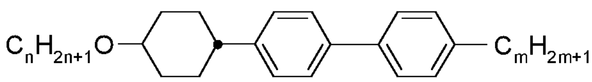

**CPP-nO-m**

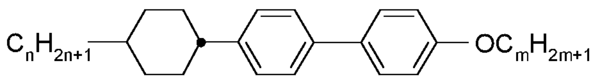

**CPP-n-Om**

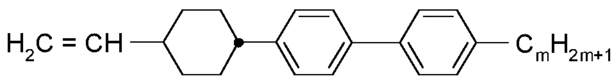

**CPP-V-m**

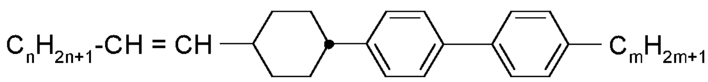

**CPP-nV-m**

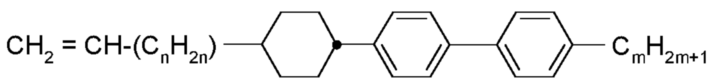

**CPP-Vn-m**

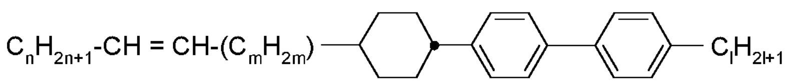

**CPP-nVm-l**

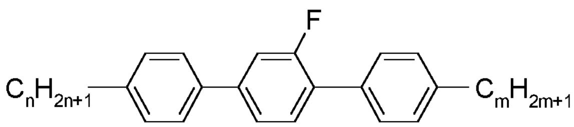

**PGP-n-m**

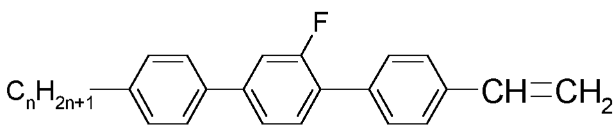

**PGP-n-V**

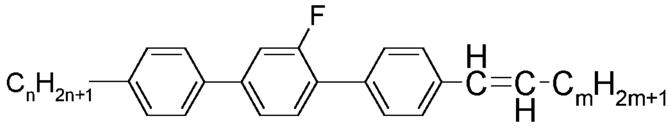

**PGP-n-Vm**

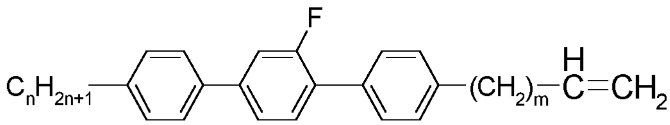

**PGP-n-mV**

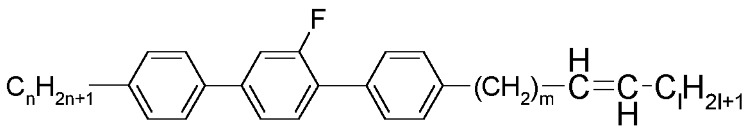

**PGP-n-mVI**

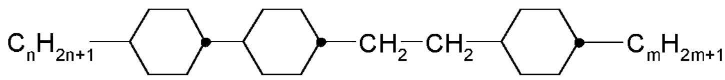

**CCEC-n-m**

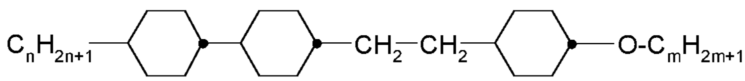

**CCEC-n-Om**

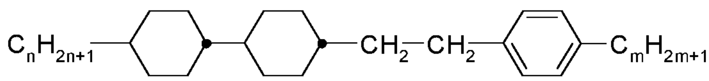

**CCEP-n-m**

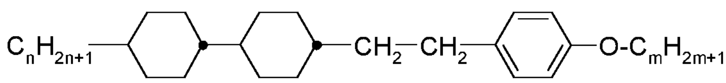

**CCEP-n-Om**

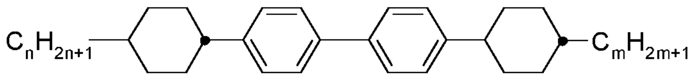

**CPPC-n-m**

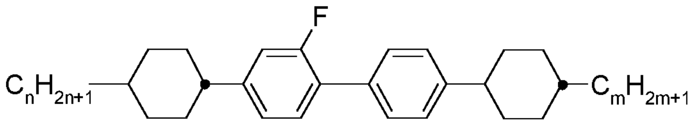

**CGPC-n-m**

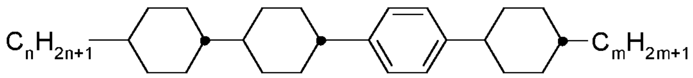

**CCPC-n-m**

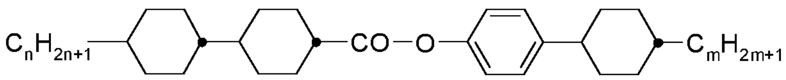

**CCZPC-n-m**

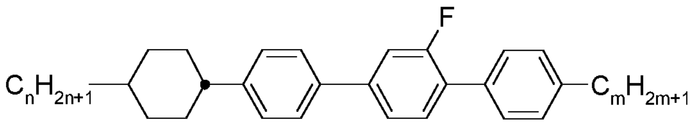

**CPGP-n-m**

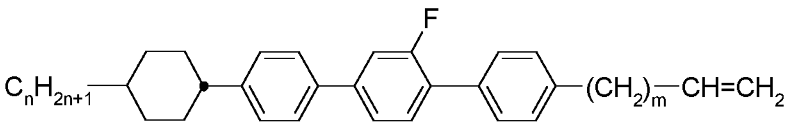

**CPGP-n-mV**

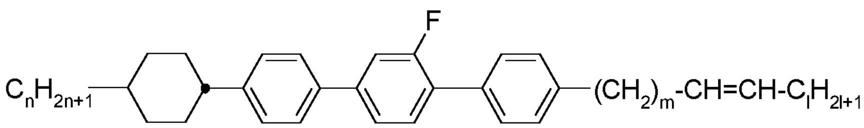

**CPGP-n-mVI**

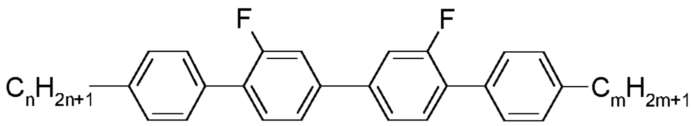

**PGIGP-n-m**

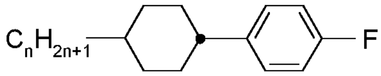

**CP-n-F**

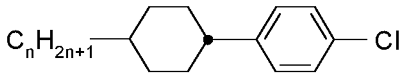

**CP-n-CL**

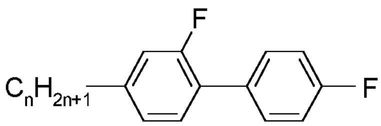

**GP-n-F**

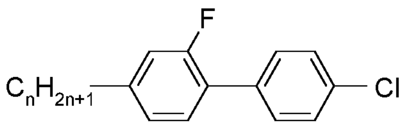

**GP-n-CL**

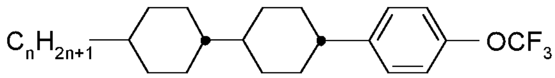

**CCP-n-OT**

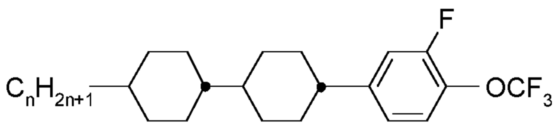

**CCG-n-OT**

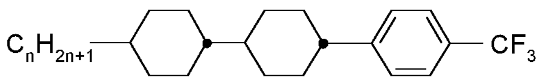

**CCP-n-T**

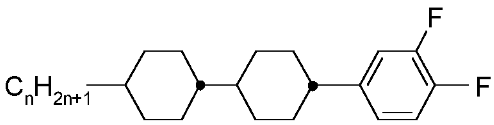

**CCG-n-F**

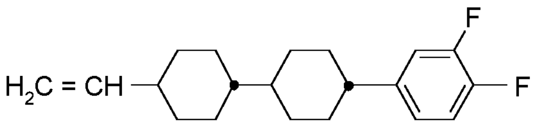

**CCG-V-F**

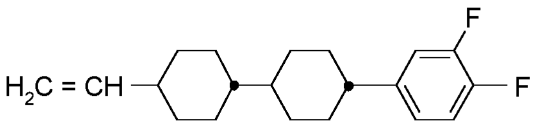

**CCG-V-F**

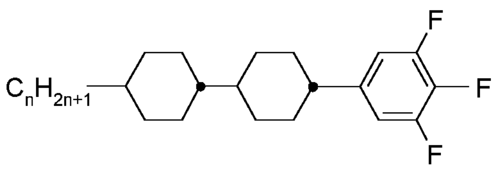

**CCU-n-F**

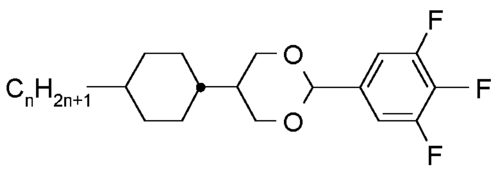

**CDU-n-F**

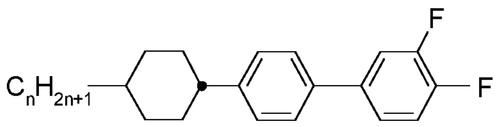

**CPG-n-F**

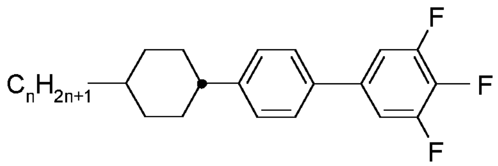

**CPU-n-F**

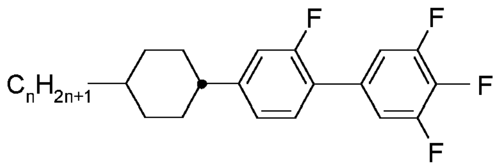

**CGU-n-F**

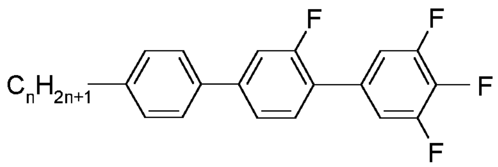

**PGU-n-F**

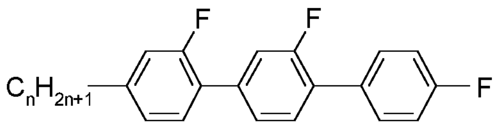

**GGP-n-F**

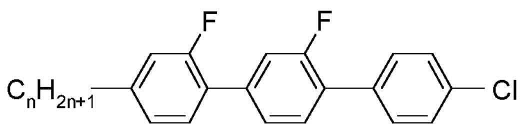

**GGP-n-CL**

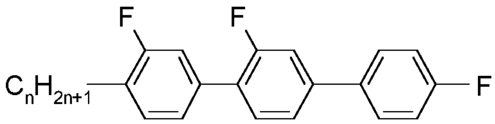

**PGIGI-n-F**

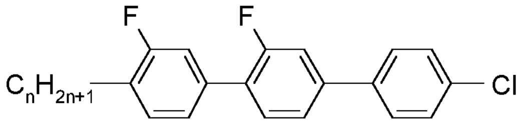

**PGIGI-n-CL**

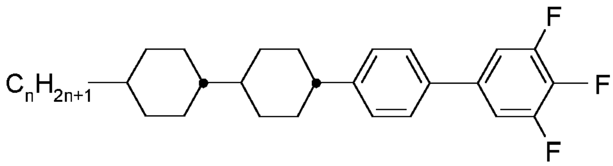

**CCPU-n-F**

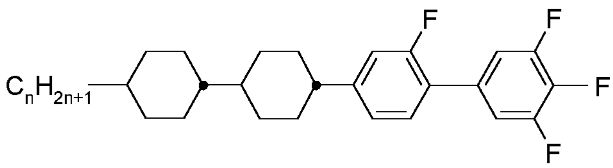

**CCGU-n-F**

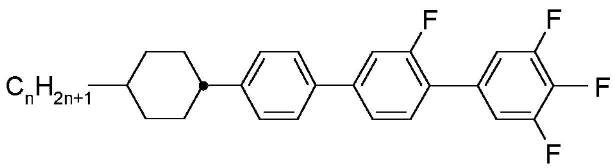

**CPGU-n-F**

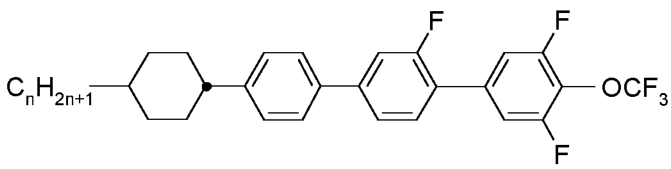

**CPGU-n-OT**

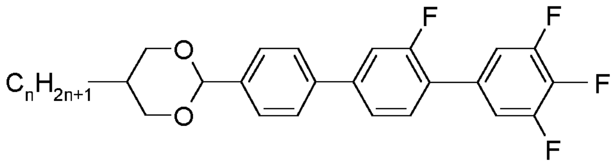

**DPGU-n-F**

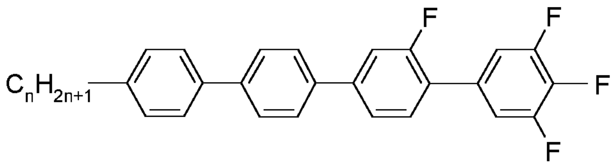

**PPGU-n-F**

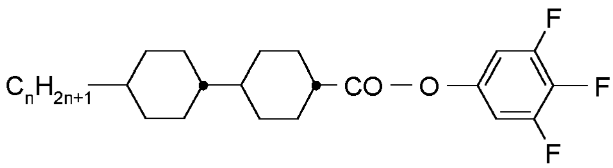

**CCZU-n-F**

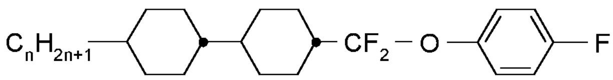

**CCQP-n-F**

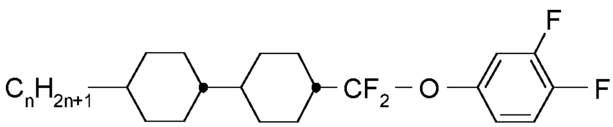

**CCQG-n-F**

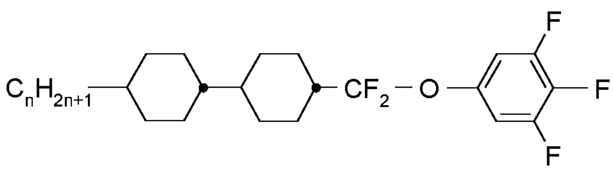

**CCQU-n-F**

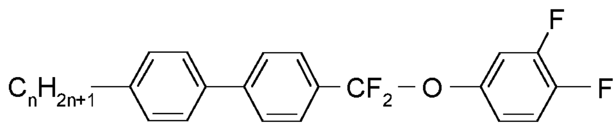

PPQG-n-F

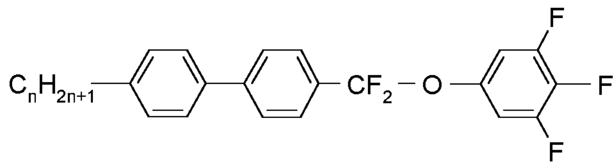

PPQU-n-F

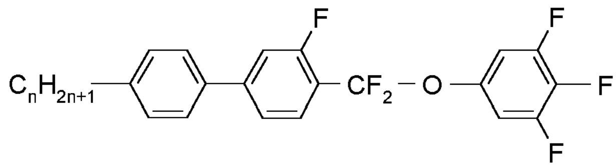

PGQU-n-F

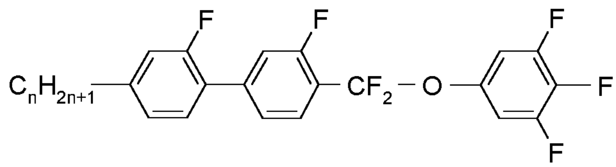

GGQU-n-F

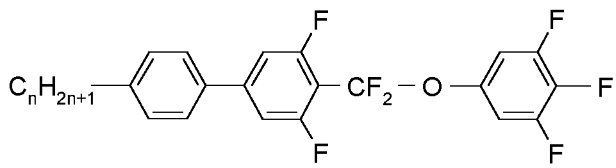

PUQU-n-F

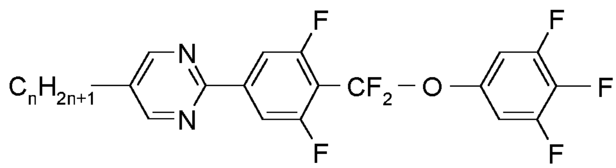

MUQU-n-F

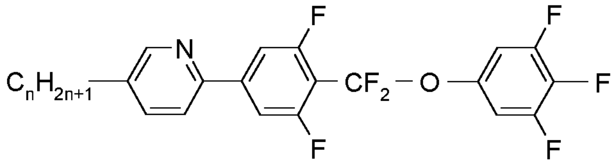

NUQU-n-F

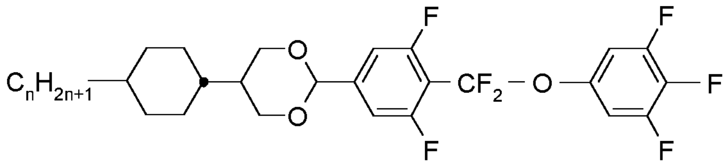

CDUQU-n-F

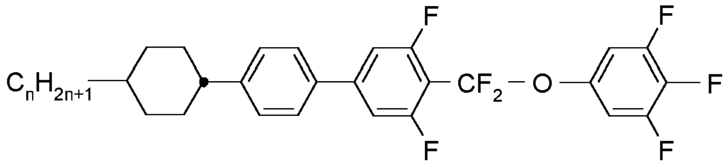

CPUQU-n-F

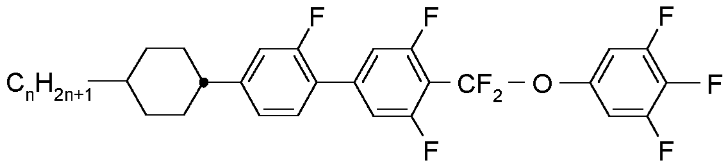

CGUQU-n-F

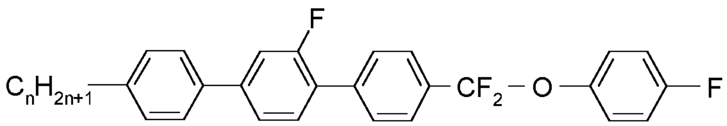

PGPQP-n-F

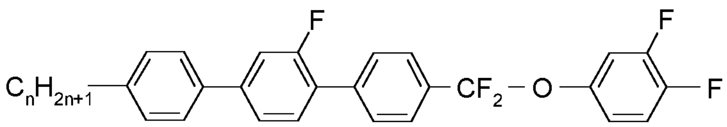

PGPQG-n-F

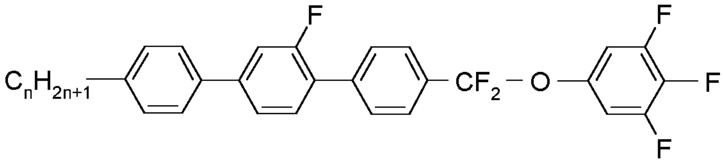

**PGPQU-n-F**

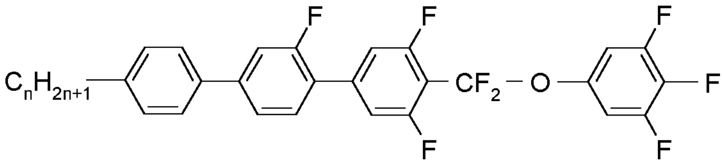

**PGUQU-n-F**

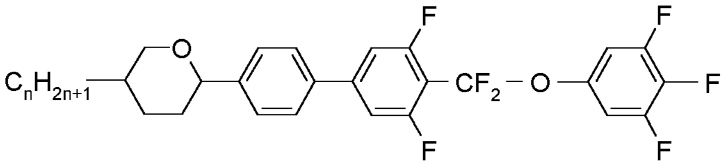

**APUQU-n-F**

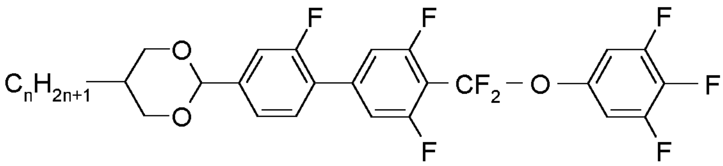

**DGUQU-n-F**

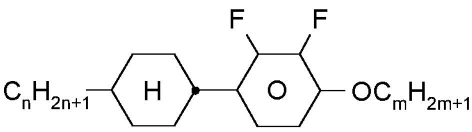

**CY-n-Om**

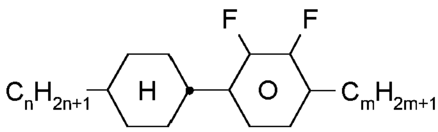

**CY-n-m**

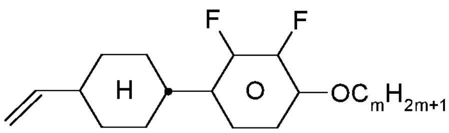

**CY-V-Om**

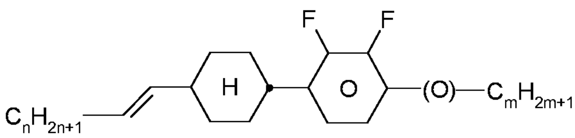

**CY-nV-(O)m**

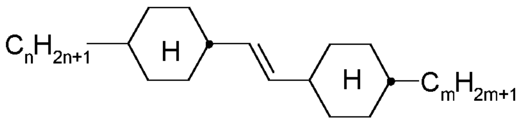

**CVC-n-m**

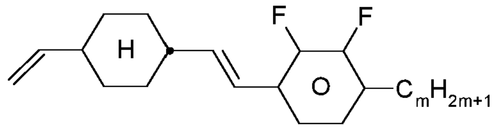

**CVY-V-m**

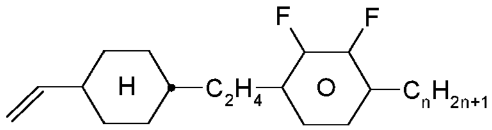

**CEY-V-m**

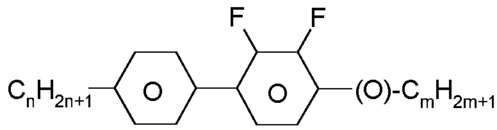

**PY-n-(O)m**

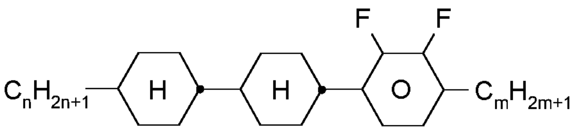

**CCY-n-m**

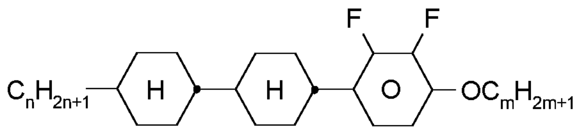

**CCY-n-Om**

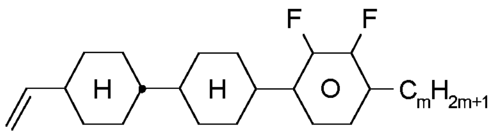

**CCY-V-m**

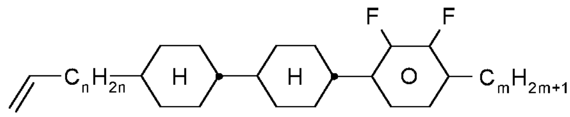

**CCY-Vn-m**

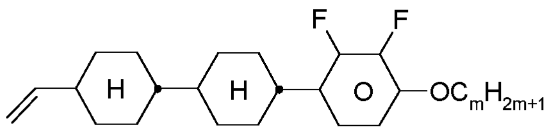

**CCY-V-Om**

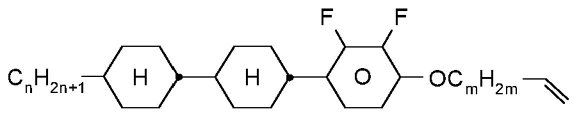

**CCY-n-OmV**

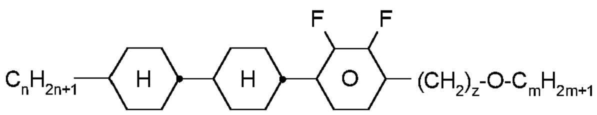

**CCY-n-zOm**

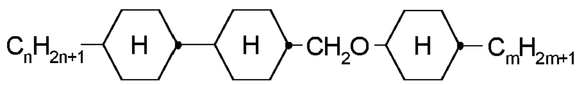

**CCOC-n-m**

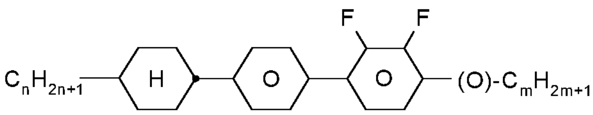

**CPY-n-(O)m**

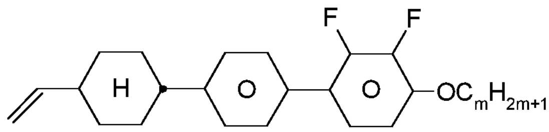

**CPY-V-Om**

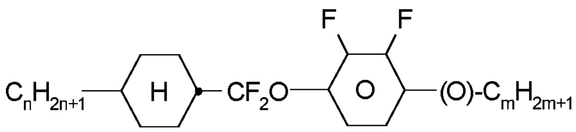

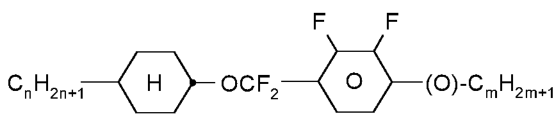

**CQY-n-(O)m** **CQIY-n-(O)m**

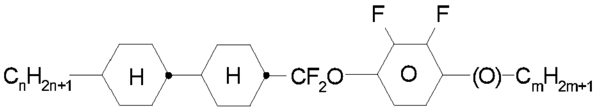
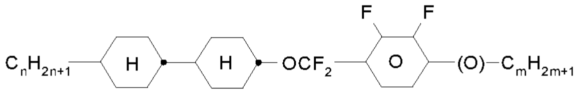

**CCQY-n-(O)m** **CCQIY-n-(O)m**

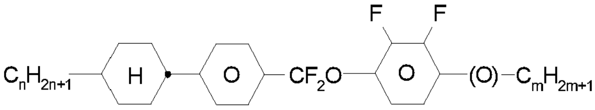
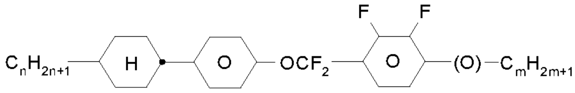

**CPQY-n-(O)m** **CPQIY-n-Om**

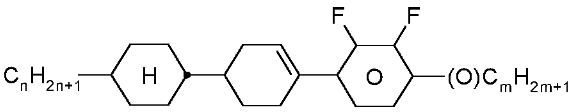
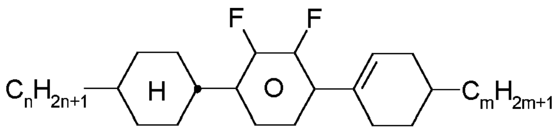

**CLY-n-(O)m** **CYLI-n-m**

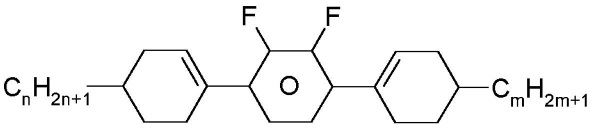
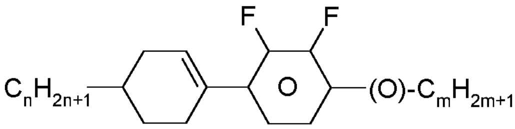

**LYLI-n-m** **LY-n-(O)m**

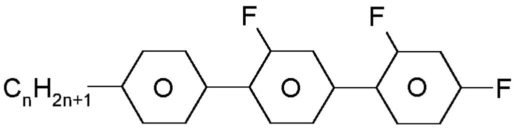
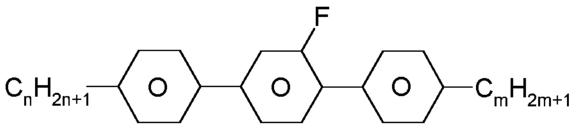

**PGIGI-n-F** **PGP-n-m**

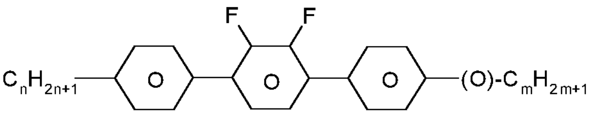
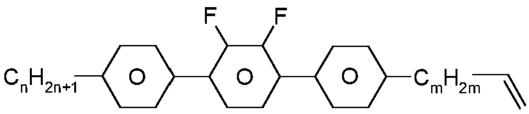

**PYP-n-(O)m** **PYP-n-mV**

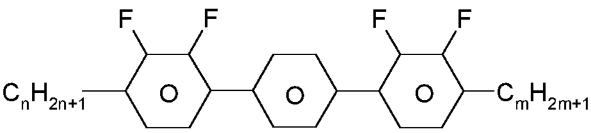
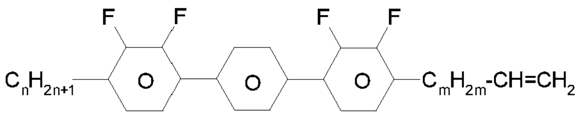

**YPY-n-m** **YPY-n-mV**

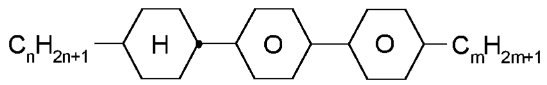

**BCH-nm**

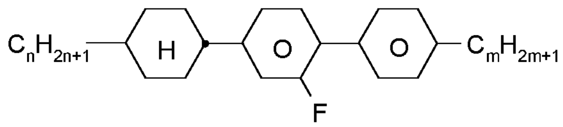

**BCH-nmF**

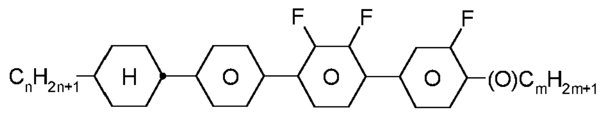

**CPYP-n-(O)m**

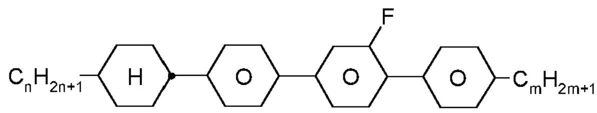

**CPGP-n-m**

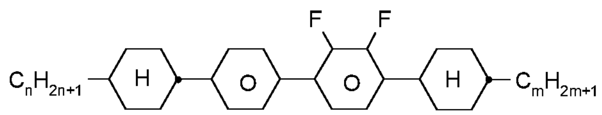

**CPYC-n-m**

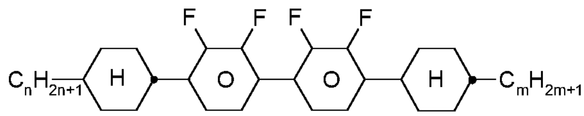

**CYYC-n-m**

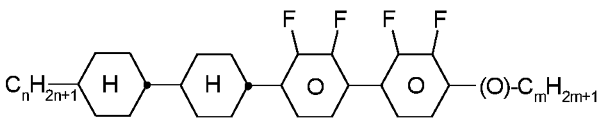

**CCYY-n-m**

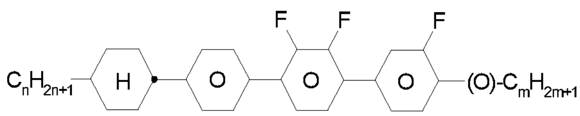

**CPYG-n-(O)m**

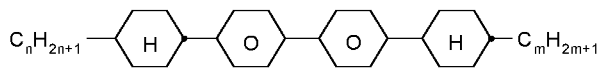

**CBC-nm**

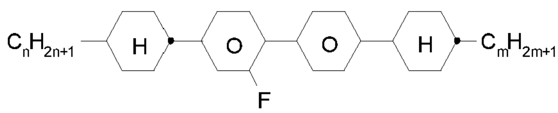

**CBC-nmF**

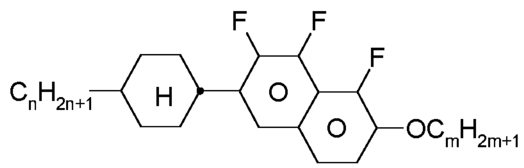

**CNap-n-Om**

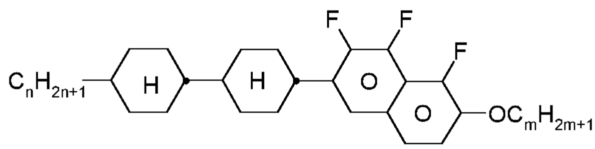

**CCNap-n-Om**

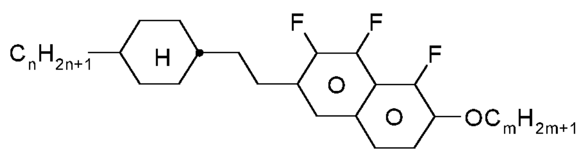

**CENap-n-Om**

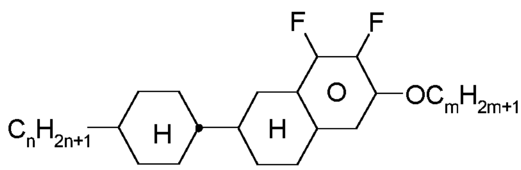

**CTNap-n-Om**

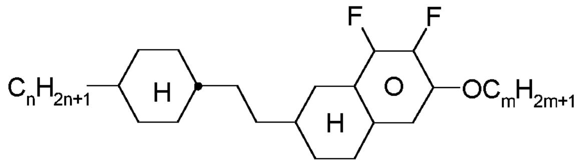

**CETNap-n-Om**

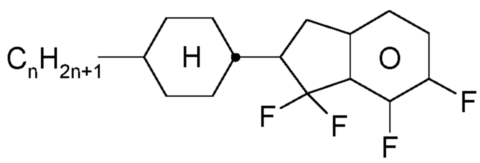

**CK-n-F**

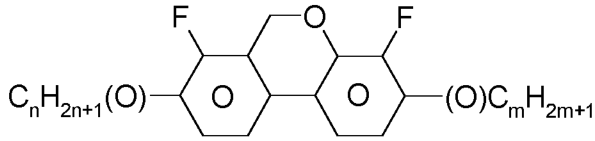

**DFDBC-n(O)-(O)m**

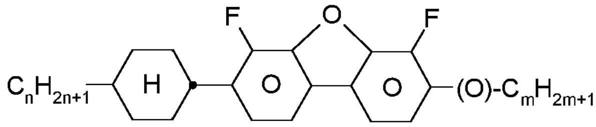

**C-DFDBF-n-(O)m**

wherein n, m and l preferably, independently of one another, denote 1 to 7.

The following table, Table E, shows illustrative compounds which can be used as additional stabilisers in the mesogenic media according to the present invention.

**Table E**

Table E shows possible stabilisers which can be added to the LC media according to the invention.

(n here denotes an integer from 1 to 12, preferably 1, 2, 3, 4, 5, 6, 7 or 8, terminal methyl groups are not shown).

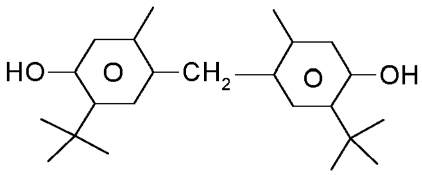

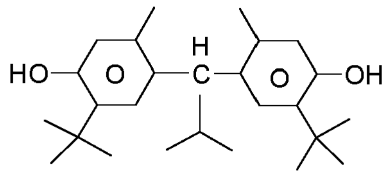

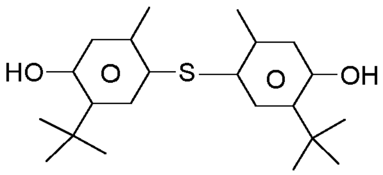

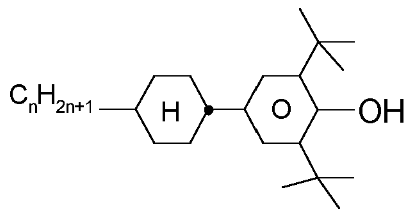

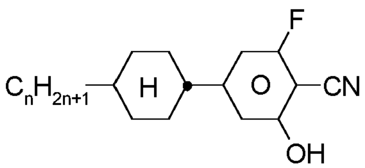

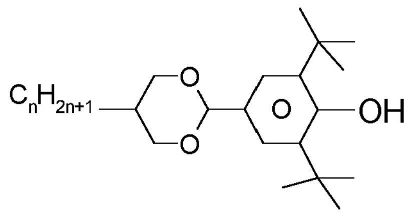

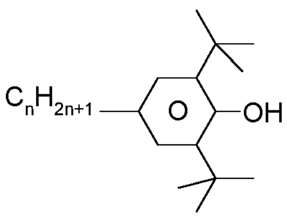

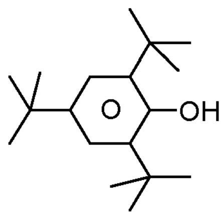

(continued)

Table E shows possible stabilisers which can be added to the LC media according to the invention. (n here denotes an integer from 1 to 12, preferably 1, 2, 3, 4, 5, 6, 7 or 8, terminal methyl groups are not shown).

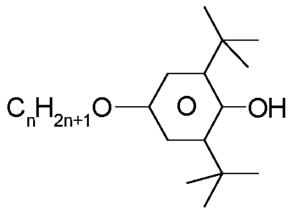

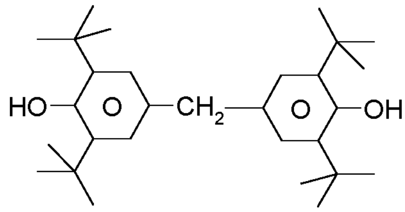

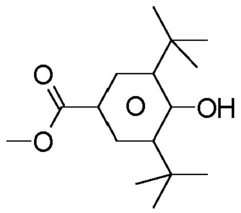

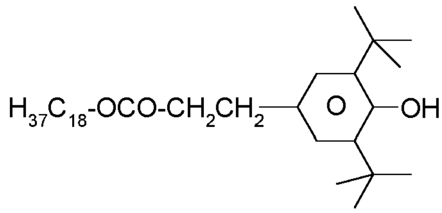

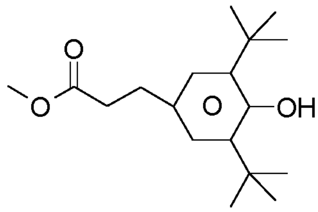

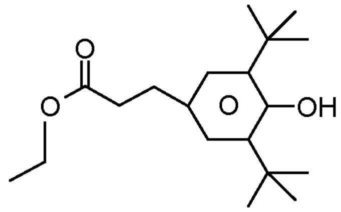

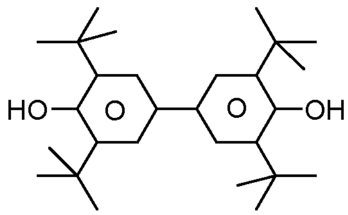

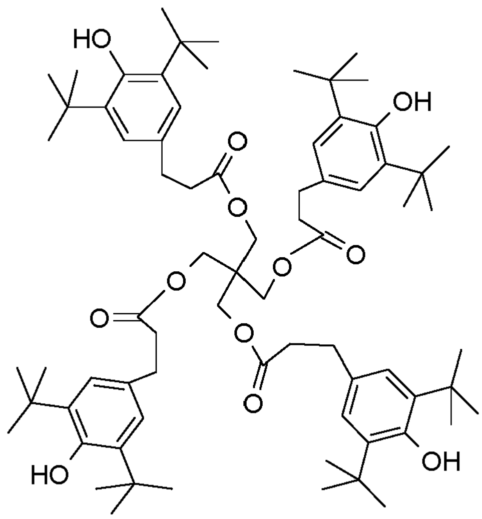

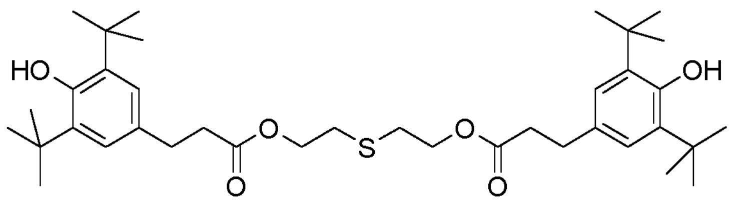

(continued)

Table E shows possible stabilisers which can be added to the LC media according to the invention.
(n here denotes an integer from 1 to 12, preferably 1, 2, 3, 4, 5, 6, 7 or 8, terminal methyl groups are not shown).

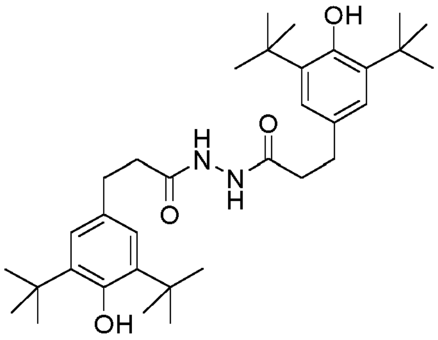

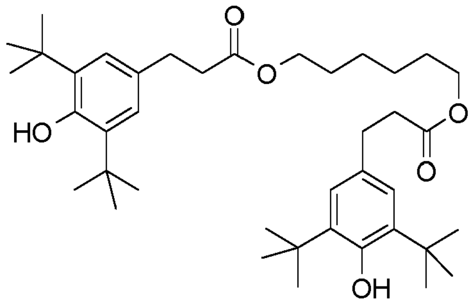

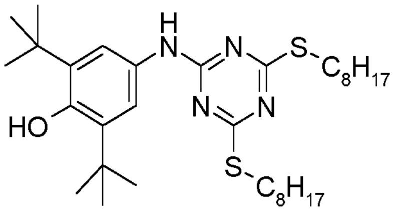

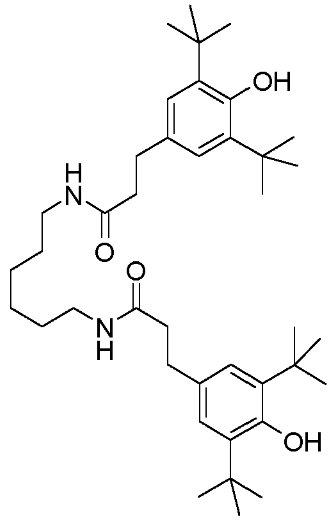

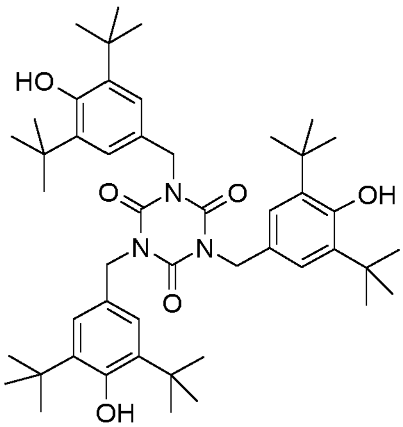

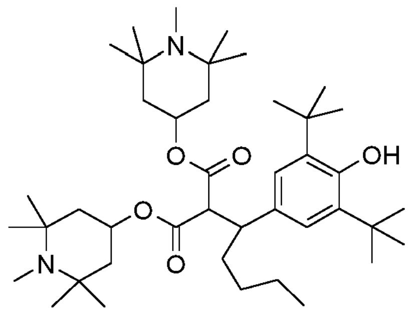

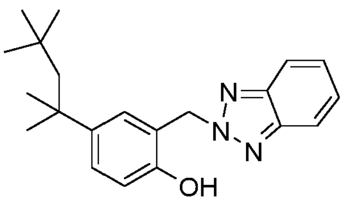

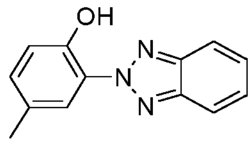

(continued)

Table E shows possible stabilisers which can be added to the LC media according to the invention.
(n here denotes an integer from 1 to 12, preferably 1, 2, 3, 4, 5, 6, 7 or 8, terminal methyl groups are not shown).

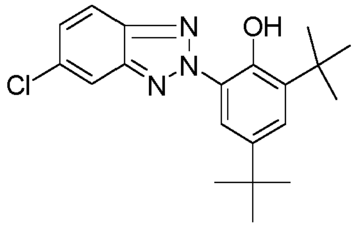

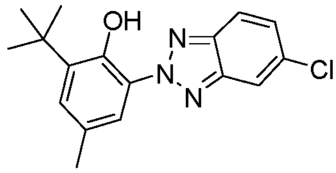

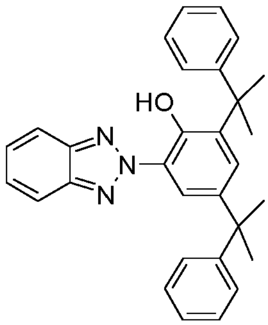

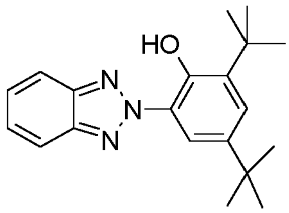

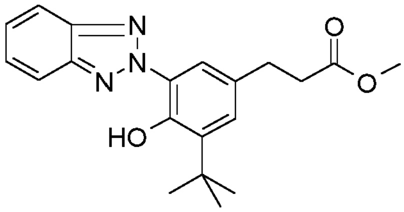

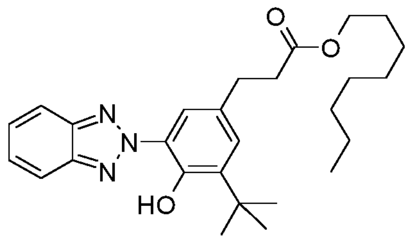

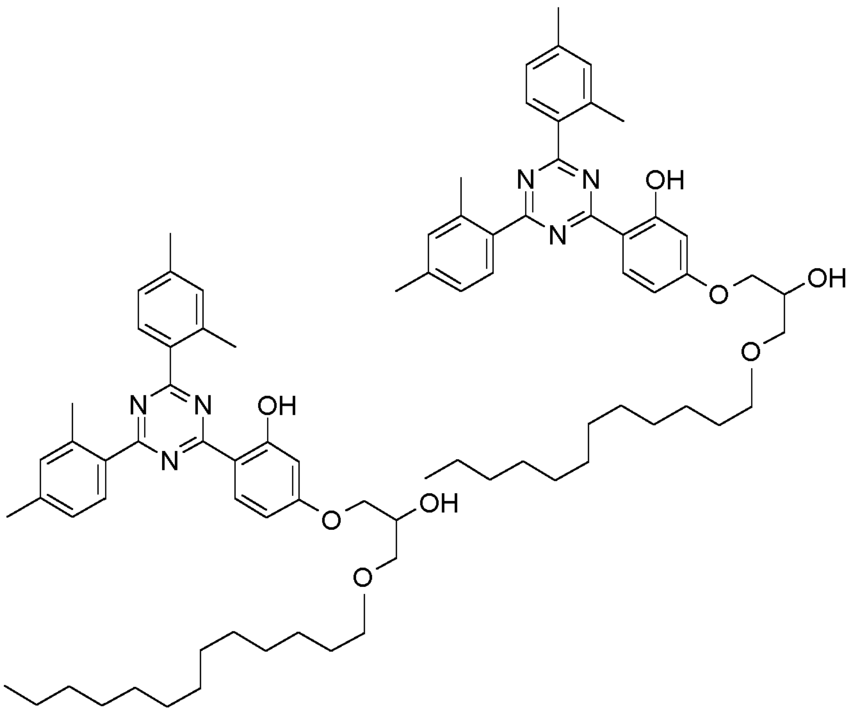

(continued)

Table E shows possible stabilisers which can be added to the LC media according to the invention.
(n here denotes an integer from 1 to 12, preferably 1, 2, 3, 4, 5, 6, 7 or 8, terminal methyl groups are not shown).

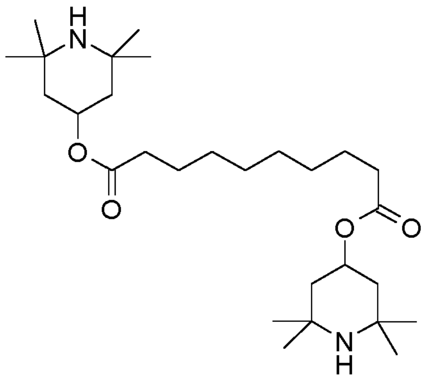

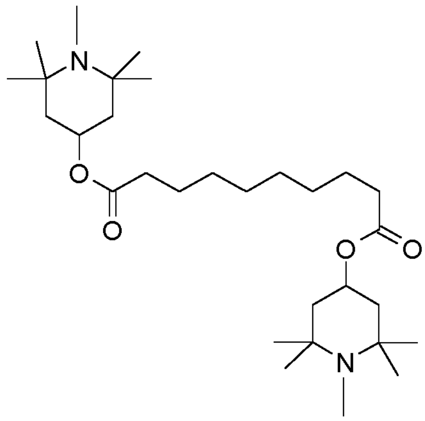

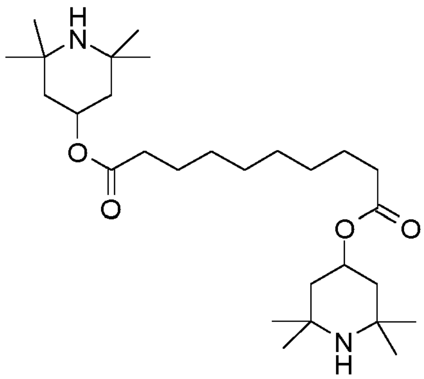

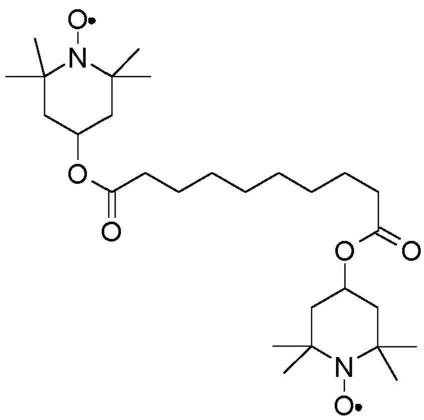

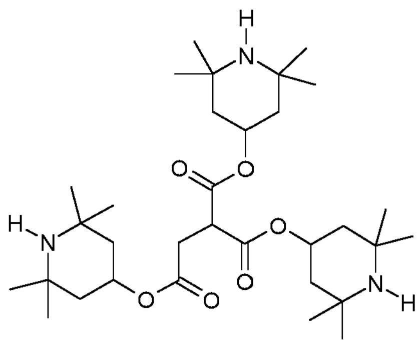

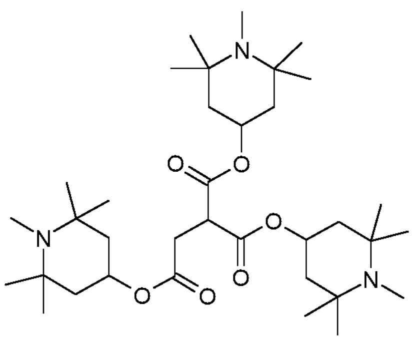

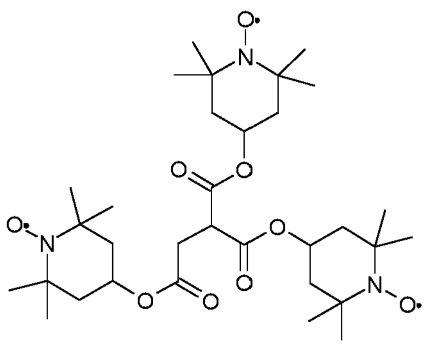

(continued)

Table E shows possible stabilisers which can be added to the LC media according to the invention. (n here denotes an integer from 1 to 12, preferably 1, 2, 3, 4, 5, 6, 7 or 8, terminal methyl groups are not shown).

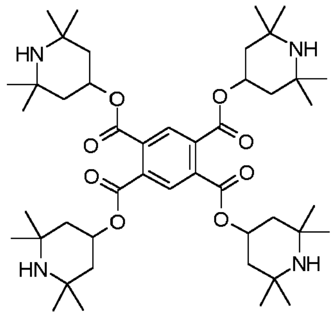

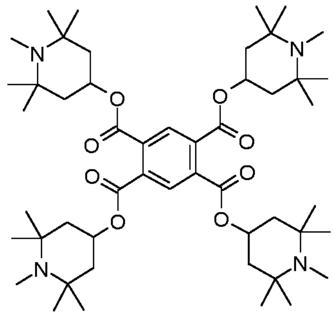

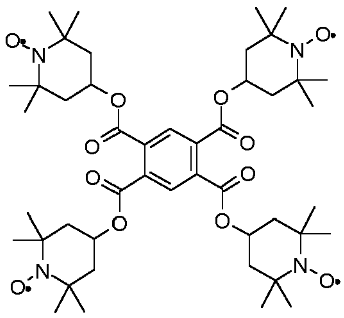

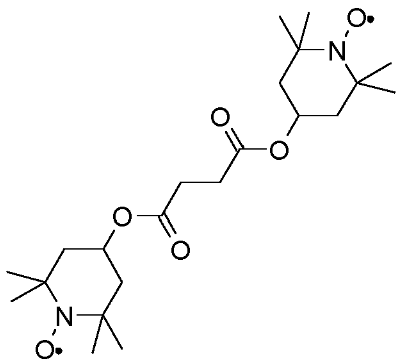

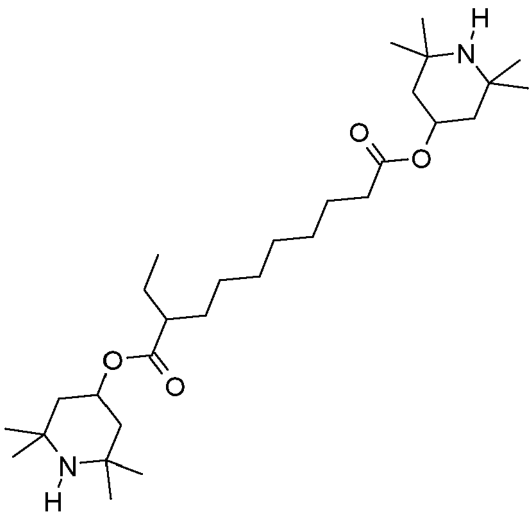

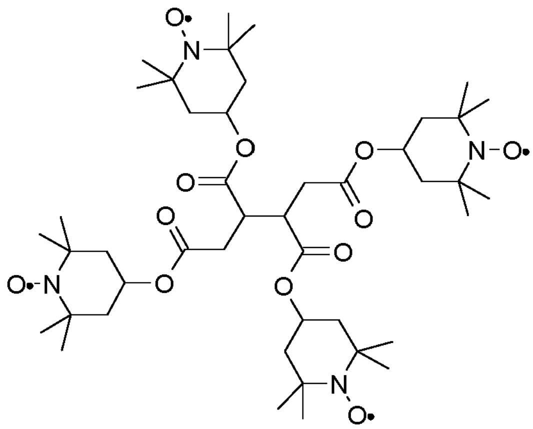

The LC media preferably comprise 0 to 10% by weight, in particular 1 ppm to 5% by weight, particularly preferably 1 ppm to 1% by weight, of stabilisers.

Table F below shows illustrative compounds which can preferably be used as chiral dopants in the mesogenic media according to the present invention.

**Table F**

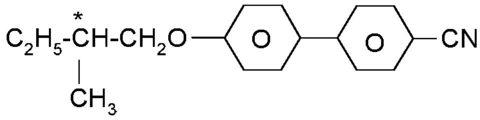

**C 15**

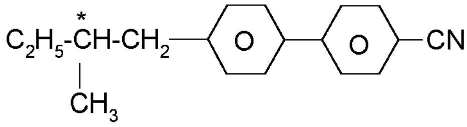

**CB 15**

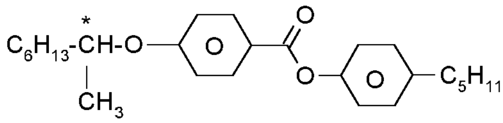

**CM 21**

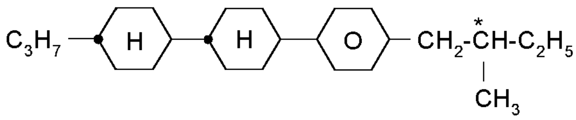

**CM 44**

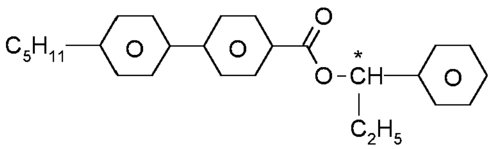

**CM 45**

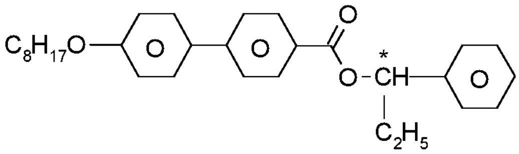

**CM 47**

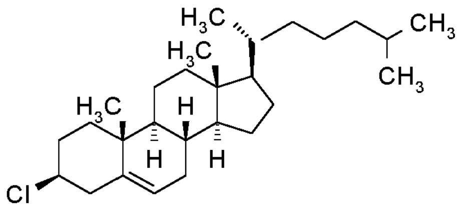

**CC**

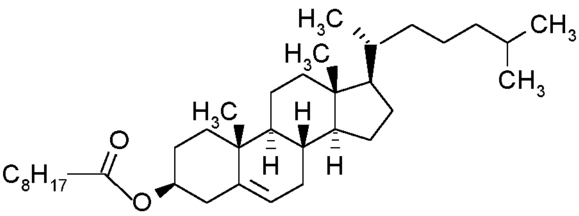

**CN**

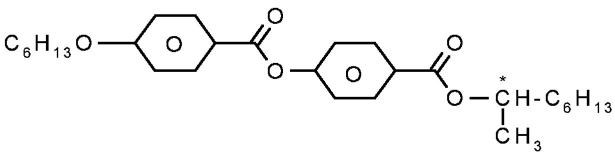

**R/S-811**

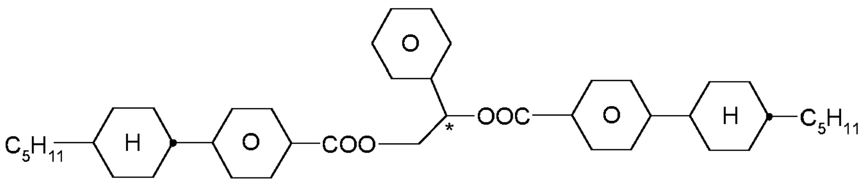

**R/S-1011**

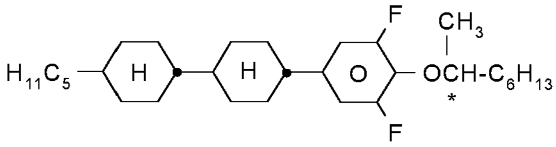

**R/S-2011**

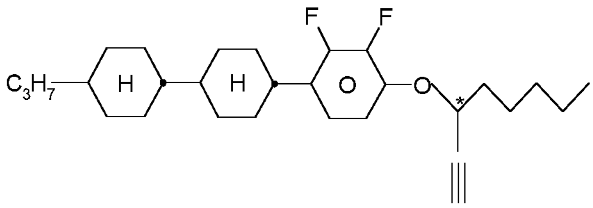

**R/S-3011**

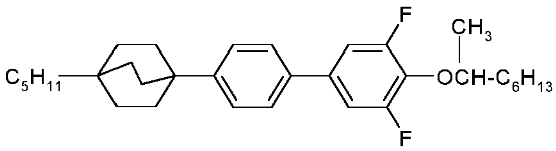

**R/S-4011**

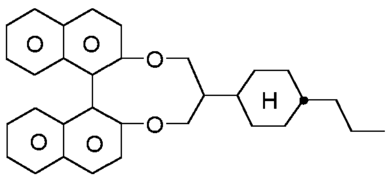

**R/S-5011**

In a preferred embodiment of the present invention, the mesogenic media comprise one or more compounds selected from the group of the compounds from Table F.

The mesogenic media according to the present application preferably comprise two or more, preferably four or more, compounds selected from the group consisting of the compounds from the above tables.

The liquid-crystal media according to the present invention preferably comprise

- seven or more, preferably eight or more, individual compounds, preferably of three or more, particularly preferably of four or more, different formulae, selected from the group of the compounds from Table D.

Hereinafter, the present invention is described in more detail and specifically with reference to the Examples, which however are not intended to limit the present invention.

Examples

**Compound Examples**

1. Synthesis Example

1.1 Synthesis of diethyl 3-(2-benzyloxyethyl)pentanedioate **1**

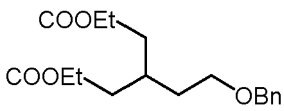

**1**

Under reflux 13.8 ml (90 mmol) of the diethyl malonate are added to mixture of 34,5 ml of a solution of sodium methylate in ethanol (20%, 50 mmol) and 40 ml ethanol. After 2h 10 g (50 mmol) of 2-bromoethoxymethylbenzene are added and heating was continued overnight. Water and MTB ether are poured into the cooled reaction mixture. The aqueous layer is extracted with MTB ether. The combined organic layers are washed with brine and dried over sodium sulfate. The solvent is evaporated. The residue is purified by silica chromatography (toluene; toluene/MTB ether 9:1). The isolated material is distilled under vacuum (0.1 mbar, 116-121°C) to give **1.**

1.2 Synthesis of 2-(2-benzyloxyethyl)propane-1,3-diol **(2)**

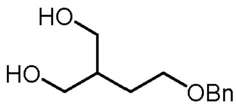

**2**

A solution of 5 g (20 mmol) of the malonate **1** in 60 ml toluene is added to a suspension of 930 mg (24 mmol) Lithium aluminum hydride in 8 ml Toluene. After 3h reflux the cooled reaction mixture is quenched with ethyl acetate. The mixture is acidified with 2mol/l hydrochloric acid (pH 3-4). The aqueous layer is extracted with MTB ether. The combined organic layers are washed with water and dried over sodium sulfate. The solvent is evaporated. The residue is purified by silica chromatography (ethyl acetate to give **2.**

1.3 Synthesis of [4-benzyloxy-2-[[tert-butyl(dimethyl)silyl]oxymethyl]butoxy]-tert-butyl-dimethyl-silane **(3)**

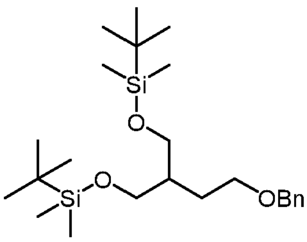

**3**

At roomtemp. 3.4 ml (25 mmol) triethyl amine are added to a mixture of 2.1 g (10 mmol) of the diol **2** and 120 mg DMAP dissolved in 30 ml dichloro methane. Afterwards a solution of 4.5 g (30 mmol) TBDMS-Cl in 15 ml dichloro methane are added to the reaction mixture at 3-4°C. After stirring 16h at roomtemp. the mixture is quenched with water. The combined organic layers are washed with brine and dried over sodium sulfate. The solvent is evaporated. The residue is purified by silica chromatography (n-heptane/ethyl acetate 19:1) to give **3.**

1.4 Synthesis of 4-[tert-butyl(dimethyl)silyl]oxy-3-[[tert-butyl(dimethyl)silyl]oxy-methyl]-butan-1-ol **(4)**

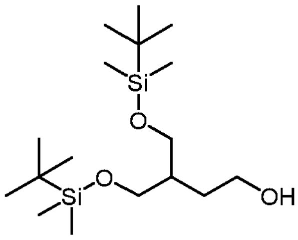

**4**

A solution of 500 mg (1 mmol) of **3** in 13 ml ethyl acetate is hydrogenated using Pd/C-5% at roomtemp.. The solvent is evaporated. The residue is purified by silica chromatography (n-heptane/ethyl acetate (gradient)) to give **4.**

1.5 Synthesis of 4-[(E)-2-(4-hydroxyphenyl)vinyl]phenol **(5)**

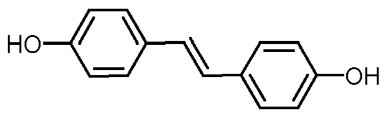

**5**

To a mixture consisting of 49.0g (95%; 137 mmol) of 1-bromo-4-[(E)-2-(4-bromophenyl)vinyl]benzene), 60 ml aqueous potassium hydroxide(47%), 200 ml water and 1200 ml dioxane, 6.3 g (6.9 mmol) Tris(dibenzylideneaceton)dipalladium(0) and 8.8 g (20.7 mmol) 2-Di-t-butylphosphine-2',4',6'-tri-i-propyl1,1 -biphenyl are added und the mixture is stirred for for 2 h under reflux. The mixture is cooled to roomtemperature, diluted with water, acidified with aques Hydrogen chloride and extracted with ethyl acetate. The combined organic phases are dried with sodium sulfate, filtrated and the solvent is evaporated. The residue is purified by silica chromatography (Bu-Cl/THF, gradient 9:1 - 1:9) and the crude product is subsequently treated with toluene and 2-propanol under reflux, respectively to give **5.**

1.6 Synthesis of tert-butyl-[2-[[tert-butyl(dimethyl)silyl]oxymethyl]-4-[4-[(E)-2-[4-(3,3-diethylpentoxy)phenyl]vinyl]phenoxy]-2-ethyl-butoxy]-dimethylsilane (6)

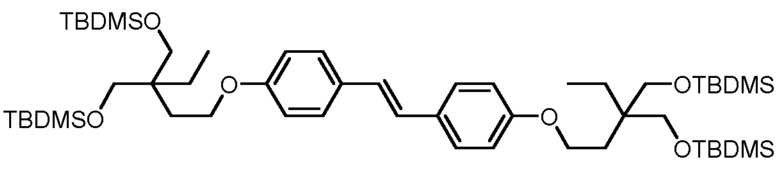

**6**

To a mixture of 10.8 g (41 mmol) Triphenyl phosphine in 15 ml THF, 8.4 ml (30 mmol) Diisopropylazodicarboxylate are added slowly not exceeding a temperature of 22°C. Afterwards, 14.8 g (40 mmol) 3,3bis[[tert-butyl(dimethyl)silyl]oxy-methyl]-pentan-1-ol in 20 ml THF and 3.8 g (20 mmol) **5** are added subsequently and the resulting mixture is stirred over night. Isolute ® HM-N is added and the solvent is evaporated. The crude product is then purified over silica gel (eleuent: n-Heptane/Butylchloride) to give 2.8 g of **6.**

1.7 Synthesis of 2-[2-[4-[(E)-2-[4-[3,3-bis(hydroxymethyl)pentoxy]phenyl]vinyl]phenoxy]ethyl]-2-ethyl-propane-1,3-diol (7)

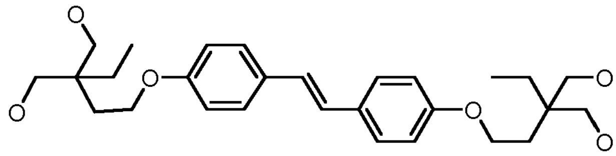

**7**

To a solution of 2.8 g (2 mmol) of **6** in 20 ml dichloromethane, 4.9 ml (30 mmol) Triethylaminotrishydrofluoride are added at roomtemperature and stirred overnight. The solvent is than evaporated and the crude product is purified is diluted in hot THF and preticipated with acetonitrile to give **7** after filtration and drying.

1.8 Synthesis of [4-[4-[(E)-2-[4-[3,3-bis(2-methylprop-2-enoyloxymethyl)pentoxy]phenyl]vinyl]phenoxy]-2-ethyl-2-(2-methylprop-2-enoyloxymethyl)butyl] 2-methylprop-2-enoate (8 - Photoalignment Additive 1)

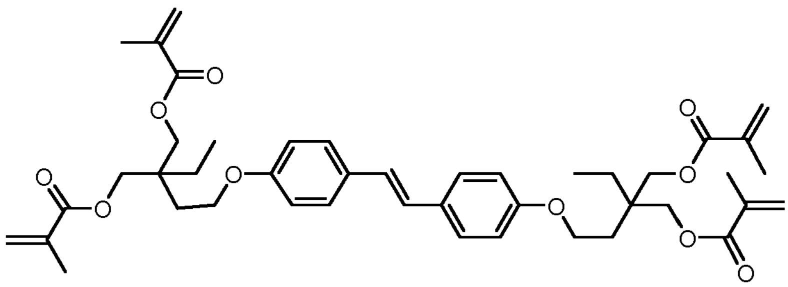

**8**

To a suspension of 1.7 g (79% 2.8 mmol) **7** in 8 ml dichloromethane, 55 mg DMAP and 0.9 ml Methacrylic acid (10.5 mmol) and 3.2 ml (17 mmol) N-(3-Dimethylaminopropyl)-N'-ethylcarbodiimid are added not exceeding a temperature of 5°C. After stirring for 1 h at 5°C the cooling is removed and the mixture is further stirred over night. The crude mixture is filtrated over silica gel (DCM) and the solvent is evaporated. The crude product is purified by column chromatography (ACN/ MTBE) to give **8** after recrystallization from acetonitrile.

2. Synthesis Example 2

2.1 Synthesis of [(E)-1,2-difluoro-2-(4-tetrahydropyran-2-yloxyphenyl)vinyl]-triethyl-silane (9)

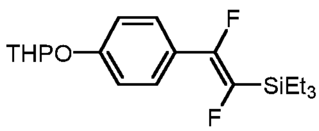

**9**

Under an inert gas athmosphere a solution of 83.4 g (32 mmol) 2-(4-bromophenoxy)tetrahydropyran in 800 ml THF is prepared and and 213 ml 1.6 mol/l Butyllithium are slowly added at a temperature of -70°C. The mixture is stirred for further 90 mins at this temperature and afterwards, a solution of 72.3 g (35 mmol) triethyl(1,2,2-trifluorovinyl)silane in 200 ml THF are added at a temperature of -70°C.. After further stirring the mixture for an additional hour, the cooling is removed and the mixture is stirred overnight. The reaction mixture is quenched with water and extrated with MTBE.

The combined organic phases are washed with bine and the combined organic phases are dried over sodium sulfate, filtrated and the solvent is removed. The crude product is purified over silica gel (Bu-Cl/n-pentane 7:3) to give 9.

2.2 Synthesis of 2-[4-[(E)-1,2-difluorovinyl]phenoxy]tetrahydropyran (10)

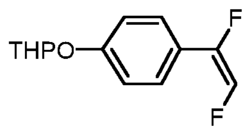

**10**

To a solution of 91 g (90%, 23 mmol) **9** in 500 ml THF, 250 ml 1mol/l Tetrabutylammoniumfluorid in THF (25 mmol) are added at a temperature of 10°C. After stirring the mixture for an additional hour at that temperature, the colling is removed and the mixture is stirred overnight.

The mixture is diluted with MTBE and water is added. The aqoues phase is extracted with MTBE and the combined organic phases are dried over sodium sulfate, filtrated and the solvent is evaporated. The crude product ist purified over silica gel (butal chloride) to give 10.

2.3 Synthesis of 2-[4-[(Z)-1,2-difluoro-2-iodo-vinyl]phenoxy]tetrahydropyran (11)

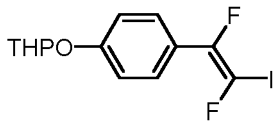

**11**

Under an inert gas athmosphere, a solution 165 ml mol/l Butyllithium (15% in n-Hexan; 26 mmol) is slowly added to a solution of 54.8 g (21 mmol) of **10** in 600 ml THF at a temperature of -70°C. After further stirring of this mixture for 1 h at that temperature a solution of 67 g Iodine in 400 ml THF is slowly added. The mixture is subsequently stirred for 30 mins at -70°C and -50°C, respectively and the cooling is removed. After the mixture is brought to roomtemperature, water is added and the aqoues phase is extracted with MTBE. The mixture is washed with sodium thiosulfate and the pH of the aques phase is adjusted to 9 with 2N sodium hydroxide. The phases are separated and the aqoues phase is extracted with MTBE. The combined organic phases are washed with brine, dried over sodium sulfate , filtrated and the solvent is evaporated. The crude product is purified over silica gel (n-Heptane/10% Ethyl acetate) to give **11.**

2.4 Synthesis of 4-[(E)-1,2-difluoro-2-(4-hydroxyphenyl)vinyl]phenol (12)

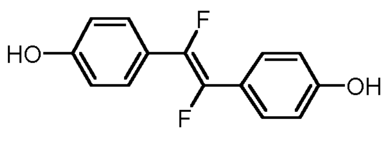

**12**

To a solution of 59 g (91%ig; 150mmol) 11 and 31 g (220 mmol) potassium carbonate in 600 ml THF and 200 ml Wasser, 810 mg CataCXium A and 750 mg Tris(dibenzylidenacetone)Palladium(0) are added under reflux while vigourously stirring. Subsequently, 21.4 g (160 mmol) (4-hydroxyphenyl)boronic acid are added and the mixture is stirred for 2 h under reflux. The mixture is diluted with water, acidified with acetic acid and extracted with MTBE. The combined organic phases are washed with water, brine, dried over sodium sulfate, filtrated and the solvent is evaporated. The crude product is purified over silica gel and recrystallized from ethylacetate/n-heptane to give 12.

2.5 Synthesis of [4-[4-[(E)-2-[4-[3,3-bis(2-methylprop-2-enoyloxymethyl)pentoxy]phenyl]-1,2-difluoro-vinyl]phenoxy]-2-ethyl-2-(2-methylprop-2-enoyloxymethyl)butyl] 2-methylprop-2-enoate (**13** - Photoalignment additive 2)

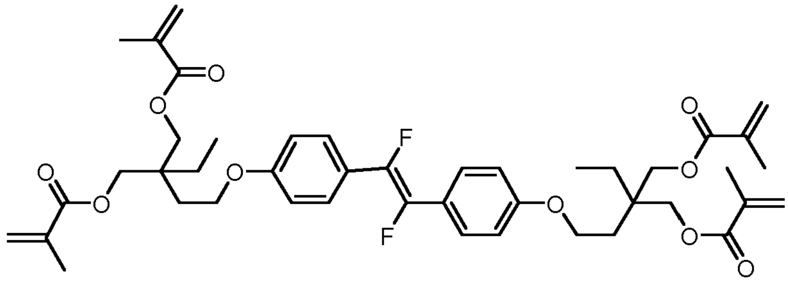

**13**

In analogy to the synthesis steps 1.6 to 1.8, 13 can be obtained from intermediate compound 12.

3. Synthesis Example 3

3.1 Synthesis of 4-[(E)-2-[4-[3,3-bis[[tert-butyl(dimethyl)silyl]oxymethyl]pentoxy]phenyl]vinyl]phenol (14)

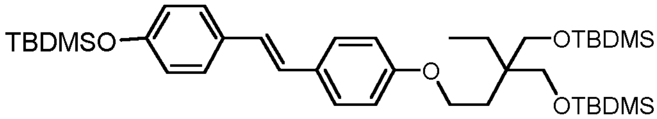

**14**

To a mixture of 11.7 g (44 mmol) Triphenyl phosphine in 15 ml THF, 9.2 ml (42 mmol) Diisopropylazodicarboxylate are added slowly not exceeding a temperature of 22°C. Afterwards, 16.4 g (40 mmol) 3,3bis[[tert-butyl(dimethyl)silyl]oxy-methyl]-pentan-1-ol in 20 ml THF and 5.0 g (96.7%, 20 mmol) **5** are added subsequently and the resulting mixture is stirred over night. Isolute ® HM-N is added and the solvent is evaporated. The crude product is then purified over silica gel (eleuent: n-Heptane/Butylchloride, 1:1) and subsequently purified over RP-silica gel (MTBE/acetonitrile, 4:6) to give a mixture of **14.**

3.2 Synthesis of 2-ethyl-2-[2-[4-[(E)-2-(4-hydroxyphenyl)vinyl]phenoxy]ethyl]propane-1,3-diol (15)

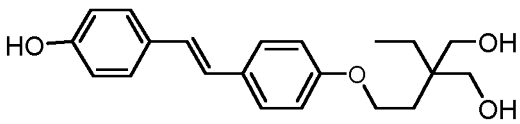

**15**

To a solution of 4.1 g (10 mmol) of **14** in 35 ml dichloromethane, 6.8 ml (56 mmol) Triethylaminotrishydrofluoride are added at roomtemperature and stirred for 2 d. Ioslute is added and the solvent is evaporated. Finally, the crude product is purified over silica gel (dichloromethane/25-50% THF; dichloromethane /50-100% MTB-Ether) to give **15.**

3.2 Synthesis of [2-ethyl-2-[(1-methylene-2-oxo-propoxy)methyl]-4-[4-[(E)-2-[4-(2-methylprop-2-enoyloxy)phenyl]vinyl]phenoxy]butyl] 2-methylprop-2-enoate (16 - Photoalignment additive 3)

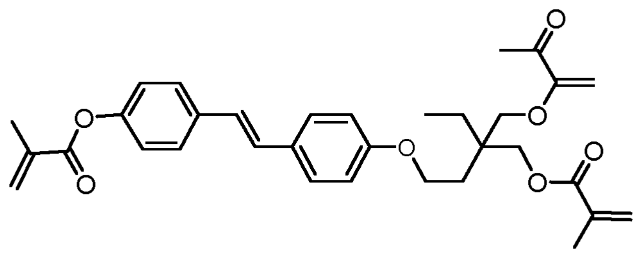

**16**

To a suspension of 1 g (88% 2.3 mmol) **15** in 6 ml dichloromethane, 45 mg DMAP and 1.2 ml Methacrylic acid (14 mmol) and 2.5 ml (17 mmol) N-(3-Dimethylaminopropyl)-N'-ethylcarbodiimid are added not exceeding a temperature of 5°C. After stirring for 1 h at 5°C the cooling is removed and the mixture is further stirred over night. The crude mixture is filtrated over silica gel (DCM) and the solvent is evaporated. The crude product is purified by column chromatography (RP-18; acetonitrile/ 10 % water) to give **16** after recrystallization from acetonitrile.

$^1$H NMR (500 MHz, Chloroform-d) δ 7.83 - 7.76 (m, 2H), 7.73 - 7.66 (m, 2H), 7.28 - 7.21 (m, 2H), 6.99 - 6.92 (m, 2H), 6.39 (p, *J* = 1.1 Hz, 1H), 6.12 (dq, *J* = 2.1, 1.0 Hz, 2H), 5.80 (p, *J* = 1.5 Hz, 1H), 5.60 (p, *J* = 1.5 Hz, 2H), 4.23 - 4.13 (m, 6H), 2.11 (dd, *J*= 1.6, 1.0 Hz, 3H), 2.02 - 1.94 (m, 8H), 1.60 (q, *J*= 7.5 Hz, 2H), 0.99 (t, *J*= 7.5 Hz, 3H).

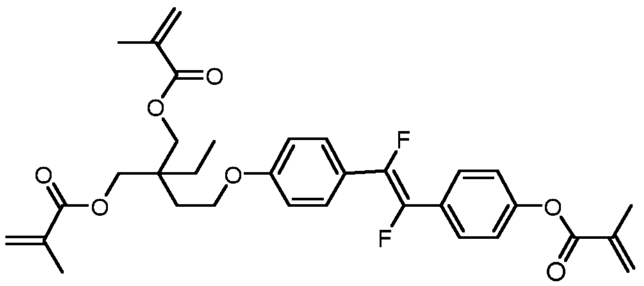

**17**

In analogy to the synthesis steps 3.1 to 3.2, 17 can be obtained from intermediate compound 12.

Utilized stabilizers for nematic host mixtures

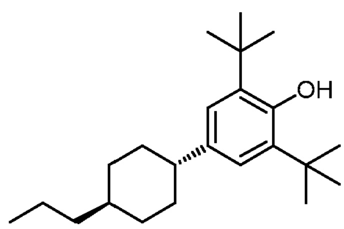

STAB-1

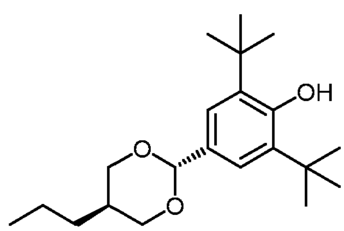

STAB-2

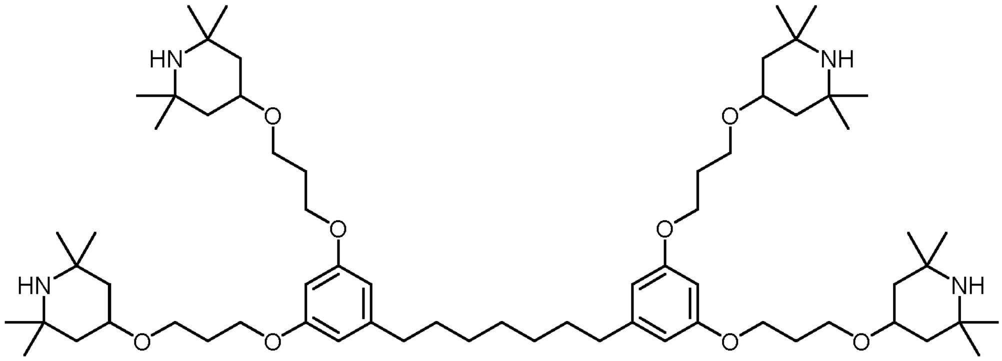

STAB-3

Utilized Nematic host mixtures

The nematic LC host mixture N-1 is prepared as indicated in the following table:

| | | | |
|---|---|---|---|
| CC-3-V | 36.00 | cl.p. [°C]: | 78 |
| CC-3-V1 | 5.00 | $n_e$ [589 nm, 20°C]: | 1.5907 |
| CCP-V-1 | 8.00 | Δn [589 nm, 20°C]: | 0.1095 |
| PGP-2-2V | 3.00 | $\varepsilon_{\parallel}$ [1 kHz, 20°C]: | 16.6 |
| CCQU-3-F | 9.5 | $\varepsilon_{\perp}$ [1 kHz, 20°C]: | 3.7 |
| PUQU-3-F | 8.5 | Δε [1 kHz, 20°C]: | 12.9 |
| APUQU-2-F | 5.00 | $K_1$ [pN, 20°C]: | 12.1 |
| APUQU-3-F | 8.00 | $K_3$ [pN, 20°C]: | 13.4 |
| PGUQU-3-F | 4.00 | $K_3/K_1$ [pN, 20°C]: | 1.11 |
| PGUQU-4-F | 8.00 | $V_0$ [V, 20°C]: | 1.01 |
| PGUQU-5-F | 5.00 | LTS bulk [h, -20°C]: | 1000 |
| Σ | 100.0 | | |

The nematic LC host mixture N-2 is prepared as indicated in the following table:

| | | | |
|---|---|---|---|
| B(S)-2O-O5 | 3.0 | Clearing point [°C]: | 98.9 |
| CC-3-V | 29.5 | Δn [589 nm, 20°C]: | 0.0922 |
| CC-3-V1 | 8.0 | $\varepsilon_{\parallel}$ [1 kHz, 20°C]: | 3.4 |
| CCH-35 | 5.5 | $\varepsilon_{\perp}$ [1 kHz, 20°C]: | 7.3 |
| CCY-3-O1 | 6.0 | Δε [1 kHz, 20°C]: | -3.9 |
| CCY-3-O2 | 8.0 | $K_1$ [pN, 20°C]: | 18.5 |
| CLY-3-O2 | 9.0 | $K_3$ [pN, 20°C]: | 19.2 |
| CLY-3-O3 | 6.0 | $V_0$ [V, 20°C]: | 2.34 |
| CLY-4-O2 | 5.0 | $\gamma_1$ [mPa s, 20°C]: | 132 |
| CLY-5-O2 | 5.0 | $\gamma_1$ [mPa s, 20°C]: | |
| COB(S)-2-O4 | 5.0 | LTS bulk [h, -10°C]: | |
| CY-3-O2 | 10.0 | LTS bulk [h, -20°C]: | |
| | 100.0 | LTS bulk [h, -30°C]: | |

The nematic LC host mixture N-3 is prepared as indicated in the following table:

| | | | |
|---|---|---|---|
| N-2 | 99.97 | Clearing point [°C]: | |
| STAB-1 | 0.03 | Δn [589 nm, 20°C]: | |
| Σ | 100.0 | $\varepsilon_{\parallel}$ [1 kHz, 20°C]: | |
| | | $\varepsilon_{\perp}$ [1 kHz, 20°C]: | |

(continued)

| | | |
|---|---|---|
| | | Δε [1 kHz, 20°C]: |
| | | $K_1$ [pN, 20°C]: |
| | | $K_3$ [pN, 20°C]: |
| | | $V_0$ [V, 20°C]: |
| | | $\gamma_1$ [mPa s, 20°C]: |
| | | $\gamma_1$ [mPa s, 20°C]: |
| | | LTS bulk [h, -10°C] |
| | | LTS bulk [h, -20°C] |
| | | LTS bulk [h, -30°C] |

The nematic LC host mixture N-4 is prepared as indicated in the following table:

| | | |
|---|---|---|
| N-3 | 99.99 | Clearing point [°C]: |
| STAB-3 | 0.01 | Δn [589 nm, 20°C]: |
| Σ | 100.0 | $\varepsilon_{\parallel}$ [1 kHz, 20°C]: |
| | | $\varepsilon_{\perp}$ [1 kHz, 20°C]: |
| | | Δε [1 kHz, 20°C]: |
| | | $K_1$ [pN, 20°C]: |
| | | $K_3$ [pN, 20°C]: |
| | | $V_0$ [V, 20°C]: |
| | | $\gamma_1$ [mPa s, 20°C]: |
| | | $\gamma_1$ [mPa s, 20°C]: |
| | | LTS bulk [h, -10°C] |
| | | LTS bulk [h, -20°C] |
| | | LTS bulk [h, -30°C] |

The nematic LC host mixture N-5 is prepared as indicated in the following table:

| | | |
|---|---|---|
| N-3 | 99.98 | Clearing point [°C]: |
| STAB-3 | 0.02 | Δn [589 nm, 20°C]: |
| Σ | 100.0 | $\varepsilon_{\parallel}$ [1 kHz, 20°C]: |
| | | $\varepsilon_{\perp}$ [1 kHz, 20°C]: |
| | | Δε [1 kHz, 20°C]: |
| | | $K_1$ [pN, 20°C]: |
| | | $K_3$ [pN, 20°C]: |
| | | $V_0$ [V, 20°C]: |
| | | $\gamma_1$ [mPa s, 20°C]: |
| | | $\gamma_1$ [mPa s, 20°C]: |
| | | LTS bulk [h, -10°C] |
| | | LTS bulk [h, -20°C] |
| | | LTS bulk [h, -30°C] |

The nematic LC host mixture N-6 is prepared as indicated in the following table:

| | | |
|---|---|---|
| N-3 | 99.97 | Clearing point [°C]: |
| STAB-3 | 0.03 | Δn [589 nm, 20°C]: |
| Σ | 100.0 | $\varepsilon_{\parallel}$ [1 kHz, 20°C]: |
| | | $\varepsilon_{\perp}$ [1 kHz, 20°C]: |
| | | Δε [1 kHz, 20°C]: |
| | | $K_1$ [pN, 20°C]: |

(continued)

| | | |
|---|---|---|
| | | $K_3$ [pN, 20°C]: |
| | | $V_0$ [V, 20°C]: |
| | | $\gamma_1$ [mPa s, 20°C]: |
| | | $\gamma_1$ [mPa s, 20°C]: |
| | | LTS bulk [h, -10°C] |
| | | LTS bulk [h, -20°C] |
| | | LTS bulk [h, -30°C] |

The nematic LC host mixture N-7 is prepared as indicated in the following table:

| | | |
|---|---|---|
| N-3 | 99.95 | Clearing point [°C]: |
| STAB-3 | 0.05 | $\Delta n$ [589 nm, 20°C]: |
| $\Sigma$ | 100.0 | $\varepsilon_\parallel$ [1 kHz, 20°C]: |
| | | $\varepsilon_\perp$ [1 kHz, 20°C]: |
| | | $\Delta\varepsilon$ [1 kHz, 20°C]: |
| | | $K_1$ [pN, 20°C]: |
| | | $K_3$ [pN, 20°C]: |
| | | $V_0$ [V, 20°C]: |
| | | $\gamma_1$ [mPa s, 20°C]: |
| | | $\gamma_1$ [mPa s, 20°C]: |
| | | LTS bulk [h, -10°C] |
| | | LTS bulk [h, -20°C] |
| | | LTS bulk [h, -30°C] |

The nematic LC host mixture N-8 is prepared as indicated in the following table:

| | | |
|---|---|---|
| N-3 | 99.9 | Clearing point [°C]: |
| STAB-3 | 0.1 | $\Delta n$ [589 nm, 20°C]: |
| $\Sigma$ | 100.0 | $\varepsilon_\parallel$ [1 kHz, 20°C]: |
| | | $\varepsilon_\perp$ [1 kHz, 20°C]: |
| | | $\Delta\varepsilon$ [1 kHz, 20°C]: |
| | | $K_1$ [pN, 20°C]: |
| | | $K_3$ [pN, 20°C]: |
| | | $V_0$ [V, 20°C]: |
| | | $\gamma_1$ [mPa s, 20°C]: |
| | | $\gamma_1$ [mPa s, 20°C]: |
| | | LTS bulk [h, -10°C] |
| | | LTS bulk [h, -20°C] |
| | | LTS bulk [h, -30°C] |

The nematic LC host mixture N-9 is prepared as indicated in the following table:

| | | | |
|---|---|---|---|
| CCY-3-O1 | 5.0 | Clearing point [°C]: | 80.5 |
| CCY-3-O2 | 5.5 | $\Delta n$ [589 nm, 20°C]: | 0.1047 |
| CCY-4-O2 | 5.5 | $\varepsilon_\parallel$ [1 kHz, 20°C]: | 3.7 |
| CLY-2-O4 | 3.0 | $\varepsilon_\perp$ [1 kHz, 20°C]: | 7.9 |
| CLY-3-O2 | 6.0 | $\Delta\varepsilon$ [1 kHz, 20°C]: | -4.2 |
| CLY-3-O3 | 6.0 | $K_1$ [pN, 20°C]: | 14.5 |
| PGIY-2-O4 | 5.0 | $K_3$ [pN, 20°C]: | 14.9 |
| CC-3-V | 38.5 | $V_0$ [V, 20°C]: | 1.98 |

(continued)

| | | | |
|---|---|---|---|
| CY-3-O2 | 9.0 | $\gamma_1$ [mPa s, 20°C]: | 102 |
| PY-3-O2 | 5.5 | $\gamma_1$ [mPa s, 20°C]: | |
| B(S)-2O-O4 | 4.0 | LTS bulk [h, -10°C]: | |
| B(S)-2O-O5 | 4.0 | LTS bulk [h, -20°C]: | |
| PYP-2-3 | 3.0 | LTS bulk [h, -30°C]: | 264 |
| Σ | 100.0 | | |

The nematic LC host mixture N-10 is prepared as indicated in the following table:

| | | |
|---|---|---|
| N-9 | 99.96 | Clearing point [°C]: |
| STAB-3 | 0.01 | $\Delta n$ [589 nm, 20°C]: |
| STAB-1 | 0.03 | $\varepsilon_\parallel$ [1 kHz, 20°C]: |
| Σ | 100.0 | $\varepsilon_\perp$ [1 kHz, 20°C]: |
| | | $\Delta\varepsilon$ [1 kHz, 20°C]: |
| | | $K_1$ [pN, 20°C]: |
| | | $K_3$ [pN, 20°C]: |
| | | $V_0$ [V, 20°C]: |
| | | $\gamma_1$ [mPa s, 20°C]: |
| | | $\gamma_1$ [mPa s, 20°C]: |
| | | LTS bulk [h, -10°C] |
| | | LTS bulk [h, -20°C] |
| | | LTS bulk [h, -30°C] |

The nematic LC host mixture N-11 is prepared as indicated in the following table:

| | | |
|---|---|---|
| APUQU-2-F | 7.5 | Clearing point [°C]: |
| APUQU-3-F | 7.0 | $\Delta n$ [589 nm, 20°C]: |
| CC-3-V | 32.5 | $\varepsilon_\parallel$ [1 kHz, 20°C]: |
| CC-3-V1 | 7.5 | $\varepsilon_\perp$ [1 kHz, 20°C]: |
| CCP-V-1 | 10.0 | $\Delta\varepsilon$ [1 kHz, 20°C]: |
| CCP-V2-1 | 10.0 | $K_1$ [pN, 20°C]: |
| CCVC-3-V | 1.0 | $K_3$ [pN, 20°C]: |
| CDUQU-3-F | 0.5 | $V_0$ [V, 20°C]: |
| DPGU-4-F | 3.5 | $\gamma_1$ [mPa s, 20°C]: |
| PPGU-3-F | 0.5 | $\gamma_1$ [mPa s, 20°C]: |
| PUQU-3-F | 6.5 | LTS bulk [h, -10°C] |
| PY-3-O2 | 13.5 | LTS bulk [h, -20°C] |
| Σ | 100.0 | LTS bulk [h, -30°C] |

The nematic LC host mixture N-12 is prepared as indicated in the following table:

| | | |
|---|---|---|
| N-11 | 99.86 | Clearing point [°C]: |
| STAB-2 | 0.04 | $\Delta n$ [589 nm, 20°C]: |
| STAB-3 | 0.1 | $\varepsilon_\parallel$ [1 kHz, 20°C]: |
| Σ | 100.0 | $\varepsilon_\perp$ [1 kHz, 20°C]: |
| | | $\Delta\varepsilon$ [1 kHz, 20°C]: |
| | | $K_1$ [pN, 20°C]: |
| | | $K_3$ [pN, 20°C]: |
| | | $V_0$ [V, 20°C]: |
| | | $\gamma_1$ [mPa s, 20°C]: |

(continued)

| | | |
|---|---|---|
| | | $\gamma_1$ [mPa s, 20°C]: |
| | | LTS bulk [h, -10°C] |
| | | LTS bulk [h, -20°C] |
| | | LTS bulk [h, -30°C] |

The nematic LC host mixture N-13 is prepared as indicated in the following table:

| | | |
|---|---|---|
| N-11 | 99.86 | Clearing point [°C]: |
| STAB-2 | 0.04 | $\Delta$n [589 nm, 20°C]: |
| STAB-3 | 0.1 | $\varepsilon_\parallel$ [1 kHz, 20°C]: |
| $\Sigma$ | 100.0 | $\varepsilon_\perp$ [1 kHz, 20°C]: |
| | | $\Delta\varepsilon$ [1 kHz, 20°C]: |
| | | $K_1$ [pN, 20°C]: |
| | | $K_3$ [pN, 20°C]: |
| | | $V_0$ [V, 20°C]: |
| | | $\gamma_1$ [mPa s, 20°C]: |
| | | $\gamma_1$ [mPa s, 20°C]: |
| | | LTS bulk [h, -10°C] |
| | | LTS bulk [h, -20°C] |
| | | LTS bulk [h, -30°C] |

The nematic LC host mixture N-14 is prepared as indicated in the following table:

| | | |
|---|---|---|
| N-11 | 99.94 | Clearing point [°C]: |
| STAB-2 | 0.04 | $\Delta$n [589 nm, 20°C]: |
| STAB-3 | 0.02 | $\varepsilon_\parallel$ [1 kHz, 20°C]: |
| $\Sigma$ | 100.0 | $\varepsilon_\perp$ [1 kHz, 20°C]: |
| | | $\Delta\varepsilon$ [1 kHz, 20°C]: |
| | | $K_1$ [pN, 20°C]: |
| | | $K_3$ [pN, 20°C]: |
| | | $V_0$ [V, 20°C]: |
| | | $\gamma_1$ [mPa s, 20°C]: |
| | | $\gamma_1$ [mPa s, 20°C]: |
| | | LTS bulk [h, -10°C] |
| | | LTS bulk [h, -20°C] |
| | | LTS bulk [h, -30°C] |

The nematic LC host mixture N-15 is prepared as indicated in the following table:

| | | | |
|---|---|---|---|
| CC-3-V | 24.5 | Clearing point [°C]: | 85.9 |
| CC-3-V1 | 12.0 | $\Delta$n [589 nm, 20°C]: | 0.0933 |
| CCP-V-1 | 13.5 | $\varepsilon_\parallel$ [1 kHz, 20°C]: | 3.4 |
| CPY-3-O2 | 1.0 | $\varepsilon_\perp$ [1 kHz, 20°C]: | 6.7 |
| CCY-5-O2 | 3.0 | $\Delta\varepsilon$ [1 kHz, 20°C]: | -3.3 |
| CLY-3-O2 | 9.0 | $K_1$ [pN, 20°C]: | 15.6 |
| CLY-4-O2 | 5.5 | $K_3$ [pN, 20°C]: | 18.0 |
| CLY-5-O2 | 5.5 | $V_0$ [V, 20°C]: | 2.46 |
| CY-3-O2 | 12.0 | $\gamma_1$ [mPa s, 20°C]: | 101 |
| CY-3-O4 | 4.0 | $\gamma_1$ [mPa s, 20°C]: | |
| CY-5-O2 | 4.0 | LTS bulk [h, -10°C]: | |

(continued)

| | | | |
|---|---|---|---|
| B(S)-2O-O4 | 4.0 | LTS bulk [h, -20°C]: | 1000 |
| B(S)-2O-O5 | 2.0 | LTS bulk [h, -30°C]: | |
| Σ | 100.0 | | |

The nematic LC host mixture N-16 is prepared as indicated in the following table:

| | | |
|---|---|---|
| N-15 | 99.9 | Clearing point [°C]: |
| STAB-3 | 0.1 | $\Delta n$ [589 nm, 20°C]: |
| Σ | 100.0 | $\varepsilon_{\parallel}$ [1 kHz, 20°C]: |
| | | $\varepsilon_{\perp}$ [1 kHz, 20°C]: |
| | | $\Delta\varepsilon$ [1 kHz, 20°C]: |
| | | $K_1$ [pN, 20°C]: |
| | | $K_3$ [pN, 20°C]: |
| | | $V_0$ [V, 20°C]: |
| | | $\gamma_1$ [mPa s, 20°C]: |
| | | $\gamma_1$ [mPa s, 20°C]: |
| | | LTS bulk [h, -10°C] |
| | | LTS bulk [h, -20°C] |
| | | LTS bulk [h, -30°C] |

The nematic LC host mixture N-17 is prepared as indicated in the following table:

| | | |
|---|---|---|
| N-15 | 99.87 | Clearing point [°C]: |
| STAB-1 | 0.03 | $\Delta n$ [589 nm, 20°C]: |
| STAB-3 | 0.1 | $\varepsilon_{\parallel}$ [1 kHz, 20°C]: |
| Σ | 100.0 | $\varepsilon_{\perp}$ [1 kHz, 20°C]: |
| | | $\Delta\varepsilon$ [1 kHz, 20°C]: |
| | | $K_1$ [pN, 20°C]: |
| | | $K_3$ [pN, 20°C]: |
| | | $V_0$ [V, 20°C]: |
| | | $\gamma_1$ [mPa s, 20°C]: |
| | | $\gamma_1$ [mPa s, 20°C]: |
| | | LTS bulk [h, -10°C] |
| | | LTS bulk [h, -20°C] |
| | | LTS bulk [h, -30°C] |

The nematic LC host mixture N-18 is prepared as indicated in the following table:

| | | |
|---|---|---|
| B(S)-2O-O4 | 4.0 | Clearing point [°C]: |
| B(S)-2O-O5 | 4.0 | $\Delta n$ [589 nm, 20°C]: |
| B(S)-2O-O6 | 2.0 | $\varepsilon_{\parallel}$ [1 kHz, 20°C]: |
| CC-3-V | 27.0 | $\varepsilon_{\perp}$ [1 kHz, 20°C]: |
| CC-3-V1 | 5.25 | $\Delta\varepsilon$ [1 kHz, 20°C]: |
| CCP-3-1 | 9.0 | $K_1$ [pN, 20°C]: |
| CCP-3-3 | 5.5 | $K_3$ [pN, 20°C]: |
| CLY-2-O4 | 2.5 | $V_0$ [V, 20°C]: |
| CLY-3-O2 | 7.0 | $\gamma_1$ [mPa s, 20°C]: |
| CLY-3-O3 | 6.0 | $\gamma_1$ [mPa s, 20°C]: |
| COB(S)-2-O4 | 3.25 | LTS bulk [h, -10°C] |
| CPY-3-O2 | 2.5 | LTS bulk [h, -20°C] |

(continued)

| | | |
|---|---|---|
| CY-3-O2 | 11.0 | LTS bulk [h, -30°C] |
| PY-1-O2 | 4.0 | |
| PY-3-O2 | 7.0 | |
| Σ | 100.0 | |

The nematic LC host mixture N-19 is prepared as indicated in the following table:

| | | |
|---|---|---|
| N-18 | 99.87 | Clearing point [°C]: |
| STAB-1 | 0.03 | Δn [589 nm, 20°C]: |
| STAB-3 | 0.1 | $\varepsilon_{\parallel}$ [1 kHz, 20°C]: |
| | 100.0 | $\varepsilon_{\perp}$ [1 kHz, 20°C]: |
| | | Δε [1 kHz, 20°C]: |
| | | $K_1$ [pN, 20°C]: |
| | | $K_3$ [pN, 20°C]: |
| | | $V_0$ [V, 20°C]: |
| | | $\gamma_1$ [mPa s, 20°C]: |
| | | $\gamma_1$ [mPa s, 20°C]: |
| | | LTS bulk [h, -10°C] |
| | | LTS bulk [h, -20°C] |
| | | LTS bulk [h, -30°C] |

The nematic LC host mixture N-20 is prepared as indicated in the following table:

| | | |
|---|---|---|
| BCH-32 | 4.0 | Clearing point [°C]: |
| CC-3-2V1 | 4.0 | Δn [589 nm, 20°C]: |
| CC-3-V | 33.0 | $\varepsilon_{\parallel}$ [1 kHz, 20°C]: |
| CC-3-V1 | 6.0 | $\varepsilon_{\perp}$ [1 kHz, 20°C]: |
| CCGU-3-F | 5.5 | Δε [1 kHz, 20°C]: |
| CCP-V-1 | 15.0 | $K_1$ [pN, 20°C]: |
| CCP-V2-1 | 4.0 | $K_3$ [pN, 20°C]: |
| CCVC-3-V | 5.0 | $V_0$ [V, 20°C]: |
| CLY-3-O2 | 6.0 | $\gamma_1$ [mPa s, 20°C]: |
| DPGU-4-F | 2.0 | $\gamma_1$ [mPa s, 20°C]: |
| PGUQU-3-F | 1.0 | LTS bulk [h, -10°C] |
| PGUQU-4-F | 2.0 | LTS bulk [h, -20°C] |
| PP-1-2V1 | 3.0 | LTS bulk [h, -30°C] |
| PPGU-3-F | 0.5 | |
| PUQU-3-F | 9.0 | |
| Σ | 100.0 | |

The nematic LC host mixture N-21 is prepared as indicated in the following table:

| | | |
|---|---|---|
| N-20 | 99.86 | Clearing point [°C]: |
| STAB-2 | 0.04 | Δn [589 nm, 20°C]: |
| STAB-3 | 0.1 | $\varepsilon_{\parallel}$ [1 kHz, 20°C]: |
| Σ | 100.0 | $\varepsilon_{\perp}$ [1 kHz, 20°C]: |
| | | Δε [1 kHz, 20°C]: |
| | | $K_1$ [pN, 20°C]: |
| | | $K_3$ [pN, 20°C]: |
| | | $V_0$ [V, 20°C]: |

(continued)

| | |
|---|---|
| $\gamma_1$ [mPa s, 20°C]: | |
| $\gamma_1$ [mPa s, 20°C]: | |
| LTS bulk [h, -10°C] | |
| LTS bulk [h, -20°C] | |
| LTS bulk [h, -30°C] | |

The nematic LC host mixture N-22 is prepared as indicated in the following table:

| | | | |
|---|---|---|---|
| CC-3-V | 29.0 | Clearing point [°C]: | 80.1 |
| CCY-3-O1 | 8.0 | $\Delta$n [589 nm, 20°C]: | 0.1052 |
| CCY-3-O2 | 6.0 | $\varepsilon_{\parallel}$ [1 kHz, 20°C]: | 3.9 |
| CCY-4-O2 | 2.0 | $\varepsilon_{\perp}$ [1 kHz, 20°C]: | 8.7 |
| CLY-3-O2 | 8.5 | $\Delta\varepsilon$ [1 kHz, 20°C]: | -4.7 |
| CLY-3-O3 | 7.5 | $K_1$ [pN, 20°C]: | 14.0 |
| CPY-2-O2 | 10.0 | $K_3$ [pN, 20°C]: | 15.4 |
| CPY-3-O2 | 7.5 | $V_0$ [V, 20°C]: | 1.91 |
| CY-3-O2 | 6.5 | $\gamma_1$ [mPa s, 20°C]: | 125 |
| PY-3-O2 | 10.0 | $\gamma_1$ [mPa s, 20°C]: | |
| Y-4O-O4 | 5.0 | LTS bulk [h, -10°C]: | |
| $\Sigma$ | 100.0 | LTS bulk [h, -20°C]: | |
| | | LTS bulk [h, -30°C]: | |

The nematic LC host mixture N-23 is prepared as indicated in the following table:

| | | | |
|---|---|---|---|
| N-22 | 99.98 | Clearing point [°C]: | 80 |
| STAB-3 | 0.02 | $\Delta$n [589 nm, 20°C]: | 0.1054 |
| $\Sigma$ | 100.0 | $\varepsilon_{\parallel}$ [1 kHz, 20°C]: | 3.9 |
| | | $\varepsilon_{\perp}$ [1 kHz, 20°C]: | 8.7 |
| | | $\Delta\varepsilon$ [1 kHz, 20°C]: | -4.7 |
| | | $K_1$ [pN, 20°C]: | 14.1 |
| | | $K_3$ [pN, 20°C]: | 15.4 |
| | | $V_0$ [V, 20°C]: | 1.92 |
| | | $\gamma_1$ [mPa s, 20°C]: | 123 |
| | | $\gamma_1$ [mPa s, 20°C]: | |
| | | LTS bulk [h, -10°C]: | |
| | | LTS bulk [h, -20°C]: | |
| | | LTS bulk [h, -30°C]: | |

The nematic LC host mixture N-24 is prepared as indicated in the following table:

| | | |
|---|---|---|
| CC-3-V | 29.0 | Clearing point [°C]: |
| CCY-3-O1 | 8.0 | $\Delta$n [589 nm, 20°C]: |
| CCY-3-O2 | 6.0 | $\varepsilon_{\parallel}$ [1 kHz, 20°C]: |
| CCY-4-O2 | 2.0 | $\varepsilon_{\perp}$ [1 kHz, 20°C]: |
| CLY-3-O2 | 8.5 | $\Delta\varepsilon$ [1 kHz, 20°C]: |
| CLY-3-O3 | 7.5 | $K_1$ [pN, 20°C]: |
| CPY-2-O2 | 10.0 | $K_3$ [pN, 20°C]: |
| CPY-3-O2 | 7.5 | $V_0$ [V, 20°C]: |
| CY-3-O2 | 6.5 | $\gamma_1$ [mPa s, 20°C]: |
| PY-3-O2 | 10.0 | $\gamma_1$ [mPa s, 20°C]: |

(continued)

| | | |
|---|---|---|
| Y-4O-O4 | 5.0 | LTS bulk [h, -10°C] |
| Σ | 100.0 | LTS bulk [h, -20°C] |
| | | LTS bulk [h, -30°C] |

The nematic LC host mixture N-25 is prepared as indicated in the following table:

| | | | |
|---|---|---|---|
| N-24 | 99.94 | Clearing point [°C]: | 80 |
| STAB-3 | 0.02 | Δn [589 nm, 20°C]: | 0.1054 |
| STAB-2 | 0.04 | $\varepsilon_{\parallel}$ [1 kHz, 20°C]: | 3.9 |
| Σ | 100.0 | $\varepsilon_{\perp}$ [1 kHz, 20°C]: | 8.7 |
| | | Δε [1 kHz, 20°C]: | -4.7 |
| | | $K_1$ [pN, 20°C]: | 14.1 |
| | | $K_3$ [pN, 20°C]: | 15.4 |
| | | $V_0$ [V, 20°C]: | 1.92 |
| | | $\gamma_1$ [mPa s, 20°C]: | 123 |
| | | $\gamma_1$ [mPa s, 20°C]: | |
| | | LTS bulk [h, -10°C]: | |
| | | LTS bulk [h, -20°C]: | |
| | | LTS bulk [h, -30°C]: | |

The nematic LC host mixture N-26 is prepared as indicated in the following table:

| | | | |
|---|---|---|---|
| N-24 | 99.91 | Clearing point [°C]: | 79.6 |
| STAB-3 | 0.05 | Δn [589 nm, 20°C]: | 0.1056 |
| STAB-2 | 0.04 | $\varepsilon_{\parallel}$ [1 kHz, 20°C]: | 4.0 |
| Σ | 100.0 | $\varepsilon_{\perp}$ [1 kHz, 20°C]: | 8.7 |
| | | Δε [1 kHz, 20°C]: | -4.7 |
| | | $K_1$ [pN, 20°C]: | 13.7 |
| | | $K_3$ [pN, 20°C]: | 15.0 |
| | | $V_0$ [V, 20°C]: | 1.89 |
| | | $\gamma_1$ [mPa s, 20°C]: | 123 |
| | | $\gamma_1$ [mPa s, 20°C]: | |
| | | LTS bulk [h, -10°C]: | |
| | | LTS bulk [h, -20°C]: | |
| | | LTS bulk [h, -30°C]: | -1 |

The nematic LC host mixture N-27 is prepared as indicated in the following table:

| | | | |
|---|---|---|---|
| N-24 | 99.86 | Clearing point [°C]: | 79.4 |
| STAB-3 | 0.1 | Δn [589 nm, 20°C]: | 0.1054 |
| STAB-2 | 0.04 | $\varepsilon_{\parallel}$ [1 kHz, 20°C]: | 4.0 |
| Σ | 100.0 | $\varepsilon_{\perp}$ [1 kHz, 20°C]: | 8.7 |
| | | Δε [1 kHz, 20°C]: | -4.7 |
| | | $K_1$ [pN, 20°C]: | 13.7 |
| | | $K_3$ [pN, 20°C]: | 14.9 |
| | | $V_0$ [V, 20°C]: | 1.89 |
| | | $\gamma_1$ [mPa s, 20°C]: | 122 |
| | | $\gamma_1$ [mPa s, 20°C]: | |
| | | LTS bulk [h, -10°C]: | |
| | | LTS bulk [h, -20°C]: | |

(continued)

| | |
|---|---|
| LTS bulk [h, -30°C]: | -1 |

The nematic LC host mixture N-28 is prepared as indicated in the following table:

| | | | |
|---|---|---|---|
| B(S)-2O-O4 | 4.0 | Clearing point [°C]: | |
| B(S)-2O-O5 | 4.0 | $\Delta n$ [589 nm, 20°C]: | |
| B(S)-2O-O6 | 2.0 | $\varepsilon_{\parallel}$ [1 kHz, 20°C]: | |
| CC-3-V | 31.0 | $\varepsilon_{\perp}$ [1 kHz, 20°C]: | |
| CC-3-V1 | 5.5 | $\Delta\varepsilon$ [1 kHz, 20°C]: | |
| CCY-3-O2 | 8.5 | $K_1$ [pN, 20°C]: | |
| CLY-2-O4 | 5.0 | $K_3$ [pN, 20°C]: | |
| CLY-3-O2 | 8.0 | $V_0$ [V, 20°C]: | |
| CLY-3-O3 | 6.0 | $\gamma_1$ [mPa s, 20°C]: | |
| CLY-5-O2 | 1.0 | $\gamma_1$ [mPa s, 20°C]: | |
| CPY-3-O2 | 4.5 | LTS bulk [h, -10°C] | |
| CY-3-O2 | 12.5 | LTS bulk [h, -20°C] | |
| CY-3-O4 | 8.0 | LTS bulk [h, -30°C] | |
| Σ | 100.0 | | |

The nematic LC host mixture N-29 is prepared as indicated in the following table:

| | | | |
|---|---|---|---|
| N-28 | 99.87 | Clearing point [°C]: | |
| STAB-1 | 0.03 | $\Delta n$ [589 nm, 20°C]: | |
| STAB-3 | 0.1 | $\varepsilon_{\parallel}$ [1 kHz, 20°C]: | |
| Σ | 100.0 | $\varepsilon_{\perp}$ [1 kHz, 20°C]: | |
| | | $\Delta\varepsilon$ [1 kHz, 20°C]: | |
| | | $K_1$ [pN, 20°C]: | |
| | | $K_3$ [pN, 20°C]: | |
| | | $V_0$ [V, 20°C]: | |
| | | $\gamma_1$ [mPa s, 20°C]: | |
| | | $\gamma_1$ [mPa s, 20°C]: | |
| | | LTS bulk [h, -10°C] | |
| | | LTS bulk [h, -20°C] | |
| | | LTS bulk [h, -30°C] | |

The nematic LC host mixture N-30 is prepared as indicated in the following table:

| | | | |
|---|---|---|---|
| B(S)-2O-O4 | 3.0 | Clearing point [°C]: | 88.9 |
| B(S)-2O-O5 | 4.0 | $\Delta n$ [589 nm, 20°C]: | 0.1152 |
| B(S)-2O-O6 | 3.0 | $\varepsilon_{\parallel}$ [1 kHz, 20°C]: | 3.7 |
| CC-3-V | 33.5 | $\varepsilon_{\perp}$ [1 kHz, 20°C]: | 8.7 |
| CC-3-V1 | 8.0 | $\Delta\varepsilon$ [1 kHz, 20°C]: | -5.0 |
| CCY-3-O2 | 5.0 | $K_1$ [pN, 20°C]: | 17.0 |
| CLY-3-O2 | 9.0 | $K_3$ [pN, 20°C]: | 17.0 |
| COB(S)-2-O4 | 10.0 | $V_0$ [V, 20°C]: | 1.95 |
| CPY-3-O2 | 9.0 | $\gamma_1$ [mPa s, 20°C]: | 123 |
| CY-3-O2 | 9.5 | $\gamma_1$ [mPa s, 20°C]: | |
| PGIY-2-O4 | 6.0 | LTS bulk [h, -10°C]: | |
| Σ | 100.0 | LTS bulk [h, -20°C]: | |
| | | LTS bulk [h, -30°C]: | |

The nematic LC host mixture N-31 is prepared as indicated in the following table:

| | | | |
|---|---|---|---|
| N-30 | 99.865 | Clearing point [°C]: | |
| STAB-3 | 0.1 | Δn [589 nm, 20°C]: | |
| STAB-1 | 0.035 | $\varepsilon_{\parallel}$ [1 kHz, 20°C]: | |
| | 100.0 | $\varepsilon_{\perp}$ [1 kHz, 20°C]: | |
| | | Δε [1 kHz, 20°C]: | |
| | | $K_1$ [pN, 20°C]: | |
| | | $K_3$ [pN, 20°C]: | |
| | | $V_0$ [V, 20°C]: | |
| | | $\gamma_1$ [mPa s, 20°C]: | |
| | | $\gamma_1$ [mPa s, 20°C]: | |
| | | LTS bulk [h, -10°C] | |
| | | LTS bulk [h, -20°C] | |
| | | LTS bulk [h, -30°C] | |

The nematic LC host mixture N-32 is prepared as indicated in the following table:

| | | | |
|---|---|---|---|
| CC-3-V | 25.0 | Clearing point [°C]: | 99.3 |
| CC-3-V1 | 7.5 | Δn [589 nm, 20°C]: | 0.1119 |
| CCGU-3-F | 2.0 | $\varepsilon_{\parallel}$ [1 kHz, 20°C]: | 8.8 |
| CCP-3-1 | 6.0 | $\varepsilon_{\perp}$ [1 kHz, 20°C]: | 2.9 |
| CCP-30CF3 | 8.0 | Δε [1 kHz, 20°C]: | 5.9 |
| CCP-50CF3 | 5.0 | $K_1$ [pN, 20°C]: | 17.9 |
| CCP-V-1 | 9.0 | $K_3$ [pN, 20°C]: | 18.6 |
| CLP-3-T | 7.5 | $V_0$ [V, 20°C]: | 1.83 |
| PCH-302 | 9.0 | $\gamma_1$ [mPa s, 20°C]: | 93 |
| PGP-2-3 | 6.0 | $\gamma_1$ [mPa s, 20°C]: | |
| PGUQU-3-F | 8.0 | LTS bulk [h, -10°C]: | |
| PGUQU-4-F | 7.0 | LTS bulk [h, -20°C]: | 1000 |
| Σ | 100.0 | LTS bulk [h, -30°C]: | |

The nematic LC host mixture N-33 is prepared as indicated in the following table:

| | | | |
|---|---|---|---|
| STAB-2 | 0.05 | Clearing point [°C]: | |
| STAB-3 | 0.1 | Δn [589 nm, 20°C]: | |
| N-32 | 99.85 | $\varepsilon_{\parallel}$ [1 kHz, 20°C]: | |
| Σ | 100.0 | $\varepsilon_{\perp}$ [1 kHz, 20°C]: | |
| | | Δε [1 kHz, 20°C]: | |
| | | $K_1$ [pN, 20°C]: | |
| | | $K_3$ [pN, 20°C]: | |
| | | $V_0$ [V, 20°C]: | |
| | | $\gamma_1$ [mPa s, 20°C]: | |
| | | $\gamma_1$ [mPa s, 20°C]: | |
| | | LTS bulk [h, -10°C] | |
| | | LTS bulk [h, -20°C] | |
| | | LTS bulk [h, -30°C] | |

The nematic LC host mixture N-34 is prepared as indicated in the following table:

| | | | |
|---|---|---|---|
| CC-3-V1 | 8.0 | Clearing point [°C]: | 100.8 |

(continued)

| | | | |
|---|---|---|---|
| CCG-V-F | 1.5 | Δn [589 nm, 20°C]: | 0.1116 |
| CCH-301 | 10.0 | $\varepsilon_{\parallel}$ [1 kHz, 20°C]: | 9.0 |
| CCH-303 | 10.5 | $\varepsilon_{\perp}$ [1 kHz, 20°C]: | 3.2 |
| CCP-30CF3 | 6.0 | Δε [1 kHz, 20°C]: | 5.8 |
| CCP-40CF3 | 4.0 | $K_1$ [pN, 20°C]: | 18.6 |
| CCP-50CF3 | 4.0 | $K_3$ [pN, 20°C]: | 18.0 |
| CCP-V-1 | 15.0 | $V_0$ [V, 20°C]: | 1.89 |
| CDUQU-3-F | 3.0 | $\gamma_1$ [mPa s, 20°C]: | 118 |
| CLP-3-T | 8.0 | $\gamma_1$ [mPa s, 20°C]: | |
| CPGP-4-3 | 3.0 | LTS bulk [h, -10°C]: | |
| PCH-302 | 10.0 | LTS bulk [h, -20°C]: | |
| PGP-2-3 | 5.5 | LTS bulk [h, -30°C]: | |
| PGUQU-3-F | 4.5 | | |
| PGUQU-4-F | 7.0 | | |
| Σ | 100.0 | | |

The nematic LC host mixture N-35 is prepared as indicated in the following table:

| | | | |
|---|---|---|---|
| STAB-2 | 0.05 | Clearing point [°C]: | |
| STAB-3 | 0.1 | Δn [589 nm, 20°C]: | |
| N-34 | 99.85 | $\varepsilon_{\parallel}$ [1 kHz, 20°C]: | |
| Σ | 100.0 | $\varepsilon_{\perp}$ [1 kHz, 20°C]: | |
| | | Δε [1 kHz, 20°C]: | |
| | | $K_1$ [pN, 20°C]: | |
| | | $K_3$ [pN, 20°C]: | |
| | | $V_0$ [V, 20°C]: | |
| | | $\gamma_1$ [mPa s, 20°C]: | |
| | | $\gamma_1$ [mPa s, 20°C]: | |
| | | LTS bulk [h, -10°C] | |
| | | LTS bulk [h, -20°C] | |
| | | LTS bulk [h, -30°C] | |

The nematic LC host mixture N-36 is prepared as indicated in the following table:

| | | | |
|---|---|---|---|
| CC-3-V | 25.0 | Clearing point [°C]: | 100.9 |
| CC-3-V1 | 3.0 | Δn [589 nm, 20°C]: | 0.1111 |
| CCP-3-1 | 6.0 | $\varepsilon_{\parallel}$ [1 kHz, 20°C]: | 8.8 |
| CCP-3F.F.F | 2.0 | $\varepsilon_{\perp}$ [1 kHz, 20°C]: | 2.9 |
| CCP-30CF3 | 8.0 | Δε [1 kHz, 20°C]: | 5.9 |
| CCP-50CF3 | 5.0 | $K_1$ [pN, 20°C]: | 17.7 |
| CCP-V-1 | 14.0 | $K_3$ [pN, 20°C]: | 18.9 |
| CLP-3-T | 8.0 | $V_0$ [V, 20°C]: | 1.81 |
| PCH-302 | 9.0 | $\gamma_1$ [mPa s, 20°C]: | 95 |
| PGP-2-3 | 5.0 | $\gamma_1$ [mPa s, 20°C]: | |
| PGUQU-3-F | 8.0 | LTS bulk [h, -10°C]: | |
| PGUQU-4-F | 7.0 | LTS bulk [h, -20°C]: | |
| Σ | 100.0 | LTS bulk [h, -30°C]: | |

The nematic LC host mixture N-37 is prepared as indicated in the following table:

| | | | |
|---|---|---|---|
| STAB-2 | 0.05 | Clearing point [°C]: | 100.4 |
| STAB-3 | 0.1 | Δn [589 nm, 20°C]: | |
| N-36 | 99.85 | $\varepsilon_{\parallel}$ [1 kHz, 20°C]: | |
| Σ | 100.0 | $\varepsilon_{\perp}$ [1 kHz, 20°C]: | |
| | | Δε [1 kHz, 20°C]: | |
| | | $K_1$ [pN, 20°C]: | |
| | | $K_3$ [pN, 20°C]: | |
| | | $V_0$ [V, 20°C]: | |
| | | $\gamma_1$ [mPa s, 20°C]: | |
| | | $\gamma_1$ [mPa s, 20°C]: | |
| | | LTS bulk [h, -10°C]: | |
| | | LTS bulk [h, -20°C]: | |
| | | LTS bulk [h, -30°C]: | |

The nematic LC host mixture N-38 is prepared as indicated in the following table:

| | | | |
|---|---|---|---|
| B(S)-2O-O4 | 2.0 | Clearing point [°C]: | 74 |
| B(S)-2O-O5 | 4.0 | Δn [589 nm, 20°C]: | 0.1092 |
| CC-3-V | 35.0 | $\varepsilon_{\parallel}$ [1 kHz, 20°C]: | 3.6 |
| CC-3-V1 | 3.0 | $\varepsilon_{\perp}$ [1 kHz, 20°C]: | 7.0 |
| CCP-V-1 | 2.0 | Δε [1 kHz, 20°C]: | -3.4 |
| CCY-3-O2 | 7.5 | $K_1$ [pN, 20°C]: | 13.9 |
| CPY-2-O2 | 6.5 | $K_3$ [pN, 20°C]: | 16.1 |
| CPY-3-O2 | 18.0 | $V_0$ [V, 20°C]: | 2.30 |
| CY-3-O2 | 15.5 | $\gamma_1$ [mPa s, 20°C]: | 89 |
| PP-1-2V1 | 6.5 | $\gamma_1$ [mPa s, 20°C]: | |
| Σ | 100.0 | LTS bulk [h, -10°C]: | |
| | | LTS bulk [h, -20°C]: | |
| | | LTS bulk [h, -30°C]: | |

The nematic LC host mixture N-39 is prepared as indicated in the following table:

| | | | |
|---|---|---|---|
| N-38 | 99.9 | Clearing point [°C]: | 74 |
| STAB-3 | 0.1 | Δn [589 nm, 20°C]: | |
| Σ | 100.0 | $\varepsilon_{\parallel}$ [1 kHz, 20°C]: | |
| | | $\varepsilon_{\perp}$ [1 kHz, 20°C]: | |
| | | Δε [1 kHz, 20°C]: | |
| | | $K_1$ [pN, 20°C]: | |
| | | $K_3$ [pN, 20°C]: | |
| | | $V_0$ [V, 20°C]: | |
| | | $\gamma_1$ [mPa s, 20°C]: | |
| | | $\gamma_1$ [mPa s, 20°C]: | |
| | | LTS bulk [h, -10°C]: | |
| | | LTS bulk [h, -20°C]: | |
| | | LTS bulk [h, -30°C]: | |

The nematic LC host mixture N-40 is prepared as indicated in the following table:

| | | | |
|---|---|---|---|
| B(S)-2O-O4 | 2.0 | Clearing point [°C]: | 74 |
| B(S)-2O-O5 | 4.0 | Δn [589 nm, 20°C]: | 0.1090 |

(continued)

| | | | |
|---|---|---|---|
| CC-3-V | 34.5 | $\varepsilon_{\parallel}$ [1 kHz, 20°C]: | 4.0 |
| CCG-V-F | 5.0 | $\varepsilon_{\perp}$ [1 kHz, 20°C]: | 7.4 |
| CLY-3-O2 | 9.0 | $\Delta\varepsilon$ [1 kHz, 20°C]: | -3.5 |
| CPY-2-O2 | 12.0 | $K_1$ [pN, 20°C]: | 13.5 |
| CPY-3-O2 | 12.0 | $K_3$ [pN, 20°C]: | 15.0 |
| CY-3-O2 | 16.5 | $V_0$ [V, 20°C]: | 2.20 |
| PP-1-2V1 | 5.0 | $\gamma_1$ [mPa s, 20°C]: | 91 |
| $\Sigma$ | 100.0 | $\gamma_1$ [mPa s, 20°C]: | |
| | | LTS bulk [h, -10°C]: | |
| | | LTS bulk [h, -20°C]: | |
| | | LTS bulk [h, -30°C]: | |

The nematic LC host mixture N-41 is prepared as indicated in the following table:

| | | | |
|---|---|---|---|
| N-40 | 99.9 | Clearing point [°C]: | 74 |
| STAB-3 | 0.1 | $\Delta n$ [589 nm, 20°C]: | |
| $\Sigma$ | 100.0 | $\varepsilon_{\parallel}$ [1 kHz, 20°C]: | |
| | | $\varepsilon_{\perp}$ [1 kHz, 20°C]: | |
| | | $\Delta\varepsilon$ [1 kHz, 20°C]: | |
| | | $K_1$ [pN, 20°C]: | |
| | | $K_3$ [pN, 20°C]: | |
| | | $V_0$ [V, 20°C]: | |
| | | $\gamma_1$ [mPa s, 20°C]: | |
| | | $\gamma_1$ [mPa s, 20°C]: | |
| | | LTS bulk [h, -10°C]: | |
| | | LTS bulk [h, -20°C]: | |
| | | LTS bulk [h, -30°C]: | |

The nematic LC host mixture N-42 is prepared as indicated in the following table:

| | | | |
|---|---|---|---|
| B(S)-2O-O4 | 4.0 | Clearing point [°C]: | 74 |
| B(S)-2O-O5 | 4.0 | $\Delta n$ [589 nm, 20°C]: | 0.1097 |
| CC-3-V | 34.5 | $\varepsilon_{\parallel}$ [1 kHz, 20°C]: | 4.0 |
| CCP-V-1 | 1.5 | $\varepsilon_{\perp}$ [1 kHz, 20°C]: | 7.4 |
| CCG-V-F | 5.0 | $\Delta\varepsilon$ [1 kHz, 20°C]: | -3.5 |
| CLY-3-O2 | 10.0 | $K_1$ [pN, 20°C]: | 13.7 |
| CPY-2-O2 | 10.0 | $K_3$ [pN, 20°C]: | 15.0 |
| CPY-3-O2 | 10.0 | $V_0$ [V, 20°C]: | 2.20 |
| CY-3-O2 | 15.5 | $\gamma_1$ [mPa s, 20°C]: | 89 |
| PP-1-2V1 | 5.5 | $\gamma_1$ [mPa s, 20°C]: | |
| | 100.0 | LTS bulk [h, -10°C]: | |
| | | LTS bulk [h, -20°C]: | |
| | | LTS bulk [h, -30°C]: | |

The nematic LC host mixture N-43 is prepared as indicated in the following table:

| | | | |
|---|---|---|---|
| N-42 | 99.9 | Clearing point [°C]: | 74 |
| STAB-3 | 0.1 | $\Delta n$ [589 nm, 20°C]: | |
| $\Sigma$ | 100.0 | $\varepsilon_{\parallel}$ [1 kHz, 20°C]: | |
| | | $\varepsilon_{\perp}$ [1 kHz, 20°C]: | |

(continued)

| | |
|---|---|
| Δε [1 kHz, 20°C]: | |
| $K_1$ [pN, 20°C]: | |
| $K_3$ [pN, 20°C]: | |
| $V_0$ [V, 20°C]: | |
| $\gamma_1$ [mPa s, 20°C]: | |
| $\gamma_1$ [mPa s, 20°C]: | |
| LTS bulk [h, -10°C]: | |
| LTS bulk [h, -20°C]: | |
| LTS bulk [h, -30°C]: | |

The nematic LC host mixture N-44 is prepared as indicated in the following table:

| | | | |
|---|---|---|---|
| CCP-3-3 | 4.0 | Clearing point [°C]: | 106 |
| CCP-V-1 | 4.0 | Δn [589 nm, 20°C]: | 0.1073 |
| CCY-3-O2 | 6.0 | $\varepsilon_\parallel$ [1 kHz, 20°C]: | 3.3 |
| CCY-3-03 | 2.0 | $\varepsilon_\perp$ [1 kHz, 20°C]: | 6.8 |
| CCY-4-O2 | 5.0 | Δε [1 kHz, 20°C]: | -3.5 |
| CLY-2-O4 | 6.0 | $K_1$ [pN, 20°C]: | 20.8 |
| CLY-3-O2 | 8.0 | $K_3$ [pN, 20°C]: | 18.4 |
| CLY-3-O3 | 6.0 | $V_0$ [V, 20°C]: | 2.36 |
| CLY-4-O2 | 5.0 | $\gamma_1$ [mPa s, 20°C]: | 143 |
| PGIY-2-O4 | 6.0 | $\gamma_1$ [mPa s, 20°C]: | |
| PYP-2-3 | 3.0 | LTS bulk [h, -10°C]: | |
| B(S)-2O-O4 | 4.0 | LTS bulk [h, -20°C]: | 504 |
| B(S)-2O-O5 | 4.0 | LTS bulk [h, -30°C]: | |
| CC-3-V | 15.0 | | |
| CC-3-V1 | 4.5 | | |
| CCH-23 | 17.5 | | |
| Σ | 100.0 | | |

The nematic LC host mixture N-45 is prepared as indicated in the following table:

| | | |
|---|---|---|
| N-44 | 99.9 | Clearing point [°C]: |
| STAB-3 | 0.1 | Δn [589 nm, 20°C]: |
| Σ | 100.0 | $\varepsilon_\parallel$ [1 kHz, 20°C]: |
| | | $\varepsilon_\perp$ [1 kHz, 20°C]: |
| | | Δε [1 kHz, 20°C]: |
| | | $K_1$ [pN, 20°C]: |
| | | $K_3$ [pN, 20°C]: |
| | | $V_0$ [V, 20°C]: |
| | | $\gamma_1$ [mPa s, 20°C]: |
| | | $\gamma_1$ [mPa s, 20°C]: |
| | | LTS bulk [h, -10°C] |
| | | LTS bulk [h, -20°C] |
| | | LTS bulk [h, -30°C] |

The nematic LC host mixture N-46 is prepared as indicated in the following table:

| | | | |
|---|---|---|---|
| CCY-3-O2 | 5.5 | cl.p. [°C]: | 85.8 |
| CLY-3-O2 | 6.0 | Δn [589 nm, 20°C]: | 0.1116 |

(continued)

| | | | |
|---|---|---|---|
| CLY-3-O3 | 6.0 | $n_e$ [589 nm, 20°C]: | 1.5970 |
| CPY-3-O2 | 11.0 | $n_o$ [589 nm, 20°C]: | 1.4854 |
| PGIY-2-O4 | 8.0 | $\Delta\varepsilon$ [1 kHz, 20°C]: | -4.1 |
| B(S)-2O-O4 | 4.0 | $\varepsilon_{\parallel}$ [1 kHz, 20°C]: | 3.6 |
| B(S)-2O-O5 | 4.0 | $\varepsilon_{\perp}$ [1 kHz, 20°C]: | 7.7 |
| B(S)-2O-O6 | 4.0 | $K_1$ [pN, 20°C]: | 16.1 |
| CC-3-V | 38.5 | $K_3$ [pN, 20°C]: | 16.6 |
| CC-3-V1 | 5.5 | $K_3/K_1$ [pN, 20°C]: | 1.03 |
| CY-3-O2 | 7.5 | $V_0$ [V, 20°C]: | 2.12 |
| Σ | 100.0 | | |

The nematic LC host mixture N-47 is prepared as indicated in the following table:

| | | | |
|---|---|---|---|
| N-46 | 99.96 | cl.p. [°C]: | 85.8 |
| STAB-3 | 0.04 | $\Delta n$ [589 nm, 20°C]: | 0.1116 |
| Σ | 100.0 | $n_e$ [589 nm, 20°C]: | 1.5970 |
| | | $n_o$ [589 nm, 20°C]: | 1.4854 |
| | | $\Delta\varepsilon$ [1 kHz, 20°C]: | -4.1 |
| | | $\varepsilon_{\parallel}$ [1 kHz, 20°C]: | 3.6 |
| | | $\varepsilon_{\perp}$ [1 kHz, 20°C]: | 7.7 |
| | | $K_1$ [pN, 20°C]: | 16.1 |
| | | $K_3$ [pN, 20°C]: | 16.6 |
| | | $K_3/K_1$ [pN, 20°C]: | 1.03 |
| | | $V_0$ [V, 20°C]: | 2.12 |

The nematic LC host mixture N-48 is prepared as indicated in the following table:

| | | | |
|---|---|---|---|
| CCP-V-1 | 16.0 | cl.p. [°C]: | 83.5 |
| CLY-3-O2 | 8.0 | $\Delta n$ [589 nm, 20°C]: | 0.0978 |
| CLY-3-O3 | 6.0 | $n_e$ [589 nm, 20°C]: | 1.5838 |
| CPY-2-O2 | 9.5 | $n_o$ [589 nm, 20°C]: | 1.4860 |
| CPY-3-O2 | 1.0 | $\Delta\varepsilon$ [1 kHz, 20°C]: | -3.0 |
| B(S)-2O-O4 | 4.0 | $\varepsilon_{\parallel}$ [1 kHz, 20°C]: | 3.4 |
| B(S)-2O-O5 | 4.0 | $\varepsilon_{\perp}$ [1 kHz, 20°C]: | 6.5 |
| CC-3-V | 38.5 | $K_1$ [pN, 20°C]: | 14.4 |
| CY-3-O2 | 13.0 | $K_3$ [pN, 20°C]: | 15.8 |
| Σ | 100.0 | $K_3/K_1$ [pN, 20°C]: | 1.10 |
| | | $V_0$ [V, 20°C]: | 2.41 |

The nematic LC host mixture N-49 is prepared as indicated in the following table:

| | | | |
|---|---|---|---|
| N-48 | 99.96 | cl.p. [°C]: | 83.5 |
| STAB-3 | 0.04 | $\Delta n$ [589 nm, 20°C]: | 0.0978 |
| Σ | 100.0 | $n_e$ [589 nm, 20°C]: | 1.5838 |
| | | $n_o$ [589 nm, 20°C]: | 1.4860 |
| | | $\Delta\varepsilon$ [1 kHz, 20°C]: | -3.0 |
| | | $\varepsilon_{\parallel}$ [1 kHz, 20°C]: | 3.4 |
| | | $\varepsilon_{\perp}$ [1 kHz, 20°C]: | 6.5 |
| | | $K_1$ [pN, 20°C]: | 14.4 |
| | | $K_3$ [pN, 20°C]: | 15.8 |

(continued)

| | |
|---|---|
| $K_3/K_1$ [pN, 20°C]: | 1.10 |
| $V_0$ [V, 20°C]: | 2.41 |

The nematic LC host mixture N-50 is prepared as indicated in the following table:

| | | |
|---|---|---|
| CC-3-V | 40.5 | cl.p. [°C]: |
| CC-3-V1 | 3.0 | Δn [589 nm, 20°C] |
| CCP-V2-1 | 2.5 | $n_e$ [589 nm, 20°C]: |
| CLP-3-T | 7.0 | $n_o$ [589 nm, 20°C]: |
| PCH-302 | 3.0 | Δε [1 kHz, 20°C]: |
| PGP-1-2V | 5.5 | $\varepsilon_{\parallel}$ [1 kHz, 20°C]: |
| PGP-2-2V | 10.0 | $\varepsilon_{\perp}$ [1 kHz, 20°C]: |
| PGP-2-3 | 1.0 | $K_1$ [pN, 20°C]: |
| PGU-2-F | 1.0 | $K_3$ [pN, 20°C]: |
| PGUQU-3-F | 3.5 | $K_3/K_1$ [pN, 20°C]: |
| PGUQU-4-F | 3.0 | $V_0$ [V, 20°C]: |
| PP-1-2V1 | 6.5 | |
| PPGU-3-F | 1.0 | |
| PUS-3-2 | 12.5 | |
| Σ | 100.0 | |

The nematic LC host mixture N-51 is prepared as indicated in the following table:

| | | | |
|---|---|---|---|
| N-50 | 99.96 | cl.p. [°C]: | 79.7 |
| STAB-3 | 0.04 | Δn [589 nm, 20°C]: | 0.1445 |
| Σ | 100.0 | $n_e$ [589 nm, 20°C]: | 1.6421 |
| | | $n_o$ [589 nm, 20°C]: | 1.4976 |
| | | Δε [1 kHz, 20°C]: | 3.0 |
| | | $\varepsilon_{\parallel}$ [1 kHz, 20°C]: | 5.8 |
| | | $\varepsilon_{\perp}$ [1 kHz, 20°C]: | 2.7 |
| | | $K_1$ [pN, 20°C]: | 16.5 |
| | | $K_3$ [pN, 20°C]: | 14.1 |
| | | $K_3/K_1$ [pN, 20°C]: | 0.85 |
| | | $V_0$ [V, 20°C]: | 2.46 |

The nematic LC host mixture N-52 is prepared as indicated in the following table:

| | | |
|---|---|---|
| APUQU-2-F | 2.0 | cl.p. [°C]: |
| CC-3-V | 44.0 | Δn [589 nm, 20°C]: |
| CC-3-V1 | 7.0 | $n_e$ [589 nm, 20°C]: |
| CLP-3-T | 4.5 | $n_o$ [589 nm, 20°C]: |
| COB(S)-2-O4 | 1.5 | Δε [1 kHz, 20°C]: |
| PCH-302 | 2.0 | $\varepsilon_{\parallel}$ [1 kHz, 20°C]: |
| PGP-2-2V | 9.0 | $\varepsilon_{\perp}$ [1 kHz, 20°C]: |
| PGU-2-F | 4.0 | $K_1$ [pN, 20°C]: |
| PGUQU-3-F | 5.5 | $K_3$ [pN, 20°C]: |
| PGUQU-4-F | 5.5 | $K_3/K_1$ [pN, 20°C]: |
| PP-1-2V1 | 2.0 | $V_0$ [V, 20°C]: |
| PPGU-3-F | 0.5 | |
| PUS-3-2 | 12.5 | |

(continued)

| | |
|---|---|
| Σ | 100.0 |

The nematic LC host mixture N-53 is prepared as indicated in the following table:

| | | | |
|---|---|---|---|
| N-52 | 99.96 | cl.p. [°C]: | 74.9 |
| STAB-3 | 0.04 | Δn [589 nm, 20°C]: | 0.1318 |
| Σ | 100.0 | $n_e$ [589 nm, 20°C]: | 1.6240 |
| | | $n_o$ [589 nm, 20°C]: | 1.4922 |
| | | Δε [1 kHz, 20°C]: | 4.9 |
| | | $\varepsilon_\parallel$ [1 kHz, 20°C]: | 7.9 |
| | | $\varepsilon_\perp$ [1 kHz, 20°C]: | 3.1 |
| | | $K_1$ [pN, 20°C]: | 14.4 |
| | | $K_3$ [pN, 20°C]: | 12.7 |
| | | $K_3/K_1$ [pN, 20°C]: | 0.88 |
| | | $V_0$ [V, 20°C]: | 1.82 |

The nematic LC host mixture N-54 is prepared as indicated in the following table:

| | | | |
|---|---|---|---|
| B(S)-2O-O4 | 5.0 | cl.p. [°C]: | 74.4 |
| B(S)-2O-O5 | 5.0 | Δn [589 nm, 20°C]: | 0.1382 |
| B(S)-2O-O6 | 4.0 | $n_e$ [589 nm, 20°C]: | 1.6400 |
| BCH-32 | 12.0 | $n_o$ [589 nm, 20°C]: | 1.5018 |
| CC-3-V | 29.0 | Δε [1 kHz, 20°C]: | -2.0 |
| CCP-V-1 | 15.0 | $\varepsilon_\parallel$ [1 kHz, 20°C]: | 3.5 |
| CLP-3-T | 0.5 | $\varepsilon_\perp$ [1 kHz, 20°C]: | 5.5 |
| COB(S)-2-O4 | 1.0 | $K_1$ [pN, 20°C]: | 14.9 |
| PP-1-2V1 | 10.5 | $K_3$ [pN, 20°C]: | 14.4 |
| PY-1-O2 | 10.0 | $K_3/K_1$ [pN, 20°C]: | 0.96 |
| PYP-2-3 | 8.0 | $V_0$ [V, 20°C]: | 2.81 |
| Σ | 100.0 | | |

The nematic LC host mixture N-55 is prepared as indicated in the following table:

| | | |
|---|---|---|
| N-54 | 99.995 | cl.p. [°C]: |
| STAB-3 | 0.005 | Δn [589 nm, 20°C] |
| Σ | 100.0 | $n_e$ [589 nm, 20°C]: |
| | | $n_o$ [589 nm, 20°C]: |
| | | Δε [1 kHz, 20°C]: |
| | | $\varepsilon_\parallel$ [1 kHz, 20°C]: |
| | | $\varepsilon_\perp$ [1 kHz, 20°C]: |
| | | $K_1$ [pN, 20°C]: |
| | | $K_3$ [pN, 20°C]: |
| | | $K_3/K_1$ [pN, 20°C]: |
| | | $V_0$ [V, 20°C]: |

The nematic LC host mixture N-56 is prepared as indicated in the following table:

| | | | |
|---|---|---|---|
| B(S)-2O-O5 | 4.0 | cl.p. [°C]: | 101 |
| CC-3-V | 22.5 | Δn [589 nm, 20°C]: | 0.0922 |
| CC-3-V1 | 8.0 | $n_e$ [589 nm, 20°C]: | 1.5722 |

(continued)

| | | | |
|---|---|---|---|
| CCH-34 | 5.0 | $n_o$ [589 nm, 20°C]: | 1.4800 |
| CCH-35 | 5.0 | $\Delta\varepsilon$ [1 kHz, 20°C]: | -4.0 |
| CCY-3-1 | 3.5 | $\varepsilon_{\parallel}$ [1 kHz, 20°C]: | 3.3 |
| CCY-3-O1 | 4.0 | $\varepsilon_{\perp}$ [1 kHz, 20°C]: | 7.3 |
| CCY-3-O2 | 8.0 | $K_1$ [pN, 20°C]: | 19.4 |
| CCY-3-O3 | 3.0 | $K_3$ [pN, 20°C]: | 19.4 |
| CLY-3-O2 | 8.0 | $K_3/K_1$ [pN, 20°C]: | 1.00 |
| CLY-3-O3 | 6.0 | $V_0$ [V, 20°C]: | 2.34 |
| CLY-4-O2 | 5.0 | | |
| CLY-5-O2 | 5.0 | | |
| COB(S)-2-O4 | 3.0 | | |
| CY-3-O2 | 10.0 | | |
| Σ | 100.0 | | |

The nematic LC host mixture N-57 is prepared as indicated in the following table:

| | | | |
|---|---|---|---|
| N-56 | 99.97 | cl.p. [°C]: | |
| STAB-3 | 0.03 | $\Delta n$ [589 nm, 20°C] | |
| Σ | 100.0 | $n_e$ [589 nm, 20°C]: | |
| | | $n_o$ [589 nm, 20°C]: | |
| | | $\Delta\varepsilon$ [1 kHz, 20°C]: | |
| | | $\varepsilon_{\parallel}$ [1 kHz, 20°C]: | |
| | | $\varepsilon_{\perp}$ [1 kHz, 20°C]: | |
| | | $K_1$ [pN, 20°C]: | |
| | | $K_3$ [pN, 20°C]: | |
| | | $K_3/K_1$ [pN, 20°C]: | |
| | | $V_0$ [V, 20°C]: | |

The nematic LC host mixture N-58 is prepared as indicated in the following table:

| | | | |
|---|---|---|---|
| APUQU-2-F | 4.0 | cl.p. [°C]: | 121.2 |
| APUQU-3-F | 6.0 | $\Delta n$ [589 nm, 20°C]: | 0.0906 |
| CC-3-V | 32.5 | $n_e$ [589 nm, 20°C]: | 1.5744 |
| CC-3-V1 | 5.0 | $n_o$ [589 nm, 20°C]: | 1.4838 |
| CCP-3-1 | 4.5 | $\Delta\varepsilon$ [1 kHz, 20°C]: | 6.1 |
| CCP-V-1 | 14.0 | $\varepsilon_{\parallel}$ [1 kHz, 20°C]: | 9.0 |
| CCP-V2-1 | 7.0 | $\varepsilon_{\perp}$ [1 kHz, 20°C]: | 2.9 |
| CCPC-33 | 3.0 | $K_1$ [pN, 20°C]: | 17.9 |
| CCPC-35 | 3.0 | $K_3$ [pN, 20°C]: | 21.1 |
| CCVC-3-V | 6.0 | $K_3/K_1$ [pN, 20°C]: | 1.18 |
| CDUQU-3-F | 7.0 | $V_0$ [V, 20°C]: | 1.80 |
| CCOC-4-3 | 2.0 | | |
| DPGU-4-F | 2.0 | | |
| PUQU-3-F | 4.0 | | |
| Σ | 100.0 | | |

The nematic LC host mixture N-59 is prepared as indicated in the following table:

| | | | |
|---|---|---|---|
| N-58 | 99.92 | cl.p. [°C]: | 120.9 |
| STAB-2 | 0.05 | $\Delta n$ [589 nm, 20°C]: | |

(continued)

| | | | |
|---|---|---|---|
| STAB-3 | 0.03 | $n_e$ [589 nm, 20°C]: | |
| Σ | 100.0 | $n_o$ [589 nm, 20°C]: | |
| | | $\Delta\varepsilon$ [1 kHz, 20°C]: | 6.2 |
| | | $\varepsilon_{\parallel}$ [1 kHz, 20°C]: | 9.1 |
| | | $\varepsilon_{\perp}$ [1 kHz, 20°C]: | 2.9 |
| | | $K_1$ [pN, 20°C]: | 18.0 |
| | | $K_3$ [pN, 20°C]: | 21.5 |
| | | $K_3/K_1$ [pN, 20°C]: | 1.19 |
| | | $V_0$ [V, 20°C]: | 1.81 |

Fabrication of display cells

Unless explicitly stated otherwise, the display cells are made with Corning AF glass of 0.7mm thickness using 6.4 μm spacer beads and XN-1500T sealant.

For measurement of electro-optics 3 μm thick PI-free IPS cells are made of substrates commercially available from SD-tech and constructed into cells using ITO electrodes having 5μm electrode spacing and a 3μm electrode width.

The cells are assembled by hand and then cured using a Omnicure 2000 Mercury lamp with with 35 $mW/cm^2$ the irradiation power is thereby measured by an Opsytec UV pad-e spectroradiometer.

Mixture examples

The following nematic LC mixtures according to the invention are prepared from the nematic host mixtures listed above and photoalignment additives of formula I, according to the compositions given in the following table.

| Mixture example | Host Mixture | c [%] of Host Mixture | Photoalignment additive | |
|---|---|---|---|---|
| | | | Compound | c [%] |
| M-1 | N-1 | 99.7 | 17 | 0.3 |
| M-2 | N-1 | 99.5 | 17 | 0.5 |
| M-3 | N-1 | 99.7 | 13 | 0.3 |
| M-4 | N-1 | 99.5 | 13 | 0.5 |
| M-5 | N-2 | 99.5 | 13 | 0.5 |
| M-6 | N-2 | 99.5 | 17 | 0.5 |
| M-7 | N-3 | 99.5 | 13 | 0.5 |
| M-8 | N-3 | 99.5 | 17 | 0.5 |
| M-9 | N-4 | 99.5 | 13 | 0.5 |
| M-10 | N-4 | 99.5 | 17 | 0.5 |
| M-11 | N-5 | 99.5 | 13 | 0.5 |
| M-12 | N-5 | 99.5 | 17 | 0.5 |
| M-13 | N-6 | 99.5 | 13 | 0.5 |
| M-14 | N-6 | 99.5 | 17 | 0.5 |
| M-15 | N-7 | 99.5 | 13 | 0.5 |
| M-16 | N-7 | 99.5 | 17 | 0.5 |
| M-17 | N-8 | 99.5 | 13 | 0.5 |
| M-18 | N-8 | 99.5 | 17 | 0.5 |
| M-19 | N-9 | 99.5 | 13 | 0.5 |

(continued)

| Mixture example | Host Mixture | c [%] of Host Mixture | Photoalignment additive | |
|---|---|---|---|---|
| | | | Compound | c [%] |
| M-20 | N-9 | 99.5 | 17 | 0.5 |
| M-21 | N-10 | 99.5 | 13 | 0.5 |
| M-22 | N-10 | 99.5 | 17 | 0.5 |
| M-23 | N-11 | 99.5 | 13 | 0.5 |
| M-24 | N-11 | 99.5 | 17 | 0.5 |
| M-25 | N-12 | 99.5 | 13 | 0.5 |
| M-26 | N-12 | 99.5 | 17 | 0.5 |
| M-27 | N-13 | 99.5 | 13 | 0.5 |
| M-28 | N-13 | 99.5 | 17 | 0.5 |
| M-29 | N-14 | 99.5 | 13 | 0.5 |
| M-30 | N-14 | 99.5 | 17 | 0.5 |
| M-31 | N-15 | 99.5 | 13 | 0.5 |
| M-32 | N-15 | 99.5 | 17 | 0.5 |
| M-33 | N-16 | 99.5 | 13 | 0.5 |
| M-34 | N-16 | 99.5 | 17 | 0.5 |
| M-35 | N-17 | 99.5 | 13 | 0.5 |
| M-36 | N-17 | 99.5 | 17 | 0.5 |
| M-37 | N-18 | 99.5 | 13 | 0.5 |
| M-38 | N-18 | 99.5 | 17 | 0.5 |
| M-39 | N-19 | 99.5 | 13 | 0.5 |
| M-40 | N-19 | 99.5 | 17 | 0.5 |
| M-41 | N-20 | 99.5 | 13 | 0.5 |
| M-42 | N-20 | 99.5 | 17 | 0.5 |
| M-43 | N-21 | 99.5 | 13 | 0.5 |
| M-44 | N-21 | 99.5 | 17 | 0.5 |
| M-45 | N-22 | 99.5 | 13 | 0.5 |
| M-46 | N-22 | 99.5 | 17 | 0.5 |
| M-47 | N-23 | 99.5 | 13 | 0.5 |
| M-48 | N-23 | 99.5 | 17 | 0.5 |
| M-49 | N-24 | 99.5 | 13 | 0.5 |
| M-50 | N-24 | 99.5 | 17 | 0.5 |
| M-51 | N-25 | 99.5 | 13 | 0.5 |
| M-52 | N-25 | 99.5 | 17 | 0.5 |
| M-53 | N-26 | 99.5 | 13 | 0.5 |
| M-54 | N-27 | 99.5 | 17 | 0.5 |
| M-55 | N-27 | 99.5 | 13 | 0.5 |
| M-56 | N-28 | 99.5 | 17 | 0.5 |

(continued)

| Mixture example | Host Mixture | c [%] of Host Mixture | Photoalignment additive | |
|---|---|---|---|---|
| | | | Compound | c [%] |
| M-57 | N-28 | 99.5 | 13 | 0.5 |
| M-58 | N-29 | 99.5 | 17 | 0.5 |
| M-59 | N-30 | 99.5 | 13 | 0.5 |
| M-60 | N-30 | 99.5 | 17 | 0.5 |
| M-61 | N-31 | 99.5 | 13 | 0.5 |
| M-62 | N-31 | 99.5 | 17 | 0.5 |
| M-63 | N-32 | 99.5 | 13 | 0.5 |
| M-64 | N-32 | 99.5 | 17 | 0.5 |
| M-65 | N-33 | 99.5 | 13 | 0.5 |
| M-66 | N-33 | 99.5 | 17 | 0.5 |
| M-67 | N-34 | 99.5 | 13 | 0.5 |
| M-68 | N-34 | 99.5 | 17 | 0.5 |
| M-69 | N-35 | 99.5 | 13 | 0.5 |
| M-70 | N-35 | 99.5 | 17 | 0.5 |
| M-71 | N-36 | 99.5 | 13 | 0.5 |
| M-72 | N-36 | 99.5 | 17 | 0.5 |
| M-73 | N-37 | 99.5 | 13 | 0.5 |
| M-74 | N-37 | 99.5 | 17 | 0.5 |
| M-75 | N-38 | 99.5 | 13 | 0.5 |
| M-76 | N-38 | 99.5 | 17 | 0.5 |
| M-77 | N-39 | 99.5 | 13 | 0.5 |
| M-78 | N-39 | 99.5 | 17 | 0.5 |
| M-79 | N-40 | 99.5 | 13 | 0.5 |
| M-80 | N-40 | 99.5 | 17 | 0.5 |
| M-81 | N-41 | 99.5 | 13 | 0.5 |
| M-82 | N-41 | 99.5 | 17 | 0.5 |
| M-83 | N-42 | 99.5 | 13 | 0.5 |
| M-84 | N-42 | 99.5 | 17 | 0.5 |
| M-85 | N-43 | 99.5 | 13 | 0.5 |
| M-86 | N-43 | 99.5 | 17 | 0.5 |
| M-87 | N-44 | 99.5 | 13 | 0.5 |
| M-88 | N-44 | 99.5 | 17 | 0.5 |
| M-89 | N-45 | 99.5 | 13 | 0.5 |
| M-90 | N-45 | 99.5 | 17 | 0.5 |
| M-91 | N-46 | 99.5 | 13 | 0.5 |
| M-92 | N-46 | 99.5 | 17 | 0.5 |
| M-93 | N-47 | 99.5 | 13 | 0.5 |

(continued)

| Mixture example | Host Mixture | c [%] of Host Mixture | Photoalignment additive | |
|---|---|---|---|---|
| | | | Compound | c [%] |
| M-94 | N-47 | 99.5 | 17 | 0.5 |
| M-95 | N-48 | 99.5 | 13 | 0.5 |
| M-96 | N-48 | 99.5 | 17 | 0.5 |
| M-97 | N-49 | 99.5 | 13 | 0.5 |
| M-98 | N-49 | 99.5 | 17 | 0.5 |
| M-99 | N-50 | 99.5 | 13 | 0.5 |
| M-100 | N-50 | 99.5 | 17 | 0.5 |
| M-101 | N-51 | 99.5 | 13 | 0.5 |
| M-102 | N-51 | 99.5 | 17 | 0.5 |
| M-103 | N-52 | 99.5 | 13 | 0.5 |
| M-104 | N-52 | 99.5 | 17 | 0.5 |
| M-105 | N-53 | 99.5 | 13 | 0.5 |
| M-106 | N-53 | 99.5 | 17 | 0.5 |
| M-107 | N-54 | 99.5 | 13 | 0.5 |
| M-108 | N-54 | 99.5 | 17 | 0.5 |
| M-109 | N-55 | 99.5 | 13 | 0.5 |
| M-110 | N-55 | 99.5 | 17 | 0.5 |
| M-111 | N-56 | 99.5 | 13 | 0.5 |
| M-112 | N-56 | 99.5 | 17 | 0.5 |

Cell filling and curing

Unless explicitly stated otherwise, the selected LC mixtures are capillary filled using capillary action at room temp., annealed for 1 h at 100°C and then irradiated at the same temperature with linearly polarised UV light (35 mW/cm$^2$, Omnicure S2000 Mercury Lamp) for the given time . The cells are then cooled to room temperature. Next, the alignment quality is studied between crossed polarisers on a light box.

| Example | Host mixture [%] | | Compound [%] | | Curing time [s] | Alignment Quality |
|---|---|---|---|---|---|---|
| M-1 | N-1 | 99.7 | 17 | 0.3 | 30 | + |
| M-1 | N-1 | 99.7 | 17 | 0.3 | 60 | ++ |
| M-1 | N-1 | 99.7 | 17 | 0.3 | 120 | ++ |
| M-1 | N-1 | 99.7 | 17 | 0.3 | 180 | ++ |
| M-2 | N-1 | 99.5 | 17 | 0.5 | 30 | ++ |
| M-2 | N-1 | 99.5 | 17 | 0.5 | 60 | ++ |
| M-2 | N-1 | 99.5 | 17 | 0.5 | 120 | + |
| M-2 | N-1 | 99.5 | 17 | 0.5 | 180 | o |
| M-3 | N-1 | 99.7 | 13 | 0.3 | 30 | o |
| M-3 | N-1 | 99.7 | 13 | 0.3 | 60 | o |
| M-3 | N-1 | 99.7 | 13 | 0.3 | 120 | + |

(continued)

| Example | Host mixture [%] | | Compound [%] | | Curing time [s] | Alignment Quality |
|---|---|---|---|---|---|---|
| M-3 | N-1 | 99.7 | 13 | 0.3 | 180 | + |
| M-4 | N-1 | 99.5 | 13 | 0.5 | 30 | + |
| M-4 | N-1 | 99.5 | 13 | 0.5 | 60 | + |
| M-5 | N-2 | 99.5 | 13 | 0.5 | 60 | + |
| M-6 | N-2 | 99.5 | 17 | 0.5 | 60 | + |
| M-7 | N-3 | 99.5 | 13 | 0.5 | 60 | + |
| M-8 | N-3 | 99.5 | 17 | 0.5 | 60 | + |
| M-9 | N-4 | 99.5 | 13 | 0.5 | 60 | + |
| M-10 | N-4 | 99.5 | 17 | 0.5 | 60 | + |
| M-11 | N-5 | 99.5 | 13 | 0.5 | 60 | + |
| M-12 | N-5 | 99.5 | 17 | 0.5 | 60 | + |
| M-13 | N-6 | 99.5 | 13 | 0.5 | 60 | + |
| M-14 | N-6 | 99.5 | 17 | 0.5 | 60 | + |
| M-15 | N-7 | 99.5 | 13 | 0.5 | 60 | + |
| M-16 | N-7 | 99.5 | 17 | 0.5 | 60 | + |
| M-17 | N-8 | 99.5 | 13 | 0.5 | 60 | + |
| M-18 | N-8 | 99.5 | 17 | 0.5 | 60 | + |
| M-19 | N-9 | 99.5 | 13 | 0.5 | 60 | + |
| M-20 | N-9 | 99.5 | 17 | 0.5 | 60 | + |
| M-21 | N-10 | 99.5 | 13 | 0.5 | 60 | + |
| M-22 | N-10 | 99.5 | 17 | 0.5 | 60 | + |
| M-23 | N-11 | 99.5 | 13 | 0.5 | 60 | + |
| M-24 | N-11 | 99.5 | 17 | 0.5 | 60 | + |
| M-25 | N-12 | 99.5 | 13 | 0.5 | 60 | + |
| M-26 | N-12 | 99.5 | 17 | 0.5 | 60 | + |
| M-27 | N-13 | 99.5 | 13 | 0.5 | 60 | + |
| M-28 | N-13 | 99.5 | 17 | 0.5 | 60 | + |
| M-29 | N-14 | 99.5 | 13 | 0.5 | 60 | + |
| M-30 | N-14 | 99.5 | 17 | 0.5 | 60 | + |
| M-31 | N-15 | 99.5 | 13 | 0.5 | 60 | + |
| M-32 | N-15 | 99.5 | 17 | 0.5 | 60 | + |
| M-33 | N-16 | 99.5 | 13 | 0.5 | 60 | + |
| M-34 | N-16 | 99.5 | 17 | 0.5 | 60 | + |
| M-35 | N-17 | 99.5 | 13 | 0.5 | 60 | + |
| M-36 | N-17 | 99.5 | 17 | 0.5 | 60 | + |
| M-37 | N-18 | 99.5 | 13 | 0.5 | 60 | + |
| M-38 | N-18 | 99.5 | 17 | 0.5 | 60 | + |
| M-39 | N-19 | 99.5 | 13 | 0.5 | 60 | + |

(continued)

| Example | Host mixture [%] | | Compound [%] | | Curing time [s] | Alignment Quality |
|---|---|---|---|---|---|---|
| M-40 | N-19 | 99.5 | 17 | 0.5 | 60 | + |
| M-41 | N-20 | 99.5 | 13 | 0.5 | 60 | + |
| M-42 | N-20 | 99.5 | 17 | 0.5 | 60 | + |
| M-43 | N-21 | 99.5 | 13 | 0.5 | 60 | + |
| M-44 | N-21 | 99.5 | 17 | 0.5 | 60 | + |
| M-45 | N-22 | 99.5 | 13 | 0.5 | 60 | + |
| M-46 | N-22 | 99.5 | 17 | 0.5 | 60 | + |
| M-47 | N-23 | 99.5 | 13 | 0.5 | 60 | + |
| M-48 | N-23 | 99.5 | 17 | 0.5 | 60 | + |
| M-49 | N-24 | 99.5 | 13 | 0.5 | 60 | + |
| M-50 | N-24 | 99.5 | 17 | 0.5 | 60 | + |
| M-51 | N-25 | 99.5 | 13 | 0.5 | 60 | + |
| M-52 | N-25 | 99.5 | 17 | 0.5 | 60 | + |
| M-53 | N-26 | 99.5 | 13 | 0.5 | 60 | + |
| M-54 | N-27 | 99.5 | 17 | 0.5 | 60 | + |
| M-55 | N-27 | 99.5 | 13 | 0.5 | 60 | + |
| M-56 | N-28 | 99.5 | 17 | 0.5 | 60 | + |
| M-57 | N-28 | 99.5 | 13 | 0.5 | 60 | + |
| M-58 | N-29 | 99.5 | 17 | 0.5 | 60 | + |
| M-59 | N-30 | 99.5 | 13 | 0.5 | 60 | + |
| M-60 | N-30 | 99.5 | 17 | 0.5 | 60 | + |
| M-61 | N-31 | 99.5 | 13 | 0.5 | 60 | + |
| M-62 | N-31 | 99.5 | 17 | 0.5 | 60 | + |
| M-63 | N-32 | 99.5 | 13 | 0.5 | 60 | + |
| M-64 | N-32 | 99.5 | 17 | 0.5 | 60 | + |
| M-65 | N-33 | 99.5 | 13 | 0.5 | 60 | + |
| M-66 | N-33 | 99.5 | 17 | 0.5 | 60 | + |
| M-67 | N-34 | 99.5 | 13 | 0.5 | 60 | + |
| M-68 | N-34 | 99.5 | 17 | 0.5 | 60 | + |
| M-69 | N-35 | 99.5 | 13 | 0.5 | 60 | + |
| M-70 | N-35 | 99.5 | 17 | 0.5 | 60 | + |
| M-71 | N-36 | 99.5 | 13 | 0.5 | 60 | + |
| M-72 | N-36 | 99.5 | 17 | 0.5 | 60 | + |
| M-73 | N-37 | 99.5 | 13 | 0.5 | 60 | + |
| M-74 | N-37 | 99.5 | 17 | 0.5 | 60 | + |
| M-75 | N-38 | 99.5 | 13 | 0.5 | 60 | + |
| M-76 | N-38 | 99.5 | 17 | 0.5 | 60 | + |
| M-77 | N-39 | 99.5 | 13 | 0.5 | 60 | + |

(continued)

| Example | Host mixture [%] | | Compound [%] | | Curing time [s] | Alignment Quality |
|---|---|---|---|---|---|---|
| M-78 | N-39 | 99.5 | 17 | 0.5 | 60 | + |
| M-79 | N-40 | 99.5 | 13 | 0.5 | 60 | + |
| M-80 | N-40 | 99.5 | 17 | 0.5 | 60 | + |
| M-81 | N-41 | 99.5 | 13 | 0.5 | 60 | + |
| M-82 | N-41 | 99.5 | 17 | 0.5 | 60 | + |
| M-83 | N-42 | 99.5 | 13 | 0.5 | 60 | + |
| M-84 | N-42 | 99.5 | 17 | 0.5 | 60 | + |
| M-85 | N-43 | 99.5 | 13 | 0.5 | 60 | + |
| M-86 | N-43 | 99.5 | 17 | 0.5 | 60 | + |
| M-87 | N-44 | 99.5 | 13 | 0.5 | 60 | + |
| M-88 | N-44 | 99.5 | 17 | 0.5 | 60 | + |
| M-89 | N-45 | 99.5 | 13 | 0.5 | 60 | + |
| M-90 | N-45 | 99.5 | 17 | 0.5 | 60 | + |
| M-91 | N-46 | 99.5 | 13 | 0.5 | 60 | + |
| M-92 | N-46 | 99.5 | 17 | 0.5 | 60 | + |
| M-93 | N-47 | 99.5 | 13 | 0.5 | 60 | + |
| M-94 | N-47 | 99.5 | 17 | 0.5 | 60 | + |
| M-95 | N-48 | 99.5 | 13 | 0.5 | 60 | + |
| M-96 | N-48 | 99.5 | 17 | 0.5 | 60 | + |
| M-97 | N-49 | 99.5 | 13 | 0.5 | 60 | + |
| M-98 | N-49 | 99.5 | 17 | 0.5 | 60 | + |
| M-99 | N-50 | 99.5 | 13 | 0.5 | 60 | + |
| M-100 | N-50 | 99.5 | 17 | 0.5 | 60 | + |
| M-101 | N-51 | 99.5 | 13 | 0.5 | 60 | + |
| M-102 | N-51 | 99.5 | 17 | 0.5 | 60 | + |
| M-103 | N-52 | 99.5 | 13 | 0.5 | 60 | + |
| M-104 | N-52 | 99.5 | 17 | 0.5 | 60 | + |
| M-105 | N-53 | 99.5 | 13 | 0.5 | 60 | + |
| M-106 | N-53 | 99.5 | 17 | 0.5 | 60 | + |
| M-107 | N-54 | 99.5 | 13 | 0.5 | 60 | + |
| M-108 | N-54 | 99.5 | 17 | 0.5 | 60 | + |
| M-109 | N-55 | 99.5 | 13 | 0.5 | 60 | + |
| M-110 | N-55 | 99.5 | 17 | 0.5 | 60 | + |
| M-111 | N-56 | 99.5 | 13 | 0.5 | 60 | + |
| M-112 | N-56 | 99.5 | 17 | 0.5 | 60 | + |
| Alignment quality: (++) excellent, (+) good, (o) acceptable, (-) poor | | | | | | |

At least acceptable uniform planar alignment is achieved with all mixtures according to the present invention and under all polarizer configurations an homogeneous transmissive state is observed.

## Claims

1. Compound of formula I,

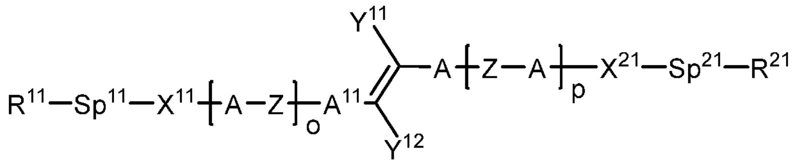

I

wherein

$A^{11}$ denotes a radical selected from the following groups: a) a group consisting of 1,4-phenylene and 1,3-phenylene, wherein, in addition, one or two CH groups may be replaced by N and wherein, in addition, one or more H atoms may be replaced by L, b) a group selected from the group consisting of

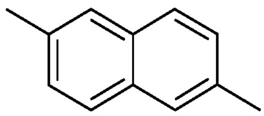
,
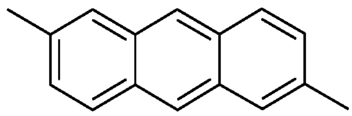
,
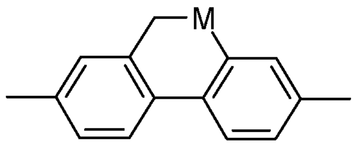
,
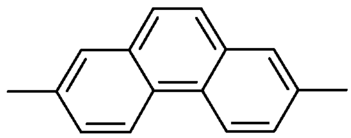
,
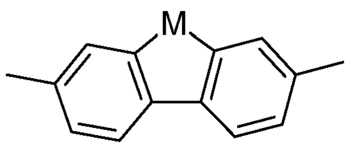
,
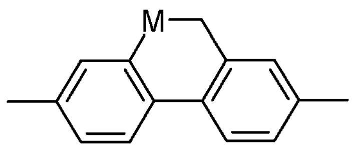
,
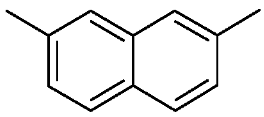
,
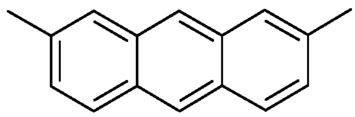
,
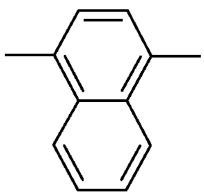
,
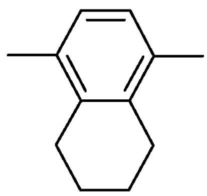
,
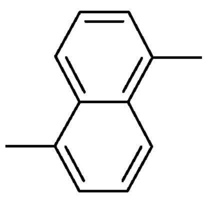
,

where, in addition, one or more H atoms in these radicals may be replaced by L, and/or one or more double bonds may be replaced by single bonds, and/or one or more CH groups may be replaced by N,
A have each, independently of one another, in each occurrence one of the meanings for $A^{11}$ or a) group consisting

of trans-1,4-cyclohexylene, 1,4-cyclohexenylene, wherein, in addition, one or more non-adjacent $CH_2$ groups may be replaced by -O-and/or -S- and wherein, in addition, one or more H atoms may be replaced by F, or b) a group consisting of tetrahydropyran-2,5-diyl, 1,3-dioxane-2,5-diyl, tetrahydrofuran-2,5-diyl, cyclobutane-1,3-diyl, piperidine-1,4-diyl, thiophene-2,5-diyl and selenophene-2,5-diyl, each of which may also be mono- or polysubstituted by L,

L on each occurrence, identically or differently, denotes -OH, -F, -Cl, -Br, -I, -CN, $-NO_2$, $SF_5$, -NCO, -NCS, -OCN, -SCN, $-C(=O)N(R^z)_2$, $-C(=O)R^z$, $-N(R^z)_2$, optionally substituted silyl, optionally substituted aryl having 6 to 20 C atoms, or straight-chain or branched or cyclic alkyl, alkoxy, alkylcarbonyl, alkoxycarbonyl, alkylcarbonyloxy or alkoxycarbonyloxy having 1 to 25 C atoms, in which, in addition, one or more H atoms may be replaced by F or Cl, or $X^{21}$-$Sp^{21}$-$R^{21}$,

M denotes -O-, -S-, $-CH_2-$, $-CHR^z-$ or $-CR^yR^z-$, and

$R^y$ and $R^z$ each, independently of one another, denote H, CN, F or alkyl having 1-12 C atoms, wherein one or more H atoms may be replaced by F,

$Y^{11}$ and $Y^{12}$ each, independently of one another, denote H, F, phenyl or alkyl having 1-12 C atoms, wherein one or more H atoms may be replaced by F,

Z denotes, independently of each other, in each occurrence, a single bond, -COO-, -OCO-, -O-CO-O-, $-OCH_2-$, $-CH_2O-$, $-OCF_2-$, $-CF_2O-$, oder $-(CH_2)_n-$ $-(CH_2)_n-$ wherein one or more non-adjacent groups may be replaced by O or S, $-CF_2CF_2-$, -CH=CH-, -CF=CF-, -CH=CH-COO-, -OCO-CH=CH-, -CO-S-, -S-CO-, -CS-S-, -S-CS-, -S-CSS- or -C≡C-,

n denotes an integer between 2 and 8,

o and p denote each and independently 0, 1 or 2,

$X^{11}$ and $X^{21}$ denote independently from one another, in each occurrence a single bond, -CO-O-, -O-CO-, -O-COO-, -O-, -CH=CH-,-C≡C-, $-CF_2-O-$, $-O-CF_2-$, $-CF_2-CF_2-$, $-CH_2-O-$, $-O-CH_2-$, -CO-S-, -S-CO-, -CS-S-, -S-CS-, -S-CSS- or -S-,

$Sp^{11}$ and $Sp^{21}$ denote each and independently, in each occurrence a single bond or a spacer group comprising 1 to 20 C atoms, wherein one or more non-adjacent and non-terminal $CH_2$ groups may also be replaced by-O-, -S-, -NH-, $-N(CH_3)-$, -CO-, -O-CO-, -S-CO-, -O-COO-, -CO-S-, -CO-O-, $-CF_2-$, $-CF_2O-$, $-OCF_2-$ -C(OH)-, -CH(alkyl)-, -CH(alkenyl)-,-CH(alkoxyl)-,-CH(oxaalkyl)-, -CH=CH- or -C≡C-, however in such a way that no two O-atoms are adjacent to one another and no two groups selected from -O-CO-,-S-CO-, -O-COO-, -CO-S-, -CO-O- and -CH=CH- are adjacent to each other,

$R^{11}$ and $R^{21}$ denotes $P^{11}$or $P^{21}$, under the condition that at least one of $R^{11}$ and $R^{21}$ denotes $P^{21}$,

$P^{11}$ each and independently from another in each occurrence a polymerizable group selected from the group consisting of acrylate, methacrylate, fluoroacrylate, furthermore vinyloxy, chloroacrylate, oxetane and epoxide groups,

$P^{21}$ denotes a group

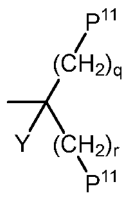
,

Y denotes H, F, phenyl or optionally fluorinated alkyl having 1-12 C atoms,

q and r denotes each and independently an integer from 0 to 8.

2. Compound according to claim 1, **characterized in that** the compound is selected selected from compounds of the sub-formulae 1-1 to I-9.

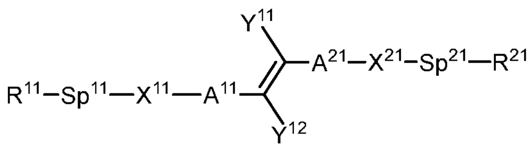

I-1

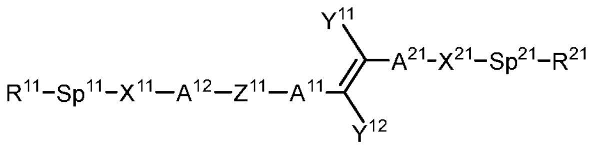

I-2

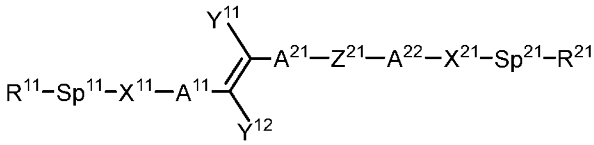

I-3

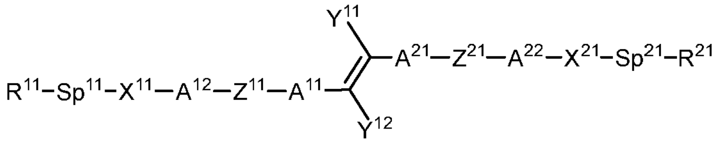

I-4

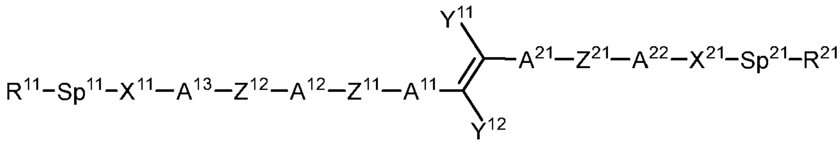

I-5

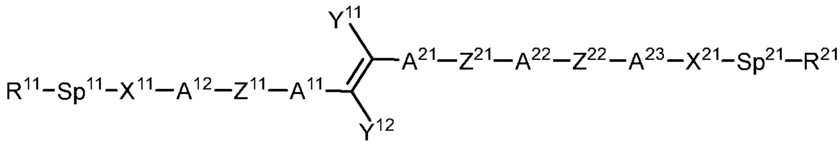

I-6

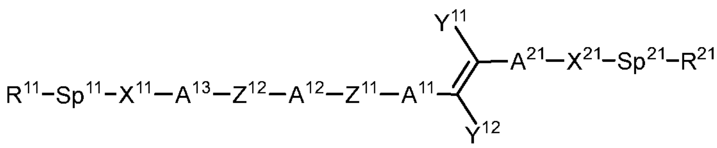

I-7

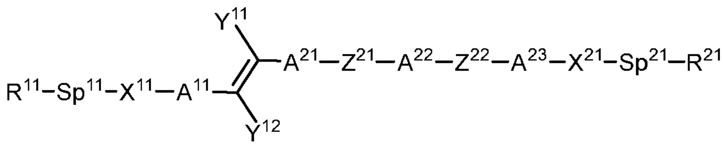

I-8

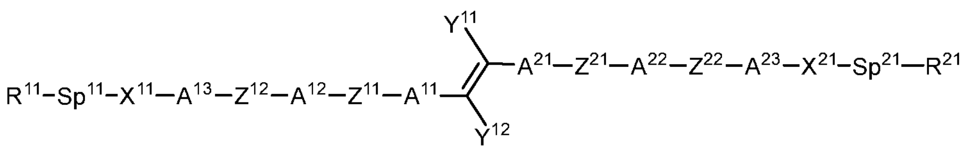

I-9

wherein $R^{11}$, $R^{21}$, $A^{11}$, $X^{11}$, $X^{12}$, $Y^{11}$, $Y^{12}$, $Sp^{11}$, and $Sp^{12}$ have in each occurrence and each and independently from another one of the meanings as given in claim 1,
$A^{12}$ to $A^{23}$ have each and independently from another one of the meanings for A as given in claim 1, and
$Z^{11}$ to $Z^{22}$ have each and independently from another one of the meanings for Z as given in claim 1.

3. Compound according to claim 1 or 2, **characterized in that** the compound are selected from the following compounds of formulae I-1a to I-4c.

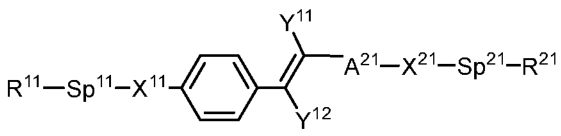

I-1a

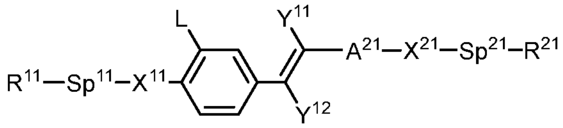

I-1b

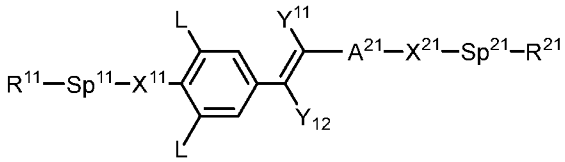

I-1c

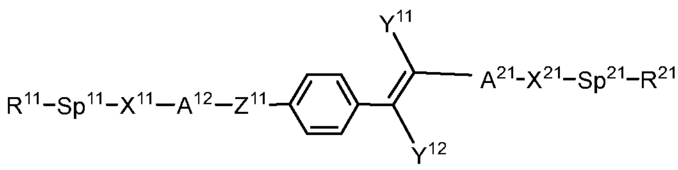

I-2a

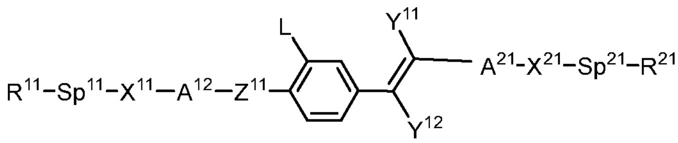

I-2b

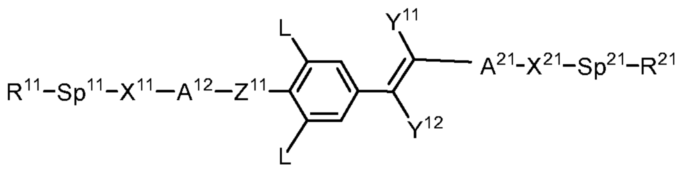

I-2c

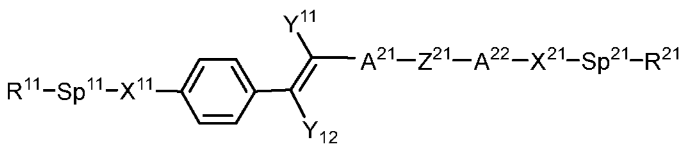

I-3a

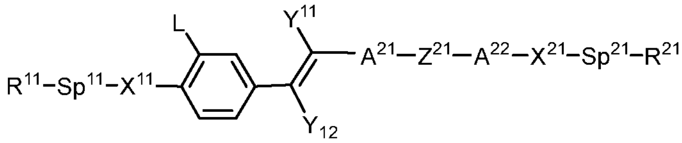

I-3b

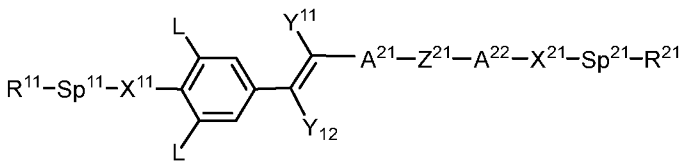

I-3c

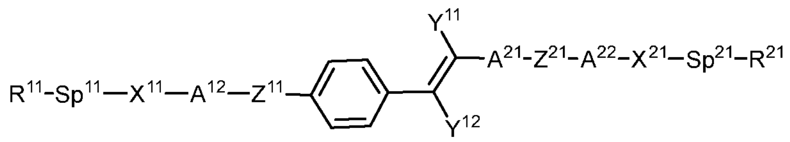

I-4a

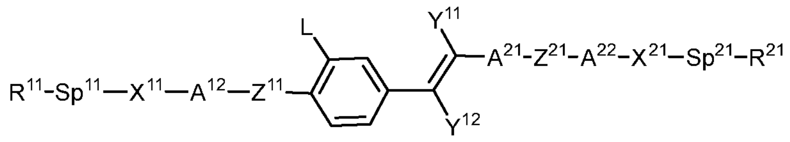

I-4b

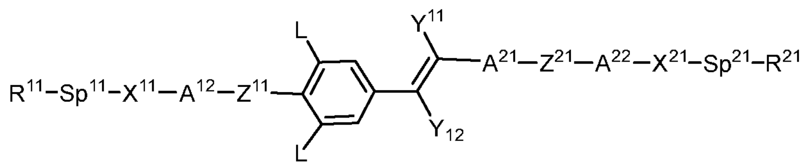

I-4c

wherein L, $R^{11}$, $R^{21}$, $A^{11}$, $X^{11}$, $X^{21}$, $Y^{11}$, $Y^{12}$, $Sp^{11}$, and $Sp^{21}$ have one of the meanings as given above in formula I, $A^{12}$ to $A^{23}$ have one of the meanings for A in formula I, and $Z^{11}$ to $Z^{22}$ have one of the meanings for Z as given above under formula I.

**4.** Compound according to one or more of claims 1 to 3, **characterized in that** it is selected from compounds of the following sub-formulae

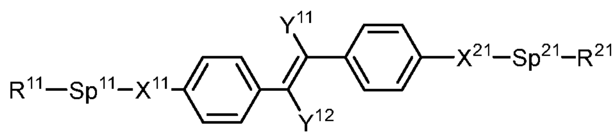

I-1a-1

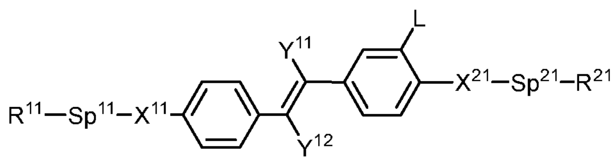

I-1a-2

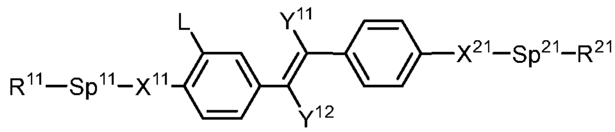

I-1b-1

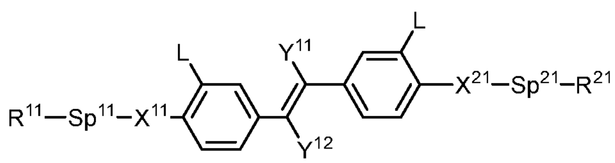

I-1b-2

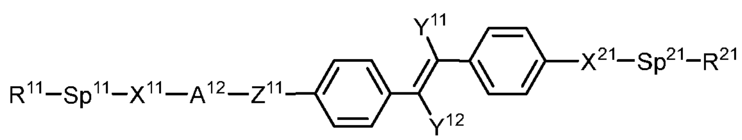

I-2a-1

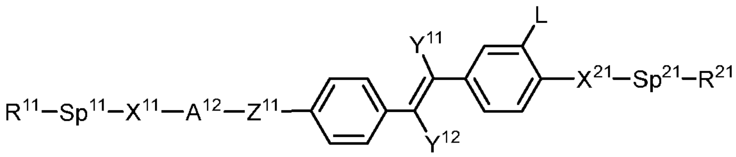

I-2a-2

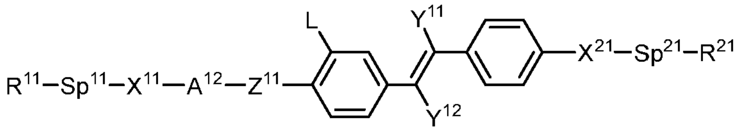

I-2b-1

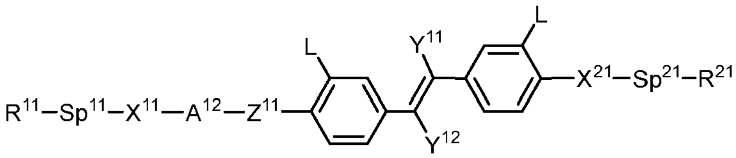

I-2b-2

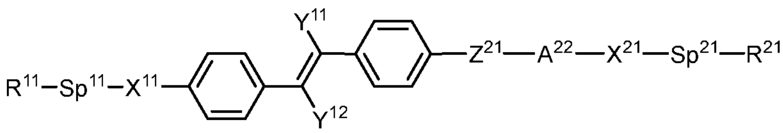

I-3a-1

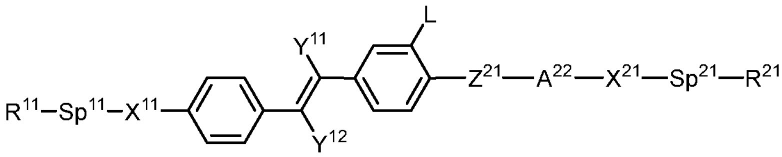

I-3a-2

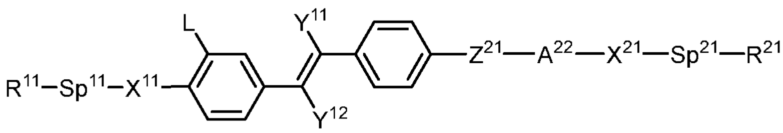

I-3b-1

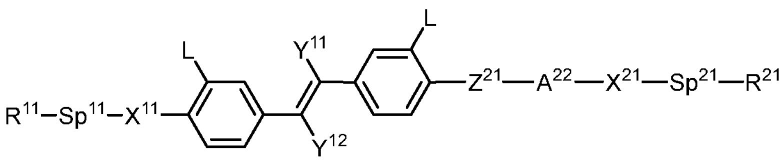

I-3b-2

wherein L, $R^{11}$, $R^{21}$, $A^{11}$, $X^{11}$, $X^{21}$, $Sp^{11}$, and $Sp^{21}$ have one of the meanings as given above in formula I, $A^{12}$ and $A^{22}$ have one of the meanings for A in formula I, and $Z^{11}$ and $Z^{22}$ have one of the meanings for Z as given above under formula I. I-3-1, 1-3-2 or I-3-3,

**5.** Compound according to one or more of claims 1 to 4, **characterized in that** it is selected from compounds of the sub-formulae

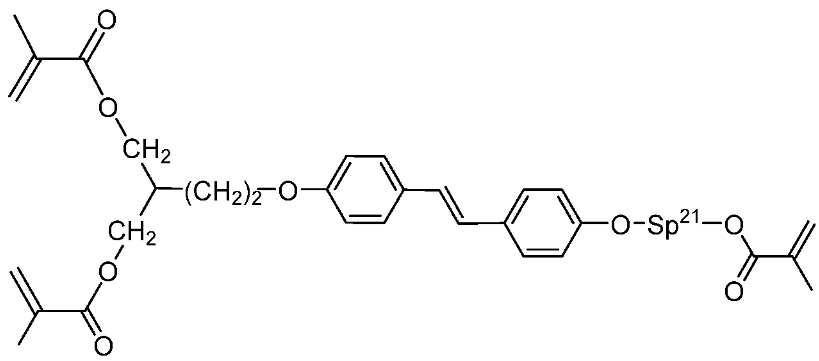

I-1a-1-1

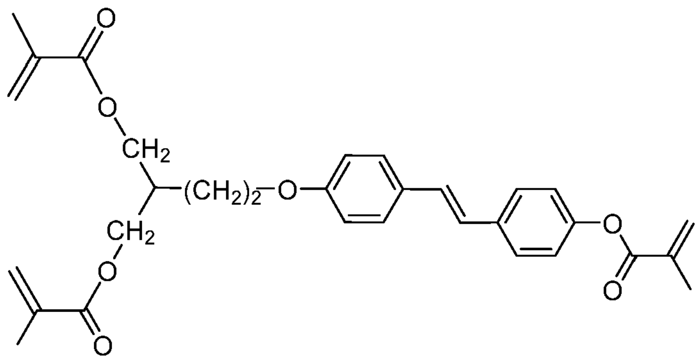

I-1a-1-2

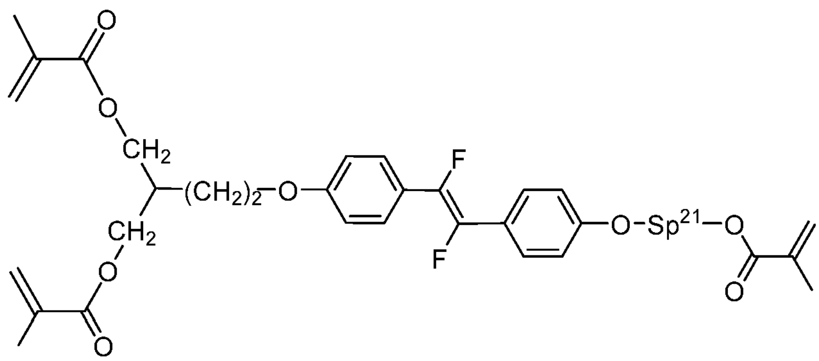

I-1a-1-3

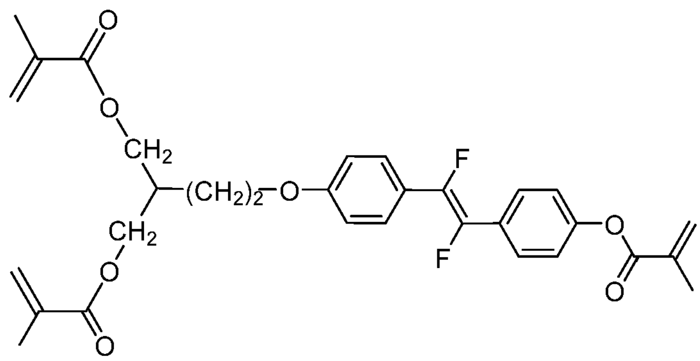

I-1a-1-4

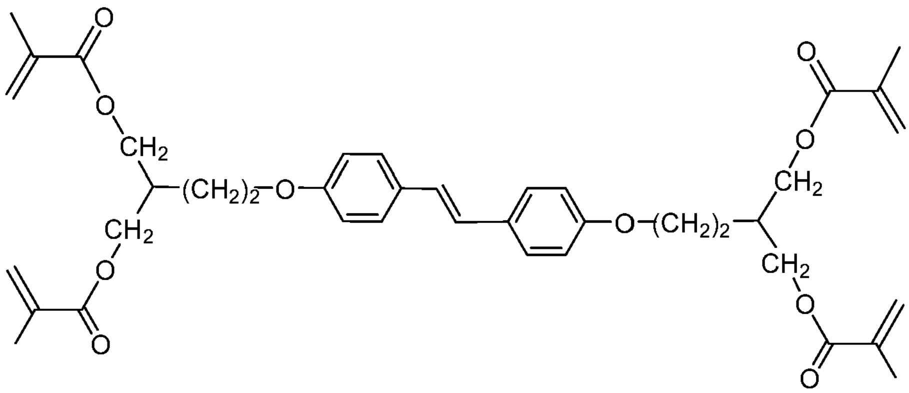

I-1a-1-5

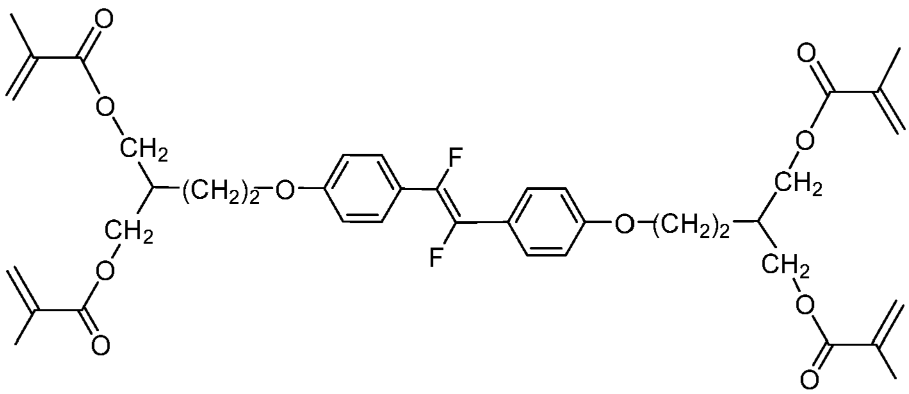

I-1a-1-6

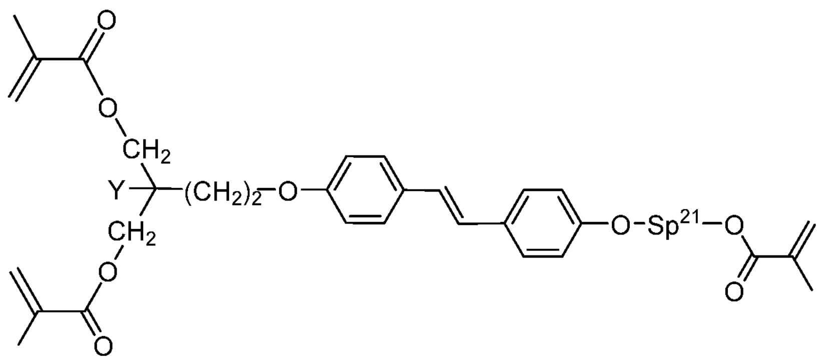

I-1a-1-7

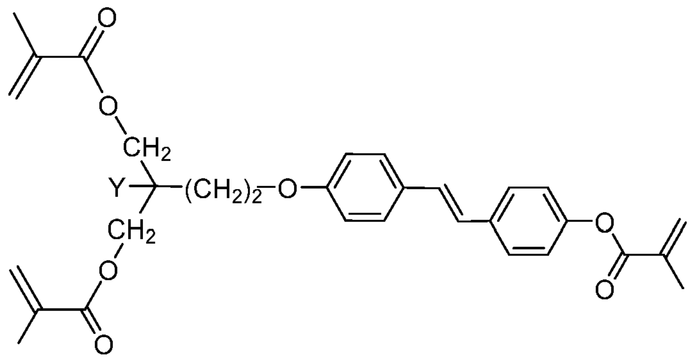

I-1a-1-8

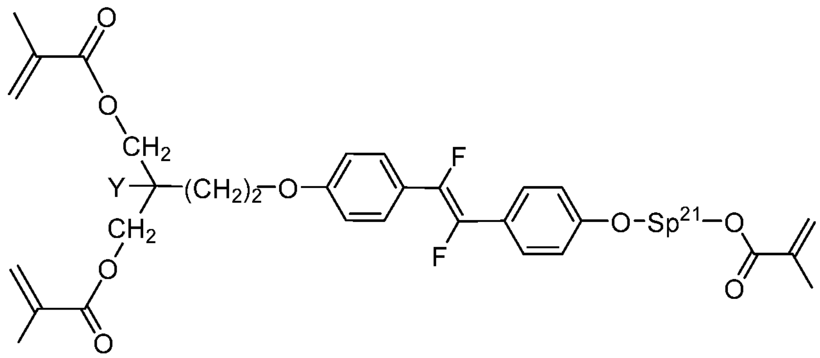

I-1a-1-9

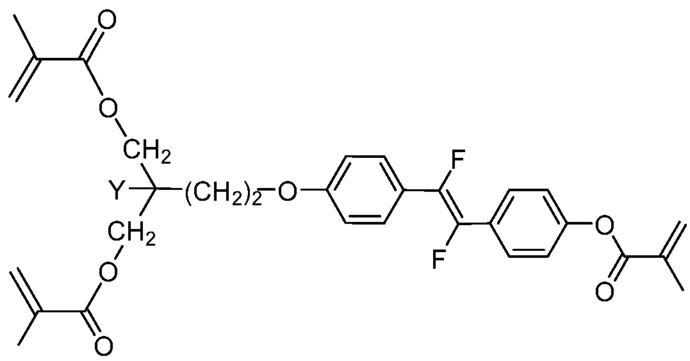

I-1a-1-10

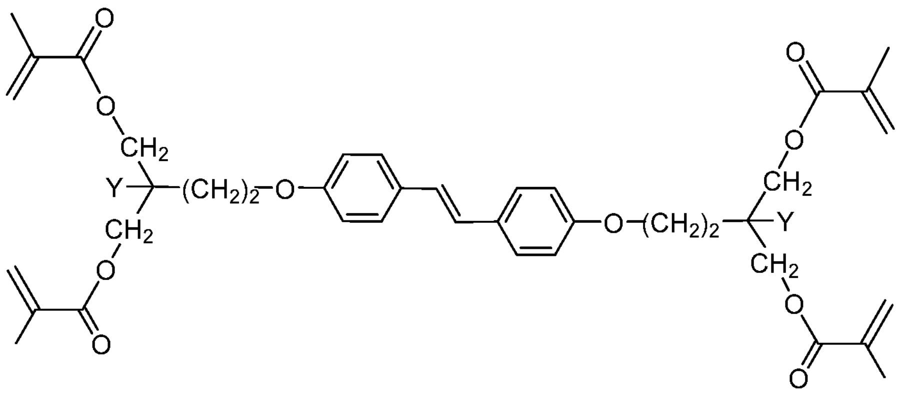

I-1a-1-11

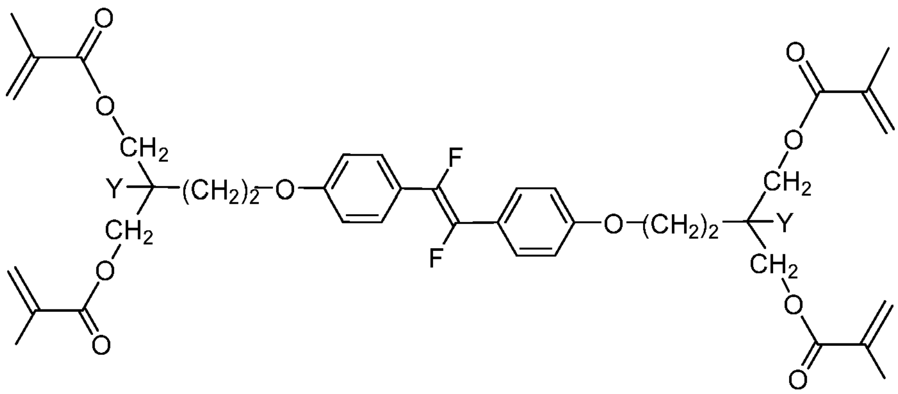

I-1a-1-12

wherein $Sp^{11}$ and $Sp^{21}$ have one of the meanings as given above in formula I, and Y denote each and independently, methyl or ethyl.

**6.** Use of compounds of formula I according to one or more of claims 1 to 5 in a liquid crystal mixture.

**7.** Liquid crystal mixture, **characterised in that** it comprises a component A) comprising one or more compounds of formula I according to one or more of claims 1 to 6, and a liquid-crystalline component B), comprising one or more mesogenic or liquid-crystalline compounds.

**8.** Liquid crystal mixture according to claim 7 **characterised in that** the total concentration of compounds of formula I in the mixture is in the range of from 0.01 to 10% by weight.

9. Liquid crystal mixture according to one or more of claims 7 or 8, **characterised in that** it additionally comprises a polymerizable component C) comprising one or more polymerizable mesogenic or polymerizable isotropic compounds.

10. Liquid crystal mixture according to claim 9, **characterised in that** the concentration of polymerizable mesogenic or polymerizable isotropic compounds is in the range of from 0.01 to 10% by weight.

11. Liquid crystal mixture according to one or more of claims 7 to 10, **characterized in that** the LC host mixture has negative dielectric anisotropy.

12. Liquid crystal mixture according to claim 11, **characterised in that** the LC host mixture comprises one or more compounds selected from the following formulae:

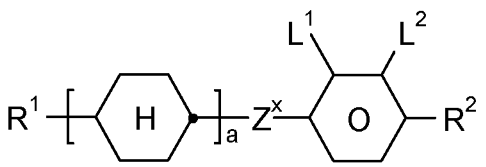

CY

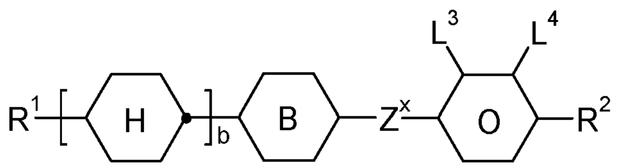

PY

wherein

a is 1 or 2,
b is 0 or 1,

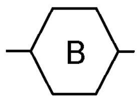

denotes

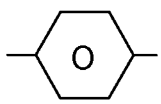 or 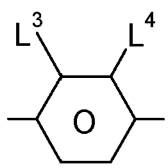

$R^1$ and $R^2$ each, independently of one another, denote alkyl having 1 to 12 C atoms, where, in addition, one or two nonadjacent $CH_2$ groups may be replaced by -O-, -CH=CH-, -CO-, -O-CO- or -CO-O- in such a way that O atoms are not linked directly to one another,
$Z^x$ denotes -CH=CH-, $-CH_2O-$, $-OCH_2-$, $-CF_2O-$ , $-OCF_2-$, -O-, $-CH_2-$, $-CH_2CH_2-$ or a single bond,
$L^{1-4}$ each, independently of one another, denote F, Cl, $OCF_3$, $CF_3$, $CH_3$, $CH_2F$, $CHF_2$.

13. Liquid crystal mixture according to one or more of claims 7 to 10, **characterised in that** the LC host mixture has positive dielectric anisotropy.

14. Liquid crystal mixture according to claim 13, **characterised in that** the LC host mixture comprises one or more compounds selected from the group consisting of the compounds of the formulae II and III,

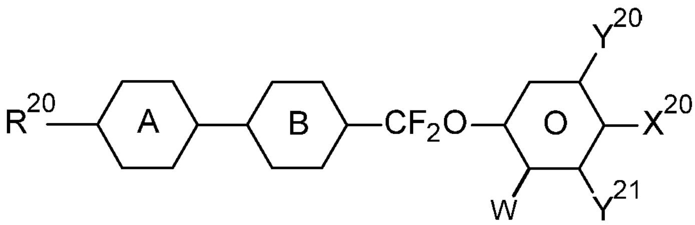

II

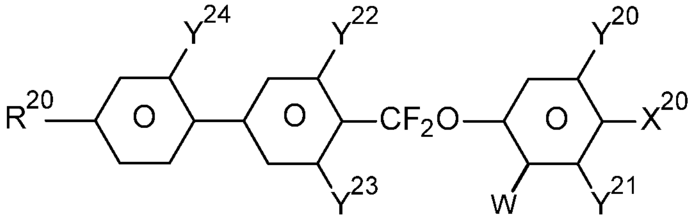

III

wherein

$R^{20}$ each, identically or differently, denote a halogenated or un-substituted alkyl or alkoxy radical having 1 to 15 C atoms, where, in addition, one or more $CH_2$ groups in these radicals may each be replaced, independently of one another, by -C≡C-, $-CF_2O-$, -CH=CH-,

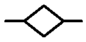,
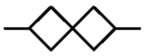,

-O-, -CO-O- or -O-CO- in such a way that O atoms are not linked directly to one another,
$X^{20}$ each, identically or differently, denote F, Cl, CN, $SF_5$, SCN, NCS, a halogenated alkyl radical, a halogenated alkenyl radical, a halogenated alkoxy radical or a halogenated alkenyloxy radical, each having up to 6 C atoms, and
$Y^{20-24}$ each, identically or differently, denote H or F,
W denotes H or methyl,

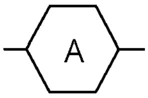 and 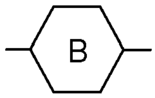

each, identically or differently, denote

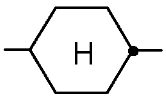,

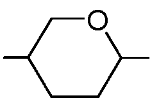 or 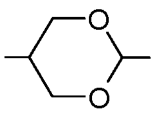.

**15.** Liquid crystal mixture according to claim 13 or 14, **characterised in that** it comprises one or more compounds selected from the group consisting of compounds of formulae XI and XII

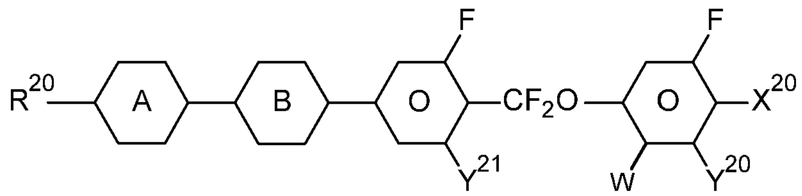 XI

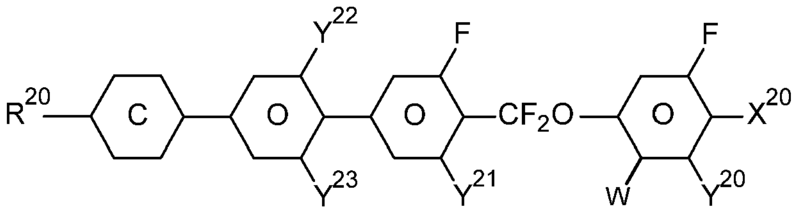 XII

wherein $R^{20}$, $X^{20}$, Wand $Y^{20-23}$ have the meanings indicated in formula III in claim 16, and

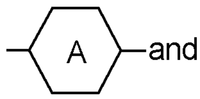 and 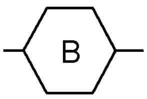

each, independently of one another, denote

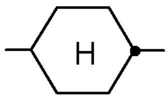 ,

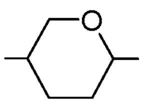 or 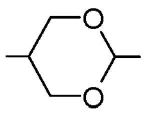

and

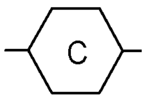

denotes

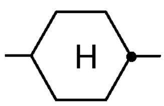 , 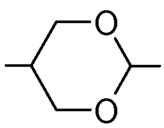 , 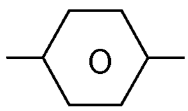

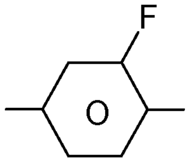 or 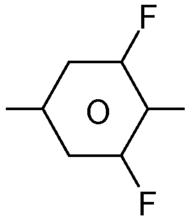

**16.** Liquid crystal mixture according to one or more of claims 7 to 15, **characterised in that** the LC host mixture comprises one or more compounds of the following formula:

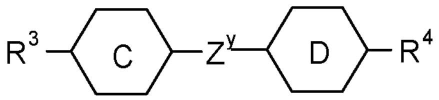

ZK

in which the individual radicals have the following meanings:

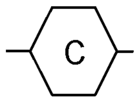

denotes

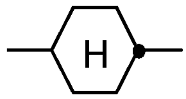, 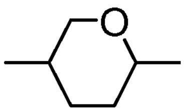, 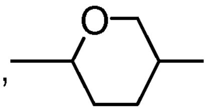,

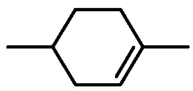 or 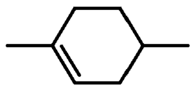,

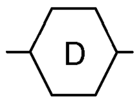

denotes

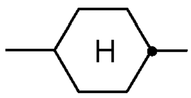 or 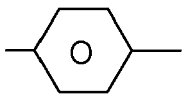,

$R^3$ and $R^4$ each, independently of one another, denote alkyl having 1 to 12 C atoms, in which, in addition, one or two non-adjacent $CH_2$ groups may be replaced by -O-, -CH=CH-, -CO-, -O-CO- or -CO-O- in such a way that O atoms are not linked directly to one another,

$Z^y$ denotes $-CH_2CH_2-$, -CH=CH-, $-CF_2O-$, $-OCF_2-$, $-CH_2O-$, $-OCH_2-$, -CO-O-, -O-CO-, $-C_2F_4-$, -CF=CF-, -CH=CH-$CH_2O-$ or a single bond.

**17.** Liquid crystal mixture according to one or more of claims 7 to 16, **characterised in that** the LC host mixture comprises one or more compounds of the following formula

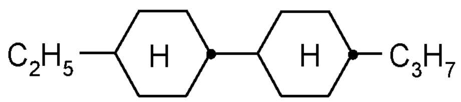

ZK1a

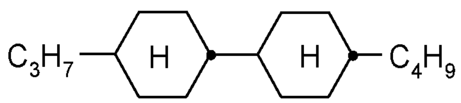

ZK1b

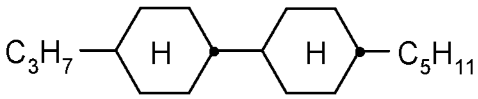

ZK1c

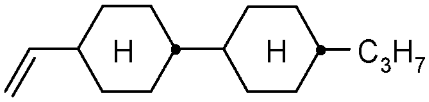

ZK3a

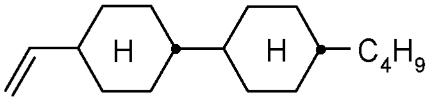

ZK3b

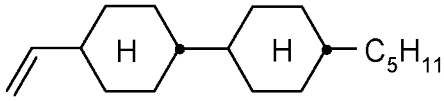

ZK3c

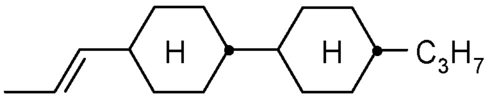

ZK3d

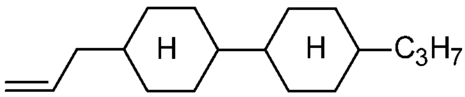

ZK3e

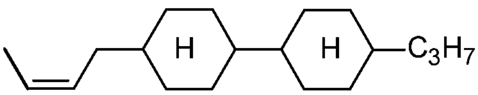

ZK3f

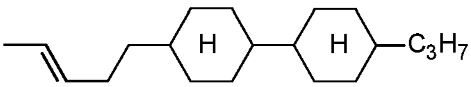

ZK3g

wherein the propyl, butyl and pentyl groups are straight-chain groups.

**18.** Liquid crystal mixture according to one or more of claims 7 to 17, **characterised in that** the LC host mixture comprises one or more compounds selected from the following formulae:

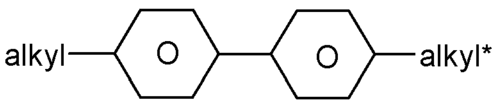

B1

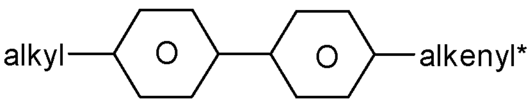

B2

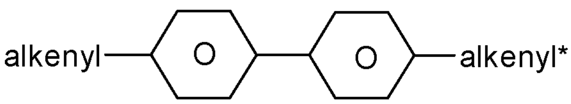

B3

in which alkyl and alkyl* each, independently of one another, denote a straight-chain alkyl radical having 1-6 C atoms, and alkenyl and alkenyl* each, independently of one another, denote a straight-chain alkenyl radical having 2-6 C atoms.

**19.** Liquid crystal mixture according to one or more of claims 7 to 18, **characterised in that** the LC host mixture comprises one or more compounds selected from the following formulae:

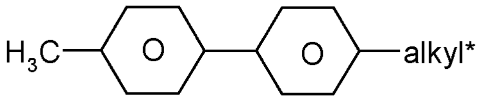
B1a

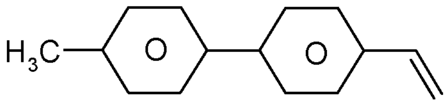
B2a

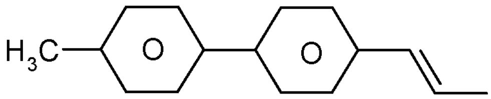
B2b

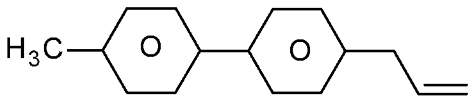
B2c

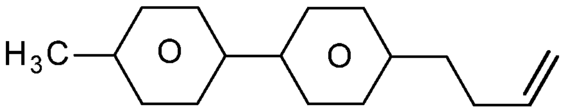
B2d

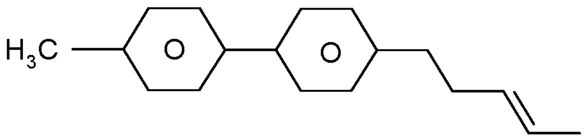
B2e

in which alkyl* denotes an alkyl radical having 1-6 C atoms.

**20.** Use of the liquid crystal mixture according to one or more of claims 7 to 19 for the fabrication of a liquid crystal display.

**21.** Process for the fabrication of a liquid crystal display, comprising at least the steps of:

- providing a first substrate which includes a pixel electrode and a common electrode for generating an electric field substantially parallel to a surface of the first substrate in the pixel region;
- providing a second substrate, the second substrate being disposed opposite to the first substrate;
- interposing a liquid crystal mixture according to one or more of claims 7 to 19;
- irradiating the liquid crystal mixture with linearly polarised light causing photoalignment of the liquid crystal;
- curing the polymerizable compounds of the liquid crystal mixture by irradiation with ultraviolet light or visible light having a wavelength of 450 nm or below.

**22.** Process according to claim 21, **characterised in that** the linearly polarised light is ultraviolet light or visible light having a wavelength of 450nm or below.

**23.** Display, obtainable by a process according to claim 21 or 22.

**24.** Display according to claim 23, wherein the LC host mixture is homogeneously aligned without the application of an

electric field.

**25.** Display according to claim 23 or 24, wherein the display is an IPS or FFS display.

**Patentansprüche**

**1.** Verbindung der Formel I,

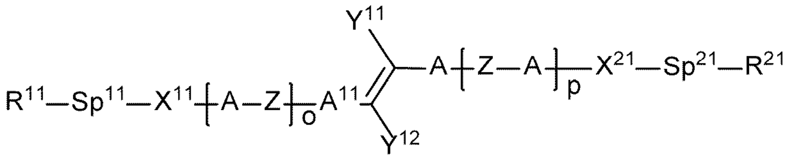

I

bei der

$A^{11}$ einen Rest bedeutet, der ausgewählt ist aus den folgenden Gruppen: a) einer Gruppe bestehend aus 1,4-Phenylen und 1,3-Phenylen, bei denen zusätzlich eine oder zwei CH-Gruppen durch N ersetzt sein können und bei denen zusätzlich ein oder mehrere H-Atome durch L ersetzt sein können, b) einer Gruppe ausgewählt aus der Gruppe bestehend aus

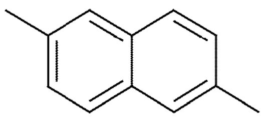 , 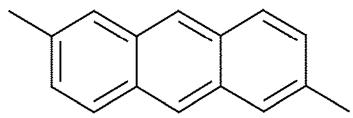 ,

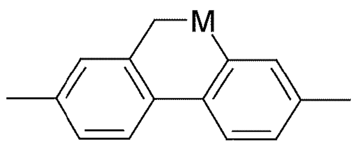 , 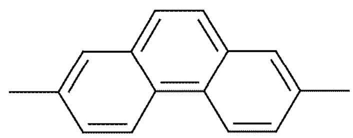 ,

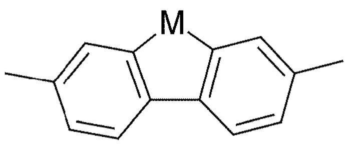 , 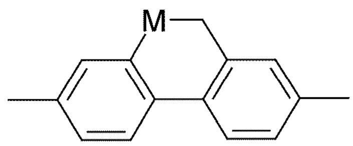 ,

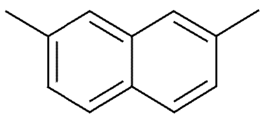 , 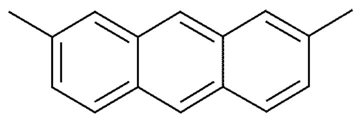 ,

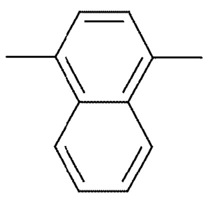 , 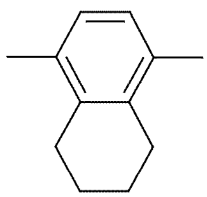 ,

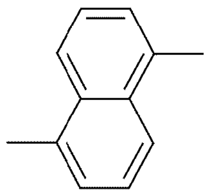,

wobei zusätzlich ein oder mehrere H-Atome in diesen Resten durch L ersetzt sein können und/oder eine oder mehrere Doppelbindungen durch Einfachbindungen ersetzt sein können und/oder eine oder mehrere CH-Gruppen durch N ersetzt sein können,

A jeweils unabhängig voneinander bei jedem Auftreten eine der Bedeutungen für $A^{11}$ besitzen oder ausgewählt sind aus a) einer Gruppe bestehend aus trans-1,4-Cyclohexylen, 1,4-Cyclohexenylen, bei denen zusätzlich eine oder mehrere nicht benachbarte $CH_2$-Gruppen durch -O- und/oder -S- ersetzt sein können und bei denen zusätzlich ein oder mehrere H-Atome durch F ersetzt sein können, oder b) einer Gruppe bestehend aus Tetrahydropyran2,5-diyl, 1,3-Dioxan-2,5-diyl, Tetrahydrofuran-2,5-diyl, Cyclobutan-1,3-diyl, Piperidin-1,4-diyl, Thiophen-2,5-diyl und Selenophen-2,5-diyl, die jeweils auch ein- oder mehrfach durch L substituiert sein können,

L bei jedem Auftreten gleich oder verschieden -OH, -F, -Cl, -Br, -I, -CN, $-NO_2$, $SF_5$, -NCO, -NCS, -OCN, -SCN, $-C(=O)N(R^z)_2$, $-C(=O)R^z$, $-N(R^z)_2$, gegebenenfalls substituiertes Silyl, gegebenenfalls substituiertes Aryl mit 6 bis 20 C-Atomen oder geradkettiges oder verzweigtes oder cyclisches Alkyl, Alkoxy, Alkylcarbonyl, Alkoxycarbonyl, Alkylcarbonyloxy oder Alkoxycarbonyloxy mit 1 bis 25 C-Atomen, worin zusätzlich ein oder mehrere H-Atome durch F oder Cl ersetzt sein können, oder $X^{21}$-$Sp^{21}$-$R^{21}$ bedeutet,

M -O-, -S-, $-CH_2-$, $-CHR^z-$ oder $-CR^yR^z-$ bedeutet, und

$R^y$ und $R^z$ jeweils unabhängig voneinander H, CN, F oder Alkyl mit 1-12 C-Atomen bedeuten, bei dem ein oder mehrere H-Atome durch F ersetzt sein können,

$Y^{11}$ und $Y^{12}$ jeweils unabhängig voneinander H, F, Phenyl oder Alkyl mit 1-12 C-Atomen bedeuten, bei dem ein oder mehrere H-Atome durch F ersetzt sein können,

Z unabhängig voneinander bei jedem Auftreten eine Einfachbindung, -COO-, -OCO-, -O-CO-O-, $-OCH_2-$, $-CH_2O-$, $-OCF_2-$, $-CF_2O-$ oder $-(CH_2)_n-$ $-(CH_2)_n-$, bei denen eine oder mehrere nicht benachbarte Gruppen durch O oder S ersetzt sein können, $-CF_2CF_2-$, -CH=CH-, -CF=CF-, -CH=CH-COO-, -OCO-CH=CH-, -CO-S-, -S-CO-, -CS-S-, -S-CS-, -S-CSS- oder -C≡C- bedeutet,

n eine ganze Zahl zwischen 2 und 8 bedeutet,

o und p jeweils und unabhängig 0, 1 oder 2 bedeuten,

$X^{11}$ und $X^{21}$ unabhängig voneinander bei jedem Auftreten eine Einfachbindung, -CO-O-, -O-CO-, -O-COO-, -O-, -CH=CH-, -C≡C-, $-CF_2-O-$, $-O-CF_2-$, $-CF_2-CF_2-$, $-CH_2-O-$, $-O-CH_2-$, -CO-S-, -S-CO-, -CS-S-, -S-CS-, -S-CSS- oder -S- bedeuten,

$Sp^{11}$ und $Sp^{21}$ jeweils und unabhängig bei jedem Auftreten eine Einfachbindung oder eine Spacergruppe mit 1 bis 20 C-Atomen bedeuten, bei der auch eine oder mehrere nicht benachbarte und nicht endständige $CH_2$-Gruppen durch -O-, -S-, -NH-, $-N(CH_3)-$, -CO-, -O-CO-, -S-CO-, -O-COO-, -CO-S-, -CO-O-, $-CF_2-$, $-CF_2O-$, $-OCF_2-$ -C(OH)-, -CH(Alkyl)-, -CH(Alkenyl)-, -CH(Alkoxyl)-, -CH(Oxaalkyl)-, -CH=CH- oder -C≡C-ersetzt sein können, jedoch derart, dass keine zwei O-Atome einander benachbart sind und keine zwei aus -O-CO-, -S-CO-, -O-COO-, -CO-S-, -CO-O- und -CH=CH- ausgewählten Gruppen einander benachbart sind,

$R^{11}$ und $R^{21}$ $P^{11}$ oder $P^{21}$ bedeuten, unter der Bedingung, dass mindestens einer von $R^{11}$ und $R^{21}$ $P^{21}$ bedeutet,

$P^{11}$ jeweils und unabhängig voneinander bei jedem Auftreten eine polymerisierbare Gruppe bedeuten, die aus der Gruppe bestehend aus Acrylat-, Methacrylat-, Fluoracrylat-, weiterhin Vinyloxy-, Chloracrylat-, Oxetan- und Epoxidgruppen ausgewählt ist,

$P^{21}$ eine Gruppe

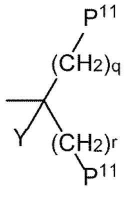

bedeutet,

Y H, F, Phenyl oder gegebenenfalls fluoriertes Alkyl mit 1-12 C-Atomen bedeutet,

q und r jeweils und unabhängig eine ganze Zahl von 0 bis 8 bedeuten.

**2.** Verbindung nach Anspruch 1, **dadurch gekennzeichnet, dass** die Verbindung ausgewählt ist aus Verbindungen der Unterformeln I-1 bis I-9,

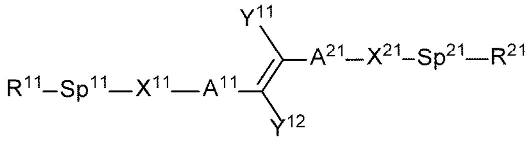

I-1

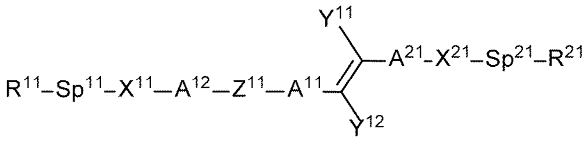

I-2

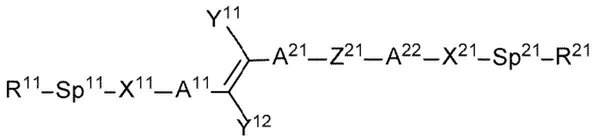

I-3

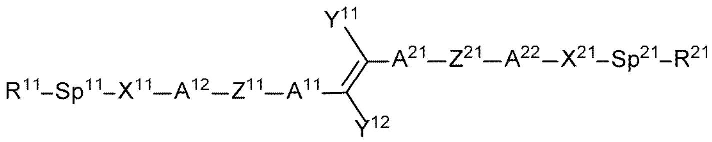

I-4

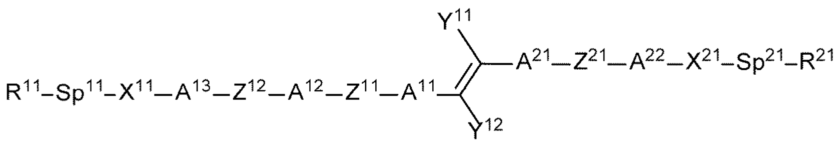

I-5

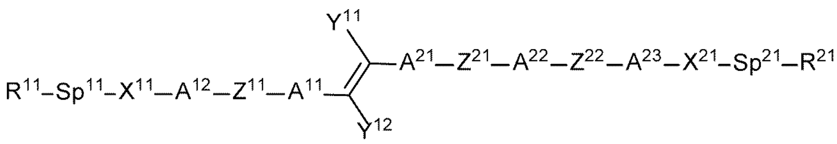

I-6

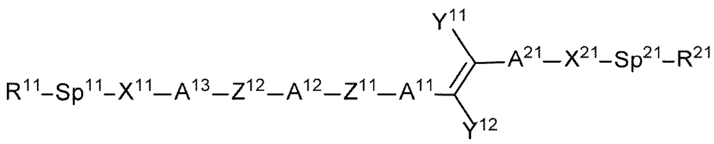

I-7

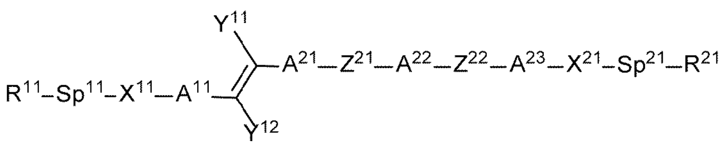

I-8

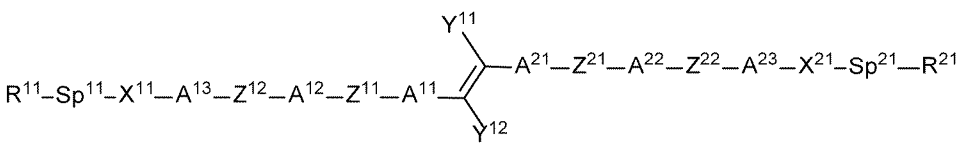

I-9

bei denen $R^{11}$, $R^{21}$, $A^{11}$, $X^{11}$, $X^{12}$, $Y^{11}$, $Y^{12}$, $Sp^{11}$ und $Sp^{12}$ bei jedem Auftreten und jeweils und unabhängig voneinander eine der Bedeutungen wie in Anspruch 1 genannt besitzen,
$A^{12}$ bis $A^{23}$ jeweils und unabhängig voneinander eine der Bedeutungen für A wie in Anspruch 1 genannt besitzen, und
$Z^{11}$ bis $Z^{22}$ jeweils und unabhängig voneinander eine der Bedeutungen für Z wie in Anspruch 1 genannt besitzen.

3. Verbindung nach Anspruch 1 oder 2, **dadurch gekennzeichnet, dass** die Verbindung ausgewählt ist aus den folgenden Verbindungen der Formeln I-1a bis I-4c,

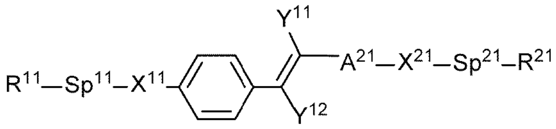

I-1a

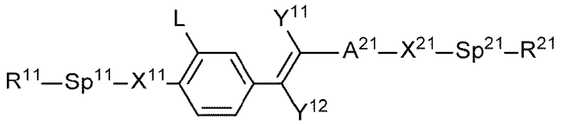

I-1b

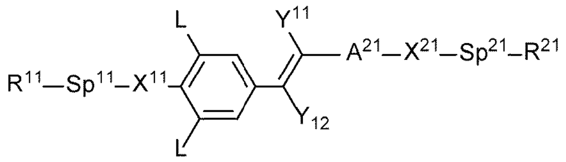

I-1c

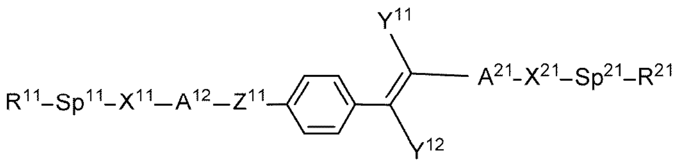

I-2a

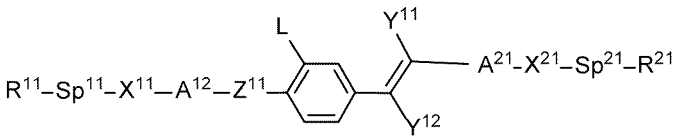

I-2b

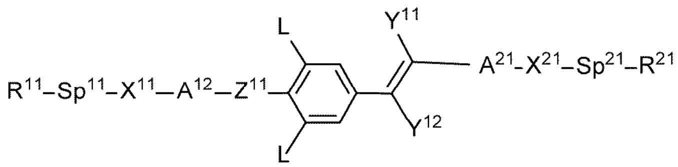

I-2c

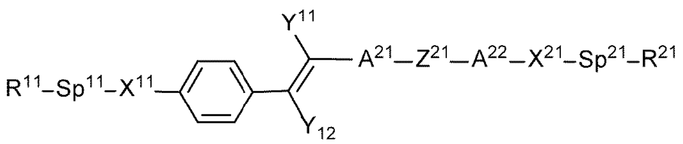

I-3a

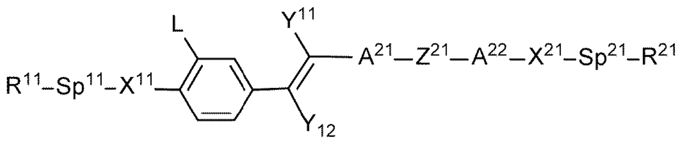

I-3b

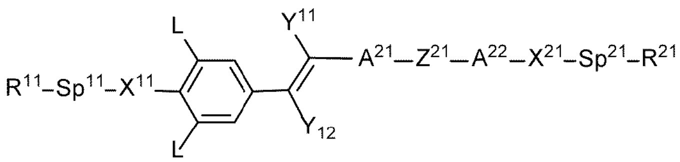

I-3c

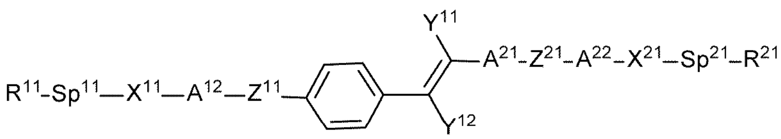

I-4a

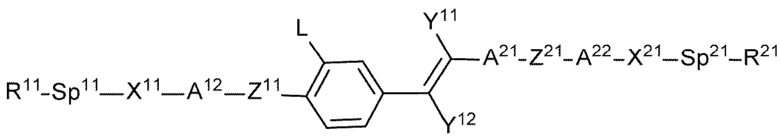

I-4b

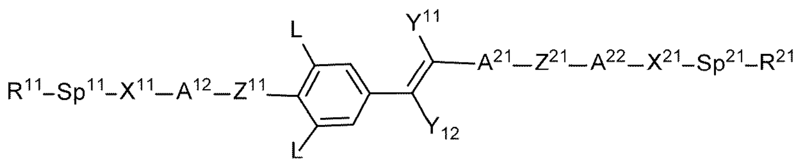

I-4c

bei denen L, $R^{11}$, $R^{21}$, $A^{11}$, $X^{11}$, $X^{21}$, $Y^{11}$, $Y^{12}$, $Sp^{11}$ und $Sp^{21}$ eine der Bedeutungen wie vorstehend in Formel I genannt besitzen, $A^{12}$ bis $A^{23}$ eine der Bedeutungen für A in Formel I besitzen und $Z^{11}$ bis $Z^{22}$ eine der Bedeutungen für Z wie vorstehend unter Formel I genannt besitzen.

**4.** Verbindung nach einem oder mehreren der Ansprüche 1 bis 3, **dadurch gekennzeichnet, dass** sie ausgewählt ist aus Verbindungen der folgenden Unterformeln

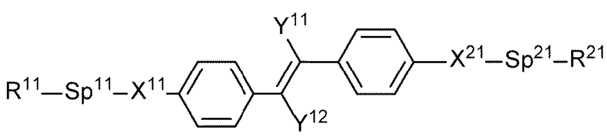

I-1a-1

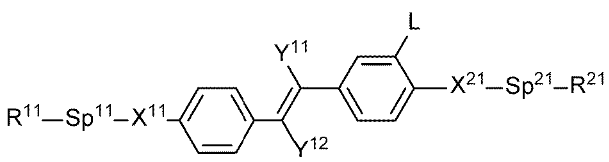

I-1a-2

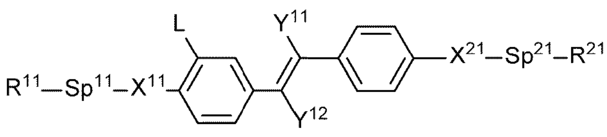

I-1b-1

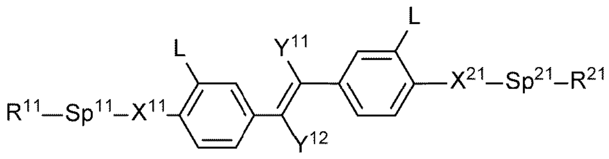

I-1b-2

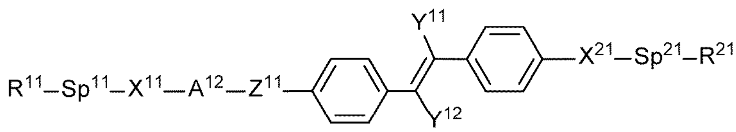

I-2a-1

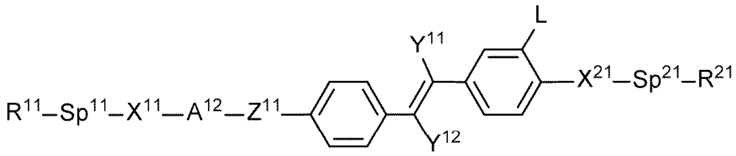

I-2a-2

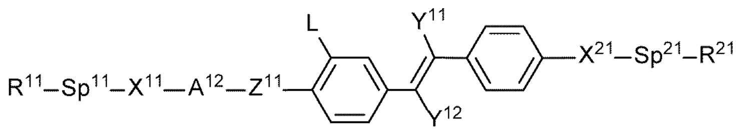

I-2b-1

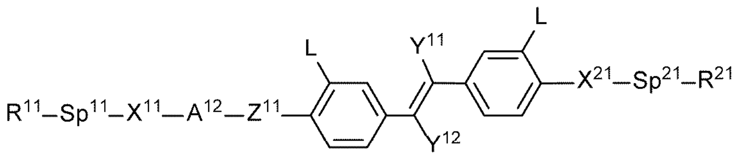

I-2b-2

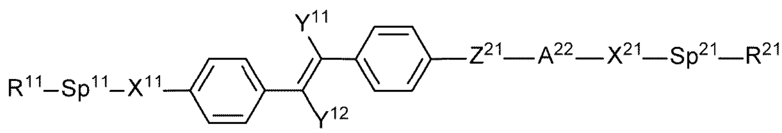

I-3a-1

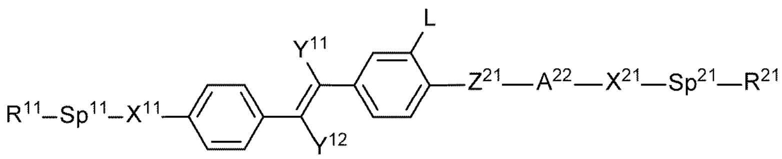

I-3a-2

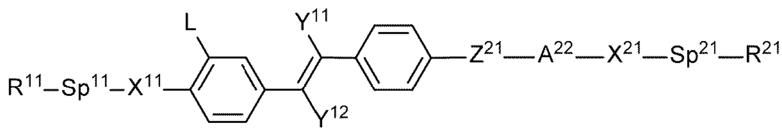

I-3b-1

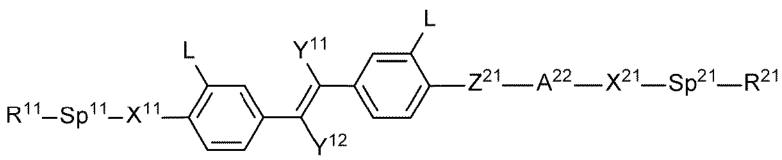

I-3b-2

bei denen L, $R^{11}$, $R^{21}$, $A^{11}$, $X^{11}$, $X^{21}$, $Sp^{11}$ und $Sp^{21}$ eine der Bedeutungen wie vorstehend in Formel I genannt besitzen, $A^{12}$ und $A^{22}$ eine der Bedeutungen für A in Formel I besitzen und $Z^{11}$ und $Z^{22}$ eine der Bedeutungen für Z wie vorstehend unter Formel I genannt besitzen.

5. Verbindung nach einem oder mehreren der Ansprüche 1 bis 4, **dadurch gekennzeichnet, dass** sie ausgewählt ist aus Verbindungen der Unterformeln

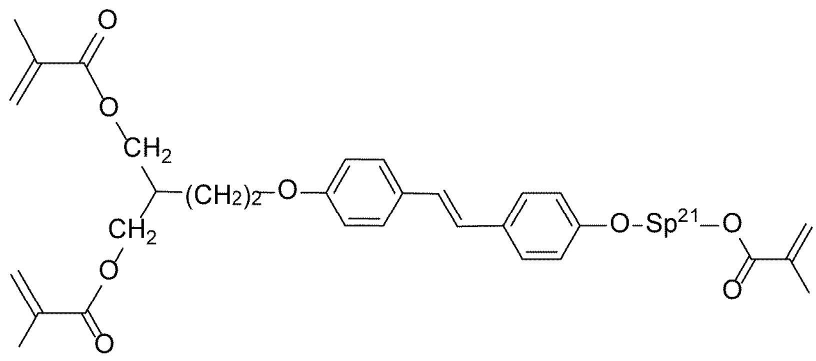

I-1a-1-1

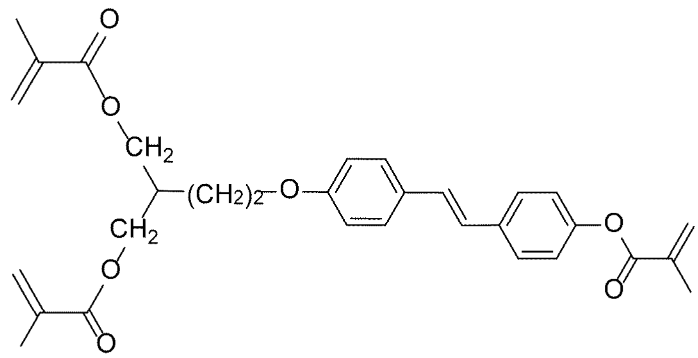

I-1a-1-2

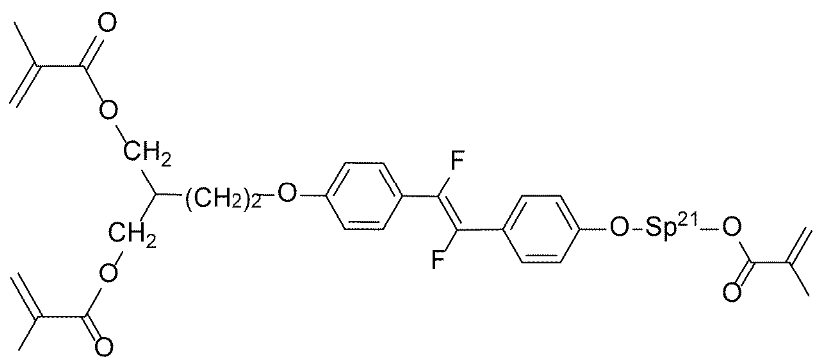

I-1a-1-3

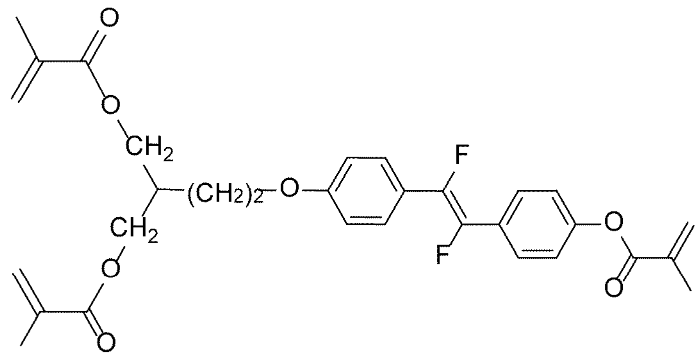

I-1a-1-4

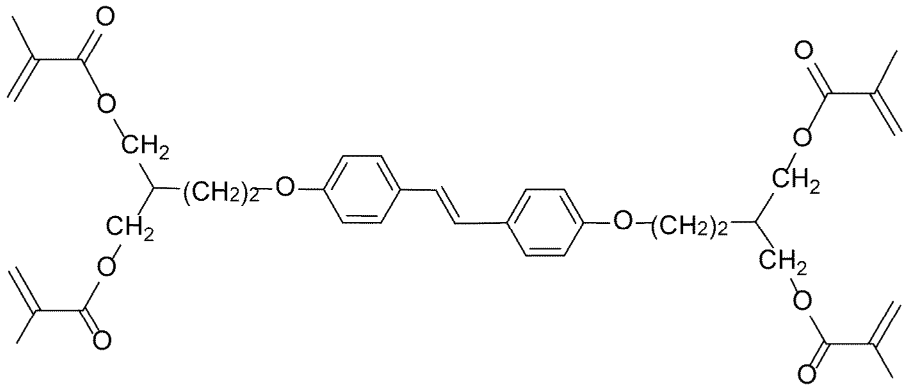

I-1a-1-5

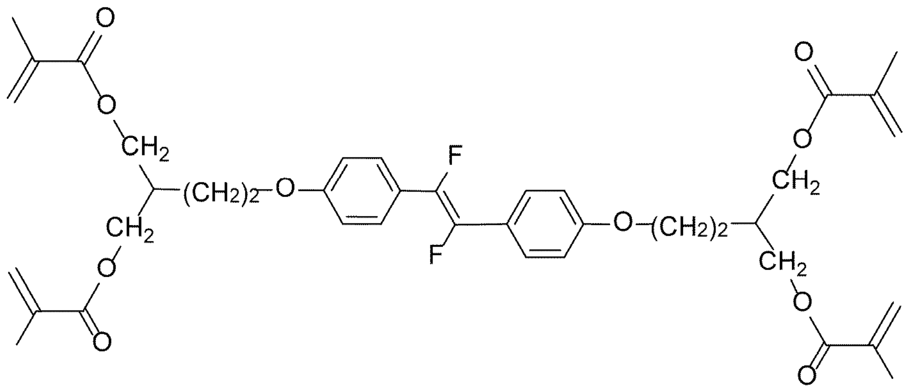

I-1a-1-6

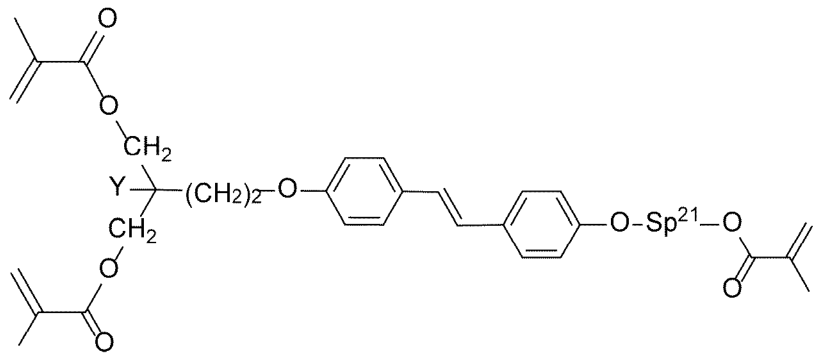

I-1a-1-7

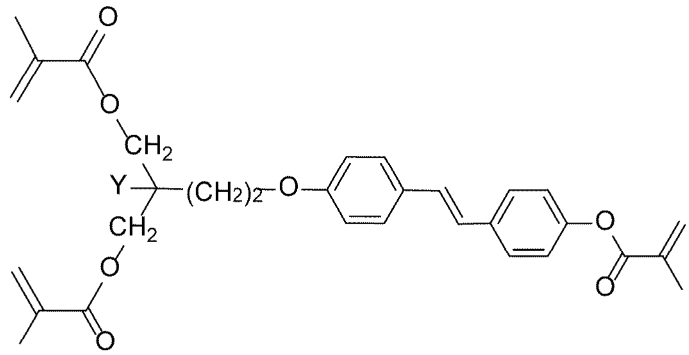

I-1a-1-8

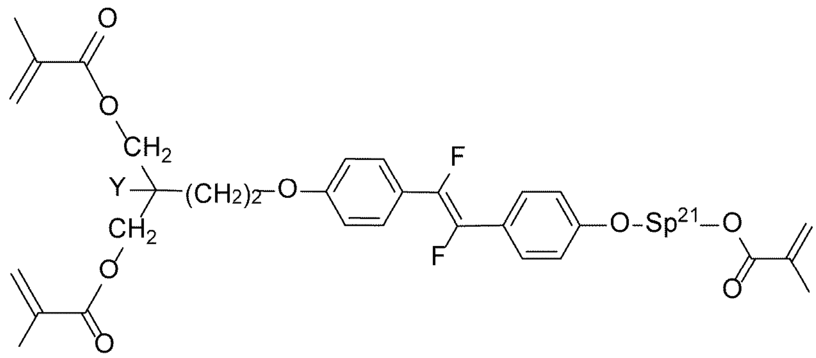

I-1a-1-9

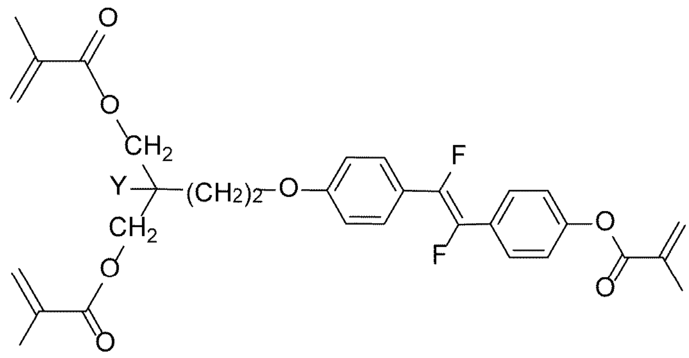

I-1a-1-10

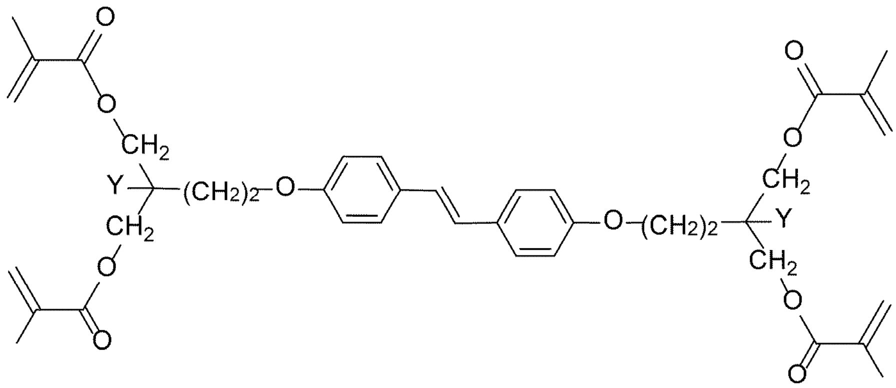

I-1a-1-11

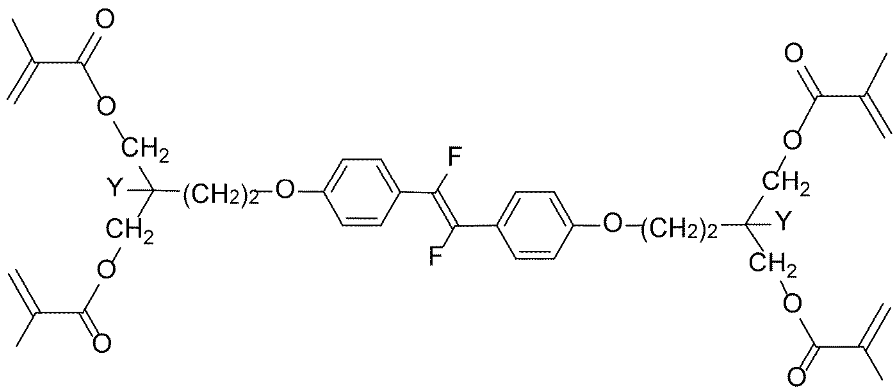

I-1a-1-12

bei denen $Sp^{11}$ und $Sp^{21}$ eine der Bedeutungen wie vorstehend in Formel I genannt besitzen und Y jeweils und unabhängig Methyl oder Ethyl bedeuten.

**6.** Verwendung von Verbindungen der Formel I nach einem oder mehreren der Ansprüche 1 bis 5 in einer Flüssigkristallmischung.

**7.** Flüssigkristallmischung, **dadurch gekennzeichnet, dass** sie eine Komponente A) enthaltend eine oder mehrere Verbindungen der Formel I nach einem oder mehreren der Ansprüche 1 bis 6 und eine flüssigkristalline Komponente B) enthaltend eine oder mehrere mesogene oder flüssigkristalline Verbindungen enthält.

**8.** Flüssigkristallmischung nach Anspruch 7, **dadurch gekennzeichnet, dass** die Gesamtkonzentration der Verbindungen der Formel I in der Mischung im Bereich von 0,01 bis 10 Gew.-% liegt.

**9.** Flüssigkristallmischung nach einem oder mehreren der Ansprüche 7 oder 8, **dadurch gekennzeichnet, dass** sie zusätzlich eine polymerisierbare Komponente C) enthaltend eine oder mehrere polymerisierbare mesogene oder polymerisierbare isotrope Verbindungen enthält.

**10.** Flüssigkristallmischung nach Anspruch 9, **dadurch gekennzeichnet, dass** die Konzentration der polymerisierbaren mesogenen oder polymerisierbaren isotropen Verbindungen im Bereich von 0,01 bis 10 Gew.-% liegt.

**11.** Flüssigkristallmischung nach einem oder mehreren der Ansprüche 7 bis 10, **dadurch gekennzeichnet, dass** die FK-Host-Mischung eine negative dielektrische Anisotropie aufweist.

**12.** Flüssigkristallmischung nach Anspruch 11, **dadurch gekennzeichnet, dass** die FK-Host-Mischung eine oder mehrere Verbindungen enthält, die ausgewählt sind aus den folgenden Formeln:

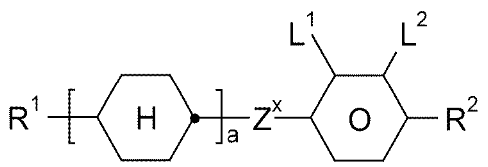

CY

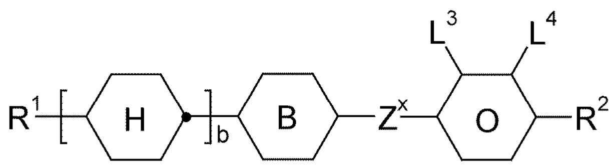

PY

bei denen

a 1 oder 2 ist,
b 0 oder 1 ist,

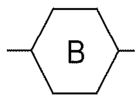

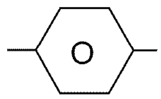

oder

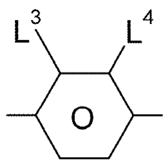

bedeutet,
$R^1$ und $R^2$ jeweils unabhängig voneinander Alkyl mit 1 bis 12 C-Atomen bedeuten, wobei zusätzlich eine oder zwei nicht benachbarte $CH_2$-Gruppen so durch -O-, -CH=CH-, -CO-, -O-CO- oder -CO-O- ersetzt sein können, dass O-Atome nicht direkt miteinander verknüpft sind,
$Z^x$ -CH=CH-, $-CH_2O-$, $-OCH_2-$, $-CF_2O-$, $-OCF_2-$, -O-, $-CH_2-$, $-CH_2CH_2-$ oder eine Einfachbindung bedeutet,
$L^{1-4}$ jeweils unabhängig voneinander F, Cl, $OCF_3$, $CF_3$, $CH_3$, $CH_2F$, $CHF_2$ bedeuten.

**13.** Flüssigkristallmischung nach einem oder mehreren der Ansprüche 7 bis 10, **dadurch gekennzeichnet, dass** die FK-Host-Mischung eine positive dielektrische Anisotropie aufweist.

**14.** Flüssigkristallmischung nach Anspruch 13, **dadurch gekennzeichnet, dass** die FK-Host-Mischung eine oder mehrere Verbindungen enthält, die ausgewählt sind aus der Gruppe bestehend aus den Verbindungen der Formeln II und III,

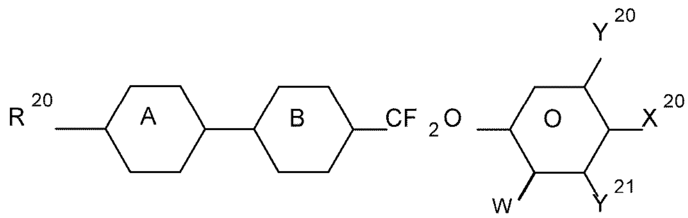

II

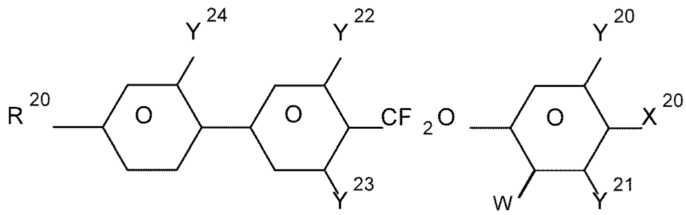

III

bei denen

$R^{20}$ jeweils gleich oder verschieden einen halogenierten oder unsubstituierten Alkyl- oder Alkoxyrest mit 1 bis 15 C-Atomen bedeuten, wobei in diesen Resten zusätzlich eine oder mehrere $CH_2$-Gruppen jeweils unabhängig voneinander so durch -C≡C-, $-CF_2O-$, -CH=CH-,

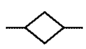 , 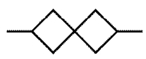 ,

-O-, -CO-O- oder -O-CO- ersetzt sein können, dass O-Atome nicht direkt miteinander verknüpft sind,
$X^{20}$ jeweils gleich oder verschieden F, Cl, CN, $SF_5$, SCN, NCS, einen halogenierten Alkylrest, einen halogenierten Alkenylrest, einen halogenierten Alkoxyrest oder einen halogenierten Alkenyloxyrest mit jeweils bis zu 6 C-Atomen bedeuten und
$Y^{20-24}$ jeweils gleich oder verschieden H oder F bedeuten,
W H oder Methyl bedeutet,

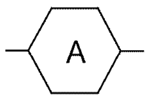 und 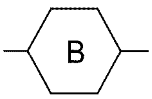

jeweils gleich oder verschieden

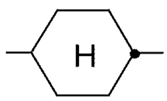 ,

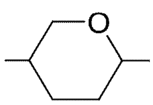 oder 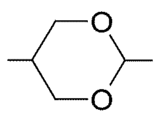 .

bedeuten.

**15.** Flüssigkristallmischung nach Anspruch 13 oder 14, **dadurch gekennzeichnet, dass** sie eine oder mehrere Verbindungen enthält, die ausgewählt sind aus der Gruppe bestehend aus Verbindungen der Formeln XI und XII

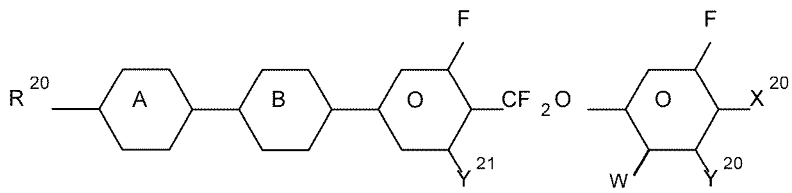

XI

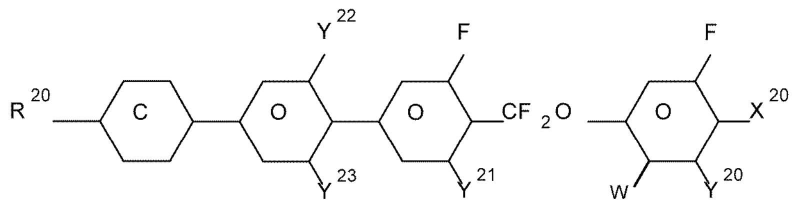

XII

bei denen $R^{20}$, $X^{20}$, W und $Y^{20-23}$ die in Formel III in Anspruch 16 angegebenen Bedeutungen besitzen und

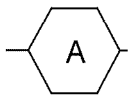 und 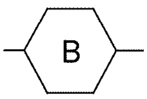

jeweils unabhängig voneinander

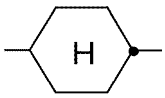 ,

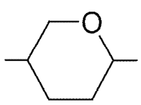 oder 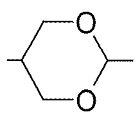

bedeuten und

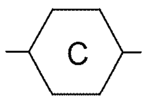

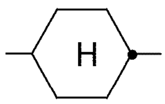 , 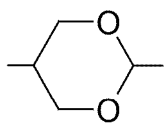 , 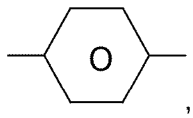 ,

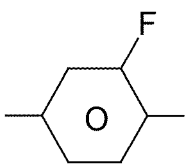 oder 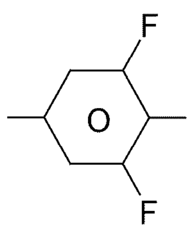

bedeutet.

**16.** Flüssigkristallmischung nach einem oder mehreren der Ansprüche 7 bis 15, **dadurch gekennzeichnet, dass** die FK-Host-Mischung eine oder mehrere Verbindungen der folgenden Formel enthält:

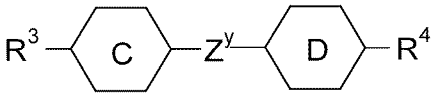

ZK

worin die einzelnen Reste die folgenden Bedeutungen besitzen:

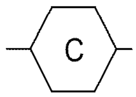

bedeutet

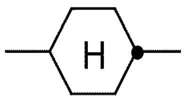 , 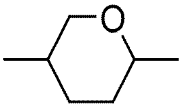 , 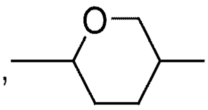 ,

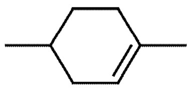 oder 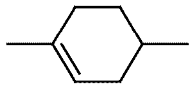 ,

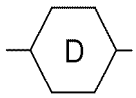

bedeutet

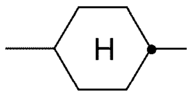 oder 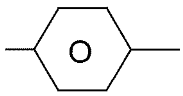 ,

$R^3$ und $R^4$ bedeuten jeweils unabhängig voneinander Alkyl mit 1 bis 12 C-Atomen, worin zusätzlich eine oder zwei nicht benachbarte $CH_2$-Gruppen so durch -O-, -CH=CH-, -CO-, -O-CO- oder -CO-O- ersetzt sein können, dass O-Atome nicht direkt miteinander verknüpft sind,

$Z^y$ bedeutet $-CH_2CH_2-$, -CH=CH-, $-CF_2O-$, $-OCF_2-$, $-CH_2O-$, $-OCH_2-$, -CO-O-, -O-CO-, $-C_2F_4-$, -CF=CF-, $-CH=CH-CH_2O-$ oder eine Einfachbindung.

**17.** Flüssigkristallmischung nach einem oder mehreren der Ansprüche 7 bis 16, **dadurch gekennzeichnet, dass** die FK-Host-Mischung eine oder mehrere Verbindungen der folgenden Formeln

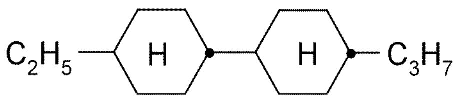

ZK1a

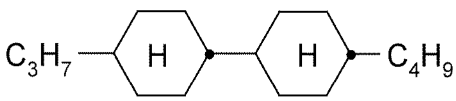

ZK1b

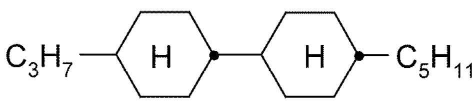

ZK1c

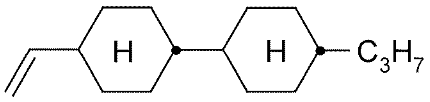

ZK3a

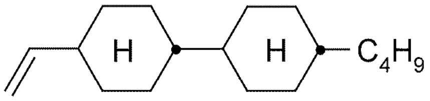

ZK3b

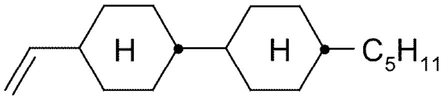

ZK3c

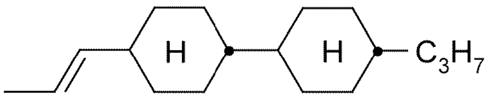

ZK3d

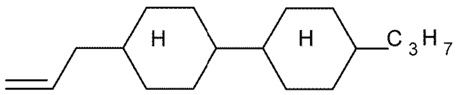

ZK3e

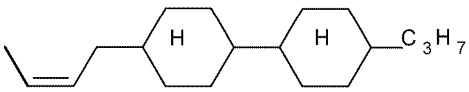

ZK3f

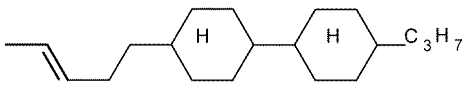

ZK3g

enthält, bei denen die Propyl-, Butyl- und Pentylgruppen geradkettige Gruppen sind.

**18.** Flüssigkristallmischung nach einem oder mehreren der Ansprüche 7 bis 17, **dadurch gekennzeichnet, dass** die FK-Host-Mischung eine oder mehrere Verbindungen enthält, die ausgewählt sind aus den folgenden Formeln:

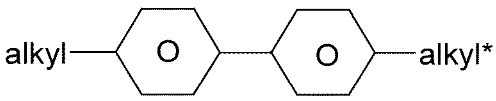

B1

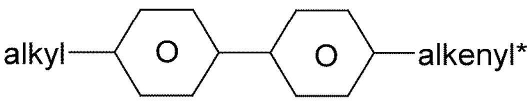

B2

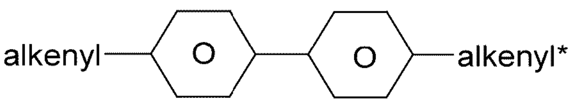

B3

worin alkyl und alkyl* jeweils unabhängig voneinander einen geradkettigen Alkylrest mit 1-6 C-Atomen bedeuten und alkenyl und alkenyl* jeweils unabhängig voneinander einen geradkettigen Alkenylrest mit 2-6 C-Atomen bedeuten.

**19.** Flüssigkristallmischung nach einem oder mehreren der Ansprüche 7 bis 18, **dadurch gekennzeichnet, dass** die

FK-Host-Mischung eine oder mehrere Verbindungen enthält, die ausgewählt sind aus den folgenden Formeln:

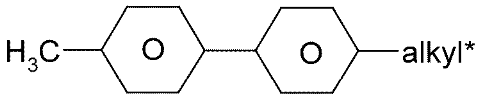
B1a

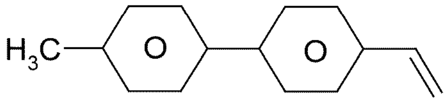
B2a

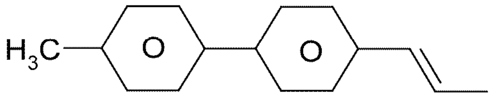
B2b

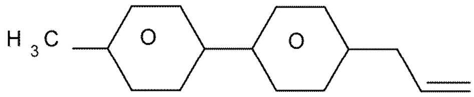
B2c

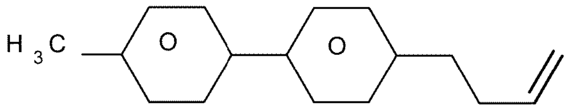
B2d

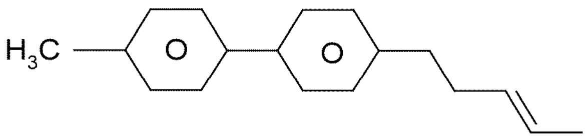
B2e

worin alkyl* einen Alkylrest mit 1-6 C-Atomen bedeutet.

**20.** Verwendung der Flüssigkristallmischung nach einem oder mehreren der Ansprüche 7 bis 19 zur Herstellung einer Flüssigkristallanzeige.

**21.** Verfahren zur Herstellung einer Flüssigkristallanzeige, umfassend mindestens die Schritte:

- Bereitstellen eines ersten Substrats, das eine Pixelelektrode und eine gemeinsame Elektrode zur Erzeugung eines elektrischen Feldes im Wesentlichen parallel zu einer Oberfläche des ersten Substrats im Pixelbereich umfasst;
- Bereitstellen eines zweiten Substrats, wobei das zweite Substrat gegenüber dem ersten Substrat angeordnet ist;
- Einfügen einer Flüssigkristallmischung nach einem oder mehreren der Ansprüche 7 bis 19;
- Bestrahlen der Flüssigkristallmischung mit linear polarisiertem Licht, was Photoorientierung des Flüssigkristalls bewirkt;
- Härten der polymerisierbaren Verbindungen der Flüssigkristallmischung durch Bestrahlen mit ultraviolettem Licht oder sichtbarem Licht mit einer Wellenlänge von 450 nm oder darunter.

**22.** Verfahren nach Anspruch 21, **dadurch gekennzeichnet, dass** das linear polarisierte Licht ultraviolettes Licht oder sichtbares Licht mit einer Wellenlänge von 450 nm oder darunter ist.

**23.** Anzeige, erhältlich durch ein Verfahren nach Anspruch 21 oder 22.

**24.** Anzeige nach Anspruch 23, bei der die FK-Host-Mischung ohne Anlegen eines elektrischen Feldes homogen orientiert wird.

**25.** Anzeige nach Anspruch 23 oder 24, bei der es sich um eine IPS- oder FFS-Anzeige handelt.

## Revendications

**1.** Composé de formule I,

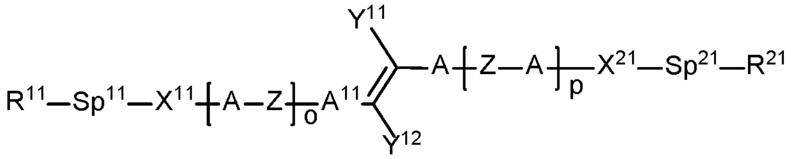

I

dans laquelle

$A^{11}$ désigne un radical choisi parmi les groupements suivants : a) un groupement constitué par 1,4-phénylène et 1,3-phénylène, où, de plus, un ou deux groupements CH peuvent être remplacés par N et où, de plus, un ou plusieurs atomes de H peuvent être remplacés par L, b) un groupement choisi dans le groupe constitué par

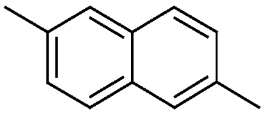

,

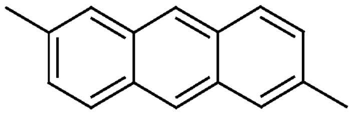

,

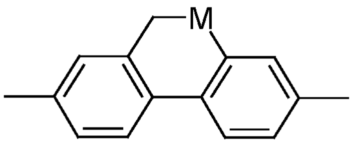

,

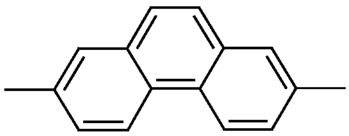

,

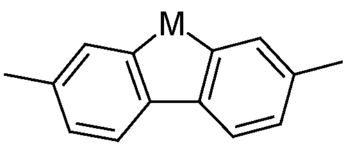

,

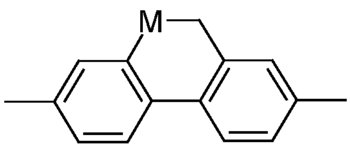

,

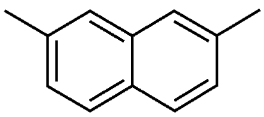

,

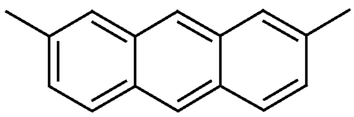

,

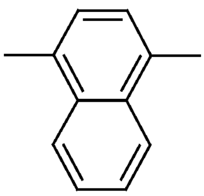

,

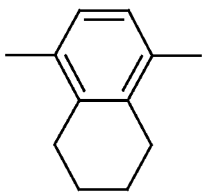

,

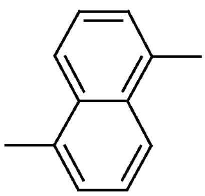

,

où, de plus, un ou plusieurs atomes de H dans ces radicaux peuvent être remplacés par L, et/ou une ou plusieurs doubles liaisons peuvent être remplacés par des liaisons simples, et/ou un ou plusieurs groupements CH peuvent être remplacés par N,

A revêtent chacun, indépendamment les uns des autres, à chaque occurrence l'une des significations pour $A^{11}$ ou sont choisis parmi a) un groupement constitué par trans-1,4-cyclohexylène, 1,4-cyclohexénylène, où, de plus, un ou plusieurs groupements $CH_2$ non adjacents peuvent être remplacés par -O- et/ou -S- et où, de plus, un ou plusieurs atomes de H peuvent être remplacés par F, ou b) un groupement constitué par tétrahydropyran-2,5-diyle, 1,3-dioxane-2,5-diyle, tétrahydrofuran-2,5-diyle, cyclobutane-1,3-diyle, pipéridine-1,4-diyle, thiophène-2,5-diyle et selénophène-2,5-diyle, chacun d'entre eux pouvant également être monoou polysubstitué par L,

L à chaque occurrence, de manière identique ou différente, désigne -OH, -F, -Cl, -Br, -I, -CN, $-NO_2$, $SF_5$, -NCO, -NCS, -OCN, -SCN, $-C(=O)N(R^z)_2$, $-C(=O)R^z$, $-N(R^z)_2$, silyle éventuellement substitué, aryle éventuellement substitué ayant de 6 à 20 atomes de C, ou alkyle, alcoxy, alkylcarbonyle, alcoxycarbonyle, alkylcarbonyloxy ou alcoxycarbonyloxy à chaîne linéaire ou ramifiée ou cyclique ayant de 1 à 25 atomes de C, où, de plus, un ou plusieurs atomes de H peuvent être remplacés par F ou Cl, ou $X^{21}$-$Sp^{21}$-$R^{21}$,

M désigne -O-, -S-, $-CH_2-$, $-CHR^z-$ ou $-CR^yR^z-$, et

$R^y$ et $R^z$ désignent chacun, indépendamment l'un de l'autre, H, CN, F ou alkyle ayant 1-12 atomes de C, où un ou plusieurs atomes de H peuvent être remplacés par F,

$Y^{11}$ et $Y^{12}$ désignent chacun, indépendamment l'un de l'autre, H, F, phényle ou alkyle ayant 1-12 atomes de C, où un ou plusieurs atomes de H peuvent être remplacés par F,

Z désigne, indépendamment les uns des autres, à chaque occurrence, une liaison simple, -COO-, -OCO-, -O-CO-O-, $-OCH_2-$, $-CH_2O-$, $-OCF_2-$, $-CF_2O-$, ou $-(CH_2)_n-$ $-(CH_2)_n-$ où un ou plusieurs groupements non adjacents peuvent être remplacés par O ou S, $-CF_2CF_2-$, -CH=CH-, -CF=CF-, -CH=CH-COO-, -OCO-CH=CH-, -CO-S-, -S-CO-, -CS-S-, -S-CS-, -S-CSS- ou -C≡C-,

n désigne un nombre entier compris entre 2 et 8,

o et p désignent chacun et indépendamment 0, 1 ou 2,

$X^{11}$ et $X^{21}$ désignent indépendamment l'un de l'autre, à chaque occurrence une liaison simple, -CO-O-, -O-CO-, -O-COO-, -O-, -CH=CH-,-C≡C-, $-CF_2-O-$, $-O-CF_2-$, $-CF_2-CF_2-$, $-CH_2-O-$, $-O-CH_2-$, -CO-S-, -S-CO-, -CS-S-, -S-CS-, -S-CSS- ou -S-,

$Sp^{11}$ et $Sp^{21}$ désignent chacun et indépendamment, à chaque occurrence une liaison simple ou un groupement espaceur comprenant de 1 à 20 atomes de C, où un ou plusieurs groupements $CH_2$ non adjacents et non terminaux peuvent également être remplacés par -O-, -S-, -NH-, $-N(CH_3)-$, -CO-, -O-CO-, -S-CO-, -O-COO-, -CO-S-, -CO-O-, $-CF_2-$, $-CF_2O-$, $-OCF_2$--C(OH)-, -CH(alkyl)-, -CH(alcényl)-,-CH(alcoxy)-, -CH(oxaalkyl)-, -CH=CH- ou -C≡C-, cependant de telle sorte que deux atomes de O ne soient pas adjacents l'un avec l'autre et que deux groupements choisis parmi -O-CO-, -S-CO-, -O-COO-, -CO-S-, -CO-O- et -CH=CH- ne soient pas adjacents l'un avec l'autre,

$R^{11}$ et $R^{21}$ désignent $P^{11}$ ou $P^{21}$, à condition qu'au moins l'un parmi $R^{11}$ et $R^{21}$ désigne $P^{21}$,

$P^{11}$ désignent chacun et indépendamment les uns des autres à chaque occurrence, un groupement polymérisable choisi dans le groupe constitué par les groupements acrylate, méthacrylate, fluoroacrylate, en outre vinyloxy, chloroacrylate, oxétane et époxyde,

$P^{21}$ désigne un groupement

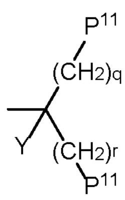

,

Y désigne H, F, phényle ou alkyle éventuellement fluoré ayant 1-12 atomes de C,

q et r désignent chacun et indépendamment un nombre entier allant de 0 à 8.

2. Composé selon la revendication 1, **caractérisé en ce que** le composé est choisi parmi les composés des sous-formules I-1 à I-9,

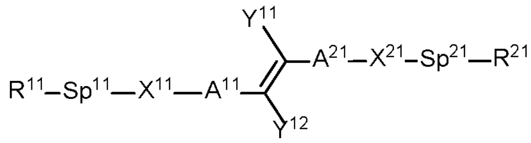

I-1

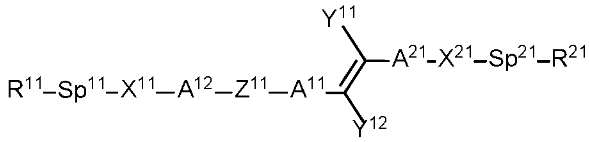

I-2

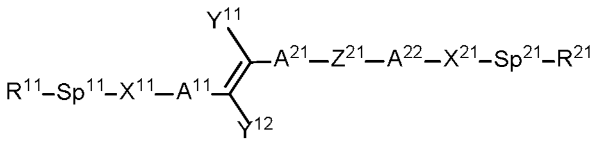

I-3

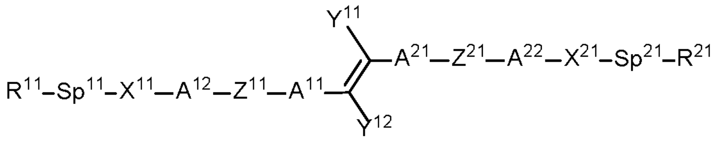

I-4

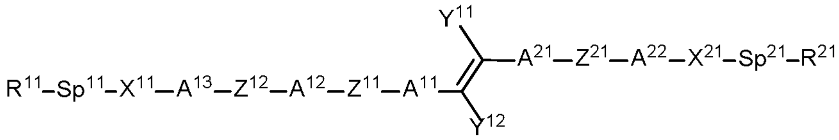

I-5

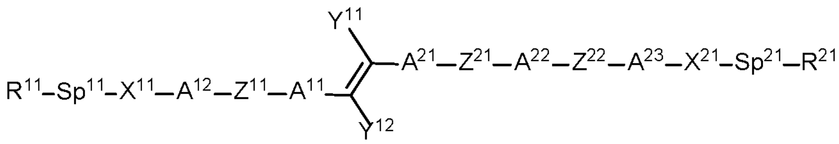

I-6

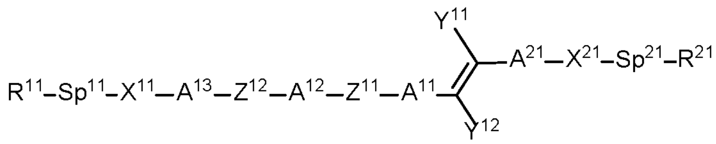

I-7

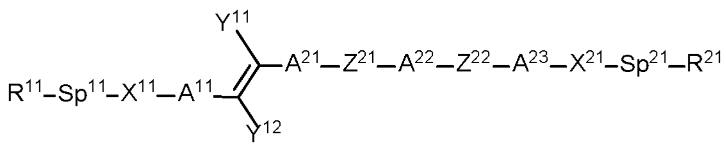

I-8

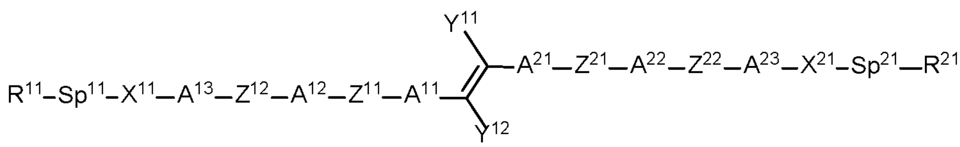

I-9

dans lesquelles $R^{11}$, $R^{21}$, $A^{11}$, $X^{11}$, $X^{12}$, $Y^{11}$, $Y^{12}$, $Sp^{11}$, et $Sp^{12}$ revêtent à chaque occurrence et chacun et indépendamment les uns des autres l'une des significations telles que données selon la revendication 1,
$A^{12}$ à $A^{23}$ revêtent chacun et indépendamment les uns des autres l'une des significations pour A telles que données selon la revendication 1, et

$Z^{11}$ à $Z^{22}$ revêtent chacun et indépendamment les uns des autres l'une des significations pour Z telles que données selon la revendication 1.

**3.** Composé selon la revendication 1 ou 2, **caractérisé en ce que** le composé est choisi parmi les composés suivants de formules I-1a à I-4c,

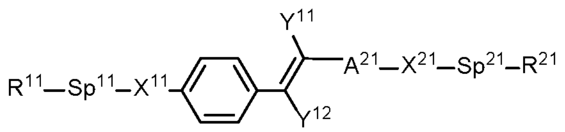

I-1a

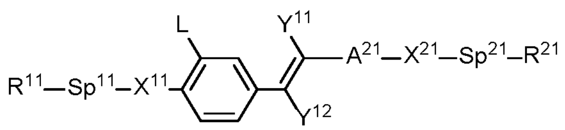

I-1b

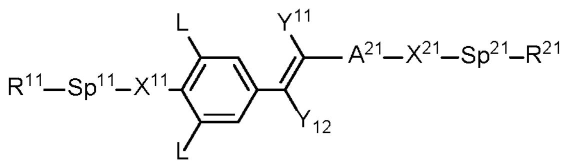

I-1c

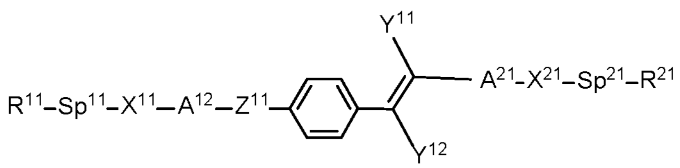

I-2a

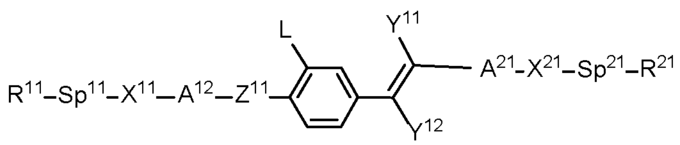

I-2b

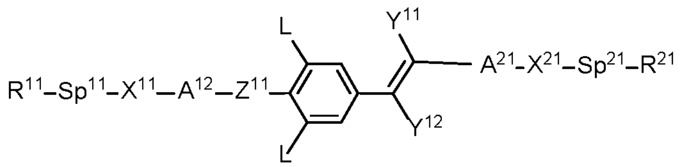

I-2c

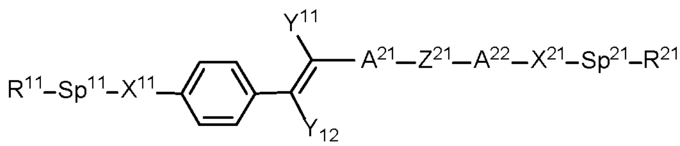

I-3a

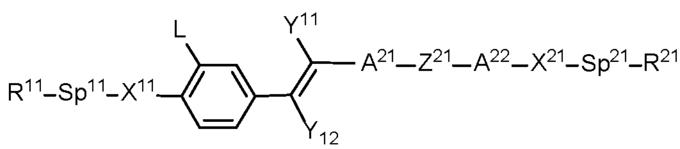

I-3b

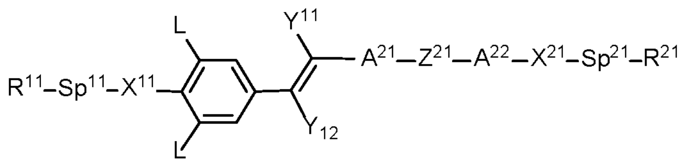

I-3c

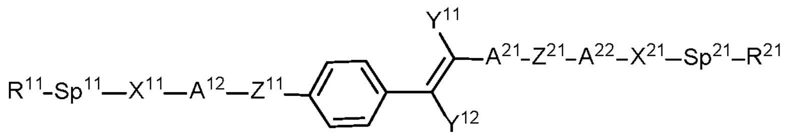

I-4a

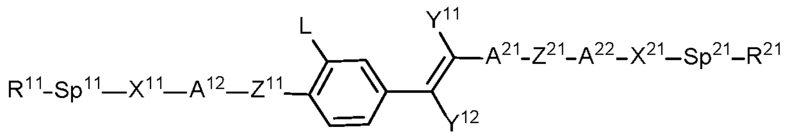

I-4b

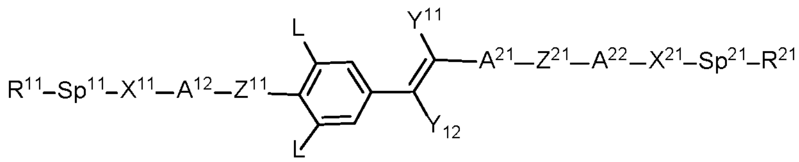

I-4c

dans lesquelles L, $R^{11}$, $R^{21}$, $A^{11}$, $X^{11}$, $X^{21}$, $Y^{11}$, $Y^{12}$, $Sp^{11}$, et $Sp^{21}$ revêtent l'une des significations telles que données ci-dessus dans la formule I, $A^{12}$ à $A^{23}$ revêtent l'une des significations pour A dans la formule I, et $Z^{11}$ à $Z^{22}$ revêtent l'une des significations pour Z telles que données ci-dessus pour la formule I.

**4.** Composé selon l'une ou plusieurs parmi les revendications 1 à 3, **caractérisé en ce qu'**il est choisi parmi les composés des sous-formules suivantes

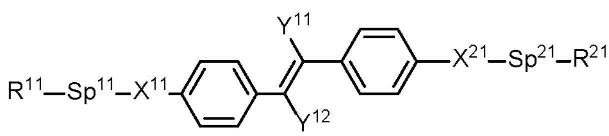

I-1a-1

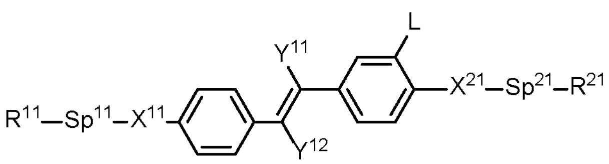

I-1a-2

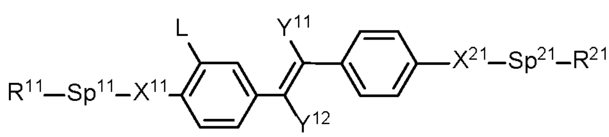

I-1b-1

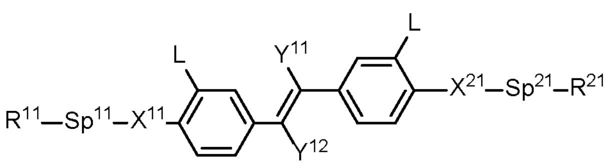

I-1b-2

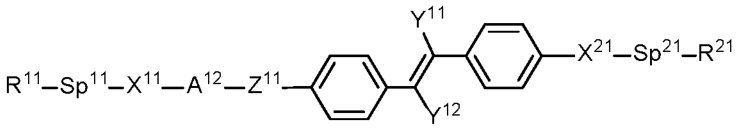

I-2a-1

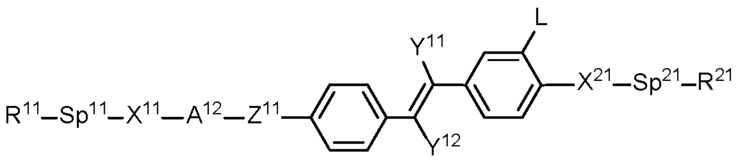

I-2a-2

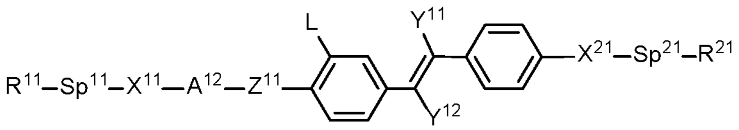

I-2b-1

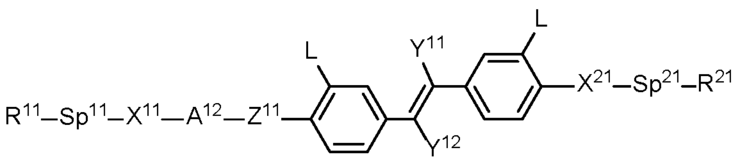

I-2b-2

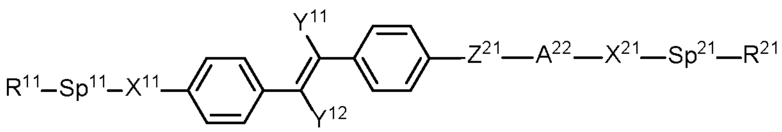

I-3a-1

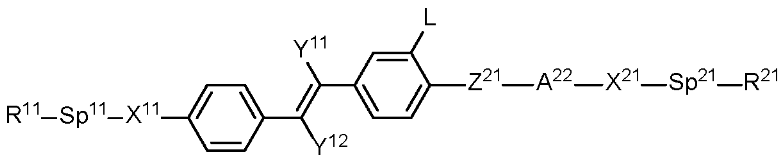

I-3a-2

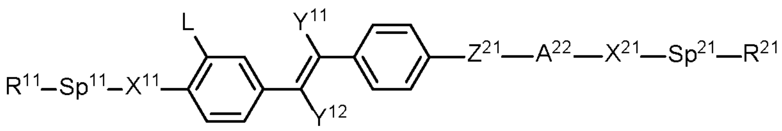

I-3b-1

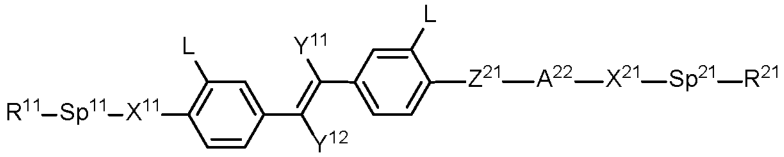

I-3b-2

dans lesquelles L, $R^{11}$, $R^{21}$, $A^{11}$, $X^{11}$, $X^{21}$, $Sp^{11}$, et $Sp^{21}$ revêtent l'une des significations telles que données ci-dessus dans la formule I, $A^{12}$ et $A^{22}$ revêtent l'une des significations pour A dans la formule I, et $Z^{11}$ et $Z^{22}$ revêtent l'une des significations pour Z telles que données ci-dessus pour la formule I.

**5.** Composé selon l'une ou plusieurs parmi les revendications 1 à 4, **caractérisé en ce qu'**il est choisi parmi les composés des sous-formules

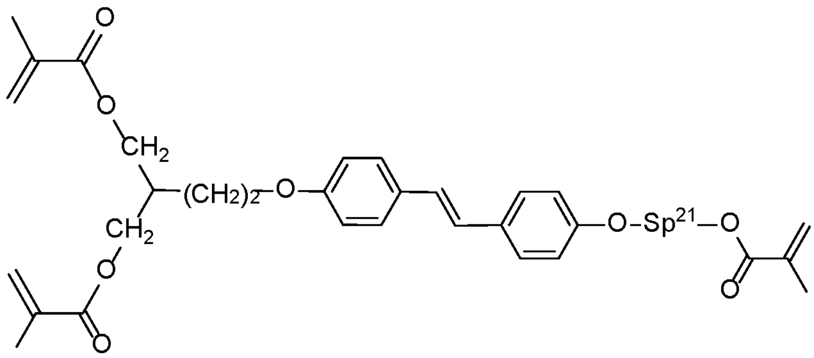

I-1a-1-1

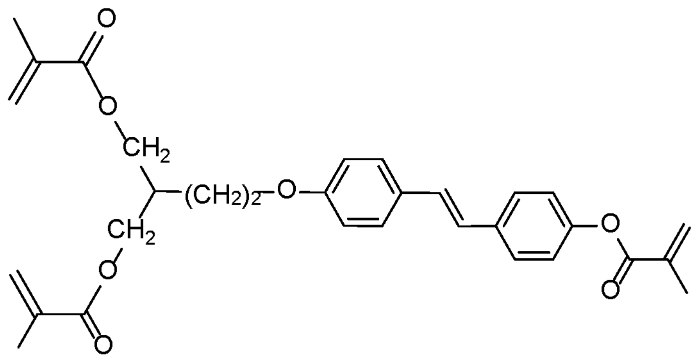

I-1a-1-2

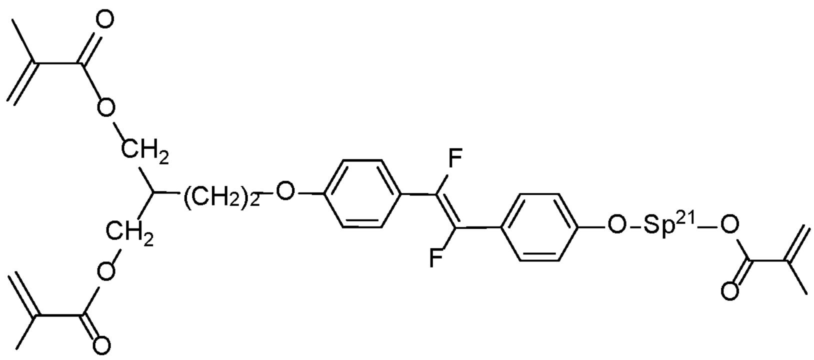

I-1a-1-3

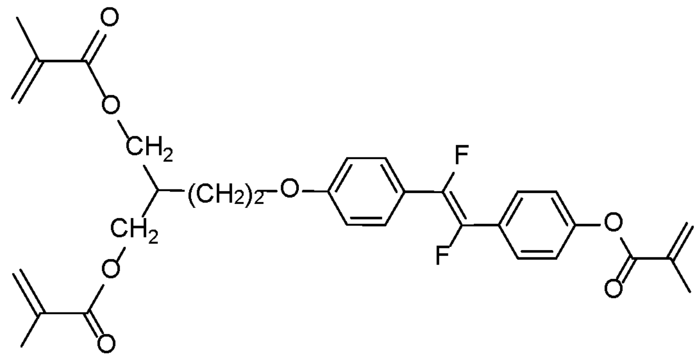

I-1a-1-4

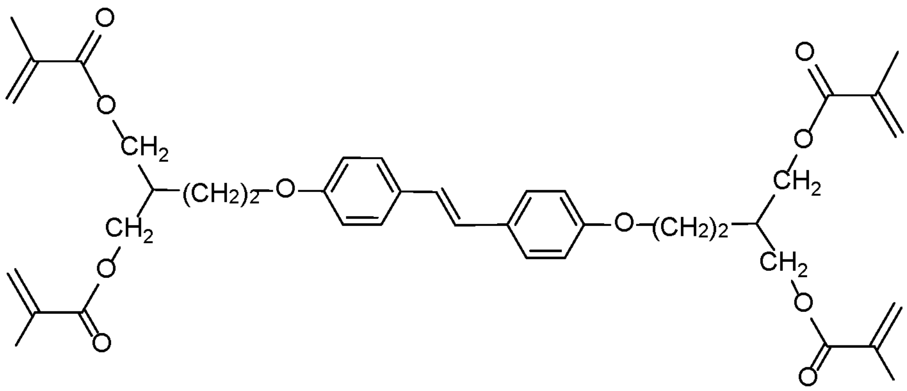

I-1a-1-5

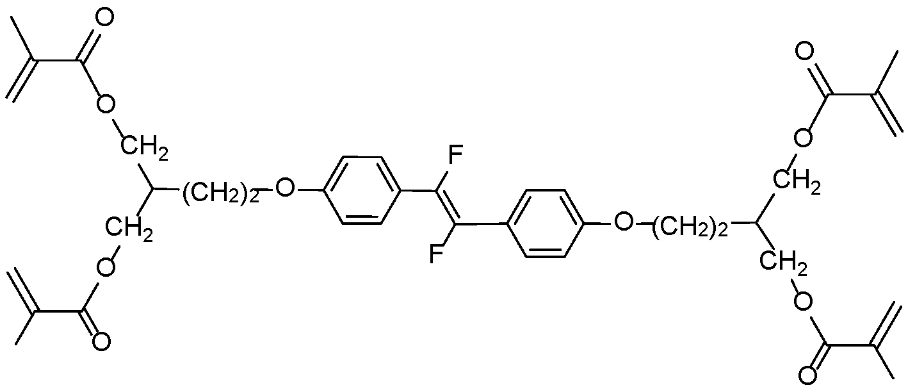

I-1a-1-6

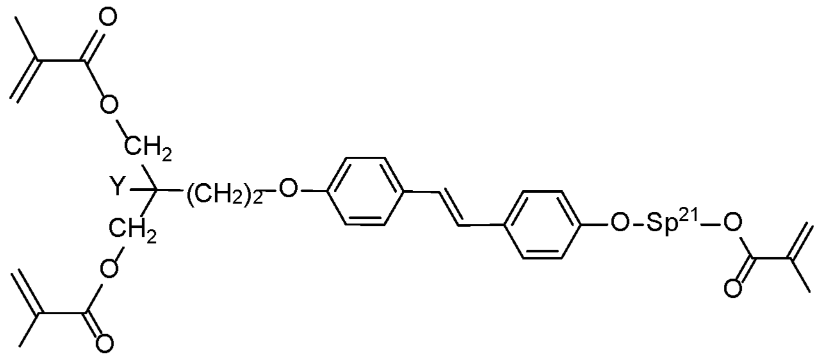

I-1a-1-7

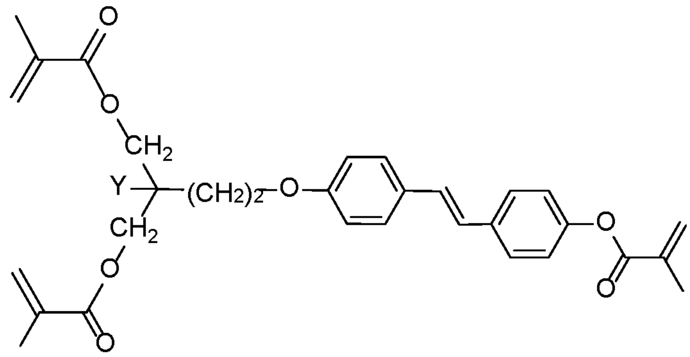

I-1a-1-8

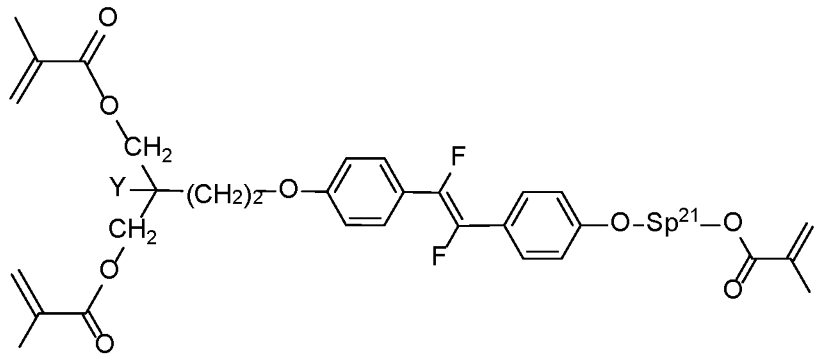

I-1a-1-9

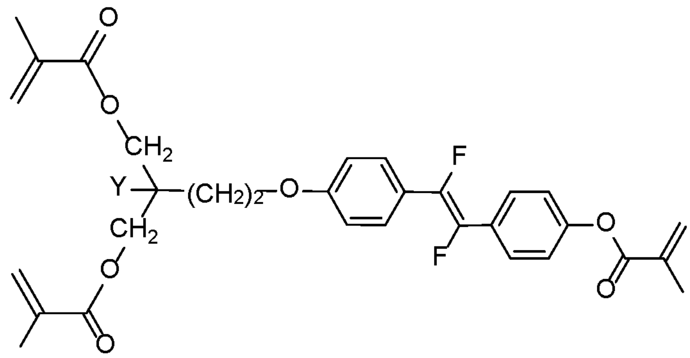

I-1a-1-10

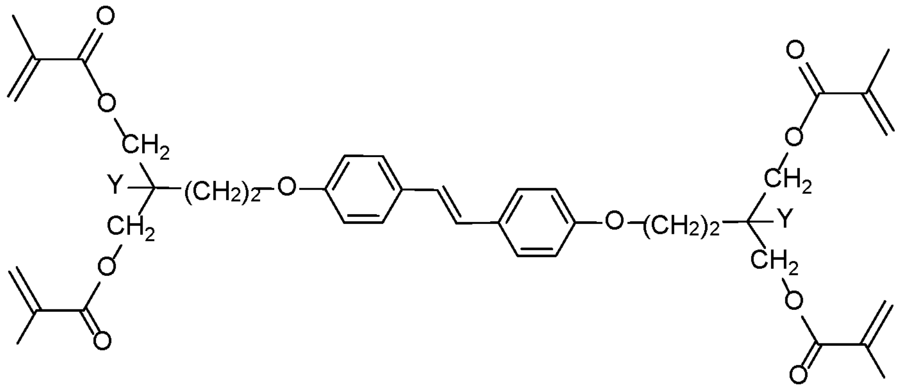

I-1a-1-11

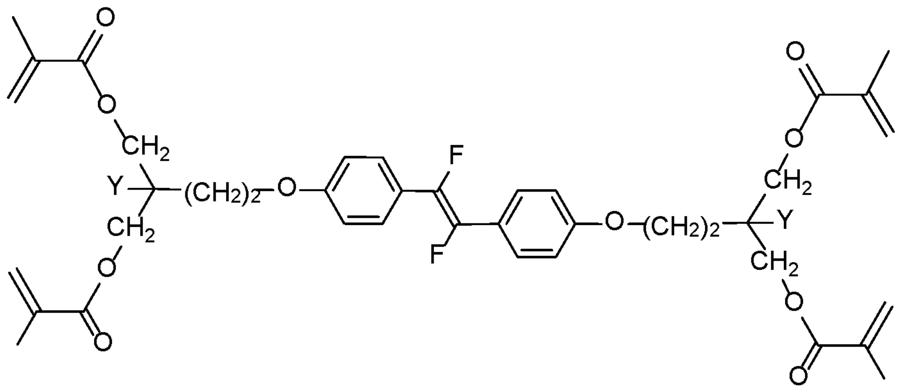

I-1a-1-12

dans lesquelles $Sp^{11}$ et $Sp^{21}$ revêtent l'une des significations telles que données ci-dessus dans la formule I, et Y désignent chacun et indépendamment, méthyle ou éthyle.

**6.** Utilisation de composés de formule I selon l'une ou plusieurs parmi les revendications 1 à 5, dans un mélange de cristaux liquides.

**7.** Mélange de cristaux liquides, **caractérisé en ce qu'**il comprend un composant A) comprenant un ou plusieurs composés de formule I selon l'une ou plusieurs parmi les revendications 1 à 6, et un composant cristallin liquide B), comprenant un ou plusieurs composés mésogènes ou cristallins liquides.

**8.** Mélange de cristaux liquides selon la revendication 7, **caractérisé en ce que** la concentration totale en composés

de formule I dans le mélange se trouve dans la plage allant de 0,01 à 10% en poids.

**9.** Mélange de cristaux liquides selon l'une ou plusieurs parmi les revendications 7 ou 8, **caractérisé en ce qu'**il comprend en outre un composant polymérisable C) comprenant un ou plusieurs composés mésogènes polymérisables ou isotropes polymérisables.

**10.** Mélange de cristaux liquides selon la revendication 9, **caractérisé en ce que** la concentration en composés mésogènes polymérisables ou isotropes polymérisables se trouve dans la plage allant de 0,01 à 10% en poids.

**11.** Mélange de cristaux liquides selon l'une ou plusieurs parmi les revendications 7 à 10, **caractérisé en ce que** le mélange hôte de LC présente une anisotropie diélectrique négative.

**12.** Mélange de cristaux liquides selon la revendication 11, **caractérisé en ce que** le mélange hôte de LC comprend un ou plusieurs composés choisis parmi les formules suivantes :

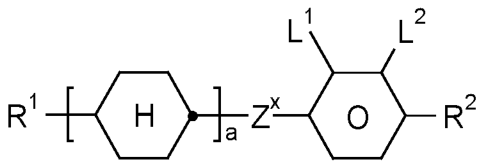

CY

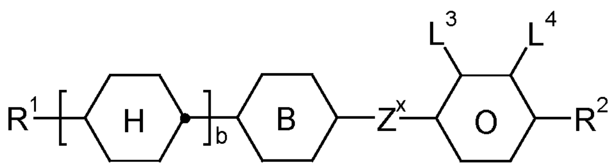

PY

dans lesquelles

a vaut 1 ou 2,
b vaut 0 ou 1,

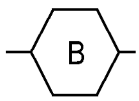

désigne

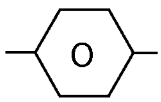 ou 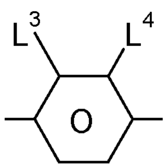

$R^1$ et $R^2$ désignent chacun, indépendamment l'un de l'autre, alkyle ayant de 1 à 12 atomes de C, où, de plus, un ou deux groupements $CH_2$ non adjacents peuvent être remplacés par -O-, -CH=CH-, -CO-, -O-CO- ou -CO-O-de telle façon que les atomes de O ne soient pas liés directement l'un à l'autre,
$Z^x$ désigne -CH=CH-, $-CH_2O-$, $-OCH_2-$, $-CF_2O-$ , $-OCF_2-$, -O-, $-CH_2-$, $-CH_2CH_2-$ ou une liaison simple,
$L^{1-4}$ désignent chacun, indépendamment les uns des autres, F, Cl, $OCF_3$, $CF_3$, $CH_3$, $CH_2F$, $CHF_2$.

**13.** Mélange de cristaux liquides selon l'une ou plusieurs parmi les revendications 7 à 10, **caractérisé en ce que** le mélange hôte de LC présente une anisotropie diélectrique positive.

**14.** Mélange de cristaux liquides selon la revendication 13, **caractérisé en ce que** le mélange hôte de LC comprend un ou plusieurs composés choisis dans le groupe constitué par les composés de formules II et III,

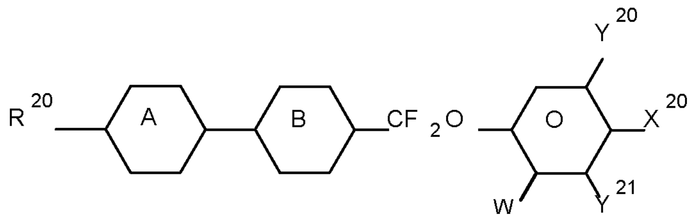

II

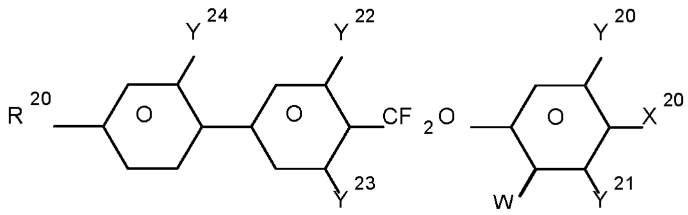

III

dans lesquelles

$R^{20}$ désignent chacun, de manière identique ou différente, un radical alkyle ou alcoxy halogéné ou non substitué ayant de 1 à 15 atomes de C, où, de plus, un ou plusieurs groupements $CH_2$ dans ces radicaux peuvent chacun être remplacés, indépendamment les uns des autres, par -C≡C-, $-CF_2O-$, -CH=CH-,

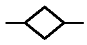 , 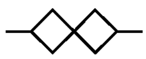 ,

-O-, -CO-O- ou -O-CO- de telle façon que les atomes de O ne soient pas liés directement l'un à l'autre,
$X^{20}$ désignent chacun, de manière identique ou différente, F, Cl, CN, SFs, SCN, NCS, un radical alkyle halogéné, un radical alcényle halogéné, un radical alcoxy halogéné ou un radical alcényloxy halogéné, chacun ayant jusqu'à 6 atomes de C, et
$Y^{20-24}$ désignent chacun, de manière identique ou différente H ou F,
W désigne H ou méthyle,

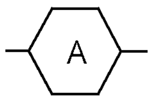 et 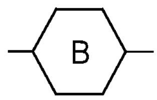

désignent chacun, de manière identique ou différente

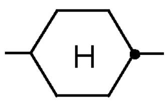 ,

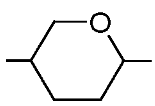 ou 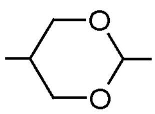 .

**15.** Mélange de cristaux liquides selon la revendication 13 ou 14, **caractérisé en ce qu'**il comprend un ou plusieurs composés choisis dans le groupe constitué par les composés de formules XI et XII

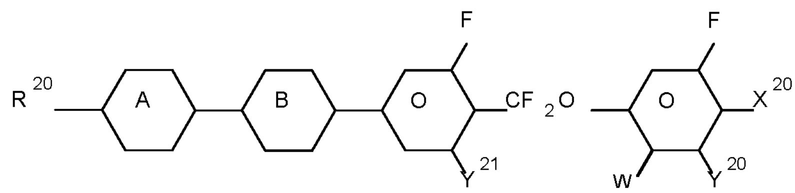 XI

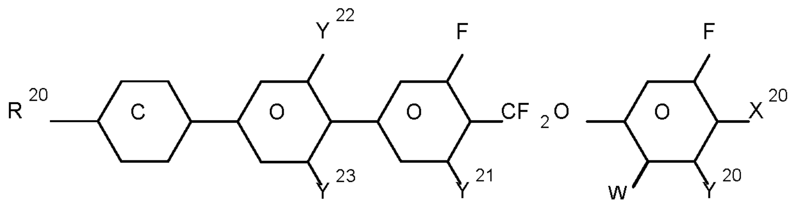 XII

dans lesquelles $R^{20}$, $X^{20}$, W et $Y^{20-23}$ revêtent les significations indiquées dans la formule III selon la revendication 16, et

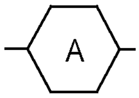 et 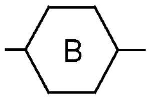

désignent chacun, indépendamment l'un de l'autre,

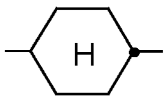 ,

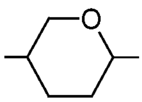 ou 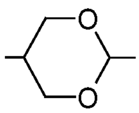

et

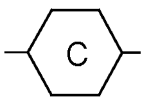

désigne

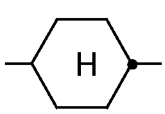 , 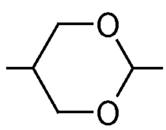 , 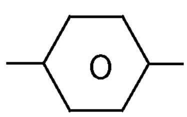

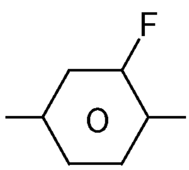 ou 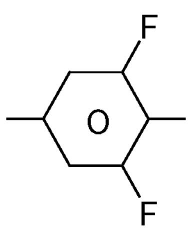

**16.** Mélange de cristaux liquides selon l'une ou plusieurs parmi les revendications 7 à 15, **caractérisé en ce que** le mélange hôte de LC comprend un ou plusieurs composés de formule suivante :

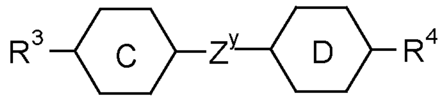

ZK

dans laquelle les radicaux individuels revêtent les significations suivantes :

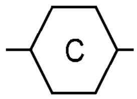

désigne

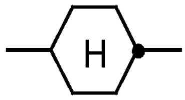 , 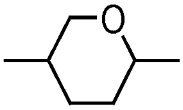 , 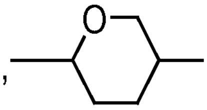 ,

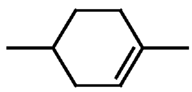 ou 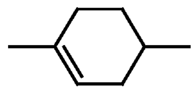 ,

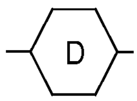

désigne

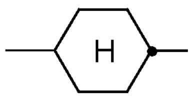 ou 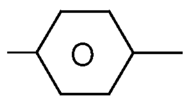 ,

$R^3$ et $R^4$ désignent chacun, indépendamment l'un de l'autre, alkyle ayant de 1 à 12 atomes de C, où, de plus, un ou deux groupements $CH_2$ non adjacents peuvent être remplacés par -O-, -CH=CH-, -CO-, -O-CO- ou -CO-O-de telle façon que les atomes de O ne soient pas liés directement l'un à l'autre,
$Z^y$ désigne $-CH_2CH_2-$, -CH=CH-, $-CF_2O-$, $-OCF_2-$, $-CH_2O-$, $-OCH_2-$, -CO-O-, -O-CO-, $-C_2F_4-$, -CF=CF-, $-CH=CH-CH_2O-$ ou une liaison simple.

**17.** Mélange de cristaux liquides selon l'une ou plusieurs parmi les revendications 7 à 16, **caractérisé en ce que** le mélange hôte de LC comprend un ou plusieurs composés des formules suivantes

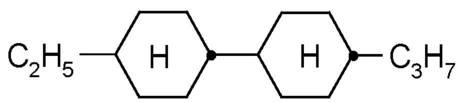

ZK1a

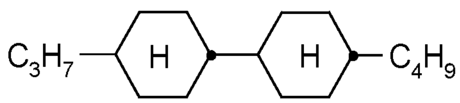

ZK1b

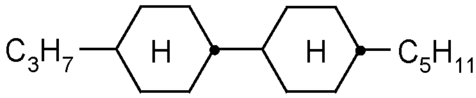

ZK1c

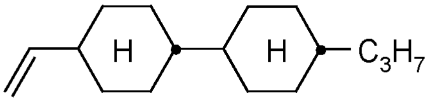

ZK3a

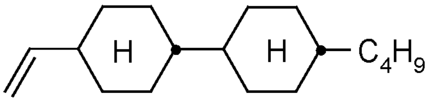

ZK3b

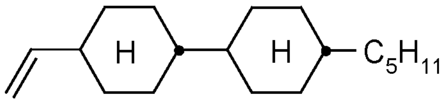

ZK3c

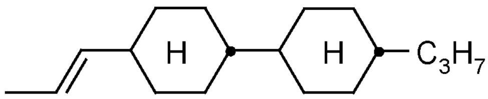

ZK3d

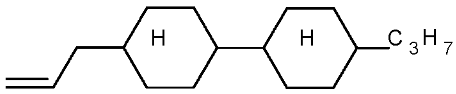

ZK3e

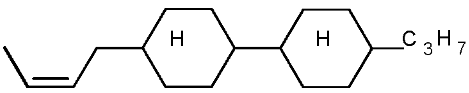

ZK3f

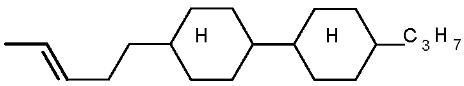

ZK3g

dans laquelle les groupements propyle, butyle et pentyle sont des groupements à chaîne linéaire.

**18.** Mélange de cristaux liquides selon l'une ou plusieurs parmi les revendications 7 à 17, **caractérisé en ce que** le mélange hôte de LC comprend un ou plusieurs composés choisis parmi les formules suivantes :

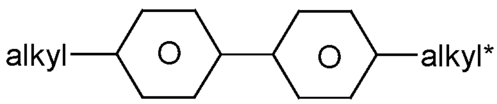

B1

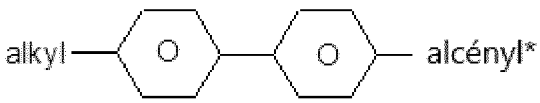

B2

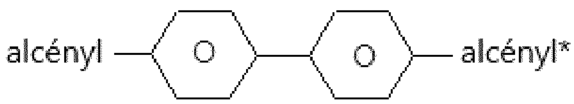

B3

dans lesquelles alkyl et alkyl* désignent chacun, indépendamment l'un de l'autre, un radical alkyle à chaîne linéaire ayant 1-6 atomes de C, et alcényl et alcényl* désignent chacun, indépendamment l'un de l'autre, un radical alcényle à chaîne linéaire ayant 2-6 atomes de C.

**19.** Mélange de cristaux liquides selon l'une ou plusieurs parmi les revendications 7 à 18, **caractérisé en ce que** le mélange hôte de LC comprend un ou plusieurs composés choisis parmi les formules suivantes :

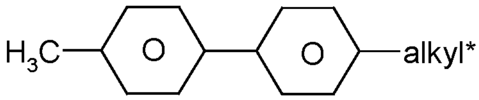

B1a

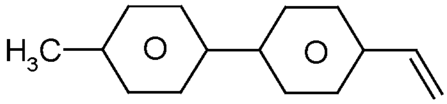

B2a

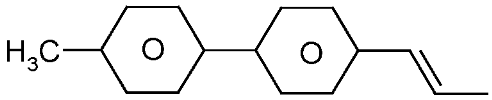

B2b

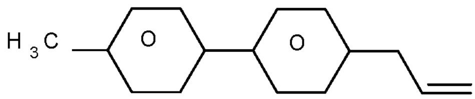

B2c

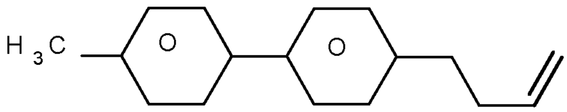

B2d

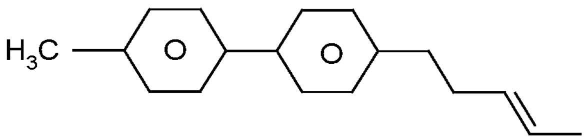

B2e

dans lesquelles alkyl* désigne un radical alkyle ayant 1-6 atomes de C.

**20.** Utilisation du mélange de cristaux liquides selon l'une ou plusieurs parmi les revendications 7 à 19, pour la fabrication d'un affichage à cristaux liquides.

**21.** Procédé de fabrication d'un affichage à cristaux liquides, comprenant au moins les étapes :

- de mise à disposition d'un premier substrat qui comporte une électrode de pixel et une électrode commune pour la génération d'un champ électrique sensiblement parallèle à une surface du premier substrat dans la région de pixel ;
- de mise à disposition d'un deuxième substrat, le deuxième substrat étant disposé à l'opposé du premier substrat ;
- d'interposition d'un mélange de cristaux liquides selon l'une ou plusieurs parmi les revendications 7 à 19 ;
- d'irradiation du mélange de cristaux liquides par une lumière polarisée linéairement, conduisant au photo-alignement du cristal liquide ;
- de durcissement des composés polymérisables du mélange de cristaux liquides par irradiation par de la lumière ultraviolette ou de la lumière visible ayant une longueur d'onde de 450 nm ou moins.

**22.** Procédé selon la revendication 21, **caractérisé en ce que** la lumière polarisée linéairement est de la lumière ultraviolette ou de la lumière visible ayant une longueur d'onde de 450 nm ou moins.

**23.** Affichage, pouvant être obtenu par un procédé selon la revendication 21 ou 22.

**24.** Affichage selon la revendication 23, dans lequel le mélange hôte de LC est aligné de manière homogène sans l'application d'un champ électrique.

**25.** Affichage selon la revendication 23 ou 24, l'affichage étant un affichage IPS ou FFS.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 10036847 A
- EP 1170626 A2
- US 6861107 B
- US 7169449 B
- US 20040191428 A1
- US 20060066793 A1
- US 20060103804 A1
- US 6177972 B
- WO 2010089092 A1
- WO 2012104008 A
- WO 2012038026 A
- EP 0763552 A
- WO 9949360 A
- WO 0005189 A
- GB 2306470 A
- WO 2017102068 A1
- WO 2018008581 A1
- WO 2018139507 A1
- EP 3466918 A1
- EP 1378557 A1
- JP 7181439 A
- EP 0667555 A
- EP 0673986 A
- DE 19509410
- DE 19528106
- DE 19528107
- WO 9623851 A
- WO 9628521 A
- WO 2012079676 A
- JP S63179323 B
- JP H10239694 B


### Non-patent literature cited in the description


- **S.H. JUNG et al.** *Jpn. J. Appl. Phys.,* 2004, vol. 43 (3), 1028
- **S.H. LEE et al.** *Appl. Phys. Lett.,* 1998, vol. 73 (20), 2882-2883
- **S.H. LEE et al.** *Liquid Crystals,* 2012, vol. 39 (9), 1141-1148
- **T.-J- CHEN et al.** *Jpn. J. Appl. Phys.,* 2006, vol. 45, 2702-2704
- **S. H. KIM ; L.-C- CHIEN.** *Jpn. J. Appl. Phys.,* 2004, vol. 43, 7643-7647
- *Appl. Phys. Lett.,* 1999, vol. 75 (21), 3264
- *Optics Express,* 2004, vol. 12 (7), 1221
- **N.A. CLARK et al.** *Langmuir,* 2010, vol. 26 (22), 17482-17488
- **VAN DER ZANDE et al.** *Liquid Crystals,* June 2006, vol. 33 (6), 723-737
- **M.H. LEE et al.** *Liquid Crystals, https://doi.org/10.1080/02678292.2018.1441459*
- *Pure Appl. Chem,* 2001, vol. 73 (5), 888
- **C. TSCHIERSKE ; G. PELZL ; S. DIELE.** *Angew. Chem.,* 2004, vol. 116, 6340-6368
- **HOUBEN-WEYL.** Methoden der organischen Chemie [Methods of Organic Chemistry. Georg-Thieme-Verlag
- **H. KELKER ; R. HATZ.** Handbook of Liquid Crystals. Verlag Chemie, 1980
- Merck Liquid Crystals - Licristal® - Physical Properties of Liquid Crystals - Description of the Measurement Methods. Merck KGaA, 1998